(19) Europäisches Patentamt
European Patent Office
Office européen des brevets

(11) EP 4 666 989 A2

(12) EUROPEAN PATENT APPLICATION

(43) Date of publication:
**24.12.2025 Bulletin 2025/52**

(21) Application number: **25210197.7**

(22) Date of filing: **12.02.2019**

(51) International Patent Classification (IPC):
***A61F 2/76*** *(2006.01)*

(52) Cooperative Patent Classification (CPC):
**A61B 5/004; A61B 5/0064; A61B 5/0082; A61B 5/1079; A61B 5/6828; A61B 8/0825; A61B 8/483; A61B 8/485; A61F 2/5046; A61F 2/76; B33Y 80/00;** A61B 5/0091; A61B 5/70; A61B 5/708; A61B 8/40; (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.02.2018 US 201862629528 P**
**14.09.2018 US 201862731376 P**

(62) Document number(s) of the earlier application(s) in accordance with Art. 76 EPC:
**19712320.1 / 3 752 049**

(71) Applicant: **Massachusetts Institute of Technology Cambridge, MA 02139 (US)**

(72) Inventors:
- **HERR, Hugh M**
  **Massachusetts 02139 (US)**
- **MOERMAN, Kevin Mattheus**
  **Massachusetts 02139 (US)**
- **SOLAV, Dana**
  **Massachusetts 02139 (US)**
- **RANGER, Bryan James**
  **Massachusetts 02139 (US)**
- **STEINMEYER, Rebecca**
  **Massachusetts 02139 (US)**
- **KU, Stephanie Lai**
  **Massachusetts 02139 (US)**
- **DAGDEVIREN, Canan**
  **Massachusetts 02139 (US)**
- **CARNEY, Matthew**
  **Massachusetts 02139 (US)**
- **PRIETO-GOMEZ, German A.**
  **Massachusetts 02139 (US)**
- **ZHANG, Xiang**
  **Massachusetts 02139 (US)**
- **FINCKE, Jonathan Randall**
  **Massachusetts 02139 (US)**
- **FEIGIN-ALMON, Micha**
  **Massachusetts 02139 (US)**
- **ANTHONY, Brian W.**
  **Massachusetts 02139 (US)**
- **LIU, Zixi**
  **Massachusetts 02139 (US)**
- **JAEGER, Aaron**
  **Massachusetts 02139 (US)**
- **YANG, Xingbang**
  **Massachusetts 02139 (US)**

(74) Representative: **D Young & Co LLP**
**3 Noble Street**
**London EC2V 7BQ (GB)**

Remarks:
This application was filed on 21.10.2025 as a divisional application to the application mentioned under INID code 62.

# (54) QUANTITATIVE DESIGN AND MANUFACTURING FRAMEWORK FOR A BIOMECHANICAL INTERFACE CONTACTING A BIOLOGICAL BODY SEGMENT

(57) Devices and methods for obtaining external shapes and internal tissue geometries, as well as tissue behaviors, of a biological body segment are provided. A device for three-dimensional imaging of a biological body segment includes a structure configured to receive the biological body segment, the structure including a first array of imaging devices disposed about a perimeter of the device to capture side images of the biological body segment and a second array of imaging devices disposed at an end of the device to capture images of a distal portion of the biological body segment. The second array has a generally axial viewing angle relative to the perimeter. A controller is configured to generate a three-dimensional reconstruction of the biological body segment based on cross-correlation of captured images from the first and second arrays.



Processed by Luminess, 75001 PARIS (FR)

(Cont. next page)

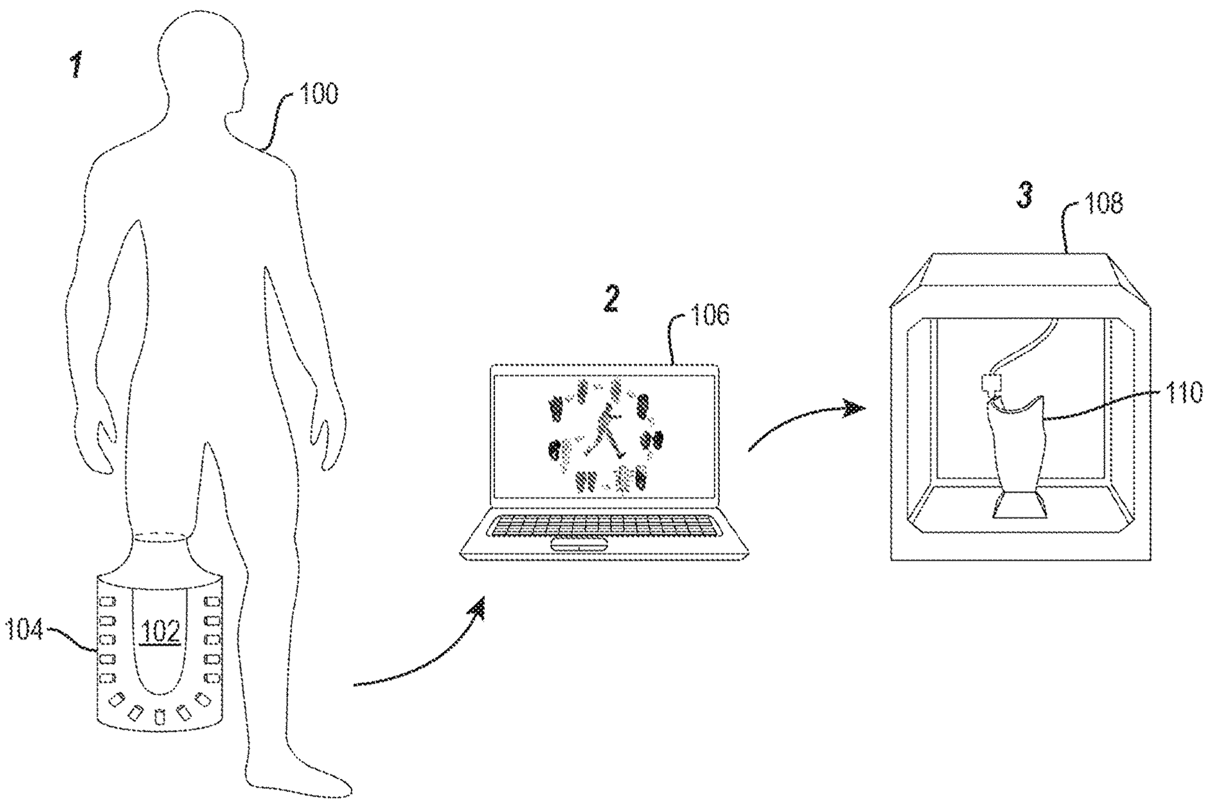


FIG. 1

(52) Cooperative Patent Classification (CPC): (Cont.)
A61B 2562/0219; A61B 2562/0261; A61F 2/7812;
A61F 2002/5049; A61F 2002/762; A61F 2002/7695

## Description

RELATED APPLICATIONS

This application claims the benefit of U.S. Provisional Application No. 62/629,528, filed on February 12, 2018, and U.S. Provisional Application No. 62/731,376, filed September 14, 2018. The entire teachings of the above applications are incorporated herein by reference.

BACKGROUND

To acquire a comprehensive data set of a biological segment for design of a prosthetic device, body imaging tools and active indenters can be used. However, current imaging efforts to obtain external segment shape, internal tissue geometries, and other properties, such as blood flow, are often bulky and expensive. Furthermore, such strategies are often limited in scope to static measurements, which are useful for initial predictive models of fit, but not with respect to dynamic interface behavior during the device's intended application.

Externally applied forces deform biological three-dimensional (3D) segments. Deformations to human tissue are sensed by mechanoreceptors that send signals to the brain. The brain perceives signals exceeding a certain threshold as some level of pain. Pain thresholds at various sites on the body vary with sensitivity to a set of parameters including pressure, shear stress, temperature, moisture, tissue depth, hydration, vascularization, and peripheral nerve anatomy. Current efforts to measure these parameters can involve handheld biological indenters that apply orthogonal indentation forces to the skin and measure tissue displacement. To localize an anatomical position of a perturbation site when using such indenters, additional imaging is often needed. Otherwise, positions must be specifically defined, which limits the number of measurement sites that can be obtained and used for prosthetic design.

There exists a need for improved imaging methods to obtain external segment shapes and internal tissue geometries, as well as tissue behaviors, of a biological body segment for prosthetic design.

SUMMARY

Devices and methods are provided for three-dimensional (3D) measurement of a biological body segment, for generating a 3D representation of a biological body segment, for manufacturing and operating biological body segment modeling devices, and for forming a biomechanical interface for a measured biological body segment. Such 3D measurement devices and methods can be used to generate a 3D image of a biological body segment, optionally under compressive loads, and optionally to also include internal features of the biological body segment, such as of musculoskeletal tissue and bone.

A device for three-dimensional imaging of a biological body segment includes a structure configured to receive the biological body segment, the structure including a first array of imaging devices disposed about a perimeter of the device to capture side images of the biological body segment and a second array of imaging devices disposed at an end of the device to capture images of a distal portion of the biological body segment. The second array has a generally axial viewing angle relative to the perimeter. The device further includes a controller configured to receive images captured from the first and second arrays and generate a three-dimensional reconstruction of the biological body segment based on cross-referencing of the captured images.

The imaging devices can be cameras, and cross-referencing can be performed by cross-correlation, including for example, three-dimensional digital image correlation (DIC), to generate a model of the biological body segment. The DIC can be based upon a pattern printed on the biological body segment, such as a speckle pattern. The controller can be further configured to transform a two-dimensional image point visible in at least two captured images to a three-dimensional image point by direct linear transformation to effect DIC. Cross-correlation of the captured images can be performed by any algorithm able to provide a contiguous representation of an imaged object based on overlapping fields of view from captured images,

Alternatively, the imaging devices of the first and/or second arrays can be ultrasound sensors, or a combination of cameras and ultrasound sensors. The structure can be a tank containing a fluid. The imaging devices of the first array can be disposed to fully surround the perimeter of the biological body segment. Optionally, the first array can be moveable relative to the structure. A mechanical perturbator can also be included within the structure, such as, for example, an ultrasound probe, an ultrasound probe including a force sensor, a flow-based perturbator comprising a nozzle configured to eject a fluid, or any combination thereof. The controller can be further configured to determine a mechanical property of the biological body segment. Such determination can be based on an inverse finite element analysis of the captured images, the captured images including images of a deformation of the biological body segment by the mechanical perturbator. Alternatively, or in addition, the determination can be based on a hyperelastography analysis of the captured images.

A method of generating a three-dimensional reconstruction of a biological body segment includes capturing side images of the biological body segment with a first array of imaging devices disposed about a perimeter of the biological body segment and capturing images of a distal portion of the biological body segment with a second array of imaging devices. The second array of imaging devices has a generally axial viewing angle relative to the perimeter. The method further includes generating a three-dimensional reconstruction of the biological body segment based on cross-correlation of the captured images.

A method of modeling a biological body segment includes obtaining images of an internal structure of the biological body segment, such as from computed tomography (CT) imaging, magnetic resonance (MR) imaging, ultrasound (US) imaging, or any combination thereof, and capturing images of an external surface of the biological body segment with a camera array. The method further includes generating a three-dimensional model of external features of the biological body segment based on cross-correlation of the captured images from the camera array and inter-digitizing the images of the internal structure of the biological body segment with the three-dimensional model to thereby generate a compound model of internal and external features of the biological body segment.

The inter-digitizing can include performing a shape registration of alignment points of the biological body segment. The method can further include imaging the biological body segment with at least one of CT, MR, and US to obtain the images of the internal structure. Alternatively, the internal structure images can be obtained from a medical image repository. The compound model can be used to generate a complementary biomechanical interface, which can, in turn, be fabricated.

Another device for three-dimensional imaging of a biological body segment includes an object including a plurality of inertial measurement units, the object configured to trace a surface of the biological body segment, and a controller. The controller is configured to receive motion data from each of the plurality of inertial measurement units, determine trajectories of the object in a three-dimensional space based on the received motion data, and generate a three-dimensional reconstruction of the biological body segment based on the determined trajectories. Each of the plurality of inertial measurement units can be a six-degree of freedom inertial measurement unit. The object can be, for example, a sphere.

Yet another 3D measurement device for a biological body segment includes an elastomeric sheath that is conformable to the biological body segment, a plurality of nodes affixed to the elastomeric sheath, a grid of electrically-conducting conduits connecting the nodes, and a plurality of first transducers at least a portion of either the electrically-conductive conduits or the nodes, whereby data collected by the first transducers can be employed to generate a 3D representation of the biological body segment. The first transducers can include at least one member selected from the group consisting of a stretch sensor and a curvature sensor.

A system for generating a 3D representation of a biological body segment includes a synthetic skin component and a handheld probe. The synthetic skin component includes an elastomeric sheath conformable to the biological body segment, a plurality of nodes affixed to the elastomeric sheath, a grid of electrically-conductive conduits connecting the nodes, and a plurality of first transducers at least a portion of either the electrically-conductive conduits or the nodes. The handheld probe includes at least one probe transducer selected from the group consisting of an ultrasound transducer, a pressure sensor, a shear sensor, a contact sensor, a temperature sensor, an inertial measurement unit (IMU), a light emitting diode (LED), and a vibration motor, whereby data collected by at least one of the first transducer and the probe transducer can be employed to generate a 3D representation of the biological body segment.

A method forming a biological body segment modeling device of the invention includes the steps of forming an elastomeric sheath that is conformable to the biological body segment, applying a plurality of nodes to the elastomeric sheath, and forming electrically-conductive interconnects between at least a portion of the nodes, wherein at least a portion of at least one of the nodes and the interconnects includes a first transducer, which can be a component selected from the group consisting of a stretch sensor, curvature sensor, ultrasound transducer, pressure sensor, shear sensor, contact sensor, temperature sensor, an IMU, an LED, and a vibration motor.

The interconnects can be serpentine, and can be formed between the nodes by forming the serpentine interconnects on a silicon wafer, transferring the serpentine interconnects to the elastomeric sheath by transfer printing, forming islands at intersections of the serpentine interconnects, and applying transducers at least a portion of the islands, whereby the transducers can measure strain at the interconnects during flexing of the elastomeric sheath and associated movement of the serpentine interconnects.

The nodes can contain ultrasound transducers in the form of ultrasonomicrometry crystals, which can be used to measure the absolute distance and changes in distance between nodes during movement of the elastomeric sheath, as well as to perform echo ultrasound to measure internal bone geometries.

Such devices and methods have many advantages. For example, such devices can provide for an inexpensive, lightweight, conformable, portable system for collecting biomechanical data across a biological segment, such as segment unloaded shape, tissue mechanical impedance, skin strain resulting from muscle and joint movement, tissue sensitivities to load, and blood flow characteristics. These data can then be used to inform the design of custom-fit interfacing devices, including but not limited to, prostheses, orthoses, exoskeletons, shoes, bras, beds, and wheelchair/bike seating.

A compact and portable measurement tool for rapid characterization of parts of the human body is also provided. Such devices can collect quantitative dynamic data that can be used to generate 3D digital models of shape, localized tissue impedances, and other biomechanical properties. For example, a lightweight, inexpensive and portable form factor can be used to obtain digital information about 3D surface shape, internal tissue geometries, tissue impedances, pain thresholds, nerve conduction, and blood flow. Such devices can also be modular and adaptable, with an option for inconspicuous integration of a custom set of biomechanical components.

In addition to biological segment surface shape and internal tissue geometries, tissue impedance is a useful data set for the design of comfortable mechanical interfaces between the human body and a synthetic device. A biological indenter component can be included to mechanically deform biological tissue in order to measure its hyperviscoelastic properties, or tissue impedances. (See, for example Zheng, Y. P., Mak, a F., & Leung, a K. (2001). State-of-the-art methods for geometric and biomechanical assessments of residual limbs: a review. Journal of Rehabilitation Research and Development, 38(5), 487-504, the relevant teachings of which are incorporated by reference herein in their entirety). Indenter data, and FEA biomechanical models derived from these data, provide useful insights into the design of apparel, shoes, prostheses, orthoses and body exoskeletons where safe and comfortable mechanical loading needs to be applied from the synthetic product to the human body.

In addition, such devices and methods can provide for the collection of accurate information on a comprehensive set of parameters to inform an accurate finite element analysis (FEA) model of a biological segment. Such a model can then be used to derive optimal interface characteristics such as equilibrium shape and mechanical impedance.

The information provided can resolve the challenge of designing mechanical devices that interface with organic tissue effectively and comfortably. Such mechanical devices include wearables such as shoes, clothing, health monitors, prosthetic sockets, and exoskeletons; as well as non-wearables such as seats and hospital beds.

A device for assessing tissue geometry and mechanical properties of a biological body segment includes a probe configured to deform soft tissue of the biological body segment, the probe including an ultrasound transducer, and a controller. The controller is configured to receive shear wave velocity data from the ultrasound transducer of soft tissue in an undeformed state, receive shear wave velocity data from the ultrasound transducer of soft tissue in a deformed state, and detect a mechanical property of the soft tissue based on a hyperelastography analysis of the received shear wave velocity data of the soft tissue in the undeformed and deformed states. The detected mechanical property can be a non-linear elastic behavior of the biological body segment. The hyperelastography analysis can includes determination of stiffness based on a large strain deformation.

Another device for detecting a mechanical property of a biological body segment includes a structure configured to receive the biological body segment, the structure including an array of imaging devices disposed to capture images about a perimeter of the biological body segment, a pressurization device, and a controller. The pressurization device is configured to apply pressure to the biological body segment to deform soft tissue of the biological body segment. The controller is configured to receive images captured by the array of the biological body segment in a plurality of deformed states, and determine a mechanical property of the biological body segment based on cross-correlation of the captured images.

The mechanical property can be a tissue characteristic, such as, for example, elasticity, modulus, stiffness, damping, and viscoelastic parameter, or a bone-to-tissue depth or a bone structure. The imaging devices can be cameras, and cross-correlation can be performed by three-dimensional digital image correlation (DIC). Alternatively, or in addition, the imaging devices can be ultrasound sensors. Where ultrasound sensors are included, additional tissue characteristics that can be determined include characteristics based upon speed of sound through the biological body segment, density, and attenuation of sound waves through the biological body segment.

A device for imaging a biological body segment includes a container defining a volume, at least one ultrasound probe supported within the volume of the container, wherein the ultrasound probe defines an ultrasound transducer surface, and a pressurizing device that applies pressure to a biological body segment that includes musculoskeletal tissue and that has been placed within the container, the ultrasound probe being arranged to image the biological body segment while the body segment is immersed in the fluid medium that is between the ultrasound transducer surface and the body segment. Optionally, a motion compensation camera can also be included.

A method of generating a three-dimensional image of musculoskeletal tissue of a biological body segment, the method including steps of immersing a biological body segment of musculoskeletal tissue into a container of fluid, the container defining a volume that is pressurizable while the biological body segment is immersed in the fluid and traverses a boundary between the container volume and an ambient volume beyond the container volume. A plurality of ultrasound images of the biological body segment is generated by at least one ultrasound probe within the container volume, the images being generated while the biological body segment is subjected to a plurality of discrete pressures within the container. A three-dimensional image of musculoskeletal tissue of the biological body segment is generated from the plurality of ultrasound images. Optionally, the three-dimensional images can be adjusted for motion compensation.

Such devices and methods can provide several advantages. For example, accurate shear wav velocity (SWV) measurements can be acquired without a probe making contact with the imaged body segment, thereby eliminating deformation consequent to any such contact and, therefore, without the need for the presence of a gel at the imaged body segment or probe. Further, three dimensional SWV measurements can be acquired of the imaged body segment while applying an external load other than the probe. As a consequence, an imaged body segment, such as a lower extremity, can be characterized while in various compressive states without interference from pressure applied by the probe. The apparatus and the method of the invention, therefore, can assist detection and monitoring of disease progression, more accurate analysis of muscle state and contraction ability, large-scale multidimensional elastography, and detailed comparative analysis among patients.

A device for assessing tissue geometry of a biological body segment includes a structure configured to receive the biological body segment, the structure including an array of imaging devices disposed to capture images about a perimeter of the biological body segment, a pressurization device, and a controller. The pressurization device is configured to apply pressure to the biological body segment to deform soft tissue of the biological body segment. The controller is configured to receive images captured by the array of the biological body segment in a plurality of deformed states and infer a geometry of a rigid internal structure of the biological body segment based on cross-correlation of the captured images. The applied pressure can be, for example, homogenous. The pressurization device can be, for example, a container containing fluid (and optional pump) or a compression garment.

A method of optimizing a design of a biomechanical interface for a biological body segment includes generating a three-dimensional model of the biomechanical interface by finite element analysis, including within the model spatially-varying and controllable internal structures, and designing the biomechanical interface with the spatially-varying and controllable internal structures. The spatially varying structures can comprise a cellular solid and/or a lattice, such as an edge-based lattice, a face lattice, or both. A spatially varying structure can be fabricated, such as by 3D printing. A biomechanical interface can, in turn, be fabricated form the spatially varying structure.

A method of designing a biomechanical interface for a biological body segment includes generating a three-dimensional model of the biological body segment and the biomechanical interface, such as, for example, a finite element analysis model. The method further includes designing the biomechanical interface with an initial fitting pressure and, using the model, determining a loading pressure of the designed biomechanical interface to at least one region of the biological body segment. The loading pressure can be determined, for example, in a simulated use case, such as standing, running, or walking. The method further includes comparing the determined loading pressure to a physiological tolerance, such as, for example, a pain threshold or a pain tolerance, and varying at least one of a compliance or a geometry of the designed biomechanical interface based on the determined loading pressure and the physiological tolerance. If the loading pressure is greater than the physiological tolerance, the process can be iteratively repeated until the determined loading pressure is below the physiological tolerance. Optionally, multiple loading pressures and/or loading pressures across multiple regions of the biological body segment can be determined, and these loading pressures can be compared to multiple physiological tolerances and/or physiological tolerances across multiple regions of the biological body segment. Additionally, within an anatomical region with a distinct physiological tolerance, a variance among two or more loading pressures can be minimized. Still further, the differential between the loading pressure and the physiological tolerance at each anatomical point and/or each anatomical region can be maximized, and/or the variance of the differentials among two or more anatomical points or anatomical regions can be minimized.

## BRIEF DESCRIPTION OF THE DRAWINGS

The foregoing will be apparent from the following more particular description of example embodiments, as illustrated in the accompanying drawings in which like reference characters refer to the same parts throughout the different views. The drawings are not necessarily to scale, emphasis instead being placed upon illustrating embodiments.

The patent or application file contains at least one drawing executed in color. Copies of this patent or patent application publication with color drawing(s) will be provided by the Office upon request and payment of the necessary fee.

FIG. 1 is a schematic illustrating stages of producing a biomechanical interface.
FIG. 2A illustrates a perspective side view of a three-dimensional imaging device.
FIG. 2B illustrates a perspective bottom view of the three-dimensional imaging device of FIG. 2A
FIG. 3 illustrates a cut-away view of an alternative three-dimensional imaging device.
FIG. 4 is a diagram illustrating calibration, data-acquisition, correlation, and post-processing procedures, and the relationship between each procedure, for a three-dimensional imaging device.
FIG. 5A is a graph illustrating checkerboard image positions and orientations with respect to a camera.
FIG. 5B is an image of detected and reprojected checkerboard corner points on an original checkerboard image.
FIG. 5C is an image of the detected and reprojected checkerboard cornerpoints on the same image as in FIG. 5B, after distortion has been removed using calculated camera intrinsic parameters.
FIG. 6A is an example of speckling pattern template.

FIG. 6B is an image of a laser-cut speckling rubber stamp.

FIG. 6C is an image of skin on which the speckle pattern of FIGS. 6A-B is applied.

FIG. 7 illustrates positions of a Triangular Cosserat Point Element (TCPE) in a reference ($t=t_0$) configuration and in a current ($t \neq t_0$) configuration with $D_3$ and $d_3$ being normal to the plane of the TCPE.

FIG. 8 illustrates an example of a synthetically deformed object (SDO), which has undergone an axial elongation with a stretch value of 1.3 (30% strain) relative to its reference model. The speckle pattern on the model is deformed locally with the cylinder.

FIG. 9A is a photo of a speckled skin indenter equipped with a force censor, the indenter including a one-dimension (1D) thin beam load cell.

FIG. 9B is a photo of a skin indenter equipped with a force censor, the indenter including a 6-axis force/torque transducer (Nano-17, ATI Industrial Automation).

FIG. 9C is a photo illustrating an example of simultaneous displacement and force measurement during indentation.

FIG. 9D is an example of simulated indentation using Finite Element Analysis (FEA).

FIG. 10 illustrates a 3D skin surface reconstruction from two sets of 12 simultaneous images each. The first set was taken with the knee in its most extended position, and the second set with the knee at a relaxed position. The local deformation from the first to the second set is depicted. The shading represents magnitude of the first and second principal Lagrangian strains, and strain directions are represented as black lines. Raw local values are shown, without any smoothing, noise reduction, or outlier removal.

FIG. 11 illustrates a 3D skin surface reconstruction from two sets of 12 simultaneous images each. The first set was taken immediately after doffing a socket and the second set was taken ten minutes later. The local deformation from the first to the second set is depicted. The local surface area change is represented. Raw local values are shown, without any smoothing, noise reduction, or outlier removal.

FIG. 12A is a schematic of an example MIMU system in the form of a sphere equipped with twelve IMUs.

FIG. 12B illustrates a sweeping profile along a biological body segment with the MIMU system of FIG. 12A.

FIG. 12C illustrates painting of a region surrounding a biological body segment with the MIMU system of FIG. 12A.

FIG. 13 illustrates a simulated spherical measurement instrument.

FIG.14A illustrates an example simulated MIMU in a three-dimensional space.

FIG. 14B illustrates the MIMU of FIG. 14A traveling along a trajectory.

FIG. 14C illustrates the MIMU of FIG. 14B continuing to travel the trajectory.

FIG. 14D illustrates the MIMU of FIG. 14C completing the trajectory.

FIG. 15A illustrates a measurement path of a simulated MIMU.

FIG. 15B illustrates a triangulated geometry for the measurement path shown in FIG. 15A.

FIG. 15C illustrates a measurement of the simulated MIMU at a later point in time than FIG. 15A.

FIG. 15D illustrates a triangulated geometry for the measurement path shown in FIG. 15B.

FIG. 15E illustrates a measurement of the simulated MIMU at a later point in time than FIG. 15C.

FIG. 15F illustrates a triangulated geometry for the measurement path shown in FIG. 15E.

FIG. 16A illustrates instrument motion for high-error simulated IMU data without calibration using an averaging correction method.

FIG. 16B illustrates instrument rotation for high-error simulated IMU data without calibration using an averaging correction method.

FIG. 16C illustrates instrument motion for high-error simulated IMU data without calibration using an instrument shape correction method.

FIG. 16D illustrates instrument rotation for the IMU for high-error simulated IMU data without calibration using an instrument shape correction method.

FIG. 17 illustrates the results of an accuracy test for motion processing and correction methods.

FIG. 18A illustrates results of a sphere geometry reconstruction using an averaging correction method.

FIG. 18B illustrates instrument trajectory over time for the geometry reconstruction test of FIG. 18A.

FIG. 18C illustrates a resulting triangulated geometry for the geometry reconstruction test of FIG. 18A.

FIG. 19 is a side view of a system including a thin elastomeric skin optimized for 3D shape capture and force localization, plus a handheld probe for other imaging and sensing.

FIG. 20 is a perspective view of one node and grid component of the system shown in FIG. 1, including one detailed edge.

FIG. 21A is a schematic representation of an unloaded parallel plate capacitive stretch sensor employed in the system of FIG. 19.

FIG. 21B is a schematic representation of the parallel plate axial stretch causing distance (d) between plates to decrease, corresponding to increased electrical capacitance.

FIG. 22A is a schematic of an unloaded simple, unipolar resistive curvature sensor employed in the system of FIG. 19 with the capacitive stretch sensor of FIG. 21A in a loaded condition.

FIG. 22B is a schematic representation of the resistive curvature sensor of FIG. 22A in a loaded condition, wherein bending causes particles to lose contact, leading to an increase in electrical resistance.

FIG. 23A is an example of a sensor employed with dual functionality for measurement of orthogonal and shear forces.

FIG. 23B is a cross-sectional side view of the sensor of FIG. 23A, showing an unloaded state overlaid with a loaded state, where the thick arrows indicate a force with both orthogonal and shear components.

FIG. 24 is a cross-sectional view of a handheld probe component for deep-tissue and high-fidelity imaging and sensing, including an ultrasound transducer at the tip and a compartment for additional electronics in the body.

FIG. 25 is an example system in the form of a sock, during its intended application.

FIGs. 26A-26D represent an example system in the form of a prosthetic socket liner for the residual limb of a transtibial amputee, during use as the leg goes through multiple poses from maximum knee bend (FIG. 26A); to maximum knee extension (FIG. 26B); and the generated visual maps of skin strain (FIG. 26C); and tissue impedance for perpendicular tissue displacements (FIG. 26D), using the data collected by embedded shape and force sensors within the liner.

FIGs. 27A-9D show use of a system that includes optimized prosthetic liners for amputees (FIGs. 27A and 27B), with multi-durometer materials corresponding to extreme skin strain values, aiming to reduce skin irritation. The various durometers are shown here in different shades in the optimized computer model (FIG. 27C), and the 3D-printed socket generated using that model (FIG.27D).

FIG. 28 is an example system in the form of a smart bra that measures breast shape and properties for custom bra fitting or monitors health of the underlying breast tissues.

FIGs. 29A and 29B are plan views of a serpentine interconnected-islands structure for electronics wiring robust to a material stretch. FIG. 29A is the structure in an unloaded condition. FIG. 29B is a representation of the structure of FIG. 29A in a loaded state. Uniaxial stretch causing the flattening of the serpentine shape.

FIG. 30 is a schematic representation of ultrasound transducers being used in a thin elastomeric skin on a human biological limb. The acoustic signal transmitted by one ultrasonomicrometry crystal can be received by the crystal itself or by other crystals in the array, and the time-of-flight can be used to derive distances through deep tissue to bone, or at the surface between crystals.

FIG. 31 is a schematic of an example ultrasound-force probe assessing a local bone depth.

FIG. 32 is a diagram of an example ultrasound-force probe system.

FIG. 33A is a photo of a prototype ultrasound-force probe.

FIG. 33B is a schematic of a tip of an ultrasound-force probe.

FIG. 34A illustrates use of an ultrasound-force probe for tissue boundary detection with a limb depicted in a frontal view.

FIG. 34B illustrates use of an ultrasound-force probe for tissue boundary detection with the limb depicted in an axial, sliced view.

FIG. 35A illustrates use of an ultrasound-force probe for indentation testing with a limb depicted in a frontal view.

FIG. 35B illustrates use of an ultrasound-force probe for indentation testing with the limb depicted in an axial, sliced view.

FIG. 36 is a diagram of modes of operation of an ultrasound-force probe.

FIG. 37A is a graph of a raw waveform obtained from an ultrasound-force probe.

FIG. 37B is a graph of a processed waveform of FIG. 37A.

FIG. 38A is an example of an accumulated detection graph to determine peaks that are most likely to represent bone depth. The two most prominent peaks after the boundary peak (right most peak) are likely bone depth representations.

FIG. 38B is an example of an edge histogram of the data presented in FIG. 38A.

FIG. 39 illustrates tilt angles of a tri-axis accelerometer.

FIG. 40 illustrates accelerometer directions for an ultrasound-force probe.

FIG. 41 is a photo of a staircase phantom for calibration of an ultrasound-force probe.

FIG. 42 is a graph of preliminary data acquired with the phantom of FIG. 41 and an ultrasound force probe from steps of depths from 16 mm to 86 mm. The time lapse was recorded for each step and the results were linearly fitted to estimate the speed of sound in the phantom to 1007 m/s.

FIG. 43A is a photo of a camera verification set up of a phantom indentation experiment from a left-view.

FIG. 43B is a photo of a camera verification set up of a phantom indentation experiment from a right-view.

FIG. 44A is a photo of an indention experiment using a left-view camera.

FIG. 44B is a photo of an indention experiment using a right-view camera.

FIG. 45A illustrates an MR scan of limb with five markers disposed around the limb for an ultrasound experiment.

FIG. 45B illustrates the MR scan of FIG. 45A with lines indicating the shortest paths to the tibia and fibula from marker 1.

FIG. 45C illustrates the MR scan of FIG. 45A with lines indicating the shortest paths to the tibia and fibula from marker 2.

FIG. 45D illustrates the MR scan of FIG. 45A with lines indicating the shortest paths to the tibia and fibula from marker 3.

FIG. 45E illustrates the MR scan of FIG. 45A with lines indicating the shortest paths to the tibia and fibula from marker 4.

FIG. 45F illustrates the MR scan of FIG. 45A with lines indicating the shortest paths to the tibia and fibula from marker 5.

FIG. 46 is an error plot of measurements obtained during a phantom staircase experiment of depths from 16 mm to 86 mm, including four trials per step.

FIG. 47 is an error histogram of depth detections from a phantom staircase experiment.

FIG. 48 is an error histogram of indentations from a phantom staircase experiment using DIC as ground truth.

FIG. 49 plots indentation, force, and tilt angle over time for an indentation experiment.

FIG. 50 is an error histogram of indentations from an in-vivo experiment using DIC as ground truth.

FIG. 51A is an error plot of depth measurements obtained from an ultrasound-force probe as compared with MRI as ground truth.

FIG. 51B is an error plot of depth measurements obtained from commercial ultrasound system as compared with MRI as ground truth.

FIG. 52A is an error histogram of the depth measurements of FIG. 51A.

FIG. 52B is an error histogram of the depth measurements of FIG. 51B.

FIG. 53 plots the estimated Phantom Device Function (PDF) of four experiments using a prototype ultrasound-force probe: phantom depth measurements, phantom indentation measurements, in-vivo depth measurements, and in-vivo indentation measurements. All trial results showed a mean error below 0.5 mm and a standard deviation of error below 2.5. Overall, the error is evenly distributed.

FIG. 54A plots the estimated PDF of a phantom depth measurement experiment.

FIG. 54B plots the estimated PDF of a phantom indentation measurement experiment.

FIG. 54C plots the estimated PDF of an in-vivo indentation experiment.

FIG. 54D plots the estimated PDF of an in-vivo depth measurement experiment.

FIG. 55 illustrates flow-induced mechanical perturbator.

FIG. 56 is an example of a hyperelastic stress-stretch curve, illustrated for an example of uniaxial loading. The slope can be used to determine an effective stiffness, which varies with stretch. An initial slope at $\lambda=1$ is for an undeformed configuration. At compressive or tensile deformations, a resistance to deformation increases.

FIG. 57A is a perspective view of an example ultrasound hyperelastography device.

FIG. 57B is a diagram illustrating use of an ultrasound hyperelastography device.

FIG. 58A is a diagram illustrating use of an ultrasound-force probe for hyperelastography measurements in an initial, nondeformed state.

FIG. 58B is an image of shear wave velocity data obtained from an ultrasound-force probe for tissue in the initial, nondeformed state of FIG. 58B.

FIG. 58C is a diagram illustrating use of an ultrasound-force probe for hyperelastography measurements in a deformed state.

FIG. 58D is an image of shear wave velocity data obtained from an ultrasound-force probe for tissue in the deformed state of FIG. 58C.

FIG. 59 is a schematic illustrating a device for three-dimensional imaging of a biological body segment.

FIG. 60 is a three-dimensional view of a setup for shear wave elastography (SWE) scanning of a calibrated phantom by scanning with a standard gel approach. The ultrasound probe is fixed to a ring stand facing downward. A layer of ultrasonic coupling gel is placed between the transducer and phantom surface.

FIG. 61 is a representation of a prior art apparatus for SWE scanning of a human lower limb by a standard gel approach, including an ultrasound probe fixed to a ring stand facing the limb, and wherein a layer of ultrasonic coupling gel is placed between the transducer and limb surface.

FIG. 62 is a setup for SWE scanning of a calibrated phantom with a water tank, wherein a ring stand is employed to secure the ultrasound transducer into a fixed position. The phantom may be moved at incremental distances away from the ultrasound transducer in the tank.

FIG. 63 is an example apparatus for SWE scanning with a water tank system, wherein a ring stand is employed to secure an ultrasound transducer at a distance from a limb that is held constant between each scan, and wherein the limb is under a load applied by a compression support garment.

FIG. 64 is another example of an apparatus that can be employed by a method of the invention to collect 3D SWE data.

FIG. 65 is a perspective view of another example device, wherein an ultrasound tank of the device can seal a biological body segment from the outside environment.

FIG. 66 is a perspective view of a single element scanning system of the invention (in the absence of a pressurizing device) with a processing unit linked to the ultrasound probe.

FIG. 67 is a series of ultrasound images with overlaid SWV maps of a calibrated phantom at 0.5 cm incremental distances away from the ultrasound transducer surface by employing a gel approach.

FIG. 68 is a series of ultrasound images with overlaid SWV maps of the phantom in the water tank. As shown, accurate measurements were achieved for both the gel (FIG. 67) and water tank (FIG. 68) methods. However, the water tank setup performed more consistently, and also allowed for measurements to be taken at more than twice the distance of the gel layer.

FIG. 69 is a plot of the mean and standard deviation values for the SWV maps shown in FIGs. 67 and 68.

FIG. 70 is a series of ultrasound images of a subject's leg under four compressive states in both longitudinal (top) and transverse (bottom) orientations, acquired by a method of the invention.

FIGs. 71A and 71B are plots of mean and standard deviation values for the SWV maps exemplified in FIG. 70, for Subject 1, and FIGs. 72A and 72B, for Subject 2.

FIG. 73 is a representation of a volumetric image data series of B-mode images collected at 10-degree increments around a subject's limb placed in 3D space.

FIG. 74 is a representation of a series of SWE images collected at 10-degree increments around a subject's limb placed in 3D space by a method of the invention.

FIGs. 75A-D represent volume results of images showing 3D changes in SWV at varying compressive loads. (A) Unloaded. (B) 8-15 mmHg compression garment. (C) 15-20 mmHg compression garment. (D) 20-30 mmHg compression garment.

FIG. 76 are plots of mean and standard deviation values for the SWV maps exemplified in FIGs. 75A-D. Similar to the 2D data, measurable changes in mean SWV at superficial muscle layers may be detected when applying an external compressive load to the limb. Further, measurements taken from the standard gel approach are comparable to those acquired in the water tank.

FIG. 77A illustrates a perspective view of an ultrasound tank system.

FIG. 77B illustrates a top view of the ultrasound tank system of FIG. 77A.

FIG. 77C illustrates a side view of the ultrasound tank system of FIG. 77A.

FIG. 78 is a diagram of an experimental electronic control system for ultrasound tank device.

FIG. 79A illustrates two example surfaces that were collected at two different time points during a scan, projected on the x-z plane.

FIG. 79B illustrates the two example surfaces of FIG. 79A projected on the x-y plane.

FIG. 80 is a diagram of a coordinate frame used for image registration and stitching process for creation of a 2D image slice.

FIG. 81A is a schematic of a calibration device to simulate controlled leg motion.

FIG. 81B is a schematic depicting a calibration procedure.

FIG. 81C is a cross-sectional rendering of the calibration device of FIG. 81A.

FIG. 81D is an example of a reconstructed ultrasound image of the phantom.

FIG. 82A is an image of two overlaid ultrasound images collected at different circumferential positions. There is clear motion present in the scan, as evidenced by the discontinuity in the skin surface near the top of the scan.

FIG. 82B is an image of the two ultrasound images shown in FIG. 82A after having undergone motion compensation using 3D camera data. Anatomy between the two images is correctly matched.

FIG. 82C is an image of an example ultrasound reconstruction with no motion compensation.

FIG. 82D is an image of the example ultrasound reconstruction of FIG. 82C after motion compensation.

FIG. 83A is an MR image of a representative slice of a research subject. The tibia bone is shown outlined.

FIG. 83B is a corresponding US image of the representative slice of FIG. 83A. The tibia bone is shown outlined.

FIG. 83C is an MR image of another representative slice of a research subject. The skin boundary is shown outlined.

FIG. 83D is a corresponding US image of the representative slice of FIG. 83C. The skin boundary is shown outlined.

FIG. 84 illustrates an MRI result for an example limb along slice planes XY, XZ, and YZ.

FIG. 85 illustrates a corresponding volume ultrasound imaging result for the example limb shown in the MRI of FIG. 84. As shown, using camera-based motion compensation, acquisition sweeps can be stitched together in 3D space to produce continuous skin and bone boundaries.

FIG. 86A illustrates surface contours of skin, tibia, and fibula from 3D ultrasound (US) data.

FIG. 86B illustrates resulting surfaces from the US contours of FIG. 86A.

FIG. 86C illustrates resulting surfaces from MRI, which were created in the same manner as the US surfaces of FIGS. 86A-B.

FIG. 86D is a 3D difference map showing differences (mm) between MRI and US skin surfaces.

FIG. 86E is a 3D difference map showing differences (mm) between MRI and US tibia surfaces.

FIG. 86F is a 3D difference map showing differences (mm) between MRI and US fibula surfaces.

FIG. 87 illustrates an example of a process for prediction of hidden internal features.

FIG. 88 illustrates another example of a process for prediction of hidden internal features.

FIG. 89 illustrates a data-driven computational design framework.

FIG. 90 illustrates an expanded virtual prototyping and optimization process.
FIG. 91 illustrates a process for forming a cosmesis based on an unaffected limb.
FIG. 92A illustrates examples of lattices of varying structures and solid formulations for modeling such structures.
FIG. 92B illustrates an FEA-based socket design.
FIG. 92C illustrates optimization of the FEA-based socket design of FIG. 92B with spatially-varying and controllable structures.
FIG. 93 illustrates an example of a cellular element with uniform density.
FIG. 94 illustrates an example of a cellular element with spatially varying density.
FIG. 95 illustrates an example of structural variations for a cellular mesh.
FIG. 96 illustrates a dual structure for a cellular mesh.
FIG. 97A illustrates a Schwarz p-surface lattice.
FIG. 97B illustrates a Schwarz d-surface lattice.
FIG. 97C illustrates a gyroid surface lattice.
FIG. 97D illustrates a Neovius surface lattice.
FIG. 97E illustrates a w-surface lattice.
FIG. 97F illustrates a pw-surface lattice.
FIG. 98A illustrates a structure defined in a template tetrahedral element.
FIG. 98B illustrates the element of FIG. 98A mapped into a general 3D tetrahedral mesh creating a continuous lattice structure.
FIG. 99 illustrates a method of creating lattice structures from general volumetric mesh descriptions.
FIG. 100 illustrates a mixed tetrahedral and hexahedral meshing.
FIG. 101 illustrates a cut-view of a volumetric mesh of a bar with spatially varying mesh density.
FIG. 102 illustrates a hexahedral element and several conversions to tetrahedral elements.
FIG. 103 illustrates lattice structures with varying densities and varying structure types for a cube.
FIG. 104 illustrates lattice structures of varying porosities based on varying strut thicknesses.
FIG. 105 illustrates lattice structures on hexahedral elements derived from edges, complimentary lattice structures that pass through face centers of the elements, and combination structures.
FIG. 106 illustrates lattice structures on tetrahedral elements derived from edges, complimentary lattice structures that pass through face centers of the elements, and combination structures.
FIG. 107 illustrates conversion of tetrahedral meshes to lattice structures for a prosthetic socket.
FIG. 108 illustrates hierarchical lattice structures on a tetrahedral input mesh.
FIG. 109A illustrates example coiled struts of varying amplitudes.
FIG. 109B illustrates 3D lattice structures including coiled struts.
FIG. 110 illustrates multi-phasic structures.
FIG. 111 illustrates a noisy/angular mesh undergoing a smoothing process.
FIG. 112 illustrates smoothing of lattice structures of an increasing number of iterations.
FIG. 113 illustrates smoothing of lattice structures while constraining boundary vertices.
FIG. 114A illustrates a solid cube for mechanical behavior analysis.
FIG. 114B illustrates the cube of FIG. 114A subjected to tension.
FIG. 114C illustrates the cube of FIG. 114A subjected to compression.
FIG. 114D illustrates the cube of FIG. 114A subjected to shear.
FIG. 115A illustrates a lattice structure for mechanical behavior analysis.
FIG. 115B illustrates the lattice structure of FIG. 115A subjected to tension.
FIG. 115C illustrates the lattice structure of FIG. 115A subjected to compression.
FIG. 115D illustrates the lattice structure of FIG. 115A subjected to shear.
FIG. 116A illustrates a response to tension of the solid cube of FIG. 114A.
FIG. 116B illustrates a response to compression of the solid cube of FIG. 114A.
FIG. 116C illustrates a response to shear of the solid cube of FIG. 114A.
FIG. 116D illustrates a response to tension of the lattice structure of FIG. 115A.
FIG. 116E illustrates a response to compression of the lattice structure of FIG. 115A.
FIG. 116F illustrates a response to shear of the lattice structure of FIG. 115A.
FIG. 116G illustrates a response to tension of the lattice structure of FIG. 115B.
FIG. 116H illustrates a response to compression of the lattice structure of FIG. 115B.
FIG. 116I illustrates a response to shear of the lattice structure of FIG. 115B.
FIG. 116J illustrates a response to tension of the lattice structure of FIG. 115C.
FIG. 116K illustrates a response to compression of the lattice structure of FIG. 115C.
FIG. 116L illustrates a response to shear of the lattice structure of FIG. 115C.
FIG. 116M illustrates a response to tension of the lattice structure of FIG. 115D.

FIG. 116N illustrates a response to compression of the lattice structure of FIG. 115D.

FIG. 116O illustrates a response to shear of the lattice structure of FIG. 115D.

FIG. 117A illustrates an example of obtaining residual limb geometries based on CT imaging. An axial CT slice with a highlighted tissue contour of the tibia is shown.

FIG. 117B illustrates highlighted tissue contours of the tibia obtained from several CT images of the subject shown in FIG. 117A.

FIG. 117C illustrates segmented voxel sets and surface models of the tibia based on the contours obtained from the CT data of FIGS. 117A and 117B.

FIG. 117D illustrates example tissue types of interest that can be obtained from imaging data, including for example, the patellar tendon, patella, femur, fibula, tibia, and external skin surface.

FIG. 118A illustrates an example of obtaining external residuum shape and tissue mechanical properties using DIC and a force probe.

FIG. 118B illustrates an example of aligning DIC data with CT data.

FIG. 118C illustrates an example model of the residuum of FIG. 118B.

FIG. 118D is a graph of experimental and simulated force-displacement curves.

FIG. 119A illustrates coronal-plane mechanical axis orientation of a lower extremity during quiet standing. The mechanical axis is perpendicular to the ground during a quiet standing posture.

FIG. 119B illustrates an enlarged view of mechanical axis orientation of FIG. 119A. The axis passes proximally from the femoral head and distally through the center of the ankle joint in the coronal plane. Relative to the knee, the coronal-plane mechanical axis passes approximately 8 mm lateral of the apex of the tibia referred to as the mechanical axis deviation (MAD).

FIG. 119C illustrates a mechanical axis line relative to a load line of a quantitatively designed transtibial socket.

FIG. 120A illustrates an example of biomechanical regions of a transtibial residual limb, shown in anterior, lateral, posterior, and medial views.

FIG. 120B illustrates an example of final fitting pressures of a representative socket for a transtibial residual limb, as shown in FIG. 120A.

FIG. 120C illustrates corresponding loading pressures for the representative socket shown in FIG. 120B in an example standing use case.

FIG. 120D illustrates a socket design that results in the fitting pressures shown in FIG. 120B and corresponding loading pressures of FIG. 120C.

## DETAILED DESCRIPTION

A description of example embodiments follows.

Devices and methods for obtaining external shapes and internal tissue geometries, as well as tissue behaviors, of a biological body segment are provided. Devices and methods for designing and fabricating a biomechanical interface, such as a prosthetic device, or a part of a prosthetic device, that interfaces with the biological body segment, are also provided.

Such devices and methods can be used to create a quantitative, subject-specific biomechanical interface for a biological body segment. Examples of biological body segments and corresponding biomechanical interfaces include an ankle-foot in the case of shoe design, breasts in the case of bra design, an amputated-residuum in the case of prosthetic socket design, buttocks in the case of seat design, and a limb or a torso, or section thereof, in the case of an exoskeletal or orthotic design.

An overview of methods included in producing a quantitative, subject specific biomechanical interface are shown in FIG. 1. At an initial stage (stage 1), data pertaining to a biological body segment 102 of a subject 100 is obtained with an imaging device 104. The imaging device 104 can measure both tissue geometry and tissue mechanical properties for use in creating a digital representation of the biological body segment. In a second stage (stage 2), a computational model 106 of the biological body segment can be generated based on the collected data to produce and optimize a digital design of a biomechanical interface. Lastly, digital fabrication (stage 3) can occur in which additive or subtractive computer-aided manufacture is conducted to produce the biomechanical interface 110, such as by use of a three-dimensional (3D) printer 108.

The determination of tissue geometry includes measurement of internal features *(e.g.,* muscle and bone architecture) and external features *(e.g.,* skin surface shape). Various non-invasive imaging methods may be employed for assessment of geometry. The determination of mechanical properties of soft tissue in-vivo includes: 1) mechanical perturbation of the tissue, 2) measurement of the response to the perturbation, and 3) analysis of these measurements.

Any physical phenomenon that mechanically interacts with tissue can be used as a mechanical perturbation. Examples include externally-applied tissue loading, such as pressures, indentations, and vibrations. The mechanical perturbation may also be physiological in nature, such as muscle activation or a study of pulsatile motions (e.g., as induced by blood vessels).

Measurement of the response of a tissue to a perturbation can include assessment of the tissue loads (such as forces and stresses), motions, and deformations. Loading, motion, and deformation measurement techniques may rely on contacting (invasive) methods or on non-contacting (non-invasive) methods. In the case of indentation of the tissue, loading may be assessed through force sensors implemented in an indenter. Alternatively, or in addition, load sensing devices may be applied to the tissue surface to assess local load (such as force, pressure, and stress) or sensing systems may be implanted to obtain load measurements.

Skin tissue shape, motion, and full-field deformation may be assessed by external, non-contacting methods, such as with use of digital image correlation (DIC), as is described further below. DIC can rely on contrasting features on the skin tissue, such as speckles that are either naturally present or artificially added as fiducial markers, to cross-correlate images obtained of the skin tissue towards generating a model of the biological body segment.

Internal shapes and deformations may be measured non-invasively using medical imaging techniques, including, for example, ultrasound (US), magnetic resonance imaging (MRI), computed tomography (CT), and near-infrared light imaging. Multiple quasi-static image data sets can be acquired, which can allow for derivation of deformation measurements by post-processing methods (*e.g.*, through the use of non-rigid registration methods). Dedicated deformation measurement techniques may also be employed, such as with ultrasound 3D strain imaging, as described further below. In the case of MR, many different dedicated deformation imaging techniques exist; an example includes spatial modulation of the magnetization (SPAMM) tagged MRI. These non-invasive medical imaging based methods can provide for both 3D internal shape data and deformation data.

During a mechanical perturbation of the tissue, if the applied load and resulting response are known, analysis techniques can be used to derive mechanical properties of the tissue. Mechanical tissue properties include, for example, stiffness, damping, modulus, elasticity, and viscoelasticity. If large deformations are used for the mechanical perturbation, inverse analysis techniques can be used, such as inverse finite element analysis (FEA). In this case, knowledge of initial shape and boundary conditions, combined with assumed mechanical properties, allows one to formulate a forward model of the experiment. The forward model can predict a tissue response to loading, which can be compared to an experimentally measured response. Next, the mechanical model and employed parameters can be iteratively updated (*e.g.*, using optimization methods, as described in Section 11 herein), with the aim of matching the experimentally observed response (*e.g.*, which may include iterative matching of tissue deformation, strains, stresses, *etc*.). Through such inverse analyses, large strain and non-linear behavior can also be studied.

The following sections describe devices and methods for use in the three stages shown in FIG. 1 of producing a biomechanical interface.

### 1. Three-Dimensional Digital Image Correlation (DIC) for Geometry and Full-field Deformation Assessment

In this section, DIC devices and methods are presented, and how data collected using DIC can inform the design of a biomechanical interface that connects a wearable device to a biological segment. Although the use of DIC for amputated residuum measurements and modeling are illustrated, it will be understood that such methods and devices can be applied equally well to the digital representation, and subsequent digital design, of any biological segment and biomechanical interface attached thereto, including but not limited to an ankle-foot in the case of shoe design, breasts in the case of bra design, an amputated-residuum in the case of prosthetic socket design, buttocks in the case of seat design, and a limb or a torso, or section thereof, in the case of an exoskeletal or orthotic design.

Local changes in the volume, shape, and mechanical properties of the residual limb can be caused by adjacent joint motion, muscle activation, hydration, atrophy, and other factors. These changes can affect socket fit quality and might cause inefficient load distribution, discomfort, and dermatological problems. Analyzing these effects can be an important step in considering their influence on socket fit and in accounting for their contribution within the socket design process.

Shape and volume changes in a residual limb can lead to changes in limb-socket interface pressure and shear stress distributions, which can, in turn, lead to socket fit problems. For instance, volume reduction might lead to increased pistoning of the residuum within the socket, areas of high stresses, typically around bony prominences, and a compromised transfer of loads between the limb and the socket.

Residual limb changes are caused by different sources, any of which may influence socket fit and function, including, for example: generalized postoperative edema resulting from surgery and/or injury to the limb; postoperative muscle atrophy; discrete, postoperative fluid collections distinct from generalized edema; and, residual limb muscle activity. These changes can be drastic, especially in the first 6-12 months post-amputation. However, mature residual limbs (e.g., at approximately 18 months or longer post-amputation) may still be subject to changes in volume and shape. The amount of daily fluctuation can vary among amputees as a function of comorbidities, prosthesis fit, activity level, and other factors.

Appropriate representation of shape and volume changes of the residual limb can be an important component of socket design strategies. Such strategies can include accounting for short term changes to shape and volume to adjust socket design(s) and thereby produce new socket design(s) as changes to the residual limb occur over time.

Methods and devices that can provide for non-invasive and low-cost systems capable of obtaining full-field deformations and mechanical properties of a residual limb were developed. Digital Image Correlation (DIC) can be employed in such methods to allow for full-field measurements of the biological body segment, which can provide a detailed description of a limb surface, including limb surface deformations, as well to allow for the ability to obtain mechanical property data when combined with a physical indenter. Such methods and devices can be used to measure displacements, deformations, and strains on almost any material.

DIC is an optical-numerical technique based on sets of images of a surface of a specimen in undeformed (reference) and deformed (current) states. DIC can be implemented both in a 2D and a 3D version. The resulting data can provide for measurement of a 3D biological limb segment's volume, shape, and deformation in order to inform the design of a biomechanical interface between the biological body and a wearable device.

A challenge for successful in-vivo measurements is that subject motion during the measurements may be unavoidable. Imaging methods in which the scanner is moved around the body segment may not be feasible due to subject motion. To overcome this problem, an imaging device for use with DIC can include multiple cameras that are synchronized with high accuracy to limit or omit a need to move cameras relative to the body segment being imaged. A further challenge with shape measurements can be determining a correct alignment of different shapes in order to compare the shapes. Using DIC, correspondence between surface points is tracked to ensure proper alignment.

*Example Device for 3D Imaging of a Biological Body Segment*

A 360° 3D digital image correlation (3D-DIC) system was developed for full-field shape and deformation measurements of the residuum. A multi-camera rig was designed for capturing synchronized image sets as well as force measurements from a hand-held indenter. Custom camera calibration and data-processing procedures were specifically designed to transform image data into 3D point clouds and automatically merge data obtained from multiple views into continuous surfaces. Moreover, a specially developed data-analysis procedure was applied for correlating pairs of largely deformed images of speckled surfaces, from which displacements, deformation gradients, and strains were calculated.

The entire procedure was validated by analyzing the strains of synthetically deformed 3D objects. First, a reference finite element (FE) model of a speckled cylinder was created. Then, different cases of prescribed deformation were simulated (e.g., homogeneous uniaxial tension, radial inflation, axial torsion). The simulated deformed objects contain the deformed state of a reference speckle pattern. The reference and deformed models were then manufactured using a multi-color 3D printer and were analyzed using the imaging system in order to evaluate its accuracy.

Furthermore, the residuum skin of two transtibial amputees were speckled with black ink using a custom made speckling stamp, and imaged in different configurations: in various knee angles and muscle contraction levels, different times after doffing of the prosthetic socket, and at different times of the day. The images were processed to obtain the associated full-field displacements and strains.

Local and subject-specific soft tissue mechanical properties were obtained by analyzing surface deformation and force measurement during indentation using inverse finite element analysis. These data can be used to accurately describe the residuum's biomechanical behavior. Characterization of the limb's geometry as well as the full-field deformations using 3D-DIC can be used to design optimal prosthetic sockets which take into account these effects.

*Materials and Methods*

*System design*

The 360-deg 3D-DIC stereo rig was developed to be suitable for measuring a residual limb in two configurations: 1) deformation analysis of the entire residual limb; and 2) indentation tests of different anatomical locations on the limb.

The following specifications were identified and taken into account for the example, experimental rig design: 1) consist of mostly inexpensive off-the-shelf components; 2) be mobile and easy to assemble and use; 3) enable the imaging of the entire residual limb simultaneously; 4) be adjustable and versatile enough to accommodate differently sized and shaped limbs; 5) be accurate enough to capture shape changes and both in-plane and out-of-plane displacements; 6) allow for the measurement of large strains; and 7) enable a fast and robust calibration procedure and acquisition.

The experimental system design consisted of multiple ($N_c$) cameras arranged in two sets: one set of $N_p$ coaxial cameras in a full circle *(e.g.,* 360 degree arrangement) pointing towards the center of the circle, to capture the proximal part of the residual limb, and a second set of $N_d$ cameras to capture the distal end of the limb. Raspberry Pi camera boards (Raspberry Pi Foundation, UK) were selected for the experimental system due to their low cost, small dimensions, the capability to control them remotely, the ability to capture images simultaneously from a large set of cameras, and the ability to transfer multiple data for further analysis.

An example of a 3D imaging device is shown in FIGS. 2A-B. The device 130 includes a structure 120a, 120b configured to receive a biological body segment 102. The structure 120a includes a first array 122 of imaging devices 128 disposed about a perimeter of the device to capture side images of the biological body segment 102. The structure 120b further includes a second array 124 of imaging devices disposed to capture images of a distal portion of the biological body segment 102. The second array 124 can have a generally axial viewing angle relative to the perimeter. The structure 120a,b is shown in FIG. 3 as comprising two parts for illustration purposes only. The structure can be unitary or can comprise two or more substructures configured to receive the segment 102. The structure can optionally include a mechanical perturbator 126, such as a mechanical indenter that includes at least one force and/or torque sensor, a flow-based indenter, a probe that includes an ultrasound sensor, or other device configured to cause a mechanical perturbation to the segment.

As shown in FIGS. 2A-B, and as included in the built experimental system, the structure is in the shape of a ring configured to surround the biological body segment, with twelve cameras included in the first array 122 and four cameras included in the second array 124. However, the structure can be any shape that enables the cameras to fully surround the biological body segment, or to enable the cameras to obtain images from about a full perimeter of the biological body segment, and each array can include any number of cameras. For example, the first array can include 4, 6, 8, 10, 12, 16, 20, 24, or more cameras, and the second array can include 1, 2, 3, 4, 5, 6, 10, or more cameras.

The first array 122 can be configured to remain stationary. Alternatively, the first array can be configured to be moveable, such as to translate axially to obtain images at different locations along a length of the biological body segment 102. Another example of a structure $120a^1$ is shown in cross-section in FIG. 3. The structure $120a^1$ is generally cylindrical in shape and includes a first array 122 of multiple subarrays 122a, 122b, 122c, each subarray including a plurality of cameras 128 and each subarray being disposed at varying locations within the structure such that a body segment can be fully imaged by simultaneous exposures of the cameras without requiring translation. Although only three subarrays are shown in FIG. 3, structure $120a^1$ can include 4, 5, 6, 7, 8, 9, 10, or more subarrays in order to scale the device so as to provide capability to image a large body segment or a full biological body. While optical cameras are shown and described, other imaging devices can be included in the first and second arrays, such as ultrasound sensors, which are capable of imaging both external and internal features of the biological body segment, as well as combination optical-ultrasound sensor devices (see, for example, Sections 3, 4, 7, 8 and 9 herein).

Returning to the experimentally-built system, a series of tests was conducted in order to test a number of cameras for providing adequate 3D reconstruction precision, as well as both in-plane and out-of-plane displacement measurements. Each pair of contiguous cameras represents a stereo-system capturing a given portion of the sample surface. For 3D reconstruction of the sample surface, two cameras image the same portion of the surface with sufficient detail. This can be achieved when the angle between the cameras is relatively small. However, accurate out-of-plane displacement measurements may require larger angles. The choice of angle $\alpha$ = 30° between 12 adjacent coaxial camera positions was found to provide a sufficient overlapping portions of image pairs for the intended application, as well as an acceptable level of distortion between image pairs, and an accurate 3D reconstruction.

Each camera unit contains a Raspberry Pi model zero W (Raspberry Pi Foundation, Cambridge, UK), and Raspberry Pi Camera Module V2 (with a Sony IMX219 8 megapixel sensor). To perform a force measurement during an indentation test, an indenter equipped with one or more force or force/torque sensors can be connected to an additional Raspberry Pi. A low-cost version with a 1-axis thin-beam force sensor (TBS-40, Transducer Techniques, Temecula, CA, USA) was designed and built. A more expensive version with two 6-axis force/torque sensors (Nano-17, ATI Industrial Automation) was also designed for the purpose of simultaneously indenting two opposing sides of the limb. All the measurement units (e.g., cameras and force sensors) are programmed to acquire simultaneous measurements with a high temporal accuracy, such that the force and image data can be accurately synchronized. In addition, LED lighting units are placed on the frame to provide adequate and uniform lighting conditions. All the measurements are then transferred to a computer for further analysis.

*System architecture and workflow*

The experimental and computational methods for obtaining 360-deg 3D full-field deformations from multiple-view image data are described in the next sections and the workflow is outlined in FIG. 4. In brief, the intrinsic and extrinsic stereo camera calibration procedures are illustrated in Blocks 1 and 2, respectively, of FIG. 4. The 2D-DIC process, which relies on the calculated camera distortion parameters, is depicted in Block 3 of FIG. 4. The transformation from 2D corresponded image points to 3D surfaces, which relies on the camera parameters calculated in Blocks 1 and 2, is depicted in Block 4 of FIG. 4. The process for obtaining the local deformation and strain from sets of corresponded meshed surfaces (undeformed and deformed), is depicted in Block 5 of FIG. 4. Lastly, the utilization of 3D-DIC in the process of soft-tissue mechanical properties evaluation is also depicted in Block 5. The procedures of each of Blocks 1-5 are further detailed below.

A library of custom MATLAB (R2017a, the Mathworks, Natick, MA, USA) codes was written which automates the entire aforementioned procedure, and allows for a fast and robust data acquisition and analysis.

*Camera intrinsic and extrinsic (stereo) calibration*

Prior to testing, the system was calibrated in a two-step procedure. The outline of the procedure is illustrated in FIG. 4. In the first step (Block 1), the intrinsic parameters of each camera were calculated. This step may be performed only once for each camera, and may not need to be repeated even if a camera pose is changed, as long as the camera lens remains untouched. The intrinsic parameters include: 1) an intrinsic matrix; 2) radial distortion coefficients of the lens; and, 3) tangential distortion parameters of the lens, each of which is described below

The intrinsic matrix, which contains the focal length, image sensor format, and principal point is as follows:

$$\begin{bmatrix} f_x & 0 & 0 \\ s & f_y & 0 \\ c_x & c_y & 1 \end{bmatrix} \qquad \mathbf{1}$$

where $(c_x, c_y)$ represents the optical center (principal point) in pixels, $(f_x, f_y)$ represents the camera's horizontal and vertical focal lengths in pixels, and s is the skew parameter which satisfies $s = \alpha_c f_x$, where $\alpha_c$ is the skew coefficient defining the angle between the x and y pixel axes. The focal length in world units, F, typically expressed in millimeters, can be obtained by the following:

$$\begin{aligned} f_x &= F s_x \\ f_y &= F s_y \end{aligned} \qquad \mathbf{2}$$

where $[s_x, s_y]$ are the number of pixels per world unit in the *x* and y directions, respectively.

The radial distortion coefficients of the lens $[k_1, k_2, k_3]$, which satisfy the relationship between the undistorted pixel locations $(x, y)$ and the distorted pixel locations $(x_d, y_d)$:

$$\begin{aligned} x_d &= x(1 + k_1 r^2 + k_2 r^4 + k_3 r^6) \\ y_d &= y(1 + k_1 r^2 + k_2 r^4 + k_3 r^6) \end{aligned} \qquad \mathbf{3}$$

where $r^2 = x^2 + y^2$.

The tangential distortion parameters of the lens $[p_1, p_2]$, which satisfy the relationship

$$\begin{aligned} x_d &= x + [2p_1 xy + p_2(r^2 + 2x^2)] \\ y_d &= y + [p_1(r^2 + 2y^2) + 2p_2 xy] \end{aligned} \qquad \mathbf{4}$$

The experimental calibration procedure utilizes the MATLAB Camera Calibration Toolbox. The calibration is achieved by obtaining and using multiple images of an asymmetric two-dimensional (planar) checkerboard pattern with a known and well-defined square size (FIG. 4, step 1a). The calibration algorithm (FIG. 4, step 1b) utilizes non-linear optimization techniques to minimize the re-projection errors of the checkerboard's corner points. The output of the algorithm are the aforementioned intrinsic parameters, which were computed for each camera and saved to be used for removing distortion from all the images taken during testing (FIG. 4, step 1c). FIGS. 5A-C illustrate the distortion removal procedure and output.

In the second step, the cameras' extrinsic parameters are calculated for the purpose of 3D reconstruction of image points (FIG. 4, Block 2). These parameters are used to map between 2D image points and 3D world points (stereo calibration), and they can be recalculated whenever the positions or the orientations of any of the cameras are changed. Numerous calibration methods exist, any of which can be used in this step. For example, the multiple checkerboard images used in the first calibration step could also be used here for stereo calibration, by taking images that are viewed by two cameras simultaneously. Nevertheless, this process is very time consuming when a large number of cameras is considered, since a large number of images has to be taken for each pair of adjacent cameras. Therefore, for this step a Direct Linear Transformation (DLT) calibration method was used. Using this method, each camera need only capture one image of a 3D calibration target, which contains control points whose 3D positions in a global reference system are known with sufficient accuracy (FIG. 4, steps 2a-2b). By comparing the 2D image coordinates of the control points with their 3D world coordinates, the associated DLT parameters can be calculated. Image distortions are removed (FIG. 4, step 2c) using the intrinsic parameters calculated in the first step; therefore, the DLT parameters in this step are estimated using a closed-form solution based on a distortion-free pin-hole camera model. The result of the stereo calibration is 11 DLT parameters per camera (FIG. 4, steps 2e-2f), which provide an explicit transformation that maps the 3D world points (typically in mm) into 2D image points on the camera sensor (typically in pixels). While the 3D calibration target can also be used for obtaining the intrinsic parameters (FIG. 4, step 1c) for distortion removal, it may be preferred to use the multiple checkerboard images for this purpose, because the checkerboard images cover a much larger portion of the camera field of view, thus providing a more accurate estimation of the camera's distortion parameters.

The basic equation describing the transformation from the coordinates {*x, y, z*} of points on the 3D object to the 2D coordinates {*u, v*} on the image planar frame involves nonlinear equations with seven unknown parameters, as follows:

$$u - u_o = -d\frac{r_{11}(x - x_o) + r_{12}(y - y_o) + r_{13}(z - z_o)}{r_{31}(x - x_o) + r_{32}(y - y_o) + r_{33}(z - z_o)}$$

$$v - v_o = -d\frac{r_{21}(x - x_o) + r_{22}(y - y_o) + r_{23}(z - z_o)}{r_{31}(x - x_o) + r_{32}(y - y_o) + r_{33}(z - z_o)} \qquad 5$$

where $\{u_o, v_o\}$ are the image coordinates of the principal point, $\{x_o, y_o, z_o\}$ is the object-space reference frame, d is the principal distance, and $r_{ij}$ are the components of the rotation matrix $\boldsymbol{R}$ from the object-space reference frame to the image-plane reference frame. Using the DLT method, the set of nonlinear equations in seven independent parameters is rearranged such that it can be converted into a set of linear equations in eleven parameters, which are not independent:

$$\begin{cases} L_1 = \dfrac{u_o r_{31} - d \cdot r_{11}}{-(x_o r_{31} + y_o r_{32} + z_o r_{33})} \\ L_2 = \dfrac{u_o r_{32} - d \cdot r_{12}}{-(x_o r_{31} + y_o r_{32} + z_o r_{33})} \\ L_3 = \dfrac{u_o r_{33} - d \cdot r_{13}}{-(x_o r_{31} + y_o r_{32} + z_o r_{33})} \\ L_4 = \dfrac{(d \cdot r_{11} - u_o r_{31})x_o + (d \cdot r_{12} - u_o r_{32})y_o + (d \cdot r_{13} - u_o r_{33})z_o}{-(x_o r_{31} + y_o r_{32} + z_o r_{33})} \\ L_5 = \dfrac{v_o r_{31} - d \cdot r_{21}}{-(x_o r_{31} + y_o r_{32} + z_o r_{33})} \\ L_6 = \dfrac{v_o r_{32} - d \cdot r_{22}}{-(x_o r_{31} + y_o r_{32} + z_o r_{33})} \\ L_7 = \dfrac{v_o r_{33} - d \cdot r_{23}}{-(x_o r_{31} + y_o r_{32} + z_o r_{33})} \\ L_8 = \dfrac{(d \cdot r_{21} - v_o r_{31})x_o + (d \cdot r_{22} - v_o r_{32})y_o + (d \cdot r_{23} - v_o r_{33})z_o}{-(x_o r_{31} + y_o r_{32} + z_o r_{33})} \\ L_9 = \dfrac{r_{31}}{-(x_o r_{31} + y_o r_{32} + z_o r_{33})} \\ L_{10} = \dfrac{r_{32}}{-(x_o r_{31} + y_o r_{32} + z_o r_{33})} \\ L_9 = \dfrac{r_{33}}{-(x_o r_{31} + y_o r_{32} + z_o r_{33})} \end{cases} \qquad 6$$

$$\Downarrow$$

$$\begin{cases} u = L_1 x + L_2 y + L_3 z + L_4 - L_9 ux - L_{10} uy - L_{11} uz \\ v = L_5 x + L_6 y + L_7 z + L_8 - L_9 vx - L_{10} vy - L_{11} vz \end{cases}$$

In order to solve for the set of 11 DLT parameters, a minimum of six non-coplanar control points are required. Nevertheless, a much larger number of control points is usually taken in order to create an overdetermined system, which utilizes the least-squares approach to reduce the effect of experimental errors.

The 3D calibration target (shown in FIG. 4, step 2a) was designed such that it captures sufficient depth in the approximate position and size of the residual limbs which are to be imaged. It features multiple black square points with known spatial positions, which are designed such that at least 150 points are visible by each camera, thus allowing for accurate estimation of the DLT parameters. The calibration target was additively manufactured using a multi-color 3D-printer (Connex Objet500, Stratasys, Eden Prairie, MN, USA) which offers high-precision (build resolution 600 dpi, accuracy of up to 200 microns). The alternating radius of the target was included in order to image points in different depths (*i.e.,* distances from the camera), which also improves the DLT parameters accuracy and helps to prevent bias.

*3D reconstruction*

The set of 11 DLT parameters $L_i^{C_j}\ (i = 1,2 \dots 11, j = 1,2)$ associated with two adjacent cameras $C_1$ and $C_2$, can then be used to transform any 2D image point which is visible by both cameras ($\{u^{C_1}, v^{C_1}\}$ and $\{u^{C_2}, v^{C_2}\}$) into 3D world points $\{x, y, z\}$, by rearranging Equation 6 into:

$$\begin{cases} u^{C_1} - L_4 = (L_1 - L_9 u^{C_1})x + (L_2 - L_{10} u^{C_1})y + (L_3 - L_{11} u^{C_1})z \\ v^{C_1} - L_8 = (L_5 - L_9 v^{C_1})x + (L_6 - L_{10} v^{C_1})y + (L_7 - L_{11} v^{C_1})z \end{cases}$$

$$\begin{cases} u^{C_2} - L_4 = (L_1 - L_9 u^{C_2})x + (L_2 - L_{10} u^{C_2})y + (L_3 - L_{11} u^{C_2})z \\ v^{C_2} - L_8 = (L_5 - L_9 v^{C_2})x + (L_6 - L_{10} v^{C_2})y + (L_7 - L_{11} v^{C_2})z \end{cases} \qquad \mathbf{7}$$

The linear equations of Equation 7 can be written in the following matrix form, where *U* is a 4X1 vector, *A* is a 4X3 matrix, and *X* is a 3X1 vector:

$$\begin{bmatrix} u^{C_1} - L_4 \\ v^{C_1} - L_8 \\ u^{C_2} - L_4 \\ v^{C_2} - L_8 \end{bmatrix} = \begin{bmatrix} L_1 - L_9 u^{C_1} & L_2 - L_{10} u^{C_1} & L_3 - L_{11} u^{C_1} \\ L_5 - L_9 v^{C_1} & L_6 - L_{10} v^{C_1} & L_7 - L_{11} v^{C_1} \\ L_1 - L_9 u^{C_2} & L_2 - L_{10} u^{C_2} & L_3 - L_{11} u^{C_2} \\ L_5 - L_9 v^{C_2} & L_6 - L_{10} v^{C_2} & L_7 - L_{11} v^{C_2} \end{bmatrix} \begin{bmatrix} x \\ y \\ z \end{bmatrix} \qquad \mathbf{8}$$

$$U = AX$$

Then, the solution for *X* can be obtained by the least-squares method:

$$X = [A^T A]^{-1} A^T U \qquad \mathbf{9}$$

Using this procedure, sets of corresponded image points from adjacent cameras found using 2D-DIC, can be transformed using the camera's DLT parameters into 3D points (FIG. 4, steps 4a-b). Consequently, 3D points representing surfaces which were reconstructed from different camera-pairs, can be merged into a continuous surface for each time-step, with points and faces that are corresponded between all the time-steps (FIG. 4, steps 4c-d).

*Application of high accuracy speckle patterns on the skin*

The accuracy and quality of DIC can be highly dependent on the quality of the speckle pattern, which is influenced by the speckle size distribution, speckle density, randomness, contrast, and edge sharpness. Most commonly, speckles are applied to a sample surface using spray paint. However, controlling for speckle size and uniformity of the pattern is rather difficult using this technique, especially on large surfaces. Therefore, in order to optimally control the speckle pattern parameters in our application, a custom-designed and manufactured stamp with a specifically-designed speckle pattern was used. A custom MATLAB code was written to produce a speckle pattern that contains the optimal density, randomness, and speckle size, according to the camera resolution and sample size (FIG. 6A). The stamp was fabricated by laser-cutting a rubber sheet according to the selected pattern (FIG. 6B). The pattern was applied to the skin using a temporary tattoo ink (FIG. 6C) which was chosen for being non-toxic and safe for use on the skin, as well as durable enough to withstand donning and doffing the liner and socket. The speckle pattern should remain unchanged throughout the measurements.

Other means of applying the speckles on the skin exist which allow accurate control of speckle size, density, and distribution. For example, water-transfer printing (also known as hydrographics), and painting or spraying ink through a stencil may also be used.

*2D Digital Image Correlation*

The 3D reconstruction of object points from pairs of images requires an accurate correspondence between object points that are viewed by two or more cameras. This correspondence can be achieved by means of 2D Digital Image Correlation (2D-DIC). In addition, 2D-DIC is used here to capture changes in the object by correlating images taken at different time frames. This way, the changes in 3D surfaces with corresponded points can be tracked, and the full-field 3D displacements and strains can be extracted. DIC relies on finding the maximum of the cross-correlation between pixel intensity subsets on two corresponding images, which gives the transformation between them.

To correlate image pairs, open-source 2D-DIC MATLAB software Ncorr was used. Ncorr incorporates a subset-based DIC algorithm with an iterative non-linear optimization scheme known as the Inverse Computational Gauss-Newton (IC-GN), which is computationally efficient.

Once images of the speckle pattern have been obtained, the images are undistorted (FIG. 4, steps 3a-3c). For each pair of cameras, Right (R) and Left (L), the reference image ($t_0$) from camera R is correlated with the current images ($t_1, t_2, \ldots, t_n$) of camera R, as well as with the reference and current images of camera L (FIG. 5, step 3d), based on an ROI that is visible in all images. This way, a single grid of points (and its corresponding mesh) can be tracked from two views, and provide the necessary correspondence for 3D reconstruction. The same grid of points is tracked in images taken in different times, which allows the computation of full-field displacements and strains.

Each set of matching points (FIG. 4, step 3e) arranged in triangular meshes, is then transformed into 3D meshed surfaces using the pre-calculated DLT parameters. The result of the last step is a set of 3D corresponded point clouds and meshed surfaces, one for each time frame. Consequently, the displacement of each point with respect to the reference configuration can be obtained, and the associated strains for each surface element can be calculated using the methods described in the next section.

The 2D-DIC algorithm is subset-based. The reference image is partitioned into smaller regions referred to as subsets. The deformation is assumed to be homogeneous inside each subset, and the deformed subsets are then tracked in each current image. Each subset is essentially a group of points with coordinates ($x_i$, $y_j$) on integer pixel locations on the reference image, arranged in a regular grid, with spacing determined by the user. The indices ($i, j$) are defined with respect to the location of the center of the subset ($x_0$, $y_0$), and all the indexed subset data points are contained in the set S [$(i, j) \in S$]. The transformation of the points ($x_i$, $y_j$) from the reference configuration to their positions ($\tilde{x}_i$, $\tilde{y}_j$) in the current configuration is constrained to a linear (first order) transformation, defined as

$$\tilde{x}_i = x_i + u + \frac{\partial u}{\partial x}(x_i - x_0) + \frac{\partial u}{\partial y}(y_j - y_0)$$
$$\tilde{y}_i = y_j + v + \frac{\partial v}{\partial x}(x_i - x_c) + \frac{\partial v}{\partial y}(y_j - y_0) \quad \mathbf{10}$$

where the displacements $\{u, v\}$ and their derivatives $\left\{\frac{\partial u}{\partial x}, \frac{\partial u}{\partial y}, \frac{\partial v}{\partial x}, \frac{\partial v}{\partial y}\right\}$ are the parameters defining the deformations and are constant for each subset. In order to find the deformation of a subset, the DIC algorithm finds the values of $\left\{u, v, \frac{\partial u}{\partial x}, \frac{\partial u}{\partial y}, \frac{\partial v}{\partial x}, \frac{\partial v}{\partial y}\right\}$ which results in the extremum of a correlation cost function, typically a normalized cross correlation criterion or a normalized least squares criterion. The cost function is a metric for similarity between the reference and the current subsets, and is based on the grayscale intensity values corresponding to specified points.

The correlation algorithm comprises two steps. In the first step, an initial guess yields the translations u and v with an integer (1 pixel) accuracy. In the second step, the Gauss-Newton (GN) iterative nonlinear optimization scheme is used to refine the initial guess and find the result with sub-pixel resolution.

*Displacement, deformation and strain calculation*

Once multiple surfaces with corresponding vertices and faces are obtained, the local deformation and strain in each surface element (FIG. 4, step 5a) can be computed. The methods presented here are based on the triangular Cosserat point element formulation described in detail in Solav (Solav D, Meric H, Rubin MB, Pradon D, Lofaso F, Wolf A (2017) Chest Wall Kinematics Using Triangular Cosserat Point Elements in Healthy and Neuromuscular Subjects. Ann Biomed Eng 45:1963-1973. doi: 10.1007/s10439-017-1840-6) and Solav (Solav D, Rubin MB, Cereatti A, Camomilla V, Wolf A (2016) Bone Pose Estimation in the Presence of Soft Tissue Artifact Using Triangular Cosserat Point Elements. Ann Biomed Eng 44:1181-1190. doi: 10.1007/s10439-015-1384-6), the relevant contents of which are incorporated herein by reference. For each triangular face, the positions of the three vertices are denoted by the vectors $\{\mathbf{X}_1, \mathbf{X}_2, \mathbf{X}_3\}$ in the reference configuration ($t = t_0$), and by the vectors $\{\mathbf{x}_1, \mathbf{x}_2, \mathbf{x}_3\}$ in the current (deformed) configuration, with $\mathbf{X}_i = \mathbf{x}_i(t = t_0)$. Then the reference and current configurations can be characterized by the director vectors $\{\mathbf{D}_1, \mathbf{D}_2, \mathbf{D}_3\}$ and $\{\mathbf{d}_1, \mathbf{d}_2, \mathbf{d}_3\}$, respectively, which are defined by:

$$\mathbf{d}_1 = \mathbf{x}_2 - \mathbf{x}_1, \mathbf{d}_2 = \mathbf{x}_3 - \mathbf{x}_1, \mathbf{d}_3 = \frac{\mathbf{d}_1 \times \mathbf{d}_2}{|\mathbf{d}_1 \times \mathbf{d}_2|}, \mathbf{D}_i = \mathbf{d}_i(t = t_0) \qquad 11$$

Note that $\mathbf{d}_3$ is a unit unit vector that is perpendicular to the plane of the triangle defined by $\mathbf{d}_1$ and $\mathbf{d}_2$, as shown in FIG. 7. In general, the vectors $\{\mathbf{D}_i\}$ do not form an orthogonal triad; therefore, in order to obtain the deformation gradient tensor of the triangular element it is convenient to define the reference reciprocal vectors $\{\mathbf{D}^i\}$ by:

$$\mathbf{D}^1 = \frac{\mathbf{D}_2 \times \mathbf{D}_3}{|\mathbf{D}_1 \times \mathbf{D}_2|}, \mathbf{D}^2 = \frac{\mathbf{D}_3 \times \mathbf{D}_1}{|\mathbf{D}_1 \times \mathbf{D}_2|}, \mathbf{D}^3 = \mathbf{D}_3 \qquad 12$$

The reciprocal vectors satisfy $\mathbf{D}^i \cdot \mathbf{D}_j = \delta^i_j$, where $\delta^i_j$ is the Kronecker delta symbol. Furthermore, the deformation gradient tensor F is defined as a second order tensor using the tensor product (outer product) operator ⊗ in the expression

$$\mathbf{F} = \mathbf{d}_i \otimes \mathbf{D}^i \qquad 13$$

The deformation gradient tensor **F** satisfies the equation $\mathbf{d}_i = \mathbf{F}\mathbf{D}_i$, which means it that it transforms the director vectors from the reference configuration to the current configuration. The associated symmetric Green-Lagrange finite strain tensor **E**, which is referenced to the reference configuration, and the Eulerian-Almansi finite strain tensor **e**, which is referenced to the present configuration, are defined by:

$$\begin{aligned} \mathbf{E} &= \frac{1}{2}(\mathbf{F}^{\mathrm{T}}\mathbf{F} - \mathbf{I}) \\ \mathbf{e} &= \frac{1}{2}(\mathbf{I} - \mathbf{F}^{-\mathrm{T}}\mathbf{F}^{-1}) \end{aligned} \qquad 14$$

where **I** is the unity second order tensor. Furthermore, the eigenvalues and eigenvectors of these strain tensors can be obtained, to represent the principal strains (magnitude and direction) in each triangular element.

Furthermore, the principal strains and their associated principal direction can be obtained from the eigen decomposition of the tensors **E** and **e**, respectively:

$$\begin{aligned} \mathbf{E} &= E_1(\mathbf{n}_1 \otimes \mathbf{n}_1) + E_2(\mathbf{n}_2 \otimes \mathbf{n}_2) + E_3(\mathbf{n}_3 \otimes \mathbf{n}_3) \\ \mathbf{e} &= e_1(\mathbf{m}_1 \otimes \mathbf{m}_1) + e_2(\mathbf{m}_2 \otimes \mathbf{m}_2) + e_3(\mathbf{m}_3 \otimes \mathbf{m}_3) \end{aligned} \qquad 15$$

where $E_i$ and $\mathbf{n}_i$ are the Lagrangian principal strains and their associated directions, and $e_i$ and $\mathbf{m}_i$ are the Eulerian principal strains and their associated directions. Since the triangle is planar, one of the principal directions must be the normal to the surface of the triangle, and its principal strain must equal zero.

Once multiple surfaces with corresponding vertices and faces are obtained, the local deformation and strain in each surface element (FIG. 4, step 5a) can be computed. The methods presented here are based on the triangular Cosserat point element formulation. For each triangular face, the positions of the three vertices are denoted by the vectors $\{\mathbf{X}_1, \mathbf{X}_2, \mathbf{X}_3\}$ in the reference configuration ($t = t_0$), and by the vectors $\{\mathbf{x}_1, \mathbf{x}_2, \mathbf{x}_3\}$ in the current (deformed) configuration, with

$\mathbf{X}_i = \boldsymbol{x}_i(t = t_0)$. Then the reference and current configurations can be characterized by the director vectors $\{\mathbf{D}_1, \mathbf{D}_2, \mathbf{D}_3\}$ and $\{\mathbf{d}_1, \mathbf{d}_2, \mathbf{d}_3\}$, respectively, which are defined by:

$$\mathbf{d}_1 = \mathbf{x}_2 - \mathbf{x}_1, \mathbf{d}_2 = \mathbf{x}_3 - \mathbf{x}_1, \mathbf{d}_3 = \frac{\mathbf{d}_1 \times \mathbf{d}_2}{|\mathbf{d}_1 \times \mathbf{d}_2|}, \mathbf{D}_i = \mathbf{d}_i(t = t_0) \qquad 16$$

Note that $\mathbf{d}_3$ is a unit unit vector that is perpendicular to the plane of the triangle defined by $\mathbf{d}_1$ and $\mathbf{d}_2$, as shown in FIG. 7. In general, the vectors $\{\mathbf{D}_i\}$ do not form an orthogonal triad; therefore in order to obtain the deformation gradient tensor of the triangular element it is convenient to define the reference reciprocal vectors $\{\mathbf{D}^i\}$ by:

$$\mathbf{D}^1 = \frac{\mathbf{D}_2 \times \mathbf{D}_3}{|\mathbf{D}_1 \times \mathbf{D}_2|}, \mathbf{D}^2 = \frac{\mathbf{D}_3 \times \mathbf{D}_1}{|\mathbf{D}_1 \times \mathbf{D}_2|}, \mathbf{D}^3 = \mathbf{D}_3 \qquad 17$$

The reciprocal vectors satisfy $\mathbf{D}^i \cdot \mathbf{D}_j = \delta^i_j$, where $\delta^i_j$ is the Kronecker delta symbol. Furthermore, the deformation gradient tensor **F** is defined as a second order tensor using the tensor product (outer product) operator ⊗ in the expression:

$$\mathbf{F} = \mathbf{d}_i \otimes \mathbf{D}^i \qquad 18$$

The deformation gradient tensor F satisfies the equation $\mathbf{d}_i = \mathbf{F}\mathbf{D}_i$, which means it that it transforms the director vectors from the reference configuration to the current configuration. The associated symmetric Green-Lagrange finite strain tensor **E**, which is referenced to the reference configuration, and the Eulerian-Almansi finite strain tensor e, which is referenced to the present configuration, are defined by:

$$\mathbf{E} = \frac{1}{2}(\mathbf{F}^{\mathrm{T}}\mathbf{F} - \mathbf{I})$$
$$\mathbf{e} = \frac{1}{2}(\mathbf{I} - \mathbf{F}^{-\mathrm{T}}\mathbf{F}^{-1}) \qquad 19$$

where **I** is the unity second order tensor. Furthermore, the eigenvalues and eigenvectors of these strain tensors can be obtained, to represent the principal strains (magnitude and direction) in each triangular element.

Furthermore, the principal strains and their associated principal direction can be obtained from the eigen decomposition of the tensors **E** and **e**, respectively:

$$\mathbf{E} = E_1(\mathbf{n}_1 \otimes \mathbf{n}_1) + E_2(\mathbf{n}_2 \otimes \mathbf{n}_2) + E_3(\mathbf{n}_3 \otimes \mathbf{n}_3)$$
$$\mathbf{e} = e_1(\mathbf{m}_1 \otimes \mathbf{m}_1) + e_2(\mathbf{m}_2 \otimes \mathbf{m}_2) + e_3(\mathbf{m}_3 \otimes \mathbf{m}_3) \qquad 20$$

where $E_i$ and $\mathbf{n}_i$ are the Lagrangian principal strains and their associated directions, and $e_i$ and $\mathbf{m}_i$ are the Eulerian principal strains and their associated directions. Since the triangle is planar, one of the principal directions must be the normal to the surface of the triangle, and its principal strain must equal zero.

*Validation and accuracy estimation*

In order to evaluate the system's accuracy, a series of tests were carried out. First, a rigid body motion test was performed, whereby a sample object undergoes a known rigid body motion with no deformation. Stereo images were taken in two configurations, before and after the applied motion, and the 3D surfaces were reconstructed using 2D-DIC and the calibration and reconstruction methods described in the previous sections herein. In a rigid body motion test, any strain value should theoretically equal to zero, such that any nonzero value represents a measurement error. In addition, the deformation between the two configurations can be used to compute the rigid body transformation between them and compared to the known transformation that was applied.

In a second test, the deformation measurement errors were examined using 3D printed synthetically deformed objects (SDO). SDOs were designed by applying different cases of deformation to a Finite Element model of a speckled solid model. The speckles on the SDO are deformed with the object (FIG. 8), such that the reference model 127 and deformed model 129 can be manufactured by means of a multi-color 3D printer, and the strains can be analyzed and compared with the simulated ones, which are considered as the ground truth.

*Photogrammetric methods combined with indentation for tissue mechanical property analysis*

In order to compute the subject-specific soft tissue mechanical properties (FIG. 4, step 5c), indentation tests are performed in which simultaneous measurements are recorded of indenter probe position, speed and force, as well as the resultant tissue surface deformation caused by the indenter (FIG. 9C). For the test, photogrammetric imaging is performed, for example, DIC, during the application of a force-sensitive indenter probe onto the body. Indentation experiments can be performed using an indenter, such as an indenter having a spherical head equipped with a force sensor. Types of force sensors include, but are not limited to, a thin beam load cell (FIG. 9A) that measures 1-axis force, or a 6-axis force/torque sensor (FIG. 9B) that measures 3D forces and torques. During data collection, the force-sensitive indenter is used to apply a force onto the biological body segment, causing a resultant tissue surface deformation or bulging around the spherical indenter head. This indenter application onto the body is performed during photogrammetric imaging, such as DIC, to capture these resultant tissue deformations, as well as the position and speed of the indenter relative to the biological segment. From these tests, the 3D model of the measured biological body segment and the indenter are then simulated using iterative inverse finite element analysis (iFEA) (FIG. 9D). In this procedure, the bulk material properties of the soft tissues are iteratively changed until an optimal match between the measured and the simulated force and tissue deformation boundary conditions is achieved. For example, the soft tissues can be modeled as hyperelastic/viscoelastic materials, and the model parameters can be determined by minimizing the error between the experimental and FEA data.

Indentation experiments may also be performed using an ultrasound transducer fitted with a force/torque sensor. Similar to what is shown in FIG 9A, the indenter probe's position may be tracked using photogrammetric imaging, such as DIC, to determine a spatial orientation of the probe, as well as a position of the probe relative to the biological segment. Using this setup, an indentation test can be performed whereby simultaneous measurements of indenter probe position, speed and force application, as well as the resultant tissue surface deformation, are recorded. Types of ultrasound data collected may include, but are not limited to: a total depth of soft tissue at anatomical points across the biological segment, a 3D bone geometry within the biological segment, b-mode imaging whereby tissue displacements and distances may be collected of various soft tissue layers (e.g., muscle, fat, skin), compression-based elastography, and shear wave elastography. Using a combination of photogrammetric imaging, such as DIC, and ultrasound, the surface of a biological body can be "painted" by a clinician using a handheld ultrasound probe. Here, for example, the probe is moved across the body's surface, and the position and speed of the probe relative to the position of the biological body segment can be determined from the photogrammetric imaging. At each probe location, bone and soft tissue geometries, as well as tissue impedances, can be determined from the ultrasound probe. These data can then be combined to form 3D external and internal tissue geometries, which can include bone *(e.g.,* as acquired with an ultrasound-force probe), as well as tissue mechanical properties as a function of anatomical location *(e.g.,* as acquired with use of an ultrasound-force probe to perform indentations).

*Photogrammetric methods for external geometry combined with other non-invasive imaging techniques for internal geometry*

Using a non-invasive imaging technique such as computerized tomography (CT), magnetic resonance imaging (MRI), or ultrasound, a scan of the biological segment can be acquired to determine the geometry of a critical internal tissue or tissues, including, but not limited to, bone, ligament, and tendon geometry, or any combination thereof. Since such tissue geometries vary little throughout the adult lifespan, such a scan need only be taken infrequently for each adult person. Such non-invasive imaging data of internal tissue geometries can then be combined with photogrammetric imaging data *(e.g.,* DIC data) of the biological segment's external shape to form a single geometric biomechanical model of the biological segment. Further, photogrammetric imaging combined with indentation tests, and inverse finite element analysis (iFEA) can be employed to obtain patient-specific tissue mechanical properties. Like the non-invasive internal imaging scan, the indentation tests need only be conducted infrequently during the person's lifespan, whereas the photogrammetric measurement of external biological segment shape can be repeated each time a new biomechanical interface, such as a socket, is manufactured for an individual. Using such an approach, imaging costs can be kept to a minimum.

*Results*

*Results: DIC Residual limb deformation*

The residual limbs of two bilateral transtibial (below knee) amputees were measured using the aforementioned system and methods in several states and configurations. Example measurements include: 1) measurements at different knee joint flexion angles (FIG. 10); 2) measurements at different times after doffing (FIG. 11); and 3) measurements obtained while indenting at different anatomical locations.

Such methods and devices can be used to capture images of an external surface of a biological body segment to generate a three-dimensional model of the biological body segment based on cross-correlation of the captured images. The three-dimensional model of external features can be inter-digitized with other image sets that provide data pertaining to internal features of the biological body segment, such as bone and tissue-to-bone depths. A compound model of internal and external features of the segment can thereby be generated. Image sets that can be combined with the generated external 3D model include, for example, CT, MR, and US imaging.

As bone structures rarely change over time, while soft tissue structures frequently change over time, such devices and methods can be used to update a previous model of a biological body segment while making use of a previously performed CT, MR, or US image set for internal features of the segment. For example, a patient may undergo an MR scan shortly after amputation, and a model of the patient's residual limb may be generated from the resulting data. As the residual limb undergoes soft tissue changes over time, rather than subjecting the patient to another costly MR scan, devices, such as device 200, can be used to quickly and easily obtain an updated model of the external features and/or mechanical properties of the residual limb, which can then be used to generate a new or revised biomechanical interface for the limb.

Optionally, or as an alternative to patient-specific MR, CT, and US data, image sets from medical image repositories, such as reference libraries of anatomical structures, can be used to supply information pertaining to internal structures of a biological body segment.

*Results: photogrammetric methods for external geometry combined with other non-invasive imaging techniques for internal geometry*

Photogrammetric methods, such as DIC-based methods, can be combined with other imaging methods, such as CT, for residual limb geometry measurements. In this example, CT is used to capture the subject-specific anatomical bone and patella-tendon geometry for a transtibial amputated residuum. Since such tissue geometries vary little throughout the adult lifespan, such a scan need only be taken infrequently for each adult person with limb amputation. For the pilot data presented here, a male volunteer and bilateral amputee (age 48, weight 77 kg, activity level K3) was recruited and placed in a supine position on a CT table (Siemens Somatom Plus). The residuum was scanned in the CT using the 32 second spiral CT with 120kVp, 210 mAs, 8mm collimation, 8mm table increment per gantry rotation and a 512 X 512 sensor matrix, reconstruction thickness 1 mm. Several slices of the CT data are visualized in FIGS. 117A and 117B. In order to construct a detailed computational model, CT slices (FIG. 117A) are segmented. In this example, six groups of segmented contours are obtained for each leg: skin, femur, tibia, fibula, patella, and patellar-tendon (FIG. 117B, highlighting the tibia). Each group of contours is then converted to smooth iso-surfaces (FIG. 117C for the tibia), and triangular surface meshes are reconstructed for all tissue contours (FIG. 117D).

In the proposed methodology, the external shape of the residuum in areas of boney protuberances can be used to align the CT-derived bone geometry within the DIC-derived external skin geometry (FIG. 118B). To better inform this alignment procedure, CT-compatible lead markers (Lead BBs, 1.5mm spherical adhesive, Beekley, X-spots Bristol, CT) are placed on the residuum skin over bony regions. Typically 4-6 markers along the anterior tibia, and an additional marker on the fibular head (if applicable), are employed. The skin surface reconstructed using DIC is registered into the CT coordinate system, by using the marker positions in both the CT and the DIC coordinate systems, and refining using the Iterative Closest Point (ICP) algorithm. The final surface model is constructed from the CT-generated bones and patellar tendon, and the DIC-generated skin.

The final geometric model assembling all surfaces is constructed from the CT-generated bones and patellar tendon and the DIC-generated skin surface. A lower cost optical imaging tool can be employed instead of DIC if patient-specific skin strain and tissue mechanical properties are deemed unnecessary for the biomechanical interface design. Average values can be employed for skin strain and tissue mechanical properties as a function of anatomical location. Examples of lower cost photogrammetric imaging mobile applications and scanning tools include, but are not limited to: 3DsizeMe, Scandy Pro, Scann 3D, Occipital Structure Sensor, iSense 3D, and Einscan 3D Pro.

*Results: Inverse Finite Element Analysis (iFEA) based subject mechanical property determination*

Indentation using a force probe, and skin deformation data obtained using DIC, are shown in FIG. 118A. Informed by CT and DIC data, an iFEA model can then be used to simulate an indentation experiment with a force-sensitive probe (FIG 118A, 118C). These model data are used to evaluate constitutive tissue parameters for each residual limb based on an iFEA minimization of the error between the experimental and model results of the force-displacement and tissue deformation response curves. In one embodiment, the non-linear elastic behavior of the tissue is modeled using the following isotropic, coupled and hyperelastic strain energy density function

$$\psi = \frac{c}{m^2}\left(\sum_{i=1}^{3}(\lambda_i{}^{m} + \lambda_i{}^{-m} - 2)\right) + \frac{\kappa}{2}(J-1)^2 \qquad \mathbf{21}$$

The material parameters $c$ and $\kappa$ have units of stress and define a shear-modulus like and bulk-modulus like parameter, respectively. The latter is seen to penalize for changes in volume as it acts on a term involving the volume ratio $J = \det(\mathbf{F})$ (with **F** the deformation gradient tensor). The unitless parameter $m$ sets the degree of non-linearity. Finally $\lambda_i$ are the principal stretches. During iFEA constitutive parameters for the patient were determined by minimizing the difference between simulated and experimental boundary conditions for a combination of indentation locations. FIG 118D shows a typical force-time curve for the experiment and FEA simulation following optimization, demonstrating the predictive capabilities of the biomechanical model. In another embodiment, the viscoelastic behaviour can also be evaluated based on an expansion of the above formulation. However, for the current biomechanical interface design embodiment featuring quasi-static evaluation, only the non-linear elastic parameters are considered. Like the non-invasive internal CT imaging scan, the indentation tests need only be conducted infrequently during the person's lifespan, whereas the optical DIC measurement of external biological segment shape would be repeated each time a new biomechanical interface, such as a socket, is manufactured for an individual. Using this approach, imaging costs can be kept to a minimum.

### 2. 3D Shape Measurement Using Multiple Inertial Measurement Units (MIMUs)

Devices and methods are provided for residual limb imaging through motion processing of coordinated inertial measurement units (IMUs). Such devices and methods can allow for improved accessibility and accuracy in limb geometry measurements. Coordinated six degree of freedom (6DOF) inertial measurement units (IMUs) can be used to calculate a trajectory in 3D space of a tracing of the limb surface. Although application to residual limb measurements is described, the devices and method can be applied equally well to the digital representation, and subsequent digital design, of any biological segment and biomechanical interface attached thereto.

The IMUs can be fixed to, or located within, an object configured to trace a surface of the residual limb. Trajectories can then be calculated for each IMU, and a correction method applied using all IMUs fixed to the instrument surface to mitigate measurement drift. The IMU trajectories can then used to generate a geometry that digitally represents the residual limb. A simulation was conducted to evaluate the feasibility of this measurement method, to provide realistic simulated measurements for a given instrument, and to inform instrument design for accurate residual limb geometry reconstruction.

An example device 200 is shown in FIGS. 12A-C. The device 200 includes an object 202 on which, or in which, multiple IMUs 204 are disposed. As illustrated, the object 202 has the form of a sphere for rolling over a surface of a biological body segment 210; however, the object 202 can be of any shape. For example, object 202 can be a glove, enabling a user to pat and/or sweep the surface of the biological body segment with their hand while wearing the glove.

As the object 202 is made to travel over the exterior surface of the biological body segment 210, a trajectory 220 of the object can be determined based on motion data of the IMUs 204. Such trajectory data can provide for points 224 and curves 226 for modeling a three-dimensional shape of the biological body segment 210 (FIG. 12B). Alternatively, or in addition, a shape of the biological body segment 210 can be determined from a three-dimensional image space in which the object "paints" a region 224 in which the biological body segment 210 is not located.

For the example simulation, an object having a rigid sphere shape was used. IMUs mounted on the exterior surface of the object created a multi-IMU (MIMU) system. Through knowledge of the MIMU's current shape (constant in the case of a rigid object), and knowledge of the locations of the IMUs on the shape, a corrective system can be employed such that the data from all IMUs can be used to record accurate motion of the MIMU system and can be used to correct the measurements from each individual IMU. For the simulation object, a set of 12 equidistant IMUs are mounted on the rigid sphere, leading to the icosahedral distribution shown in FIG. 12A (displayed as wireframe).

In one approach, illustrated in FIG. 12B, the set of all IMU measurements can be used to calculate accurate motion data of the MIMU system. A single point or region of the MIMU system can be defined as a path tracer point. This point can then be made to touch a biological body segment for which the shape is to be recorded. By sweeping multiple paths across the biological body segment with the MIMU system, a dense point set can be obtained from which surface geometry can be reconstructed.

Another approach, illustrated in FIG. 12C, relies on operations in a virtual 3D image space. For instance, a 3D image space can be defined (*e.g.,* as containing zeros), which can be visualized as a black image intensity (FIG. 12C). Regions 224 visited by the MIMU system can then be assigned a different image intensity (e.g. ones), which can be displayed as white. In this way, the MIMU system can paint around the biological body segment, or any other object of interest, thereby revealing it. A benefit of this system is that it does not matter where the MIMU system touches the object of interest, and users can freely and rapidly sweep the entire surface. The act of painting in this sense can be used to alter the 3D image intensities in a binary sense (*e.g.,* 0's become 1's) if the region has been visited. However, repeatedly passing over the same region may also alter the image intensity in a non-binary sense. For instance, the magnitude of the image intensity can denote confidence which starts at 0 and approaches 1 if the MIMU system has passed over that region of space multiple times. Also, the core of the object can paint with a highest intensity (*e.g.*, representing a highest confidence), and an intensity can decay towards the object's boundary. In this way, the MIMU system can act as a soft and blurry eraser in the image space. Multiple passes of the MIMU system can fully define a white space around the object of interest. The latter approach, featuring a scalar image space representing MIMU coverage confidence, can help correct for potential MIMU system location determination errors.

*Methods*

A simulation was conducted to evaluate the proposed measurement process and instrument design. The simulation first generates a measurement instrument consisting of a number of IMUs on the surface of a 3D object, creating the MIMU system. Data from each IMU during measurement is then simulated using a virtual IMU method, incorporating realistic sources of error to mimic physical sensors. A measurement trajectory was generated to imitate the physical measurement process. Simulated data from the generated instrument following the trajectory was then analyzed to assess the viability of the measurement method and to guide instrument design.

*Geometry Measurement with IMUs*

A geometry may be measured and reconstructed by calculating a position trajectory of an object guided across its surface. The method can include measuring residual limb geometry using motion processing with 6DOF inertial measurement units (IMUs), each consisting of a three-axis accelerometer and a three-axis gyroscope. The measurement instrument can consist of multiple IMUs fixed to objects with known relative IMU positions and orientations.

During the measurement process, the instrument can be guided across the surface of the residual limb, *e.g.*, in a light back-and-forth rubbing motion over a short period of time. Trajectories can be calculated for each IMU, and the relative trajectories of IMUs fixed to the same surface can be used to apply a correction method to prevent drift in calculating the instrument trajectory. Next, a surface reconstruction method can be applied to the corrected position trajectory of the instrument in order to generate a 3D shape, which represents the geometry of the residual limb.

*Calibration and Sensor Error Management*

IMUs can be subject to significant intrinsic error, which poses challenges to accurate motion processing. Four common sources of error are axis misalignment, constant offset, sensitivity scale factor, and noise, although additional error may result due to other error sources, including, for example, nonlinearity and moving bias. The simulation presented assumes accurate calibration to determine axis misalignment, offset, and sensitivity, neutralizing their effects on raw IMU data and filtering to mitigate the effects of noise.

IMU calibration methods include the use of high-accuracy turntables and in-field calibration methods, which require no external devices. Accuracy varies significantly across calibration methods; for the purposes of obtaining a residual limb geometry, a high-precision calibration method is assumed. The simulation therefore mitigates error by executing the motion processing methods using the exact error parameters applied to the raw simulated data by the virtual IMU.

*Coordinated IMU Trajectory Correction*

The propensity of IMUs for dramatic error accumulation over a short period of time merits the introduction of multiple sensors for a single trajectory calculation. A common method for IMU trajectory calculation correction is coordination of the IMU with a Global Positioning System (GPS) to improve position accuracy. However, the small-scale requirements of residual limb geometry reconstruction and the goal of a self-contained system suggest that the GPS correction method is impractical for this application. Another method of IMU trajectory correction is the introduction of redundant IMUs, which can improve IMU trajectory measurements.

The motion processing method used in limb measurement can have an implicit correction method integrating the redundant IMUs to prevent unacceptable levels of measurement drift. Two distinct methods of trajectory correction were designed for this simulation: correction by averaging the trajectories from multiple IMUs, and correction by constantly constricting the positions and orientations of IMUs on a fixed surface to their known relative values.

The averaging correction method is a computationally inexpensive position and orientation correction strategy, relying on the idea that points on a fixed surface experience position and orientation changes at the same rate. The averaging correction method for orientation first performs the basic orientation calculation method. An approximate angular velocity for each IMU is calculated by taking the difference between orientations at consecutive time steps. This angular velocity is averaged across all IMUs, and each IMU orientation trajectory is recalculated using the overall average IMU angular velocity. The position averaging correction process is slightly more involved: since each IMU experiences its own orientation trajectory throughout the motion path, each set of accelerometer data (*e.g.*, one per IMU) is first be converted to the Earth's reference frame. The velocity is then calculated using the Euler method, and an average velocity is calculated across all IMUs. In the Earth frame, each IMU experiences the same angular velocity, so this velocity is assigned to each IMU, and the Euler method is used again to calculate position at each IMU.

The instrument shape correction method applies a correction to all IMU orientation and position calculations at every time step. The goal of the correction process is to restrict all orientation and position methods at every point in time to their known relative positions and orientations on the measurement instrument shape. The method involves knowledge of exact IMU positions and orientations on the instrument. The orientation shape correction method relies on the fact that all IMUs fixed to the instrument maintain the same relative orientation throughout the entire measurement process. For IMUs fixed on a given surface, although each IMU experiences orientation changes throughout the span of measurement, the normalized difference between each IMU orientation remains the same. The instrument shape correction method for orientation calculates the initial normalized orientation difference across all IMUs, and constructs a shift matrix for each IMU of the same size as the orientation matrix. The normalized orientation difference matrix ***N*** may be calculated using Eqn. 22, where ***A*** and ***B*** are 3x3 arrays containing the axis vectors for unique IMUs on the instrument surface.

$$\boldsymbol{N} = \begin{bmatrix} \sqrt{\left(A_{x_1} - B_{x_1}\right)^2 + \left(A_{x_2} - B_{x_2}\right)^2 + \left(A_{x_3} - B_{x_3}\right)^2} \\ \sqrt{\left(A_{y_1} - B_{y_1}\right)^2 + \left(A_{y_2} - B_{y_2}\right)^2 + \left(A_{y_3} - B_{y_3}\right)^2} \\ \sqrt{\left(A_{z_1} - B_{z_1}\right)^2 + \left(A_{z_2} - B_{z_2}\right)^2 + \left(A_{z_3} - B_{z_3}\right)^2} \end{bmatrix} \qquad \mathbf{22}$$

At each time step, an orientation shift is applied to each IMU, and the normalized difference between each IMU pair is calculated given the updated (shifted) orientation for each IMU. The orientation shift matrices are calculated to minimize the system, Eqn. 23, for each set of two IMUs fixed to the measurement instrument (where $\boldsymbol{S_A}$ and $\boldsymbol{S_B}$ are shift matrices for two unique IMUs, and $\boldsymbol{N_0}$ is the initial normalized difference matrix for the two IMUs). The minimized system provides a unique shift variable assigned to each of the total orientation components in the system (9n components, where n is the number of IMUs). After the shift matrices are calculated, each orientation matrix is updated by simply adding the corresponding shift matrix.

$$\begin{bmatrix} \sqrt{\left(\left(A_{x_1} + S_{A_{x_1}}\right) - \left(B_{x_1} + S_{B_{x_1}}\right)\right)^2 + \left(\left(A_{x_2} + S_{A_{x_2}}\right) - \left(B_{x_2} + S_{B_{x_2}}\right)\right)^2 + \left(\left(A_{x_3} + S_{A_{x_3}}\right) - \left(B_{x_3} + S_{B_{x_3}}\right)\right)^2} - N_{0_x} \\ \sqrt{\left(\left(A_{y_1} + S_{A_{y_1}}\right) - \left(B_{y_1} + S_{B_{y_1}}\right)\right)^2 + \left(\left(A_{y_2} + S_{A_{y_2}}\right) - \left(B_{y_2} + S_{B_{y_2}}\right)\right)^2 + \left(\left(A_{y_3} + S_{A_{y_3}}\right) - \left(B_{y_3} + S_{B_{y_3}}\right)\right)^2} - N_{0_y} \\ \sqrt{\left(\left(A_{z_1} + S_{A_{z_1}}\right) - \left(B_{z_1} + S_{B_{z_1}}\right)\right)^2 + \left(\left(A_{z_2} + S_{A_{z_2}}\right) - \left(B_{z_2} + S_{B_{z_2}}\right)\right)^2 + \left(\left(A_{z_3} + S_{A_{z_3}}\right) - \left(B_{z_3} + S_{B_{z_3}}\right)\right)^2} - N_{0_z} \end{bmatrix} \qquad \mathbf{23}$$

The position shape correction method, like the orientation correction method, relies on the knowledge that relative positions of the IMUs remain constant throughout the measurement span. As in the averaging position correction method, at each point in time the *x, y* and *z* positions are first be converted to the Earth reference frame. Following a similar process to the orientation correction method, the position correction method first calculates the absolute value of the difference in *x, y,* and *z* positions for each pair of IMUs, as shown in Eqn. 24; M is the resultant difference vector for two unique IMUs with positions C and D, respectively.

$$M = \begin{bmatrix} |C_x - D_x| \\ |C_y - D_y| \\ |C_z - D_z| \end{bmatrix} \quad \mathbf{24}$$

As in the orientation correction method, at each time step, a position shift is applied to each IMU, and the normalized difference between each IMU pair is calculated using the shifted position for each sensor. Position shift matrices are calculated to minimize the system, where $\boldsymbol{S_C}$ and $\boldsymbol{S_D}$ are position shift vectors for two unique IMUs, and $\boldsymbol{M_0}$ is the initial normalized difference array for the IMU pair.

$$\begin{bmatrix} \left|(C_x + S_{C_x}) - (D_x + S_{D_x})\right| - M_{0_x} \\ \left|(C_y + S_{C_y}) - (D_y + S_{D_y})\right| - M_{0_y} \\ \left|(C_z + S_{C_z}) - (D_z + S_{D_z})\right| - M_{0_z} \end{bmatrix} \quad \mathbf{25}$$

After the shift vectors are calculated, each IMU position vector is updated by adding the corresponding shift matrix.

Both the orientation and position shape correction methods can be applied at each time step, across all IMUs. The shape correction method is significantly more computationally expensive than the averaging correction method.

*3D Geometry Reconstruction*

After generating an array of corrected position matrices for each IMU, an overall instrument position matrix is generated. The instrument position is calculated by taking the average position trajectory across all IMUs, after adjusting each IMU trajectory to begin at the origin. Finally, the instrument position is adjusted to account for the offset between the instrument origin and the geometry surface.

*Simulation*

The goal of the simulation is the generation and processing of realistic IMU data in order to test the viability of the proposed 3D imaging method for residual limbs. The user first sets inputs for desired quantities, according to the metrics in Table 1.

After the user sets parameters, the simulation sets a motion path including a position trajectory over time and iterative quaternions to update orientation at each time step.

The simulation then generates a measurement instrument of the specified shape, and places the desired number of IMUs at random locations on the instrument surface. Each IMU is assigned intrinsic error parameters according to the specified error level.

**Table 1: Input parameters for three-dimensional geometry measurement simulation**

| Input Parameter | Description |
|---|---|
| *sim_time* | Simulation time (seconds) |
| *sampling_rate* | IMU sampling rate (Hz) |
| *imu_num* | Number of IMUs fixed to measurement instrument surface |
| *ADC_length* | Resolution of IMU analog-to-digital converter (bits) |
| *A_range* | Upper bound for accelerometer measurement (g) |
| *G_range* | Upper bound for gyroscope measurement (°/s) |
| *geom_shape* | Shape of geometry for simulated measurement |
| *instrument_shape* | Shape of instrument for simulated measurement |
| *correction_method* | Correction method to improve IMU measurement accuracy |
| *error_level* | IMU intrinsic sensor error magnitude coefficient (0-10; 0 indicates no error and 10 indicates high error) |

(continued)

| Input Parameter | Description |
| --- | --- |
| *error_type* | Allows error to be applied only partially to accelerometers and gyroscopes for testing purposes |

Next, the virtual IMU method produces realistic accelerometer and gyroscope data for each IMU on the surface of the instrument, following the previously calculated trajectory. The orientation and position of each IMU is calculated, and a correction method for each is applied (*e.g.,* the averaging correction method or the shape correction method). Trajectories from all IMUs are then combined to produce an overall instrument trajectory, which is corrected to account for the offset between the instrument center and the geometry surface. Finally, the simulation generates a 3D surface reconstruction to represent the geometry measured by the instrument.

*Virtual IMU*

The virtual IMUs used in these simulations were designed to reflect physical IMUs by taking into account realistic sensor error and measurement limitations intrinsic to an analog-to-digital converter (ADC). It accepts as inputs the trajectory and IMU parameters produced in the simulated test path and IMU setup routines, and applies them to produce realistic accelerometer and gyroscope data for each IMU on the measurement instrument.

For a series of *N* measurements over time, for each sensor, the virtual IMU accepts an *N*x3 position matrix, an Nx4 quaternion matrix (representing iterative rotations), a 3x3 initial orientation, and a time vector of length *N*. To account for intrinsic IMU error, it also receives a 3x6 IMU axis alignment matrix, a 3x2 offset matrix, a 1x2 sensitivity matrix, an *N*x6 noise matrix, and a scalar ADC resolution for the IMU.

To produce simulated gyroscope data, the angular velocity of the sensor is first calculated at each time step directly from the quaternion matrix. The resultant angular velocity is adjusted to reflect the misaligned coordinate frame, and divided by the sensitivity to convert the data to least standard bits (LSB), the standard units for raw IMU data. Finally, the virtual IMU adds offset and noise to the gyroscope data. To produce simulated accelerometer data, the initial orientation is first adjusted to reflect the misaligned coordinate frame, then rotated through time according to the quaternion matrix. The acceleration is calculated from the position matrix, and gravity is added. The resultant acceleration is adjusted to reflect axis misalignment, and divided by the sensitivity to convert the data to LSB. Finally, offset and noise are added to the accelerometer data. Error parameters (for sensitivity, axis misalignment, and constant offset) are randomly generated according to a normal distribution about the ideal (zero error case) value with a maximum magnitude determined by the user.

*Instrument Generation*

The instrument generation method simulates an instrument of the shape and number of IMUs provided by the user in the overall simulation parameters (*e.g.*, as shown in FIGS. 12A-C, a rigid sphere containing an icosahedral distribution of IMUs). The method calculates the *x, y,* and z coordinates of the instrument shape, which is centered at the origin, and places the specified number of IMUs at random locations on the instrument surface. The IMUs are placed such that the *x* and *y* vectors for each IMU lie tangent to the instrument surface, orthogonal to the z vector which extends away from the surface, as shown in FIG. 13. In FIG. 13, IMU locations 234 are shown as black dots with axes indicated in grey. A centerline 238 illustrates an overall orientation of the MIMU system 200 from the origin to the top of the instrument.

Although the simulation uses a spherical measurement instrument, the overall simulation is robust and can accept any instrument shape. For a new instrument shape design, the *x, y,* and *z* coordinates of the shape centered at the origin can be specified, and IMU locations and initial IMU orientations can be provided.

*Basic Simulated Sample Trajectory*

A basic sample trajectory was used to test data generation and motion processing, and to compare motion processing correction methods. The trajectory 240 (shown in FIGS. 14A-14D, using a spherical instrument 200) consists of a circular motion in the x, y, and z directions and rotation about all three axes, starting and ending at the origin with a known initial trajectory velocity and an iterative rotation quaternion matrix for the simulated measurement instrument. The test path was chosen for simplicity and smoothness.

*Measurement Path Generation Method*

The proposed measurement method for residual limb imaging involves moving a measurement instrument, the MIMU, in a relatively rapid back-and-forth motion around the shape of a limb, gathering IMU data over time. The data is then used to generate a 3D geometry. For the simulation to accurately reflect the measurement process, a randomized path generation method mimics the expected motion to gather simulated measurement data for a given shape geometry over a set period of time.

The randomized measurement trajectory consists of a series of short paths tangent to the geometry surface. The trajectory begins at a set initial location on the geometry shape, chooses a random direction and travels tangent to the surface in that direction, then after a short period of time, switches directions for another short period of time, and so on, periodically wrapping the trajectory curve to the surface to maintain tangency. This method may be applied to generate a randomized simulated measurement path about any known 3D geometry. The measurement path generation method maintains constant orientation of the instrument throughout the measurement process, but could be easily modified to include arbitrary orientation motion to further demonstrate the viability of the measurement process.

There is a direct correlation between the time span of the measurement process and the accuracy and precision of the geometry generated by the measurement routine. FIGS. 15A, 15C, and 15E depict the measurement path for the spherical geometry over a series of increasing time spans, and FIGS. 15B, 15D, and 15F depict a resulting triangulated geometry for each of the paths.

Note that the geometries produced in the figure are a product of the generated trajectory and have not been converted to IMU data and passed through the motion processing and correction methods.

*Results*

Performance evaluation was conducted in two stages. First, the two motion processing correction methods were compared at a variety of simulation settings using the basic test trajectory to evaluate the functionality and limitations of each, and to determine which was more appropriate for a realistic geometry measurement. Second, the full simulation was used to generate a motion path for a measurement instrument surveying a chosen geometry, generate the instrument and simulated data for all IMUs, and motion processing and correction were applied to calculate the instrument trajectory in order to produce a final shape geometry. The full simulation provided insight on the viability of the overall measurement process.

*Evaluation of Trajectory Correction Methods*

To evaluate the success of the motion processing methods and trajectory correction, the measurement process was simulated using a variety of parameters. Both correction methods (*i.e.,* averaging and shape correction) were tested without the use of calibration to dramatically demonstrate the results of each correction strategy. As a result, the motion processing results shown are not representative of the capabilities of the motion processing method; the calculated trajectories depicted in these plots are expected to demonstrate significantly lower accuracy than the standard case.

The results demonstrated in FIGS. 16A-D show that motion processing using the designed trajectory correction methods (Corrected) is significantly more accurate than motion processing without trajectory correction (Basic), and provides a qualitative measure of performance for representative data sets using a simulation with no calibration and a high-error IMU (error level 10, in the simulation parameters described in Table 2). Since the system is entirely uncalibrated for these examples, deviation of the measured trajectory from the actual trajectory is significant; however, in the cases in which correction is applied, the behavior of the calculated trajectory (Corrected) is notably closer to the actual trajectory behavior (Actual), even when the system still exhibits large discrepancies due to the lack of calibration. The actual trajectory (Actual) represents the ground truth trajectory defined by simulation input data.

To confirm the viability of the motion processing and correction methods, a test was conducted simulating data following the position trajectory shown in FIGS. 14A-14D, scaled to a diameter of 0.2 meters. Simulated data was collected at a rate of 1 kHz with an IMU error level of 0.1 (indicating an IMU of very high accuracy), with an instrument consisting of ten calibrated IMUs randomly positioned on the surface of a sphere. Millimeter-level accuracy was achieved for the first 5 seconds of motion, with unacceptable levels of drift starting to accumulate towards 10 seconds; the error over time is shown in FIG. 17 for both the Corrected and Basic results. Here the Corrected curve shows the results when the correction methods are applied, whereas the Basic curve shows the results when trajectory correction methods are not employed.

*Evaluation of Geometry Reconstruction*

Geometry reconstruction was attempted using simulated measurement paths. The simulation parameters used in the evaluation are outlined in Table 2, with a noise only error applied to mimic perfect calibration conditions. The error level was set to 1 to represent a high-accuracy IMU. The simulation time was set to 30 seconds to allow comparison between the motion processing results and the ideal geometry reproduction (from only the simulated trajectory, without IMU motion processing) shown in FIGS. 15A-15F. A simulation time of 30 seconds was also selected to provide a good measurement of shape progress while not allowing significant error to accumulate. For measurement of a physical system with a necessary error bound on the order of millimeters, as in the case of measurement of residual limbs, higher accuracy may be achieved using a series of short measurements (for example, in the range of five to ten seconds). The simulation results are shown in FIGS. 18A-C.

**Table 2: Simulation input parameters for 3D geometry reconstruction evaluation**

| Input Parameter | Value | Units |
|---|---|---|
| Simulation Time | 30 | seconds |
| Sampling Rate | 100 | Hz |
| Number of IMUs | 10 | N/A |
| ADC Length | 32 | bits |
| Accelerometer Range | 2 | g |
| Gyroscope Range | 250 | °/s |
| Instrument Shape | sphere | N/A |
| Correction Method | averaging | N/A |
| Error Level | 1 | N/A |
| Error Type | noise only | N/A |

*Discussion*

The simulation and measurement process can be evaluated by considering two distinct parts: the geometry measurement method (*e.g.*, assuming ideal IMUs) and the IMU correction process (*e.g.*, independent of geometry measurement trajectory).

The geometry measurement method was simulated over a realistic measurement path consisting of a series of short, randomized trajectories tracing the surface of a given geometry. Qualitative results (depicted in FIGS. 15A-15F) demonstrated that the proposed physical measurement process was sufficient to reproduce a given geometry, assuming a perfectly calibrated system.

However, since the physical world is not conducive to a perfect system, two correction methods were tested to coordinate multiple IMUs on a measurement instrument in order to prevent drift and allow accurate measurement of a geometry given the desired motion path over a substantial period of time. Both the averaging and instrument shape correction methods demonstrated improvement in trajectory calculation (compared to calculating trajectory with no similar correction method in place). Of the two, the instrument shape correction method is recommended for improving trajectory calculation using coordinated IMUs. Although it is more computationally intense than the averaging method, it provided more accurate reproduction of a sample trajectory given a system of many uncalibrated, high-error IMUs.

When all processes were combined to simulate geometry measurement and reconstruction of a sphere, accuracy on the order of millimeters was achieved over an approximate range of 5-10 seconds for an instrument incorporating a low-error IMU system. Assuming a measurement process would allow the combination of multiple datasets, this range should allow meaningful geometry to be collected for a residual limb. The results of the simulation therefore suggest that the proposed process is a viable measurement method for residual limb geometry.

*Conclusion*

A simulation was designed to test the viability of measuring residual limb geometry using motion processing with inertial measurement unit (IMU) sensors and to provide insight on instrument design. The simulation generated a measurement instrument consisting of randomly spaced IMUs on a 3D shape. Each IMU was generated using a virtual 6DOF IMU that incorporated realistic sources of error based on variability in commercial sensors. These errors and a given trajectory were used to produce simulated accelerometer and gyroscope data. Two categories of trajectory were used in this simulation: a regular curve with simple sinusoidal motion in each direction, and a randomized trajectory mimicking the physical measurement process of surveying a 3D shape (in this case, a residual limb) using a light rubbing motion over the shape's surface. The simulation then applied motion processing methods to the simulated data, and used correction routines coordinating the data from multiple IMUs on the surface of the instrument, using trajectory averaging and correction according to the relative positions and orientation of the IMUs.

The trajectory correction methods in combination with the motion processing routine used in this simulation demonstrated high accuracy over short periods of time with the simple sinusoidal motion path, showing accuracy on the order of millimeters for very high quality IMUs up to five seconds, with unacceptable levels of drift accumulating towards ten seconds. The motion processing method for the randomized shape-measuring simulated trajectory, while demonstrating general performance similar to that seen in the ideal measurement case (using perfect IMUs), did not demonstrate this level of accuracy; however, this is most likely due to the high prevalence of sharp corner turns in the simulated path. As such, during actual measurement, smooth motion can be prioritized while measuring a residual limb.

### 3. Instrumented Wearable Systems for Shape and Mechanical Property Assessment

A 3D measurement device for a biological body segment, a system for generating a 3D representation of a biological body segment, and a method of forming a biological body segment modeling device are provided.

In one embodiment, the invention is a 3D measurement device for a biological body segment. The measurement device includes an elastomeric sheath conformable to the biological body segment. A plurality of nodes is affixed to the elastomeric sheath. A grid of electrically conductive conduits connects the nodes. A plurality of first transducers is at least a portion of either the electrically-conductive conduits or the nodes, whereby data collected by the first transducers can be employed to generate a 3D representation of the biological body segment. The device can further include a plurality of second transducers at least a portion of the other of the electrically-conductive conduits or their nodes at which the first transducers are located. The first and second transducers can each, independently, be emitters or sensors.

The first transducers can include, for example, at least one member of the group consisting of a stretch sensor and curvature sensor. For example, in one embodiment, the first transducers are at the electrically-conductive conduits and include at least one member of a stretch sensor, curvature sensor, and ultrasound transducer. For example, the transducers can include stretch sensors. Examples of suitable stretch sensors include at least one parallel plate capacitive stretch sensor. The transducers include curvature sensors. The curvature sensors can include at least one member of the group consisting of a unipolar resistive curvature sensor and a bipolar resistive curvature sensor. In another embodiment, the transducers can include at least one curvature sensor and at least one stretch sensor.

The second transducers can include at least one member of the group consisting of biomechanical sensors, biometrical sensors, and feedback components. Examples of suitable feedback components include LEDs and vibration motors that provide feedback to the user. In at least one embodiment, the second transducers specifically include at least one member selected from the group consisting of ultrasound transducers, inertial measurement units (IMUs), light emitting diodes (LEDs), vibration motors, and sensors of skin modulus, pressure, shear force, temperature, heart rate, respiratory rate, blood oxygenation, electrodermal response, molecular composition of sweat, moisture, tissue depth, hydration, vascularization, and peripheral nerve anatomy. Ultrasound transducers are sensors and actuators that may specifically include ultrasonomicrometry crystals comprised of piezoelectric and polycrystalline materials such as ceramic PZT4 and PZT8, or non-piezoelectric and single crystal materials such as lead magnesium niobate / lead titanate (PMN-PT), lead indium niobate / lead magnesium niobate / lead titanate (PIN-PMN-PT), lithium niobate ($LiNbO_3$), barium titanate ($BaTiO_3$), strontium titante ($SrTiO_3$), zinc oxide (ZnO), and synthetic quartz. These ultrasound transducer crystals may transmit acoustic signals or receive acoustic signals, or serve both functions. A transmitter-receiver pair of crystals or a single dual-function crystal can be used to measure the time-of-flight of a signal. Given velocity of transmission (approximately 1540 m/s for speed of sound through human biological tissue) and this measured time-of-flight, a measurement of distance may be derived, where the distance is that between a transmitter-receiver pair of crystals or twice the distance between a single dual-function crystal and an acoustically reflective surface. A single omnidirectional transmitter crystal may send a signal to multiple receiver crystals. A single omnidirectional receiver crystal may receive signals from multiple transmitter crystals. Any combination of receiver, transmitter, or dual-function crystals may be arranged in a network array structure to obtain multiple time-of-flight measurements, such as between nodes of the present invention. Ultrasound signal depth of penetration and resolution vary with frequency. For human biological tissue, an ultrasound signal frequency of 5-20 MHz is desired for surface imaging of targets such as carotid arteries, whereas an ultrasound signal frequency of 2-5 MHz is desired for deeper imaging of targets such as superficial patella bone, and an ultrasound signal frequency of less than 2 MHz is desired for even deeper imaging of targets such as deep femur bone. In another embodiment, at least a portion of the second transducers can include a plurality of different types of components.

At least a portion of the electrically-conductive conduits can include an elastomeric component, whereby the electrically conductive conduits are essentially elastic. In a specific embodiment, the electrically-conductive conduits further include conductive particles. The electrically conductive conduits can have a serpentine shape.

The biological body segment can include at least one member of the group consisting of a human chest, abdomen, face, arm, hand, leg, and foot. In a specific embodiment the biological body segment is a human chest and the elastomeric sheath is a component of a bra, wherein the nodes include at least one component selected from the group consisting of skin modulus sensors and ultrasound sensors. In a specific embodiment, the bra further includes at least one component selected from the group consisting of an electrocardiogram (EKG) sensor, respiration rate sensor, pressure and shear force sensor, inertial measurement units, temperature sensor, heart rate sensor, blood oxygenation sensor, electrodermal response sensor, molecular composition of sweat sensor, moisture sensor, tissue depth sensor, hydration sensor, vascularization sensor, LED, and vibration motors.

In yet another embodiment, the invention is a system for generating a 3D representation of a biological body segment. In this embodiment, the system includes a synthetic skin component and a handheld probe. The synthetic skin component can include an elastomeric sheath conformable to the biological body segment, a plurality of nodes affixed to the elastomeric sheath, a grid of electrically-conductive conduits connecting the nodes, and a plurality of first transducers at least a portion of either the electrically-conductive conduits or the nodes, whereby data collected by the first transducers can be employed to generate a 3D representation of the biological body segment. The handheld probe can include at least one probe transducer selected from the group consisting of an ultrasound transducer, pressure sensor, shear force sensor, temperature sensor, and IMU.

In an optional embodiment, the system further includes a plurality of second transducers at least a portion of the other electrically-conductive conduits and the nodes at which the first transducers are located.

In yet another embodiment, the invention is a method of forming a biological body segment modeling device. In this embodiment, the method includes forming an elastomeric sheath that is conformable to the biological body segment, applying a plurality of nodes to the elastomeric sheath, and forming electrically conductive interconnects between at least a portion of the nodes, wherein at least a portion of at least one of the nodes and the interconnects includes a first transducer. In a specific embodiment, at least a portion of the other of the nodes and the interconnects includes a second transducer. The interconnects can include an elastomeric component. In addition, the interconnects can further include at least one electrically-conductive component selected from the group consisting of carbon black, silver, eutectic gallium-indium (EGaIn), and carbon nanotubes.

Interconnects can be serpentine. The serpentine interconnects can be formed between the nodes by forming the serpentine interconnects on a silicon wafer, transferring the serpentine interconnects to the elastomeric sheath by transfer printing, forming islands at intersections of the serpentine interconnects, and applying transducers (e.g., sensors) at least a portion of the islands, where the transducers can measure strain at the interconnects during flexing of the elastomeric sheath and associated movement of the serpentine interconnects.

An embodiment of the present invention is a system that includes an elastomeric sheath that is worn on a biological segment of interest. The elastomeric sheath captures 3D surface shape, biomechanical properties of tissue, and internal bone geometries.

In one embodiment, shown in FIG. 19, 3D measurement device 10 is made of elastomeric sheath 12 and embedded grid 13 that includes a plurality of nodes 14 and edges, e.g., electrically-conductive conduits 16. Grid 13 can be quadrilateral or have some other suitable topology. Device 10 of FIG. 19 is an ankle-foot device, and the user can easily roll elastomeric sheath 12 on to biological body segment 18 over skin surface 20 of at least a portion of biological body segment 18 like a traditional cloth sock. Elastomeric sheath 12 acts as a scaffold that can support the device components and the required electronics. Examples of suitable elastomers of elastomeric sheath 12 include, but are not limited to, thermoplastic elastomers, styrenic materials, olefenic materials, polyolefins, polyurethane thermoplastic elastomers, polyamides, natural and synthetic rubbers, polydimethylsiloxane (PDMS), polybutadiene, polyisobutylene, poly(styrene-butadiene-styrene), polyurethanes, polychloroprene, and silicones. The degree of flexibility of device 10 may vary in parts of elastomeric sheath 12 by using elastomers of different flexibilities. Elastomeric sheath 12 can be a material with low Young's modulus, for example, 50 MPa, 30 MPa, 25 MPa, or less. Thickness of elastomeric sheath 12 may also vary depending on the application, the properties of the elastomeric material, etc. For instance, elastomeric sheath 12 can, in some embodiments, have a thickness of 10 nm to 10 $\mu$m, 50 nm to 5 $\mu$m, or 100 nm to 1 $\mu$m.

A plurality of transducers is located at least a portion of either the electrically-conductive conduits or the nodes, whereby data collected by the transducers can be employed to generate a 3D representation of the biological body segment. In one embodiment, the transducers are at nodes 14. Device 10 also includes additional transducers, which are located at electrically-conductive conduits 16 between nodes 14.

The transducers at the electrically-conductive conduits (e.g., the first transducers) and the transducers at the nodes (e.g., the second transducers) can be employed to evaluate, inter alia, pressure, stretch, shear, proximity, or touch in a variety of applications. For example, as shown in FIG. 20, dielectric stretch sensors 22 and curvature sensors 24 are located at electrically-conductive conduits 16. Electrically-conductive grid 13, in one embodiment, includes a neutral, unloaded topology, with each node 14 spaced, for example, about 2 cm from neighboring nodes 14. Also, typically, device 10 has an electrically-conductive unloaded interior volume that is smaller than the volume of the biological segment being measured (e.g., ankle-foot complex in FIG. 19).

As can be seen in FIG. 19 and FIG. 20, extending from each node 14 is a plurality of edges, e.g., electrically-conductive conduits 16, configured as, for example, a four quadrilateral mesh configuration, or a six triangular mesh configuration (not shown). Stretch sensors 22 detect edge length, or distance between nodes 14. These sensors can be capacitive, as illustrated in FIG. 21A and FIG. 21B, with two parallel plates 26 oriented parallel to biological body segment 18 at surface 20 and separated by the dielectric elastomer 28. As sensor 22 stretches from the position of FIG. 22A to the position of FIG. 22B, the distance (d) between plates 26 decreases, corresponding to increased capacitance. Curvature sensors 24 detect edge 16 bend radius and direction.

These sensors may be resistive, as illustrated in FIG. 23A and FIG. 23B, with patterned conductive ink particles 30 suspended in the elastomeric substrate 32. As sensor 24 bends, particles 30 begin to lose contact, thereby increasing resistance, which can be converted to a measurable voltage.

In one embodiment, curvature sensors 24 are bipolar to distinguish directionality, but unipolar sensors are also viable because concavity of human body parts is generally easily observable and measurements can thus be corrected during data processing if needed. Stretch sensors 22 and curvature sensors 24 may be capacitive, resistive, piezoelectric, fiber optic, or conductive fabric/thread/polymer-based. Suitable commercial stretch sensors 22 and curvature sensors 24 can be used, e.g., custom sensors from StretchSense Ltd. As these sensors consist of multiple (n) layers, the overall system will have a neutral mechanical plane (NMP), which is the zero-stress plane under bending stress, to provide flexibility features. The position of NMP within the system is affected by the property of the functional layer that can either be heterogeneous or have one or more properties that are inhomogeneous. With respect to the first layer ($i$ = 1), the location ($y_{neutral}$) of the NMP can be calculated from the summation of $n$ layers, plane-strain modulus ($\bar{E}_i$), and thickness of the ith layer ($t_i$). (See, for example Zheng YP, Mak a F, Leung a K (2001) State-of-the-art methods for geometric and biomechanical assessments of residual limbs: a review. J Rehabil Res Dev 38:487-504, the relevant teachings of which are incorporated by reference in their entirety).

$$y_{neutral} = \frac{\sum_{i=1}^{n} \bar{E}_i t_i \left(2 \sum_{j=1}^{i} t_j - t_i\right)}{2 \sum_{i=1}^{n} \bar{E}_i t_i} \qquad \mathbf{26}$$

Without any external forces being applied or sensors being used, an embodiment of the invention can process this stretch and curvature information, including the absolute value of or change under load in electrical parameters like capacitance, resistance, and voltage, or mechanical parameters like strain, bend radius, interference pattern, and position of zero-stress plane to calculate the relative spatial coordinates of each node 14 in the biological segment reference frame (*e.g.,* the foot in FIG. 19), as well as the contour of the surface at points between nodes 14, toward the generation of a digital 3D shape model. This method may reduce the number of nodes 14 required versus existing imaging methods, essentially allowing a lower resolution mesh with smoother and better-informed interpolation between nodes 14. Overall resolution of the 3D shape model may be controlled by careful design of physical mesh parameters, primarily the number of conduits 16 and nodes 14, and the sensitivity of sensors 22, 24.

The system can have the NMP that passes through the active material (e.g., piezoelectric, dielectric, ferroelectric or pyroelectric materials) in the sensing elements, actuating elements, or both. If the device has multiple layers including an active material layer, then the NMP can be located within the piezoelectric layer. In some embodiments, this reduces the strain on the active material during possible bending during daily activities. For example, the active material can exhibit a strain of less than 5%, 4%, 3%, 2%, 1%, 0.5%, 0.1%, or 0.05% for a device bending radius of 10-1000 mm, 10-500 mm, 10-200 mm, 20-200 mm, 20-500 mm, 20-1000 mm, 30-200 mm, or 30-500 mm, respectively.

In one embodiment, nodes 14 of grid 13 include thin, flexible, high-performance integrated circuits 34. As shown in FIG. 20, the embodiment of each circuit 34 shown therein includes at least a thin-film microcontroller 36 and a radiofrequency antenna (RF) 38 to pre-process and transmit data from the sensors wirelessly to an off-board non-volatile memory storage unit (not shown). Alternatively, low-energy Bluetooth (BLE) may be used in place of RF 38. Each circuit 34 also includes thin biomechanical sensors, such as dielectric elastomer pressure and shear force sensors 40, which can be resistive or capacitive, and ultrasound transducers 49, which can transmit and receive acoustic signals. Sensors 40 can also include a piezoelectric material. Examples of suitable piezoelectric materials are berlinite (AlPO4), quartz, Rochelle salt, topaz, tourmaline-group minerals, gallium orthophosphate (GaPO4), langasite (La3Ga5SiOi4), barium titanate (BaTiO3), lead titanate (PbTiO3), lead zirconate titanate (Pb[ZrxTi1-x]O3, 0<x<1) (commonly referred to as PZT), lead magnesium niobate (PMN), lead magnesium niobate-lead titanate (PMN-PT), potassium niobate (KNbO3), lithium niobate (LiNbO3), lithium tantalate (LiTaO3), sodium tungstate (Na2WO3), zinc oxide (ZnO), sodium potassium niobate ((K,Na)NbO3) (also known as NKN), bismuth ferrite (BiFeO3), Sodium niobate (NaNbO3), Bismuth titanate (Bi4Ti3O12), Sodium bismuth titanate (NBT), polyvinylidene fluoride (PVDF), poly[(vinylidenefluoride-co-trifluoroethylene] [P(VDF-TrFE3)].

Sensors with dual functionality for orthogonal and shear forces currently exist in research pipelines. (See, for example, Gere JM & Timoshenko SP. (2003) Mechanics of Materials: Solutions Manual, Nelson Thomes, Cheltenham, UK, and Charalambides A & Bergbreiter S. "A novel all-elastomer MEMS tactile sensor for high dynamic range shear and normal force sensing." J. Micromech. Microeng. vol. 25, no. 9, Sep. 2015, the relevant teachings of both of which are incorporated by reference herein in their entirety). An example of a node component that detects both orthogonal and shear forces is shown in FIG. 23A and FIG. 23B.

Elastomer base 161 supports sensor plates composed of the same elastomer infused with an electrically conductive material selected from the group consisting of carbon black, silver, eutectic gallium-indium (EGaIn), and carbon nanotubes. Each of four larger "pillars" 165, which are ultrasound transducers, (such as ultrasound transducers 49 in FIG. 20) surrounded by four smaller "electrodes" 164, (such as shear force sensors 40 in FIG. 20). Thin electrical conduits of the same material composition act as wires 162 connecting the sensors to ground, and wires 163 transmit electrical data to the rest of the node circuit. Orthogonal and shear force deformations applied to node component 166 as shown in FIG. 23B generate changes in capacitance between the pillars 165 and their respective electrodes 164. As force is applied, the geometry of the sensor elastomer 161 and internal pillar 165 changes to a loaded elastomer 168 and pillar 167 geometry.

Circuits may also include, without limitation, ultrasound transducers, a sensory feedback component, such as an LED or a vibration motor, and sensors of temperature, moisture, tissue depth, hydration, vascularization, and peripheral nerve anatomy. Ultrasonomicrometry crystals 49, shown in FIG. 20, can be included to measure absolute or change in distances between nodes, as well as internal bone geometries. Microscale light-emitting diodes 42 can be included to provide visual feedback. For example, once node 14 has been actuated by optional handheld imaging probe 44, shown in FIG. 19 and a reading is taken, LED 42 switches on to mark node 14 as read, both to eliminate redundancy and ensure that none were missed. The LED 42 may form part of a user interface that is provided at device 10. All circuit wiring 46, shown in FIG. 20, can be flexible and stretchable due to filling channels in the elastomer of circuit wiring 46 with conductive particles that are not rigidly constrained as would be solid metal. For power, the entire grid of circuits can be connected by wires 48 from wiring 46 to external battery 50 that can be positioned in a carrier 54 (FIG. 19) and can be mounted by a suitable adhesive or hook-and-loop fasteners 52 elsewhere on the body. Battery carrier 54 can also house external memory storage unit 56. Although the components are all relatively low-energy, they are numerous, and decoupling battery 50 from elastomeric wires 48 allows physical battery size to not be a limitation. As battery technology advances toward materials with superior storage capacity, such as zinc-polymer or lithium-sulfur and graphene, integration of batteries into elastomeric wirings or wireless charging may be possible. In one embodiment, the invention is modular, with the ability to customize selection of included sensors. Strip 58 of elastic adhesive material lines top 60 of elastomeric sheath to aid in keeping device 10 from slipping down biological body segment 18 (*e.g.,* ankle of FIG. 19), although the inner surface (not shown) of elastomeric sheath 12 may also need to be sticky, without leaving residue, due to its low durometer.

Handheld imaging probe 44, illustrated in FIG. 19 and FIG. 24, optionally expands the capabilities of system 10. Some desirable biomechanical properties generally cannot be measured with existing transducers that are sufficiently thin, flexible, stretchable, or robust. Specifically, traditional ultrasound transducers typically need to be a certain thickness in order to image deep tissue. Thus, imaging probe 44, shown in cross-section in FIG. 24, integrates acoustic lens 62 at contact interface 64, acoustic matching layer 66, piezoelectric elements (PZT) 68, electrodes 70, and backing material 72 for ultrasound imaging.

All electronics 69 are packaged in space 74 behind ultrasound components 76, including a 9-axis inertial measurement unit (IMU) 77 for displacement measurements, microcontroller 80, and RF antenna 82 or low-energy Bluetooth (BLE) to pre-process and transmit data wirelessly to the same off-board memory storage unit (not shown). Flexible micro-coaxial cable 84 extends out to power and read the sensors (not shown). Probe 44 can also include a battery (not shown) for power. Because probe 44 is far less restricted in volume and power within acoustically insulating rigid outer shell 86, electronics with higher rates of data acquisition, resolution, or range of applied forces can be used. As ultrasound technology advances in reliability and penetration depth, capacitive micromachined ultrasonic transducers (CMUTs) may emerge as a viable alternative to PZT. Probe 44 can be used to take measurements at nodes 14 of grid 17 (FIG. 19), which are physically recognizable by the tester (either the user himself or a medical professional), and thus may be immediately correlated in space and time in a digital 3D model. Silicones such as ECOFLEX® Supersoft Silicone (Smooth-On, Inc.) are essentially transparent to ultrasound and, therefore, generally will not interfere with the signal, but may actually serve as the coupling medium.

Device 10 and probe 44 described herein form a complete measurement system that maximizes portability and versatility while significantly reducing the bulk and cost associated with the imaging technologies described earlier. This makes it more practical to use in remote geographic locations, as well as more affordable for people in all financial situations worldwide. An embodiment of the invention is useful as a data collection tool to inform improved design, and secondarily as a diagnostic tool to assess health of the tissues of a biological segment (e.g., the foot and ankle in FIG. 19). A wide range of forces may be applied to characterize a comprehensive range of tissue responses, including the maximum forces tolerated before pain is perceived (hereinto referred to as the pain threshold). Acquired data can be processed to generate visual maps of tissue properties - for example, viscoelasticity or skin strain, both of which are useful factors in the fit and comfort of interfacing mechanical devices, including, but not limited to, shoes, prosthetic limbs, orthoses, exoskeletons, and bras. A map of localized biomechanical tissue viscoelastic responses, unloaded segment shape, and internal tissue geometries can be used to generate a FEA model of the biological segment, and that model can them be employed to design both the unloaded shape and spatially or temporally-varying impedance of a fitted wearable device.

The design framework may specify thicker cushioning where the user's bone is closer to the skin surface and thus more vulnerable to injury, and a more breathable fabric weave where the user's skin is prone to developing blisters due to moisture accumulation. In the case of shoe design, this model may then be matched to a set of appropriate shoe components, or even sent to a 3D printer to instantly fabricate an entirely custom-fit shoe.

In addition to applying forces with fingers or the external probe to a static sock-covered foot, the present invention enables more complex force applications. Because the system is so unobtrusive and self-contained, the user can walk while wearing the sock alone, and embodiments can collect dynamic data on how the tissue behaves during the time-course of an activity that the foot experiences in daily life, as illustrated in FIG. 25.

The user rolls on elastomeric sheath 12 with embedded grid 17 and nodes 14 of edge and node components that capture: 3D shape of the foot-ankle complex; applied forces; viscoelastic tissue properties (e.g., tissue stiffness and damping); and internal bone geometries. Forces can be applied to each node 14 with the fingers and/or the handheld imaging probe (*e.g.,* probe 44) to collect data on biomechanical properties such as viscoelastic tissue properties and soft tissue depth that are relevant design inputs for a wearable device such as a custom-fit shoe. The user can also walk on elastomeric sheath 12, with or without a shoe, to generate the exact forces experienced during walking, while device 10 and, optionally, probe 44 collects dynamic pressure, shear force, and other data. These data can then be used to generate a 3D FEA model of the ankle-foot complex and the corresponding digital design of a custom-fit shoe 88, which can then be sent to a manufacturer or 3D printed 90 immediately in-house.

The user can also run, jump, kick, or do anything else to complete a data set that is representative of intended applications. Elastomeric sheath 12 can also be worn inside of a shoe during data collection, serving to measure the static or dynamic interface behavior between foot and shoe. Tissue parameters can vary widely with time and activity, and as a result, shoe fit varies too, resulting in discomfort, pain, or injury. By capturing these changes, embodiments of the invention can inform a more adaptive shoe design than current artisanal methods ever can.

It should be evident that embodiments of the invention can take the form of devices other than socks for applications to body parts other than feet. Alternatives to the probe component will not be discussed, because it can be universally applied to any body segment.

One alternative embodiment is a measurement socket liner 12' that rolls on over the residual limb of a person with limb amputation (*e.g.*, a transtibial amputee as in FIGS. 27A-D). It functions identically to the ankle-foot sock embodiment, collecting biomechanical tissue data in combination with the external imaging probe toward designing better prosthetic sockets and liners, as in FIGS. 26A-D.

The lines of minimum extension are shown here as the digital model of computed skin strain (FIG. 26A), and the variable-thickness socket liner generated from a 3D-printed mold with inextensible material placed along lines of minimum skin extension (FIG. 26B). Results of using an embodiment of the invention also include optimized prosthetic sockets for amputees, with multi-durometer materials corresponding to locations of varying mechanical properties on the residual limb (FIG. 26C), to increase comfort of the 3D-printed socket generated using that module (FIG. 26D).

Examples of suitable prosthetic sockets and liners are described, for example, in U.S. Ser. No. 13/836,835, filed March 5, 2013, published as US 2013/0282141, the relevant teachings of which are incorporated herein by reference in their entirety. Also incorporated by reference in their entirety are the relevant teachings of International Application No. PCT/US2017/013154, filed January 12, 2017 and published as WO2017/123729, which claims priority to U.S. Ser. No.: 62/278,158, filed January 13, 2016. The improved socket could comprise multiple materials to provide continuously variable impedances to provide rigid support or soft cushioning where needed. The improved liner could use variable material thicknesses to accommodate anatomical points of high skin strain during joint excursion such as a flexed knee. It could also have threads of increased stiffness along lines of minimum skin extension for enhanced structural integrity, while still permitting high compliance in regions of high skin extension to reduce skin irritation caused by high shear forces at the liner-biological skin interface. The novel socket liner may be worn alone for static data collection, or under the prosthetic socket to analyze socket fit and dynamic interface behavior.

An alternative embodiment, shown in FIG. 28, is a smart bra 300 that performs measurements or periodic monitoring of breast health using embedded sensors. This is particularly important for early detection of breast cancer, which presents with unusual tissue stiffness, abnormal discharge, elevated thermal profiles, increased vascularization, and tumorigenesis. These are currently identified by medical examination using MRI, CT, or X-ray mammograms, relying on unreliable and oft-forgotten self-examinations to prompt medical visits. An embodiment of the present invention takes the form of a regular women's bra 302, with two cups 304, two shoulder straps 306, and band 308. Surface of bra 302 directly interfacing with the skin of an individual (not shown) is lined with a thin elastomeric material (310) that is embedded with a mesh 312 of nodes 314 and edges 316, similar to the previously described embodiments. Mesh 312 enables 3D shape capture to detect shape and shape variations, as well as the measurements of applied shear and normal forces applied to the breast tissue, or other critical parameters such as temperature.

With a complete model of breast shape and viscoelastic properties, a custom bra can be fabricated to perfectly fit the individual. The unique data collected with bra 300 can be fed into algorithms that precisely dimension the corresponding bra model from the selected style template (*e.g.*, strapless, wireless, push-up) for automatic fabrication. This substantially increases efficiency and privacy by eliminating guesswork, trial-and-error in store fitting rooms, and the need for assistance from strangers.

Skin modulus sensors 318 are integrated into some or all of nodes 314 to provide additional indicators of unusual tissue stiffness, whether associated with cancer, pregnancy, menstruation, injury, or otherwise. Ultrasound transducers 320 are positioned at nodes 314 that fall within thicker padding 322 in lower section 324 of cups 304, to minimize obtrusiveness. These serve to directly image abnormal deep tissue formation, vascularization, and nerve anatomy. If viable ultrasound sensors are too thin to image at desired tissue depths, then a handheld ultrasound probe, such as probe 44, can easily be employed in conjunction with smart bra 300. EKG and respiration rate sensors 326 can be located in bands 308 to the sides of cups 304 to monitor heart rate, respiratory rate, and rate variability. This functionality is especially useful for athletic bras, which are worn during activities when monitoring heart rate and respiration can be critical. This data may be provided directly to the user or used to calculate calories burned.

Wiring 328 can be embedded anywhere, including below cups 304 where traditional structural underwire is located. Power elements, transmission elements, and other electronics 330 can be positioned in band 308 between cups 304, where they are least conspicuous. These electronics can include, for example, microprocessors, RF antennas, LED's, and vibration motors or other actuators. All collected shape, skin modulus, ultrasound, temperature, and other data is compiled by the microprocessor to detect tumor growth and other tissue, cardiovascular, or respiratory abnormalities. Feedback is provided to the user either through subtle signals from actuators or through wireless transmission to an external device such as a smartphone (not shown). This feedback includes alerts when abnormalities are detected and when adequate force is applied to the tissue for handheld probe 44 ultrasound imaging during self-examination. The bra outer surface, likely interfacing with clothing, is lined with traditional bra materials such as cotton and polyester, or digitally fabricated woven fabrics. Regular monitoring may be desirable in other parts of the body, such as the abdomen, arms, or legs, for other purposes, such as tumor detection, compartment syndrome, blood flow, wound healing, temperature, and hydration - the invention may be extended to these applications.

The present invention, in another embodiment, can take the general form of a mesh embedded in a thin elastomeric sheet of material and arranged in any appropriate topology for the measurement of several useful features relevant to the design of a mechanical interface between a device and a biological segment. These useful features include: biological segment shape; skin strains caused by joint and/or muscle movements; tissue viscoelastic properties such as stiffness and damping; and internal tissue geometries such as 3D bone shape, skin thickness, and the density and thickness of skin, muscle and fat layers. It may be fabricated in a number of ways. A two-part mold may be created via 3D printing (Connex 500, Objet Ltd) at fine resolution for more complex 3D shapes as found in socks and socket liners, or by using masks to etch patterns on silicon for simpler 2D planar profiles as is sufficient for bra cups. Soft elastomers like polydimethylsiloxane (PDMS) or super-soft silicone rubbers can be poured or injected into the mold in thin layers, with electronics being embedded between the appropriate layers. Complete electronics, like a thin LED and a microcontroller, can be placed directly, while simpler elements, such as potential dielectric elastomeric pressure/shear sensors and wiring, can be integrated into the elastomer.

If, in one embodiment, a mold is employed to form channels in a desired pattern in an initial layer of a poured elastomer, then these channels can be filled with an elastomer mixed with some percentage of suspended carbon black, silver, or other conductive powder; with a conductive fluid like eutectic gallium-indium (EGaIn); or with carbon nanotubules, all of which can stretch without loss of signal. If a metallic trace or other less elastic material is desired for its electrical properties, integrated sensors and wires can still be made stretchable by serpentine geometry, as illustrated in FIGS. 29A-29B.

As shown in FIGS. 29A-29B, grid 140 includes serpentine interconnects with required nodes 144 for sensor placement. Fabrication of grid 140 can be conducted on a silicon wafer and then transfer-printed onto a target polymer substrate. In one embodiment, a silicon wafer is initially coated with a 50 nm thick poly(methyl methacrylate) (PMMA) layer and 1.2 μm thick poly(pyromellitic dianhydride-co-4,40-oxydianiline) amic acid (PI) layer to provide a tacky surface for the base of the structures. The PMMA layer can be created by spin-coating at 3,000 rpm for 30 seconds and baked at 180 oC for 2 minutes on a hotplate. The PI layer can also be created by spin-coating at 4,000 rpm for 30 seconds and procured at 150oC for 1 minute. Afterward, the serpentine interconnects 142 can be defined through the evaporation and photolithographic patterning of gold (Au)/chromium (Cr) with 200 nm and 10 nm thicknesses, respectively. The electrodes can be Ag, Al, Au, Co, Cr, Cu, Fe, Mo, Nb, Ni, W, Zn, Zr, Au/Ti, Cr/Au, Pt/Cr, Ti/Au, Ti/Pt electrodes, or some combination thereof. This structure is then encapsulated with another 1.2 μm thick PI layer to protect it from the immersion in acetone at 100oC. PVA tape can be used to retrieve the structures. A layer of titanium (Ti)/silicon dioxide (SiO2) with 4 nm and 40 nm thicknesses, respectively, is then deposited on a back side of serpentine 142 interconnects to provide an adhesive layer to bond onto the target substrate. Sensors are later printed on top of the nodes 144 which are connected to adjacent nodes 144 by serpentine interconnections 142. Interconnections 142 can be strained without undergoing fracture, and may undergo strain of, for example, 0.5%, 1%, 5%, 10%, 15%, 25%, 30%, 40%, 50%, or larger without fracturing. Stable nodes (e.g., device components) can be engineered to undergo compression, elongation, and/or twisting in order to withstand deformation without fracturing.

In any of the embodiments described above, the nodes can contain ultrasound transducers in the form of ultrasonomicrometry crystals, shown in FIG. 30. In the example shown, the crystals are embedded in the elastomeric sheath 150 at each node. A crystal 151 may transmit an ultrasonic pulse through an acoustic medium such as biological tissue 152. An omnidirectional ultrasonic pulse may either echo off an acoustically reflective surface such as bone 153 and be received by the same crystal, or transmit directly to a different receiving crystal 154 at a different node in the network array. The crystals may be electrically connected to peripheral electronics via some combination of traditional wiring, serpentine interconnects, wireless communication such as Bluetooth or RF. The crystals can be used to measure absolute values and changes in distance between nodes during movement of the elastomeric sheath, as well as to perform echo ultrasound to measure internal bone geometries.

Transducers described herein that can be used to acquire data, such as data about the biological body segment and the environment. Acquired data can be post-processed in MATLAB® technical computing software (The MathWorks, Inc.) or other software in any language. For 3D shape capture, length and curvature data are used to calculate node coordinates and edge splines, which are used to create a visual 3D model of the measured body segment, as well as tissue displacements and displacement rates caused by an applied tissue load. For force sensing, system inputs and outputs can be employed for system identification to identify a transfer function that describes the physical response of the tissue system to an applied perturbation. The collective responses at each measurement site can be visually coded and mapped to the 3D model of the measured biological body segment to generate maps of pain, sensitivity, tissue impedances, or other properties of interest. This comprehensive model can then be used to improve the form and functionality of superior human-device interfaces.

## 4. A Low-Cost Single Element Ultrasonic Sensing System for Assessment of Tissue Geometry and Mechanical Properties

A hand-held apparatus that can measure both skin-to-bone depth and soft tissue mechanical properties, and methods for using such an apparatus, are provided. The device and method can include gathering and processing data from an ultrasound transducer, a force sensor, and an accelerometer. The procedure of use can include placing the apparatus at various regions on the limb while maintaining a slight contact pressure to acquire skin-to-bone depth, and indenting the apparatus into the limb to acquire soft tissue mechanical properties.

Such devices can be used for tissue boundary detection using a single element ultrasonic transducer while providing for a low-cost, light-weight apparatus that measures both the tissue boundaries and the soft tissue mechanical behavior using ultrasonic sensing and force sensing.

An ultrasound bone depth sensing and indentation device 400 is shown in FIG. 31. The device 400 includes an ultrasonic transducer 402, a force sensor 404, an accelerometer 406, and a microcontroller 408. As illustrated, a second microcontroller 410, a force sensor calibration printed circuit board, is included. In operation, the device 400 can be placed against a surface 432 of a biological body segment 430, illustrated as a residual limb having soft tissue 434 and bone 436. A skin-to-bone depth 420 can be assessed. The device 400 can also be used to apply pressure to the biological body segment 430, such as by indenting the soft tissue, to obtain displacement data for the assessment of soft tissue characteristics or mechanical properties of the body segment 430.

### *Ultrasonic Sensing for Tissue Boundaries*

Ultrasonic sensing, among other available imaging technologies such as X-rays, CT, and MRI, is non-radiative (unlike X-rays and CT), more affordable (especially with respect to MRI), and can be non-invasive. Widespread for both industrial and medical use, ultrasonic technology is a mature field where sensors of a variety of types specifically designed for various applications are commonly available. While medical ultrasound machines are already significantly more affordable and accessible than MRI scanners, individual ultrasonic transducers are even more compact and at low cost. At present, ultrasonic technologies in-vivo use either an echo-based technique that measures the reflected signal, or a signal-between-two-crystal technique, which is known as sonomicrometry.

Sonomicrometry is a particular technique among ultrasonic technologies that measures the distance between piezoelectric elements or crystals (piezoelectric elements and crystals both refer to the fundamental part of an ultrasonic transducer. In this section, the terms piezoelectric elements, crystals, ultrasonic transducers, ultrasonic crystals, and transducers are used interchangeably as referring to a single ultrasonic sensing unit). In the literature, sonomicrometry is also considered a reliable method for in-vivo measurements. The main difference between sonomicrometry and an echo-based approach is that, instead of measuring the reflected ultrasound wave at the same location where it is transmitted, sonomicrometry measures the time lapse for ultrasound to travel from one crystal to another through the medium. In an in-vivo setting, sonomicrometry casts a constraint on the stability of the positions of the crystal pairs. In practice, the crystals are invasively implanted to the human organs or other body parts of measurement. However, echo-based techniques can provide a non-invasive solution.

The most common form of an echo-based technique in the medical field is commercial ultrasound imaging machines. Ultrasonic measurements are routinely performed in hospitals not only as part of diagnostic assessment such as of various organs, tumors, and the fetus during pregnancy, but also as a guidance in surgeries. Commercial ultrasound machines use a probe that contains arrays of ultrasonic transducers to produce images based on the reflected signals at tissue boundaries. One can use image segmentation and tissue characterization to determine tissue boundaries.

Another form of an echo-based technique is the use of a single transducer, as opposed to an array of transducers. In this study, a single element ultrasonic transducer was used, which performed both the role of the transducer and the receiver. A single transducer not only has the benefit lower cost than a probe containing an array of transducers, but it also introduces further simplicity and design flexibility. By evaluating the feasibility of using a single transducer to detect tissue boundary and indentation, this study attempts to fill the gap of non-invasive low-cost techniques and the use of single transducers in the medical domain.

*Sound in the Human Body*

As sound waves propagate through tissue boundaries, such as from fat to muscle or muscle to bone, some of the waves are reflected while others are refracted. The perturbation to ultrasound waves on propagation through soft tissue is sufficiently small that refraction and defocussing artifacts can often be neglected. Situations where these effects may become significant often takes place through fatty tissues, such as the breast, or through the skin/fat/muscle complex of the abdominal surface.

The sound signal attenuates as it propagates through the human body. The reflected sound waves are the receiver signals to the ultrasonic transducers. The attenuation is a function of the traveling distance and the wave's frequency: the farther the sound wave travels, the greater the attenuation. The shape of attenuation depends on the transducer, but most commonly captures a form similar to exponential decay. The attenuation in soft tissues increases approximately linearly as frequency increases.

*Transducer Selection Considerations*

In the context of tissue boundary detection and indentation sensing using a single element transducer, the most important trade-offs in meeting a specific design requirement include axial resolution, focus area, and the dimension of the transducer. Transducer frequency, diameter, and type are the determining factors and thus the most common design considerations, and will be discussed in the following sub-sections.

A typical transducer includes a matching layer, piezoelectric crystal, backing material, acoustic insulator, electrical shield, case, and wire. The matching layer optimizes acoustic impedance of the transducer and the medium; the piezoelectric crystal transfers mechanical and electrical energy; and the backing material provides damping to minimize ringing after pulse.

*Transducer Frequency*

Ultrasound range is defined by frequencies larger than 20 kHz. Typical medical ultrasonic imaging uses frequency range from 2 to 15 MHz. The speed of sound is 1450 m/s in fat and 1550-1630 m/s in muscle. In ultrasound beam-forming, calculations typically assume a fixed sound speed *(e.g.,* 1540 m/s). In this study, speed of 1540 m/s was assumed in all limb measurements.

The wavelength $\lambda$ of the ultrasonic wave is given by:

$$\lambda = \frac{c}{f} \qquad \textbf{27}$$

where c is the speed of sound, and $f$ is the frequency of the ultrasonic wave. From Eqn. 27, ultrasonic waves of 1 to 15 MHz have 1.54 to 0.10 mm wavelength. In this study, an apparatus which operates in a 1 MHz frequency was used. The wavelength determines the axial resolution of the ultrasonic transducer. Axial resolution describes how finely the signal can tell apart two structures that are parallel to the sound beam's main axis, *i.e.,* if two structures are aligned in the direction of the sound beam, how far apart they have to be for the transducer to differentiate them. Axial resolution is particularly important for a single-element transducer since it receives a one-dimensional signal. In addition, axial resolution is also a function of the number of cycles in the triggering electrical pulse. Depending on the driver system that the application adapts, pulses of different shapes, duration, and cycles may differ. Square waves, sine waves, or waves in between of one to ten cycles are the most common in commercial driver systems.

The axial resolution r is given by:

$$r = \frac{1}{2}\lambda n \qquad \textbf{28}$$

where n is the number of cycles per pulse. In the case of a 1 MHz transducer driven by one cycle per pulse, which is the case in this study, the axial resolution is 0.77 mm. The resolution becomes finer as the wavelength decreases or as the frequency increases. For applications such as computer aided prosthetic socket design, 0.77 mm axial resolution can be sufficient.

The pulse repetition rate can be adjusted according to the transducer frequency to ensure that all echoed signals have been received before the next pulse is generated. Overlapping signals not only increase noise but cause confusion in interpreting the received signal. In this study, a trigger rate of 1000 Hz was used, which means triggering a pulse every 1 ms, which is a significantly longer than the time it takes to the echoed sound wave to decay completely (0.4 ms), thus a sufficiently low pulse repetition rate.

*Transducer Diameter*

Another physical characteristic of the transducer is its diameter. The diameter of the transducer, together with its frequency, determine its focal depth. The sound beam of a transducer is often divided into three distinctive regions: near zone, far zone, and focal zone. The beam converges in the near zone, and is the most concentrated at the focus in the focus zone. As the wave propagates away from the focus out of the focus zone into the far zone, the beam diverges. Because of the variations within the near field, accurate analysis can be difficult. In soft tissue, the natural focus N is given by:

$$N = \frac{D^2 f}{4\lambda} \qquad \textbf{29}$$

where $D$ and $f$ are the diameter and the frequency of the transducer, respectively.

*Transducer Categories*

Ultrasonic transducers can be of different types, such as contact transducers, dual element transducers, angle beam transducers, delay line transducers, etc. Different types offer different advantages. For instance, for near surface detection, dual element transducers have less noise due to pulse artifact while delay line transducer improves near-surface detection by adding additional materials between the crystal and the contact surface.

*Force and Ultrasonic Sensing for Mechanical Properties of Soft Tissues*

By adding force sensing to an ultrasonic device, the same apparatus can be used for both tissue boundary detection and indentation experiments. The simultaneous measurement of displacement and force during indentation can allow for the computation of mechanical properties of the body segment by comparing the measured mechanical response with a simulated response, such as by using inverse finite element analysis (FEA) methods. An inverse FEA method can be used with indentation experiments. Measurements can be taken to obtain a force-displacement history, and consequently the tissue mechanical properties can be obtained. Here, by combining force and displacement sensing in the same apparatus, force-displacement histories can be acquired during indentation experiments.

FEA methods are suitable for modeling the mechanical properties of the limb because they allow for the modelling of large changes, large deformations, and time-dependent recovery (*e.g.*, viscoelastic behavior). Using FEA, the non-linear elastic behavior of soft tissue can be modeled using hyper-elastic formulations, and viscoelasticity can be modeled using the quasi-linear theory of viscoelasticity. Having measurements of distinctively representative anatomical regions, an inverse FEA based optimization routine determines the material parameters for each soft tissue location of measurement using the force-time and force-displacement curves obtained during indentation. Since these parameters are descriptive of anatomically distinctly representative locations, one can then model the entire limb using the acquired parameters. As described in Sengeh DM, Moerman KM, Petron A, Herr H (2016) Multi-Material 3-D Viscoelastic Model of a Transtibial Residuum from In-vivo Indentation and MRI Data. J Mech Behav Biomed Mater. doi: 10.1016/j.jmbbm.2016.02.020, a robotically actuated system was used for an indentation experiment. Conversely, in this study unconstrained (*e.g.*, handheld) indentation experiments were conducted and were shown to be sufficient to determine the modeling parameters using inverse FEA methods.

*Methods*

Using a single transducer for tissue boundary detection can be challenging because interpretation of a one-dimensional ultrasound signal is nontrivial. Peaks of the highest amplitude are not necessarily due to tissue boundaries, especially when searching for the shortest skin-to-bone depth. To overcome this problem, a statistical method to interpret the results collected from a sweep across the limb at a certain location was used.

In this work, a prototype device was built that incorporates the sensors and other electronics in a hand-held apparatus to demonstrate feasibility. The prototype demonstrates the feasibility of a compact, low-cost device using ultrasonic and force sensing for tissue boundary detection and indentation experiment.

*Overall System Structure*

An overview of the system is shown in FIG. 32. The system 500 includes a PC 502, a data acquisition system 504 (*e.g.,* PicoScope), a pulser/receiver 506, and a probe assembly 510. The probe assembly 510 includes a microcontroller 512, an ultrasound transducer 514, a force sensor 516, and an accelerometer 518. For the example prototype device that was built, Table 3 contains the part/device numbers of each component and their descriptions of an example, prototype system.

In the probe assembly, the ultrasound transducer measures the tissue boundaries and tissue depths (displacements); the force sensor measures the forces due to contact and indentation; and, an accelerometer measures accelerations for self-weight compensation.

Data collection parameters such as the number of total sets of data and preprocessing thresholds, were directly instructed from the PC end, while the driving and receiving parameters, such as filter frequency, gain, and damping factor of the ultrasonic transducer, were controlled by the knobs in the pulser/receiver. While such a configuration was used for the prototype system, controllers for operation of the probe 510 can optionally be integrated into the probe.

In this study, a JSP DPR300 pulser/receiver was used, which was set to have 55 dB gain, 1 to 3 MHz band pass filter, PRF rate of 1, pulser amplitude of 10, pulser energy of low 1, and damping of 10 in echo mode). The PicoScope and the microcontroller have independent data acquisition systems, and data logging is centralized in the PC.

**Table 3: Component names and description**

| Parts/Device | Parts/Device Name | Description |
|---|---|---|
| Pulser/Receiver | JSR DPR300 | High voltage pulser and low noise receiver |
| Data Acquisition System | PicoScope 2205A | Digital oscilloscope with programming support |
| Ultrasound Transducer | Olympus C548-SM | 1MHz 10mm diameter angle beam transducer |
| Force Sensor | SingleTact S8-10N | 10N capacitive force sensor |
| Accelerometer | Adafruit ADXL326 | 5V triple axis accelerometer (+/-16g) |
| Microcontroller | Arduino Nano | Arduino Nano 3.0 |

*Transducer Selection*

In this case studies all experiments were conducted using a transducer with a 1 MHz angle beam and 10 mm transducer diameter (C548-SM from Olympus). This setup was found suitable for measurement between 20 and 200 mm. However, for areas where the skin-to-bone distance is below 20 mm, a more suitable transducer shall be used.

*Transducer in Probe Assembly*

The specifications of the ultrasonic transducer used in this work are summarized in Table 4. Due to the focus value, this transducer suited for measuring tissue depths larger than 16.23 mm. In addition, two tissue boundaries, which are 0.77 mm apart or more, can be distinguished. This transducer is a basic low-profile transducer that is easy for modeling and system design.

**Table 4: Transducer characteristics**

| | |
|---|---|
| Diameter [mm] | 10 |
| Frequency [MHz] | 1 |

(continued)

| | |
|---|---|
| Natural Focus in Soft Tissue [mm] | 16.23 |
| Axial Resolution | 0.77 |

*Further Transducer Selection*

For lower limb prosthetic sockets, the nearest distance between a bone and the skin is the tibia-to-skin distance, which can be as small as 5mm at certain regions. For accurate tissue boundaries detection of these regions, a transducer of focus 5 mm or closer can be desirable. To achieve a 0.5 mm axial resolution, according to Eqn. 27 and Eqn. 28, one needs a minimum 5 MHz transducer. With a 5 MHz transducer, according to Eqn. 29, one needs a transducer of diameter 2.5 mm or less. The specifications optimized for tissue depths of 5mm are summarized in Table 5. Depending on commercial availability, one might have to trade off focus, diameter, and axial resolution.

**Table 5: A possible transducer for near-skin tissue boundary detection**

| | |
|---|---|
| Diameter [mm] | 2.5 |
| Frequency [MHz] | 5 |
| Natural Focus in Soft Tissue [mm] | 5 |
| Axial Resolution | 0.5 |

*Probe Assembly Design*

The probe assembly was designed to be a hand-held apparatus, fitting the selected components compactly into one assembly, as shown in FIG. 31 and 33A. In this design, the ultrasonic transducer is aligned with the force sensor in the axial direction of the probe assembly.

Further details of a tip 500 of the hand-held device are shown in FIG. 33B. The tip 500 includes an ultrasonic transducer 502 and force sensor 504. The force sensor 504 is pre-loaded with a rubber pad 512 where fixed pressure is applied by Loctite fixed screws 514, as shown in FIG.33B. The probe assembly compacts all three sensors, as well as the microcontroller (e.g., Arduino Nano), into one hand-held device for ease of use.

*Data acquisition procedures*

The following procedures were followed in using the probe for tissue boundaries detection and during the indentation experiments.

*Tissue Boundaries Detection Procedure*

An example of a tissue boundary detection method is shown in FIGS. 34A-34B. The device 400 is rotated about the biological body segment 430, for example, in the direction shown by arrow 440. The rotation permits for measurements along multiple axes, such as axes 442, 444.

During the experimental procedure, the following steps were followed for tissue boundary detection tests: 1. Generously apply ultrasound gel at the location of measurement on the limb; 2. Lightly touch the limb with the probe head, adjust by sliding and rotating, to make a direct contact between the probe head and the skin; 3. Rotate the probe around the limb in the plane of the cross-sectional view from the top to an extreme where the majority of the probe is touching the skin; 4. Start data logging on PC; and, 5. Slowly rotate the probe around the limb in the plane of the cross-sectional view from the top to the other extreme where the majority of the probe is touching the skin.

*Indentation Experiment Procedure*

An example of a tissue boundary detection method is shown in FIGS. 35A-35B. The device 400 is pressed into the biological body segment 430, for example, in the direction shown by arrow 450.

During the experimental procedure, the following steps were followed for indention tests: 1. Generously apply ultrasound gel at the location of choice on the limb; 2. Start data logging on PC. Be sure that the probe is not touching anything at start for calibration purposes; 3. Lightly touch the limb with the probe head, quickly adjust by sliding and rotating, to make a direct contact between the probe head and the skin; 4. Press into the limb at speed of experiment requirement; 5. Repeat at different indentation speeds as necessary.

*Data Logging*

Ultrasound, force, and acceleration data was logged at the PC end via MATLAB based PC-to-Arduino and PC-to-PicoScope interfaces. For Arduino, MATLAB Support Package for Arduino Hardware version 17.1.1 was installed. For PicoScope, the PicoScope SDK and PicoScope 2000 Series MATLAB Generic Instrument Driver version 1.8 were installed. Simple triggering for block data mode was used to acquire sections of waveforms. The trigger was set to DC coupling, +/- 5V range, 0.1 ms time interval, and 5004 data points to collect. The sampling rate was thus 50.04 MHz, which is well above the Nyquist frequency of 2 MHz (twice the transducer's 1 MHz frequency).

The system can have two modes: a visual feedback mode and a blind mode. An overview of the system 600 is shown in FIG. 36. Both visual feedback mode and blind mode include a data acquisition stage 610. Visual feedback mode further includes a preprocessing stage 620. In visual feedback mode, pre-processed waveforms are displayed, together with reference lines including peaks over predetermined threshold and areas above thresholds such as in FIG. 37B. The visual feedback mode can be used as a training mode, which allows the user to learn and adjust to how signal strength is associated with the amount of ultrasound gel, the amount of pressure, and the transducer motion. After the user becomes familiar with using the transducer, blind mode can be available for an improved frame rate, *i.e.* more data sets are recorded for the same period of recording time.

*Visual Feedback Mode*

As shown in FIG. 36, once data logging begins, the PC requests a block of waveform from PicoScope (step 612), force and acceleration data from the Arduino (steps 614 and 616), and a time-stamp. Before the next round of data acquisition, the waveform signal is processed and displayed.

Each 0.1 ms long waveform captures a single pulse and its echo. A series of steps are taken to determine if the captured waveform is both properly triggered and contains information regarding tissue boundaries/depths (steps 622-628). If the waveform is either poorly triggered or doesn't contain useful information, the waveform and other data at this instance are discarded. The raw measured waveform, as well as the processed waveform are then displayed on the PC (step 630), and move as the next waveform is processed.

FIG. 37A-B shows an example of a raw waveform and a corresponding processed waveform. FIG. 37A is a typical waveform measured in-vivo. The burst right after 0 ms is the artifact from the triggering of the transducer. The smaller peaks between 0.03 and 0.08 ms are echoed waves at various boundaries. Depending on the angle and contact, the amplitude of the peaks may vary significantly. These are likely to be echoes from fat-to-tissue, tissue-to-bone, and other boundaries where acoustic properties vary. In this example, there is one echoed wave that is significantly larger in amplitude than the other around 0.55 ms, and it corresponds to the tissue-to-bone boundary, as verified by MRI. However, it is common to observe multiple large amplitude peaks, or none.

FIG. 37B plots the upper envelope of the filtered raw waveform, after removing the first peak which is associated with the triggering artifact. Note that the x-axis in the bottom subplot is distance instead of time. The distance d between the transducer and the surface from which the wave echoed is obtained by

$$d = \frac{ct}{2} \qquad \mathbf{30}$$

where c is the speed of sound (1540 m/s in soft tissue), and t is time. The red squares are locations where the upper envelope exceeds a certain threshold (i.e. the detection multiplied by the threshold, where the detection is a binary recording.), and the blue line denotes the peak(s) in the upper envelope that exceeds another threshold. The significance of these reference lines and further details on finding tissue boundaries using these waveforms are discussed in the next sub-sections.

Due to the processing and display, the frame rate (*i.e.,* how often a new block of waveforms is captured) is significantly slower compared to the blind mode. Since in processing, non-valid waveforms are discarded, the time to capture 500 valid data sets (*e.g.,* waveform, force, acceleration, and timestamps) varies. On average, it takes about 36 seconds to capture 500 data sets, which rounds to approximately 14Hz.

*Blind Mode*

Once the user is familiar with using the probe, blind mode can be used for an improved frame rate and thus overall more data in a given time, thus making experiments shorter and easier. As shown in FIG. 36, blind mode lacks the preprocessing and displaying process 620.

In the tested blind mode, typically 700 samples per trial were captured, which took approximately 14 seconds, thus a frequency of 50 Hz, which is much higher than the 14 Hz achieved in visual feedback mode.

*Processing of Ultrasonic Signal*

Medical ultrasound typically assumes a fixed speed of sound in the human body. With this assumption, one can calculate the distance between the transducer and the boundary where sound is echoed by measuring the time lapse between the trigger and the received echo. However, due to noise, multiple reflection sites, non-direct relationship between the wave amplitude and the type of boundary it is associated with, and a one-dimensional narrow field of view, it can be difficult to locate the bones in the limb using the raw measured signal. In particular, to detect the bone location using a single frame of waveform, the probe axis can be aligned with the bone whose location is unknown to the user. Therefore, in order to make the bone location detection procedure more user friendly, data driven, and more automatic, here a histogram and edge based method were applied.

In indentation experiments, in addition to the bone boundary, one can also use a particularly reliable echo site of the tissue-to-skin boundary on the opposing side of the limb. This is, when the ultrasonic wave enters the limb, goes through the limb, and echoed to the transducer from the boundary between the tissue and the skin.

*General Waveform Processing*

On the pulser/receiver side, the waveforms are bandpass filtered and amplified. In post processing on the PC, the waveforms are processed in the same way as in the visual feedback mode. A 128th order bandpass FIR filter in the shape of a Hamming window with boundaries at 0.9 and 1.1 MHz is used to filter the waveform. Although the pulser/receiver has a built in filter, it is often in a wide bandwidth. Further filtering in post processing may not be necessary but can remove additional noise.

The upper envelope is then abstracted from the filtered waveform to make thresholding easier and remove ambiguity in peak detection. In the filtered waveform, the exact location of peaks may vary by one or more cycles within the echoed wave, even when the overall shape matches. A cycle here refers to one going up and down in the raw ultrasound signals. At a boundary when the ultrasound wave echoes, there are typically multiple cycles in the echo. The envelope returns can provide a more consistent result for similar waveforms.

*Processing method for indentation experiments*

For in-vivo indentation experiments, at the beginning, the furthest edge is recorded and used as depth. In processing a new waveform, the edge that is the closest to the previous depth is recorded to be the new depth. This is because, in indentation experiments, movements are made significantly slower than 50 Hz, and thus, by using the previous value, a much more accurate displacement can be obtained.

For the tissue-mimicking silicone gel indentation experiments, the echo signal is typically clean and the wave with the greatest amplitude is returned at the hard surface boundary. Thus, in indentation experiment waveform processing, the depth is simply determined by the peak of the waveform of the greatest amplitude.

*A Histogram and Edge Based Method for Tissue Boundaries Detection*

For tissue-boundary detection, the probe was used to sweep across the limb, through which process a cross-sectional view was obtained. In this process, a series of 700 waveforms were recorded. The waveforms were processed as described above, and two plots were generated for determination of the bone locations.

For skin-to-bone depth detection, all locations in the upper envelope where values exceed a threshold are saved in a *detection* array, in the form of a binary matrix (See for example lines 638 and 640 in FIG. 37B). Moreover, peaks in the upper envelope which exceed a certain threshold (not necessarily the same threshold as the one used for detection) are also saved in an *edges* array (See for example line 642 in FIG. 37B). FIG. 38A shows an example of accumulated detections, and FIG. 38B shown an example of a histogram of the edges.

FIG. 38A indicates where the significant echoes are as the probe sweeps across the cross-section of the limb. The peaks in this plot represent places where it is statistically likely to have substances that cause significant reflection of sound waves. The example in FIG. 38A is typical for lower limb tissue boundaries detection. In this particular trial, the block around 100 mm corresponds to the wave which echoes at the tissue-to-skin boundary where the sound wave propagates out of the limb, the blocks at 40 to 50 mm and 70 to 80 mm are reflections from the tibia and fibula, respectively, and the blocks at 20 to 30 mm are artifacts.

From FIG. 38A one can determine that tibia and fibula are approximately 40 to 50 mm, and 70 to 80 mm from the probe, respectively. Having the window in mind, one can look at the histogram of the edges shown in FIG. 38B. As the probe 'sweeps' across a particular bone, the distance is the shortest when it directly faces the bone. Therefore, in a block in a histogram, it can be considered that the most likely distance is the shortest detected distance, *i.e.,* the highest values on the left side of a block. In the example in FIG. 38B, for the block in the window of 40 to 50 mm, 43.13 mm can accordingly be chosen, and in the block in the window of 70 to 80 mm, 70.37 mm can be chosen. These are the peaks in the respective blocks located on the left side. Statistically the peaks represent the most likely edges within the window that are the shortest. Recall that edges as shown in FIG. 37B with line 638, are the peaks in the waveforms, and the time lapse (that is converted into distances in these plots) is fundamentally how ultrasound detects acoustic boundaries, or in this application, the distance from the location of probing on the skin to the respective bones inside of the limbs.

*Self- Weight Compensation*

In indentation experiments, force-depth history is measured. Here, the force is exerted on the skin by the probe head. However, the weight of the probe adds additional force to the force sensor as it determines the force from the probe to the skin in indentation experiments. Accelerometers are used to determine the tilt angle of the probe to compensate for this effect.

*Sensor Calibration*

The force sensor and the accelerometer can both require calibration. Although the SingleTact force sensor comes with a calibration PCB and exhibits a linear behavior to force, in this assembly, the rubber pad in pre-load caused additional non-linearity. Thus, the force sensor was calibrated after assembly of the probe. The force sensor was calibrated with calibration weights. Although this particular force sensor showed a significantly lower repeatability rate than desired, it can be easily improved in next generation prototypes by using a load cell instead.

Gravity was used as reference for the calibration of the accelerometer. The recorded raw data are listed in Table 6.

**Table 6: Accelerometer Raw Data**

| | Maximum reading [V] at +*g* | Minimum reading [V] at -*g* |
|---|---|---|
| *x* | 2.1457 | 1.3539 |
| *y* | 2.1114 | 1.3148 |
| *z* | 2.2581 | 1.3832 |

*Self-Weight Compensation*

With a 3-axis accelerometer, tilt can be determined with improved sensitivity and accuracy combining x, y, and z signals. Pitch, roll, and tilt are calculated in the following equations.

$$\alpha = \arctan\left(\frac{x}{\sqrt{y^2 + z^2}}\right)$$
$$\beta = \arctan\left(\frac{y}{\sqrt{x^2 + z^2}}\right) \qquad \textbf{31}$$
$$\gamma = \arctan\left(\frac{z}{g}\right)$$

Where *x*, y, *z* are the accelerometer readings as labeled, *g* is the gravity constant, and $\alpha$, $\beta$, $\gamma$ are the pitch, roll, tilt respectively (FIG. 39).

The angle between handle of the probe and the direction perpendicular to contact surface is $\beta$, as shown in FIG. 40. The additional force by the probe weight on the force sensor to subtract is then given by:

$$w_c = \begin{cases} w_2 \cos(\beta) & 0 \le \beta < \frac{\pi}{2} \\ w_1 \cos(\beta) & \frac{\pi}{2} \le \beta < \pi \end{cases} \qquad \textbf{32}$$

where $w_c$ is the weight to compensate, $w_1$ is the head weight (from the probe head to the force sensor), $w_2$ is the tail weight (from the force sensor to the tail of the probe).

*Cost Breakdown*

With an effort to further miniaturize the system, the parts of a system can include an ultrasonic transducer, a reliable force sensor, an accelerometer, a PCB with a microcontroller, a battery, and its assembly. Table 7 below shows the breakdown of the estimated cost of each part. Overall, the system can cost approximately $1200, as opposed to about $20,000 to $75,000 for an ultrasound imaging system, and $400,000+ for an MRI scanner.

With the system described in this study, instead of a PCB and a battery, a PicoScope ($139.00), a Pulser/Receiver (approx. $3000), and an Arduino Nano (approx. $4) were used; instead of a more reliable force sensor, such as a load cell, a force sensing capacitor (approx. $74) was included. The total cost of the example, prototype system as described in this study is approximately $3707, which is significantly below the cost for ultrasound imaging systems and MRI machines.

**Table 7: Cost Breakdown of the System after Further Miniaturization**

| Component | Cost estimate |
|---|---|
| Force sensor | $400 |
| Ultrasonic transducer | $400 |
| Accelerometer | $20 |
| PCB with microcontroller | $250 |
| Battery | $60 |
| Housing/assembly | $70 |
| Total | $1200 |

*Experiments*

To verify that the system design meets the objectives of an accurate tissue boundary and indentation detection using low profile ultrasonic transducers, in-vivo experiments were performed and results were compared to results from MRI scans and Digital Image Correlation (DIC). To understand the limits of ultrasonic sensing for this application, the tissue boundary detection results were also compared to those obtained using a commercial ultrasound imaging system. Furthermore, a phantom staircase study for both depth and indentation were performed to reduce the inaccuracy introduced by human factors in in-vivo experiments. Specifically, two sets of four experiments were performed: depth and indentation experiments in a phantom staircase, and skin-to-bone distance and indentation experiments in-vivo.

*Depth Sensing Verification by a Phantom Staircase*

To test the accuracy of depth measurement as a result of a single ultrasonic transducer, a 19.7cm x 9.4cm x 5.6cm staircase was built, filled with silicone gel (SYLGARD 527 A&B Dow Coming, MI, USA, A:B ratio 1:1, cured in incubator set to 60 °C for four hours) which exhibits similar mechanical properties as soft tissue, as shown in FIG. 41. The walls of the staircase are made of Acrylic, and the steps are 3D printed using VeroClear material (Stratasys). The steps were designed to have 10 mm increment; and the staircase is filled 4 mm (as manually measured by a ruler) from the top such that the distances from the phantom (gel) surface to the bottom of the steps are 6 mm, 16 mm, 26 mm, 36 mm, 46 mm, 56 mm, 66 mm, 76 mm, and 86 mm. This distance was manually verified by a ruler from the outside of the transparent Acrylic wall.

Preliminary data were collected to determine the speed of sound in the phantom. In each step from 16 to 86 mm, the time lapse was manually measured in a digital oscilloscope. Manual measurements were made with the foreknowledge of the fixed step incremental distance. The speed of sound was calculated by linearly fitting to the truth depths using a least squares method. The results of the measurements and fitting is plotted in FIG. 42. The speed of sound was found to be 1007 m/s.

Additional data was collected from the 16mm to 86mm steps to determine the accuracy and repeatability of depth detection. In each step, 4 trails were conducted, and the measured distances were compared to the true depths.

*Indentation Verification by stereo-imaging in Phantom*

To verify depth change detection in indentation experiments, experiments were conducted with the phantom staircase shown in FIG. 41. A group of four experiments, each with four trials, were conducted at steps of depths 26, 46, and 76 mm with a repeated trial at depth 46 mm. These are representative of shallow, medium, and deep steps. Two cameras were used to capture pairs of synchronized pictures every five seconds. The probe assembly and the limb were labeled with black dots of 5 mm diameter for image processing purposes, as shown in FIGS. 43A and 43B. The indentation experiment uses procedure as previously described, and the stereo-imaging system was used to measure the 3D displacement of the indenter at each step, to be used for validation of the ultrasound measurement accuracy.

The stereo-imaging algorithm takes the dot groups from left and right cameras, and computes the 3D position of the centroid of each dot in each frame. The position of the probe head is then calculated based on the dots' positions and the distance from the dots to the probe head. Finally, the indentation is calculated by finding the projection of translation of the probe head position with respect to the direction of the probe handle in the first set of images:

$$d = \mathbf{n} \cdot \mathbf{ab} \qquad \mathbf{33}$$

where *d* is the indentation distance, **n** is the normalized vector pointing in the direction of the probe handle, **a** is the position of the probe head in this frame, and **b** is the position of the probe head in the reference frame.

*Indentation Verification by DIC in-vivo*

At the same location in a lower limb in a healthy individual, four trials of indentation experiments were performed with the same setup as described with respect to the phantom indentation verification experiments. The views of the cameras of the experiment are shown in FIGS. 44A and 44B.

*A Comparison with a Commercial Ultrasound System with Respect to MRI in-vivo*

To test the accuracy of skin-to-bone distance measurement in-vivo, we compared the accuracy of results obtained by method as described in previous section in five distinctive locations on the lower limb of a healthy individual, and compared results to that from a commercial ultrasound imaging instrument using MRI scan measurements as ground truth.

Five MRI markers around the center of the limb distributed around the limb are attached to the limb as the MRI scan is taken, as shown in FIGS. 45A-F. The locations of the MRI markers were then marked with temporary tattoo ink to be consistent in the following experimentations.

The distances from each marker location to tibia and fibula are measured from the MRI scan by manually selecting the line of measurement and converting to distance in the physical world, as shown in the lines of FIGS. 45B-F. The measurement data are shown in Table 8. In the following experiments, the distances obtained from the MRI scan as shown in Table 8 are considered the ground truth. The system measures the shortest distances from the location of probing to the bones, *i.e.* tibia and fibula. The longest distance to each bone are measured for the purpose of interpreting the data in cases such as when the distance is too short for the transducer of selection or when the results are not obvious.

**Table 8: Measurements obtained from the system.**

| Marker Number | 1 | 2 | 3 | 4 | 5 |
|---|---|---|---|---|---|
| Shortest distance to tibia from marker [mm] | 12 | 28.8 | 64.2 | 73.4 | 46 |
| Longest Distance to Tibia from Marker [mm] | 28 | 63 | 75.5 | 80.77 | 56.5 |
| Shortest Distance to Fibula from Marker [mm] | 43 | 63 | 75.8 | 41.23 | 24.1 |
| Longest Distance to Fibula from Marker [mm] | 51.3 | 66 | 84.7 | 52 | 30.1 |

From each marker location, the distance from the marker to tibia and fibula are measured by manually clicking on the MRI image and converting the distance on the image to distance in the limb. Only distances larger than 20mm were considered for data processing. Following the previously described procedure for tissue boundary detection, four trials were conducted for each marker location. The shortest distances to tibia and fibula were processed.

To determine how the performance compares to commercial ultrasound machines, measurements were also taken using a commercial ultrasound system (Telemed SmartUs EXT-1M), in a similar manner: at each marker location, the probe was slowly moved around the limb in a sweeping motion, and a sequence of images was recorded. Due to unavailability of 1 MHz option in the commercial ultrasound machine, the 5 MHz option was used.

*Results*

*Depth Sensing Validation using a Phantom Staircase*

With respect to in-vivo measurements, depth detection in the phantom is relatively straight-forward, because there are not multiple tissue boundaries. FIG. 46 shows the depth measured using the single element transducer device against the true depth, for each of the steps. The overall mean error was 0.36 mm and the standard deviation was 1.1 mm. The larger standard deviation observed in the 86 mm step might be a result of interference due the echo from the corners of the staircase. The error distribution is plotted in figure FIG. 47 and detailed in Table 9.

These results confirm that the prototype system is suitable for depth sensing in the range of interest. Since the speed of sound is 1007 m/s in the phantom and 1540 m/s in soft tissue, the phantom results are approximately equivalent to a 24 mm to 131 mm range in soft tissue.

**Table 9: Phantom staircase depth detection experiment results**

| Metric | Magnitude [mm] |
|---|---|
| Mean error | 0.359 |
| Maximum absolute error | 2.300 |
| Minimum absolute error | 0.000 |
| Standard deviation | 1.109 |

*Indentation Validation using Stereo-Imaging in Phantom*

The depth and displacement results obtained from the prototype system were compared to those obtained using the stereo-imaging system, as shown in FIG. 48 and Table 10. In these trials, the results showed a similar error mean (-0.1289 mm) and standard deviation (1.1086 mm) as in the depth sensing results. This result confirms that the system is sensitive enough for indentation tests in phantom.

**Table 10: Phantom staircase indentation experiment results**

| Metric | Magnitude [mm] |
|---|---|
| Mean error | 0.129 |
| Maximum absolute error | 3.352 |
| Minimum absolute error | 0.112 |
| Standard error deviation | 1.383 |

FIG. 49 shows an example of results from an indentation experiment. As indentation (displacement) increases, the depth decreases, and the force at the probe tip increases. As previously described, the force measurement was relatively inaccurate due to probe design. However, the data demonstrates the feasibility of combining force and displacement sensing during an indentation test using the described apparatus. The title angle, calculated from acceleration data, shows that throughout the indentation procedure, the probe orientation was kept relatively constant.

*Indentation Validation using Stereo-Imaging in-vivo*

The indentation errors with respect to stereo-imaging measurements are shown in FIG. 50 and Table 11. The mean and standard deviation errors were -0.18 mm and 0.47 mm, respectively. Therefore, the system is appropriate for indentation experiment for prosthetic socket design. Phantom and in-vivo indentation results showed similar accuracy.

Table 11: **In-vivo indentation experiment results**

| Metric | Magnitude [mm] |
|---|---|
| Mean error | -0.181 |

(continued)

| Metric | Magnitude [mm] |
|---|---|
| Maximum absolute error | 1.358 |
| Minimum absolute error | 0.011 |
| Standard error deviation | 0.472 |

*Comparison to a Commercial Ultrasound System*

The depth results obtained from the prototype system were compared to those obtained from MRI, as shown in FIG. 51A. The depth results obtained from a commercial ultrasound system were also compared to those obtained from MRI, as shown in FIG. 51B.

The mean and standard deviation errors with respect to MRI measurement results are listed in Table 12, and the error histograms are shown in FIGS. 52A-B. The prototype system showed comparable results to those obtained using commercial ultrasound system.

**Table 12: Evaluation metrics for the prototype ultrasound-force probe, and a clinical US system, compared to MRI**

| Metric | Prototype probe data [mm] | Clinical US probe data [mm] |
|---|---|---|
| Mean error | 0.226 | 0.477 |
| Maximum absolute error | 4.840 | 5.800 |
| Minimum absolute error | 0 | 0.400 |
| Standard error deviation | 2.218 | 3.284 |

*Overall Comparison*

Overall, the system demonstrated relatively stable performance both in phantom and in-vivo. FIGS. 53 and 54A-D recapitulate the results described above and show the error distributions of the depth and indentation experiments. As shown in FIG. 54A-D, the results show a similar distribution of error that centers on zero and has a standard deviation of less than 2 mm.

*Discussion*

The results presented here demonstrate that a single ultrasonic transducer is suitable for skin-to-bone distance detection in-vivo in limbs, and that a system using a single ultrasonic transducer, a force sensor, and other correction sensors is suitable for both skin-to-bone depth measurement and indentation experiments.

The ultrasound-force system can be further modified. For example, the pulse repetition rate can be optimized. In the prototype system, the pulse repetition rate was simply set to maximum to avoid overlapping echo. As communication bandwidth improves, pulse repetition rate can optimized for a better temporal resolution. Such ultrasound-force systems can include probes of varying types for improved near-skin bone detection. In the prototype system, the transducer is optimal for detections of distances larger than 20 mm. As discussed above, one or more transducers can be added to the assembly. A separate probe assembly can be used for an improved near-skin detection. Additionally, all components can be centralized to increase frame rate. In the prototype system, the frame rate is limited by communication bandwidth, which is limited due to multiple devices in the system. One way to improve frame rate is to centralize all components such that communication or on-board data storage is performed by a single micro-controller with minimum layers in the pipeline.

The ultrasound-force system can also be further miniaturized. For example, the system can become more compact and portable with a single PCB serving the roles of pusler/receiver, data acquisition system, on-board data logging system, and communication system to a PC, force sensor, and accelerometer. Speed of sound assumption errors can be minimized. As described above, accuracy can be improved by using a more accurate speed of sound. By looking at patient information, such as BMI, in combination with inference from echo close to the transducer, one can improve accuracy by a better estimation of the assembly of tissue types.

To make computer-aided biomechanical interface design more accessible, this study described a system that can a) detect the skin-to-bone depth and b) perform indentation experiments for tissue mechanical properties evaluation. Low profile sensing, including single element ultrasonic sensing, force sensing, and acceleration sensing, were included.

The hand-held apparatus in the system is light-weight, compact, and affordable, while also demonstrating comparable results to a commercial ultrasound imaging system.

### 5. Flow induced mechanical perturbation

Some methods for mechanically perturbing a material for mechanical property analysis rely on contact. An example of such a method is indentation, whereby an indenter initiates contact with the material in order to deform it. Such contacting methods can be undesirable in some situations. For example, a material can be negatively affected by contact. For instance, the material may be sensitive such that contact creates discomfort or elicits changes in the material. As another example, a contacting mechanical perturbation system may obstruct the view of the mechanically perturbed site. For instance, in the case of indentation, the indenter system may obstruct an optical strain imaging system. Furthermore, a state of the material directly beneath the indenter may not be visible. As another example, analysis of an indentation experiment may require computational modeling techniques, such as finite element analysis. If sliding between the mechanical perturbation device and the material region of interest occurs, the sliding contact interface may be represented in computational models for accuracy. Appropriate modeling of contact can be challenging and is computationally intensive. Furthermore, computational modeling of the sliding/friction conditions creates and additional set of unknown parameters requiring investigation, especially if zero friction or infinite friction assumptions are not realistic.

An alternative methodology to induce a mechanical perturbation similar to an indentation is provided. Devices and methods of mechanically perturbating a tissue that do not contact the material region and do not obstruct the view at the mechanical perturbation site are described.

A flow based mechanical perturbation is proposed here whereby a medium exits a nozzle creating a focused jet capable of locally distorting a material. The medium can be a fluid, including gas or liquid. By adjusting features of the nozzle (*e.g.,* nozzle geometry, pressure, flow, and time varying patterns), different mechanical perturbations can be effected. The medium used for the flow may also be altered during the course of the experiment with a desired temporal variation. Medium properties may be altered over time (*e.g*., density, color, temperature, and opacity).

Fiducial markers can also be employed in the medium (*e.g*., particles allowing for methods such as particle image velocimetry). Constant or non-constant flow jets (*e.g.,* pulsatile, vibrating, or other types of time varying profiles) may be used and can be either laminar or turbulent in nature. Furthermore, a focal distance of the jet and shape of the jet may be held adjustable, held constant, or may be perturbed in time.

An example of a flow-based perturbator 710 is shown in FIG. 55. The flow-based perturbator includes a nozzle end 712 that can be used to create a local tissue deformation 720 of the skin and underlying soft tissues of biological body segment 730, shown in FIG. 55 as being a human arm.

Such flow-based devices and methods offer an ability to create a local deformation without requiring contact between the device and the material being measured. By using a transparent medium (*e.g*., air) to apply the mechanical perturbation, the entire deformed site (including regions which would be located directly under an indenter tip in the case of indentation) can be studied using optical strain imaging methods, such as digital image correlation (DIC).

Analysis of the mechanical properties from a flow experiment can rely on quasi-static or dynamic analysis. Closed-form solutions may be employed or computational modelling approaches featuring fluid-structure interactions may be employed.

A response to an applied flow may be time-varying in nature. As such, analysis may also focus on propagating features, such as waves, including analysis of observed wave lengths (*e.g.,* spatial wave lengths), wave attenuations, wave modes, wave mode conversions, and harmonic wave analysis.

Measurements of the mechanical perturbation may focus on the material region, for example, using optical or non-optical imaging methods. However, measurements and analyses may also focus on the medium that is flowing and the characteristics of the flow as it encounters the material region, for example, particular flow characteristics may depend on the mechanical properties of the material it mechanically perturbs.

The flow may be moved across a region, and multiple jets may be combined for mechanical property investigation.

### 6. Hyperelastography: Elastography for finite strain mechanical property analysis

*Linear elastography techniques*

In elastography, mechanical properties are determined from vibrations and wave propagations. The local spatial wavelength and wave attenuation inform local elastic and viscoelastic parameters. In Magnetic Resonance Elastography (MRE), wave motions are measured using phase contrast MRI methods. These provide 3D displacement data. Direct inversion methods have been proposed to convert the time varying displacement data to so-called elastograms, which are images representing mechanical properties, such as local shear moduli. Elastography methods focus on small displacements and, therefore, only provide linear elastic (*e.g.*, infinitesimal strain) mechanical property estimates. Since soft tissue is non-linear elastic and stiffness is a function of deformation, conventional elastography techniques, at best, inform a current or initial stiffness state and do not inform large-strain and non-linear elastic behavior.

In elastography, spatially varying maps of constitutive parameters are formed through analysis of the equation of balance of linear mechanical momentum. In the absence of body forces, this is written as:

$$\rho \frac{\partial^2 \mathbf{u}}{\partial t^2} = \boldsymbol{\nabla} \cdot \boldsymbol{\sigma} \qquad \mathbf{34}$$

where $\rho$ is the material density, **u** the displacement vector (and $\frac{\partial^2 \mathbf{u}}{\partial t^2}$ represents the acceleration vector), and σ the Cauchy stress tensor. In linear elastography, the material is assumed to be linear elastic (and/or linearly viscoelastic) as described by Hooke's law. Hooke's law for a linear elastic and isotropic material is given by:

$$\boldsymbol{\sigma} = \lambda \mathrm{tr}(\boldsymbol{\varepsilon})\mathbf{I} + 2\mu\boldsymbol{\varepsilon} = \lambda(\nabla \cdot \mathbf{u})\mathbf{I} + \mu(\boldsymbol{\nabla}\mathbf{u} + (\boldsymbol{\nabla}\mathbf{u})^{\mathrm{T}}) \qquad \mathbf{35}$$

where σ is the Cauchy stress, ε represents the linear strain tensor:

$$\boldsymbol{\varepsilon} = \frac{1}{2}(\mathbf{F}^{\mathrm{T}} + \mathbf{F}) - \mathbf{I} = \frac{1}{2}((\boldsymbol{\nabla}\mathbf{u})^{\mathrm{T}} + \boldsymbol{\nabla}\mathbf{u}) \qquad \mathbf{36}$$

The scalars μ and λ are material parameters called the Lamé parameters. The material bulk modulus can be derived from:

$$\kappa = \lambda + \frac{2}{3}\mu \qquad \mathbf{37}$$

For infinitesimal strains the Cauchy stress is equivalent to the Kirchoff stress such that $\boldsymbol{\sigma} \approx \boldsymbol{\tau} = \frac{\partial \Psi}{\partial \epsilon}$ allowing expression of the Cauchy stress as:

$$\boldsymbol{\sigma} = \lambda \mathrm{tr}(\boldsymbol{\varepsilon})\mathbf{I} + 2\mu\boldsymbol{\varepsilon} = \lambda(\nabla \cdot \mathbf{u})\mathbf{I} + \mu(\boldsymbol{\nabla}\mathbf{u} + (\boldsymbol{\nabla}\mathbf{u})^{\mathrm{T}}) \qquad \mathbf{38}$$

The material elasticity tensor for an isotropic linear elastic material is defined as:

$$\mathbb{C} = \lambda\mathbf{I}\otimes\mathbf{I} + \mathbf{2}\mu\mathbf{I}\overline{\underline{\otimes}}\mathbf{I} \qquad \mathbf{39}$$

Thus, the dyadic product notation can be introduced: $(\mathbf{M}\otimes\mathbf{N})_{ijkl} = M_{ij}N_{kl}$, $(\mathbf{M}\underline{\otimes}\mathbf{N})_{ijkl} = M_{ik}N_{jl}$ and $\left(\mathbf{M}\overline{\underline{\otimes}}\mathbf{N}\right)_{ijkl} = \frac{1}{2}\left(M_{ik}N_{jl} + M_{il}N_{jk}\right)$, for arbitrary second order tensors **M** and **N.**

By combining the equation of linear momentum with Hooke's law, one obtains the following partial differential equation (PDE):

$$\rho \frac{\partial^2 \mathbf{u}}{\partial t^2} = \mu\nabla^2\mathbf{u} + (\lambda + \mu)\,\nabla(\nabla \cdot \mathbf{u}) \qquad \mathbf{40}$$

In Magnetic Resonance Elastography (MRE), the PDE can be solved by assuming linear elasticity, isotropy, local homogeneity, as well as state harmonic oscillations (the latter induced by an external actuator). Solving the system leads to estimates of the local shear modulus μ, which may be complex to allow for viscoelasticity, on a voxel by voxel basis.

In ultrasound elastography (USE), the PDE is often approached differently. The displacement **u** is decomposed into a longitudinal component $\mathbf{u_L}$ and a shear component $\mathbf{u_S}$, and the PDE is rewritten as:

$$\frac{\partial^2 \mathbf{u_S}}{\partial t^2} - \frac{1}{{c_S}^2} \nabla^2 \mathbf{u_S} = 0 \qquad \mathbf{41}$$

where $\boldsymbol{c_S}$ denotes the shear wave propagation velocity:

$$c_S = \sqrt{\frac{\mu}{\rho}} \qquad \mathbf{42}$$

Ultrasound based assessment of local shear wave velocities therefore allows for the computation of the local shear modulus.

Both MRE and USE offer 3D maps of local estimates of the material shear modulus. Although elastography methods offer a per-voxel shear modulus, such data cannot be directly used in computational modelling based design as it does not provide for large strain formulations.

*Hyperelastography*

Although inverse FEA offers a means to evaluate large strain and non-linear elastic formulations, it does not easily offer a means to derive spatially varying maps of constitutive behavior. In contrast, MRE can be used to obtain maps of spatially varying constitutive parameters. However, elastography techniques only offer estimates of linear elastic parameters. Since linear elasticity is not suitable for large strain analysis and does not capture the known non-linear elastic behavior of soft tissue (see FIG. 56), elastography findings are not directly usable for large strain applications, such as computational-modelling-based prosthetic socket design.

A hyperelastography approach is provided to overcome these shortcomings. Hyperelastography is a hybrid approach whereby large strain hyperelastic formulations are used in a framework that combines finite element analysis and elastography techniques.

Similar to standard elastography, one may start with the balance of mechanical momentum (Eqn. 34). However, the Hookean Cauchy stress can be substituted by a general hyperelastic Cauchy stress form, leading to:

$$\rho \frac{\partial^2 \mathbf{u}}{\partial t^2} = \nabla \cdot \boldsymbol{\sigma} = \nabla \cdot \left( J^{-1} \mathbf{F} \frac{\partial \Psi}{\partial \mathbf{E}} \mathbf{F}^{\mathrm{T}} \right) \qquad \mathbf{43}$$

Here $\Psi$ may represent any suitable hyperelastic strain energy function.

In non-linear continuum mechanics, the constitutive behavior of materials is represented by strain energy density functions. Many such functions have been proposed, but here, the discussion focusses on the following coupled hyperelastic formulation:

$$\Psi = \frac{c}{2m}\left(\mathrm{tr}\left(\mathbf{E}^{(m)}\right) - \mathrm{tr}\left(\mathbf{E}^{(-m)}\right)\right) + \frac{\kappa'}{2}(J-1)^2 = \frac{c}{m^2}\sum_{i=1}^{3}\left({\lambda_i}^m + {\lambda_i}^{-m} - 2\right) + \frac{\kappa'}{2}(J-1)^2 \qquad \mathbf{44}$$

with c and $\kappa$' representing shear and bulk modulus like parameters with unites of stress. The tensor $\mathbf{E}^{(m)}$ is a finite (Lagrangian) strain tensor of the class:

$$\mathbf{E}^{(m)} = \begin{cases} m \neq 0 & \frac{1}{m}(\mathbf{U}^{\mathrm{m}} - \mathbf{I}) \\ m = 0 & \ln(\mathbf{U}) \end{cases} \qquad \mathbf{45}$$

with **U** the right stretch tensor which can be related to the right-Cauchy-Green tensor **C** and the deformation gradient tensor **F** as:

$$\mathbf{C} = \mathbf{U}^2 = \mathbf{F}^{\mathrm{T}}\mathbf{F} \qquad \mathbf{46}$$

The eigenvalues or principal components of **C** are $C_i = \lambda_i^2$ (with $i$ = 1, 2, 3), *i.e.,* the squared principal stretches.

The scalar $J = \det(F) = \lambda_1\lambda_2\lambda_3$ is called the Jacobian or volume ratio.

Derivatives of the strain energy function with respect to deformation measures yields stress measures, for example:

$$\mathbf{S} = \frac{\partial\Psi}{\partial\mathbf{E}}, \mathbf{P} = \frac{\partial\Psi}{\partial\mathbf{F}} \text{ and } \boldsymbol{\tau} = \frac{\partial\Psi}{\partial\epsilon} \quad \mathbf{47}$$

where P, S and $\tau$ represent the Piola-Kirchoff stress tensor, the second Piola-Kirchoff stress tensor and the Kirchoff stress tensor respectively. The Cauchy stress can be evaluated using:

$$\boldsymbol{\sigma} = J^{-1}\mathbf{F}\left(\frac{\partial\Psi}{\partial\mathbf{F}}\right)^{\mathrm{T}} = J^{-1}\mathbf{F}\mathbf{P}^{\mathrm{T}} = J^{-1}\mathbf{F}\mathbf{S}\mathbf{F}^{\mathrm{T}} = J^{-1}\boldsymbol{\tau} \quad \mathbf{48}$$

The material stiffness or elasticity tensor $\mathbb{C}$ can be derived from the strain energy density function $\Psi$ using:

$$\mathbb{C} = \frac{\partial\mathbf{S}}{\partial\mathbf{E}} = \frac{\partial^2\Psi}{\partial\mathbf{E}^2} \quad \mathbf{49}$$

where E = $\mathbf{E}^{(2)}$ is the Green-Lagrange strain tensor.

Since the hyperelastic formulations reduce to Hooke's law for infinitesimal deformations an initial shear modulus $\mu_0$ can be computed which is equivalent to the Hookean shear modulus $\mu$. The initial slope due to the initial modulus is shown at $\lambda = 1$ in FIG. 56.

The initial shear modulus of a hyperelastic formulation can be derived from its initial slope. For the above formulation, the simple relationship$\mu = \mu_0 = c$ can be obtained. Therefore, by performing elastography in the undeformed state, one can directly provide a spatially varying map of the hyperelastic parameter c, leading to c(x), where x denotes a position vector. For the above form, this leaves the spatially varying hyperelastic parameter m(x) unknown. However, next, one or more experiments can be conducted, featuring large strain mechanical perturbations, for example, indentations at different levels. In these deformed states the elastography experiment can be repeated. Next the spatially varying hyperelastic parameters *c*(x) and *m*(x) can be derived, since *m*(x) locally determines the degree of stiffness enhancement in response to deformation. Two types of approaches can be followed, one relying on large strain measurements, and one relying on computational modelling (although combinations are also possible).

If analysis is based on large strain measurements (*e.g.,* using SPAMM tagged MRI), and if all the induced large deformations are known, and the apparent initial and final shear moduli are derived from elastography, fitting of the hyperelastic form is similar to locally fitting the dashed line shown in FIG. 56 to the multiple slope (apparent shear moduli) assessments.

Alternatively, inverse FEA can be employed. Using FEA, the current stiffness tensor, at any point and at any desired state of deformation, can be exported. These can be compared to the apparent shear moduli observed in elastography experiments. Therefore, it is possible to formulate an objective function allowing for the minimization of the difference between the simulated and experimental: 1) initial shear moduli, 2) final or intermediate apparent shear moduli, and 3) the deformation and load boundary conditions.

The inverse FEA optimization approach may be staggered such that the spatially varying *c*(x) (derived from the elastography experiment in the initial state) are held constant at first while a single *m*, which is not spatially varying, is determined to best match the overall experimental force and deformation boundary conditions. The results of this first step are then refined in subsequent optimization steps, whereby *m*(x) is allowed to vary spatially. This process may also be incremental in complexity, for example, by first solving for 2 *m* parameters, then 3 *m* parameters, up to the full spatially varying field. The grouping of the regions where m is equivalent may be informed by similar degrees of stiffness enhancement, such as by studying the difference between shear moduli in the initial and subsequent elastography experiments. Additional optimization steps may allow the field c(x) to be adjusted to provide an overall best match to the experimental data.

As the above shows, using large strain experiments and multiple elastography measurements allows for the computation of spatially varying hyperelastic parameters. This hyperelastography technique can be expanded to include viscoelasticity and anisotropy. For instance, local fiber directions may be included in the finite element model, *e.g.,* as derived from diffusion tensor MRI. Such fiber directions can be incorporated in the MRE inversion to yield initial constants for anisotropic Hookean forms (e.g., transverse isotropy), which can inform initial moduli of anisotropic large strain formulations.

Even for initially isotropic materials, anisotropic elastography inversion techniques may be required to cope with deformation induced anisotropy. In the large strain deformed state non-linear elasticity induces changes in the degrees of anisotropy, since deformation induces stiffness changes. As a consequence, isotropic materials may be deformed to become orthotropic, while transversely isotropic materials may become triclinic under large deformations. However, with knowledge of the initial fiber directions and other structural directions (none in the case of isotropy, and measureable from diffusion tensor MRI for muscle tissue), and with knowledge of the large deformations (derivable from inverse FEA or from dedicated imaging methods), the set of material constants to be solved can be locally reduced. For instance, the local eigen-decomposition of the elasticity tensor, as per the Kelvin mapping, can be employed.

The hyperelastography technique can also be employed to determine an unloaded state of tissue where it is unknown. Many tissues in the human body are naturally in a pre-loaded state. Unknown spatially varying pre-stresses and pre-strains therefore exist. Resolving the initial state locally for a material is challenging. In FIG. 56, for a hyperelastic material of this form, the initial and unloaded state presents with the lowest stiffness (resistance to increments of tensile or compressive load), *i.e.,* the initial unloaded state presents as the minimum stiffness state. Therefore, if a material region is subjected to known tensile and compressive strains (with respect to some chosen reference frame), while multiple elastography data sets are simultaneously recorded, it is possible to determine a minimum stiffness state. Once a configuration is identified for which a material region presents with the minimum stiffness, this configuration may serve, for that region, as the new initial or reference configuration. Once identified, this configuration can be used to retrospectively adjust the recorded deformations to represent deformations with respect to this configuration as the initial state.

FIGS. 57A-B show a schematic of an ultrasound hyperelastography device 800 that includes a guide 810 and an ultrasound probe 820. The ultrasound hyperalstography device can be used for indentation with simultaneous ultrasound-based elastography imaging. As illustrated in FIG. 57A, the device includes a stand 812; however, the device can also be a handheld device. Application of the device 800 is shown in FIG. 57B, with the ultrasound probe 820 applying a deformation to a biological body segment 830. The device can be used to perform elastography in an initial (e.g., undeformed) configuration and one or more deformed configurations. Stiffness enhancement can be observed due to the non-linear elastic and hyperelastic nature of tissue. Dedicated FEA models can then aide in the determination of hyperelastic constants.

FIGS. 58A-D demonstrate use of a handheld ultrasound-force probe 850, such as described in Section 4 herein, for hyperelastography measurements. FIG. 58A illustrates application of the probe 850 to a biological body segment 830 without deformation to record initial elastography data, an example of which is shown in FIG. 58B. The probe 850 can then be used to apply a deformation 860, as shown in FIG. 58C, to record elastography data pertaining to the deformed state, an example of which is shown in FIG. 58D.

### 7. Shape Imaging and Pressurization for Tissue Mechanical Property Analysis

Devices and methods for non-invasive imaging-based mechanical property assessment are provided. An example of an imaging device 900 is shown in FIG. 59. A biological body segment 902 or tissue region is placed within a structure 910, which as illustrated in FIG. 59, is a tank. The structure 910 includes a plurality of imaging devices 912, 914, 916. The device 910 of FIG. 59 is shown in cross-section, with imaging devices 912, 914, and 916 being disposed to capture images that provide a full field of view of the biological body segment 910. For example, the structure can include imaging devices 912, which are disposed about a perimeter of the structure 910 and are configured to capture images of the sides of the biological body segment 902. The structure 910 can further include at least one imaging device 914, which has a generally axial viewing angle relative to the perimeter. Optionally, additional imaging devices 916 can be included to provide additional viewing angles. While the structure 910 is shown as having fifteen imaging devices in one cross-section, additional imaging devices can be included about the perimeter of the structure in cross sections not illustrated in FIG. 59, and fewer or additional imaging devices can be included.

As illustrated in FIG. 59, the structure 910 is a tank, which can be filled with a fluid medium, such as liquid or gas. A seal 920 can be included, which seals about a portion 904 of the biological body segment 902, such that another portion 906 of the segment 902 is subject to pressures applied within the tank 910. A pressure within the tank can be altered by removing or adding fluid medium (e.g., by pumping). Pressure changes can thereby then provide the mechanical perturbation by causing associated shape changes of the biological body segment. The shape of the tissue region 906 at multiple states can be imaged based on the array of sensing elements, including imaging devices 912, 914, 916. The imaging devices can be cameras, ultrasound elements, or other non-invasive imaging elements, provided that the medium employed is functionally transparent with respect to the technique employed. Based on pressure control and/or measurement, and measurement of the shape changes to the body segment, one is able to compute the mechanical properties of the tissue region, *e.g.,* through the use of computational methods such as inverse finite element analysis.

Where some, or all, of the imaging devices are optical cameras, methods of obtaining an external geometry of the biological body segment in both undeformed and deformed states can be used, such as with Digital Image Correlation methods, as described in Section 1 herein. Information pertaining to internal geometry of the biological body segment can also be obtained, such as with cloaking methods, as described in Section 10 herein.

Where some, or all, of the imaging devices are ultrasound, both internal and external geometries of the biological body segment can be obtained, at both deformed and undeformed states. The ultrasound devices can also be configured to collect shear wave velocity data for hyperelastography analysis, as described in Section 6 herein.

An advantage of such devices is that a biological body segment can be quickly imaged since translation of the imaging devices relative to the segment is not required. Another advantage of such devices is that data pertaining to deformations and mechanical perturbations can be quickly obtained without requiring translation of a mechanical perturbator relative to the segment. Yet another advantage of such devices is that the application of pressurization can be less invasive than mechanical perturbations caused by an indenter. Furthermore, while an indenter can potentially obstruct a field of view of an imaging device during an indentation experiment, application of pressure by a fluid medium does not cause such obstructions. Where some or all of the imaging devices are ultrasound, the fluid medium can be one that permits propagation of ultrasound waves to enable imaging.

A device can optionally include imaging devices of varying types. For example, both optical cameras and ultrasound sensors can be included. With such devices, multiple data acquisition types can be combined for the formation of a model of the biological body segment being imaged. For example, DIC can be used to generate an external model of the biological body segment and US can be used to generate an internal model of the segment.

The application of pressurization can alternatively be combined with mechanical perturbators, such as flow-based perturbators as described in Section 5, and/or with other compressive load methods and devices, as described in the following section, Section 8.

## 8. Three-Dimensional Imaging of Musculoskeletal Tissue Under Compressive Loads

It is reported that 57% of persons with transtibial amputation suffer from moderate to severe pain when wearing a prosthetic limb. Improper fit of the prosthetic socket, the cup-like interface connecting a residual limb to a remainder of the prosthesis, can lead to several pain-causing pathologies including neuromas, inflammation, soft tis- sue calcifications, and pressure sores. A person with amputation may choose not to wear their prosthesis if it is not comfortable; thus, it plays a critical role in physical rehabilitation and subsequent future health outcomes. The current standard for prosthetic socket fabrication is plaster casting, a mostly subjective process performed by a prosthetist. Though this artisanal method can be effective in some instances, it is expensive, time consuming, and often requires several iterations in order to achieve a desirable fit. A quantitative, reproducible, and data-driven procedure for socket creation could have substantial clinical impact.

For a person with amputation using a prosthetic lower limb, both static and dynamic loads are maintained by transferring forces from the socket to the limb soft tissue. Therefore, biomechanical understanding of the tissues throughout the socket-limb interface is essential when trying to derive a comfortable socket design. There have been several advances in scanning of the residual limb and manipulating this data as input to computer-aided design/manufacturing (CAD/CAM) of prosthetic sockets. Nevertheless, most studies either (a) focus only on the external shape of the limb and do not take into account quantified internal tissue distributions or compliance data, which can be important for analyzing and simulating accurate loading conditions, or (b) use expensive scanning tools that are only available in specialized facilities. An attractive alternative that may address these limitations is musculoskeletal (MSK) ultrasound (US) imaging.

There have been many recent developments in application of US imaging to the MSK field due to its inherent advantages of real-time performance, high tissue resolution, relative speed, and accessibility as compared to other imaging modalities. For example, computed tomography (CT) exposes the patient to ionizing radiation, while magnetic resonance imaging (MRI) is not always possible, particularly in cases where the patient may have medical implants or combat injuries where metal shrapnel may be present. Alternatively, 3D US is a low-cost and widely available option for obtaining volumetric and diagnostically useful images, particularly in rehabilitation applications. Intensive training or radiation protection is not necessary for its use, and its hardware is portable, thus allowing for use at the bedside and making it more accessible in tertiary or more resource-constrained facilities.

In clinical applications of MSK imaging, it is often sufficient to achieve a reconstructed volume that does not contain the complete anatomy of the imaged body segment (e.g., diagnosing a local pathology or guiding an intervention such as soft tissue biopsy may only require a regional field of view). However, of particular interest, is reconstructing an image volume of an entire limb that could subsequently be used in soft tissue modelling, load simulation, and computational mechanical interface design (e.g., CAD of prosthetic sockets), both of which require complete 3D anatomical information. To acquire a 3D US scan of a limb, several approaches have been pursued. One conceivable solution involves covering the imaged body segment in gel, scanning up and down around the body segment, and stitching the collected images into a volume. However, since there is direct contact between the transducer and body surface, soft tissues are deformed; if tissue deformation is not appropriately accounted for, it can lead to an inaccurate portrayal and biomechanical characterization of the anatomy. Further, contact during scanning can cause motion of the limb, thereby generating yet another source of error.

To address these challenges, the transducer and imaged body segment can be submerged into a water bath to achieve unloaded 3D US imagery However, some of the limiting factors of such methods include: (i) the mechanical setup can be cumbersome, (ii) limb motion can degrade image resolution and motion is difficult to compensate for, and (iii) results may not allow for accurate differentiation between tissue types. Image feature-based registration for motion compensation and spatial compounding can be used, but such methods do not allow for rapid volumetric imaging.

Patient-specific determination of the mechanical properties of musculoskeletal tissues can be important to diagnosis in a variety of clinical applications such as muscle injury, neuromuscular disease, and training. It can also be important for determining proper interventions in rehabilitation medicine, including stretching, strengthening and spasticity treatments, all of which rely on altering muscle biomechanical properties. However, quantifying the mechanical behavior of musculoskeletal tissues is difficult due to its anisotropic behavior and complex composition of active and passive components.

Analogous to palpation procedures used by clinicians, medical imaging-based elastography techniques have been developed to quantify and map the mechanical properties of soft tissue in vivo. This involves applying a mechanical stimulus to the tissue, and subsequently imaging the resulting deformation to obtain a qualitative or quantitative measure of stiffness. One such method that has been applied to skeletal muscle is magnetic resonance elastography (MRE). Here, shear waves are generated by an electro-mechanical transducer, and when combined with a motion sensitizing gradient in the MR pulse sequence, produces a quantitative map of stiffness. Despite its success, this approach is limited by cost, accessibility, and a range of other technical factors such as posture restriction.

An appealing alternative to MRE is ultrasound shear wave elastography (SWE), a modality that is now incorporated into many modern clinical ultrasound systems. This technique utilizes an acoustic radiation force (ARF) impulse, generated directly by the ultrasound transducer, to produce shear waves in the tissue. Conventional B-mode imaging, in conjunction with speckle tracking methods, can be used to monitor real-time tissue displacements caused by the shear waves in order to create a map of shear wave velocities (SWV) in meters per second. In some simplification cases, the shear modulus can be related to the Young's Modulus by the following relationship: $E=3\rho c^2$, where $\rho$ is tissue density, c is SWV, and E is the estimated Young's modulus - in general, a low speed corresponds to a soft tissue, and a high speed corresponds to a hard tissue. Unlike some other forms of elastography, like strain imaging, SWE provides a quantitative stiffness value on an absolute scale.

Recently, SWE has been applied to skeletal muscle in a variety of applications. Some relevant examples include: (i) determination of shear modulus over a normal range of tension for skeletal muscle, (ii) inference of muscle stiffness across a wide range of contraction intensities, and (iii) establishing normative trends in passive skeletal muscle in adulthood. In addition, the technique has been validated for measurement in human muscle for different probe angles and orientations. Despite the potential of SWE for quantifying the stiffness of musculoskeletal tissue, there are several limitations related to its repeatability. For example, SWV measurements are dependent on applied transducer force - this has been shown in various studies including those focused on breast, liver, kidney, and muscle. In each of these studies, the operator's applied force directly affected the investigated tissue properties, leading to an error in the resulting stiffness modulus. Similarly, another study indicated a high reliability when SWV measurements were acquired under rigorous conditions, but reported only "moderate" reproducibility in scenarios that more closely mirror clinical practice. It has also been shown that using different clinical systems may produce varying results.

Consequently, methods to rapidly and easily assess muscle tissue composition in multiple clinical settings with minimal patient burden are needed, as well as methods to standardize measurement procedures in order to obtain meaningful data for diagnosis.

Furthermore, unlike MRE, which allows for full volume imaging, the traditional approach to ultrasound SWE involves only acquiring a local measurement within a region of interest (ROI). As such, SWE does not easily enable analysis of three-dimensional anatomical structures and pathologic features. It is also not straightforward to apply a transducer-independent external load (which would provide insight into the mechanical response of the tissue) to the imaged body segment since the transducer must make direct contact in order to produce an image. To this end, a complete 3D ultrasound dataset that includes a combined illustration of tissue morphology and biomechanics in multiple dimensions, is desirable. From a clinical standpoint, this would cost-effectively provide the examiner with a detailed spatial understanding of the tissue structures for diagnosis and intervention. Additionally, the imaging data combined with finite element analysis (FEA) may also inform more realistic ultrasound-based constitutive modeling of biological soft tissue.

A need exists for an apparatus and method for a generating three-dimensional imaging of musculoskeletal tissue under compressive loads that eliminates or minimizes the above-referenced problems.

Devices and methods for imaging a biological body segment that includes musculoskeletal tissue are provided. Examples of suitable biological body segments include surgically-altered or truncated limbs for which a prosthetic is to be fitted.

An example of a prior art device for imaging a biological body segment is shown in FIG. 60, which is a three-dimensional view of setup 1010 for SWE scanning of calibrated phantom 1012 using a standard gel approach. Ultrasound probe 1014 (transducer) is fixed to ring stand 1016 facing downward. Layer 1018 of an ultrasonic coupling gel is placed between ultrasound probe 1014 (transducer) and phantom surface 1020. Transducer surface 1022 is applied, typically with pressure, to phantom surface 1020. FIG.61 is a representation of prior art apparatus 1024 for SWE scanning a human lower limb 1026 by a standard gel approach, including ultrasound probe 1028 fixed to ring stand 1030 facing limb 1026, and wherein layer 1032 of ultrasonic coupling gel is placed between the ultrasound probe 1028 transducer and limb surface 1034 of human lower limb 1026.

Another embodiment of the invention is shown in FIG. 62, and is device 1040 for imaging calibrated phantom 1042 that simulates musculoskeletal tissue. Device includes container 1044 defining volume 1046, and at least one ultrasound probe 1048 supported within volume 1046 of container 1044 by a support, such as ring stand 1050. Phantom 1042 can be moved among incremental distances away from ultrasound probe 1048 in container 1044.

FIG. 63 is a perspective view of device 1052 of the invention for SWE scanning. As shown therein, ring stand 1050 is employed as a support to secure ultrasound probe 1054 within volume 1056 of container 1058 at a distance from biological body segment 1060. Biological body segment 1060 is under a load applied by shear support compressive garment 1062.

Examples of other suitable containers include open top or hermetically sealed containers suitable for containing a fluid within the volume of container. Container can be made of any material suitable for containing a suitable fluid within the container, such as is known in the art. Examples of suitable materials include plexiglass and glass. The shape of the container can be of any shape suitable for containing a fluid in which a biological body segment of interest can be at least partially immersed. Examples of suitable shapes include squares, rectangles, and cylinders. An example of a suitable fluid for use with the device of the invention is water.

Ultrasound probe 1054 defines an ultrasound transducer surface 1064. In one embodiment, at least one of the ultrasound probes includes a single element transducer. In another embodiment, at least one of the ultrasound probes can generate an echo ultrasound image. In one embodiment, ultrasound probe 1054 is movable relative to container 1058, such as by being movable along support 1050. In another embodiment, device 1052 includes a plurality (not shown) of ultrasound probes 1054, at least a portion of which are movable relative to container 1058. In a specific embodiment, ultrasound probes 1054, are located at different distances from a common region of focus or interest of a biological body segment within volume 1056 of container 1058 and immersed in fluid 1066, such as water, contained within container 1058. In another embodiment, ultrasound probes 1054 are independently movable relative to each other and to container 1058. A suitable mechanism by which ultrasound probes 1054 can be moved relative to container is a motor.

A pressurizing device applies pressure to biological body segment 1060 placed within container 1058, whereby pressure is applied to biological body segment 1060 greater than that of an unpressurized ambient medium in which the biological body segment 1060 is immersed and which is between ultrasound probe surface 1064 and biological body segment 1060, wherein ultrasound probe 1054 or, alternatively, the plurality of ultrasonic probes are arranged to image a region of interest of the biological body segment 1060. In one embodiment, such as is shown in FIG. 63, pressurizing device includes compression sock or stocking 1062, or some other suitable garment that applies a substantially uniform compression load to external surface 1068 of the biological body segment immersed in fluid in the container.

In another embodiment, shown in FIG. 64, device 1070 of the invention includes ultrasound support 1072 having a ring 1074 on which ultrasound probe 1076 is directly or indirectly mounted, such as by post-linking ultrasound probe 1076 to ring 1074. Device 1070 further includes water tank 1086 and seat 1078 mounted on top of water tank 1086. 3D-printed mount 1080 secures ultrasound probe 1076 to the rotating portion of ring 1074 , thus allowing for circumferential rotation of probe 1076 around biological body segment 1082 the limb at a fixed radius. SWE imagery can be collected at 10-degree increments around biological body segment 1082 in a longitudinal direction along the length of biological body segment 1082. Subject 1084 is situated above tank 1086, and is asked to submerge biological body segment 1082 into tank 1086. Subject 1084 is then asked to place their foot flat on bottom 1090 of tank 1086 in order to minimize motion of the submerged biological body segment 1082. Compression stocking 1088 applies uniform compression load to biological body segment 1082.

In this embodiment, ultrasound probe 1076 is movable about a central portion of tank 1086 (container) occupied by biological body segment 1082 by actuation of motor 1092 at a juncture between ultrasound probe 1076 and ring 1074. In another embodiment, the device includes a plurality of rings to each of which is linked an ultrasound probe. The rings can have different diameters and need not be concentric. For example, in one embodiment, at least a portion of the ultrasound probes are mounted on respective rings of at least one support at different distances from a common center of the rings, or from a point bisecting the line between distinct centers of the rings in which the ultrasound probes are supported. In one embodiment, at least one of the ultrasound probes is arranged to form a first image, and at least one of the ultrasound probes is arranged to form a second image transverse to the first image.

In yet another embodiment, shown in FIG. 65, device 10118 of the invention includes container 10120 and an elastomeric layer 10126 that defines a boundary 10122 between volume 10124 of container 10120 and atmosphere. Seal 10128 at boundary 10122 partitions volume 10124 of container 10120 from the atmosphere when biological body segment 10130 traverses boundary 10122, whereby container 10120 can be pressurized relative to an ambient pressure of the atmospheric volume by pressurizing device 10132, such as a pressurizing motor in fluid communication with volume 10124 of container 10120. The device 10118 can optionally include a camera 10136 positioned at a bottom of the tank for motion compensation, as described more fully in Section 9 herein.

In one embodiment of the invention, shown in FIG. 66, device 10140 of the invention (but for a pressurizing device, not shown) includes processing unit 10142 linked to ultrasound probe 10144, whereby images obtained by ultrasound probes 10144 can be employed to generate a three-dimensional representation of the biological body segment. As shown in FIG. 66, processing unit 10142 includes DAQ hardware 10146, pulser/receiver 10148, and myRIO 10150. In one embodiment, processing unit 10142 can employ finite element analysis to generate a three-dimensional representation of a biological body segment.

In yet another embodiment, a device of the invention further includes an ultrasound probe that includes an elastomeric sheath that conceals the biological body segment from the remainder of the embodiment of the container, wherein the ultrasound probes include nodes at the elastomeric sheath, and further including a processing unit that conform to a three-dimensional image of the position of the nodes relative to each other, whereby the ultrasound images can be obtained of the biological body segment at different pressures applied to the biological body segment by the pressurizing device.

In still another embodiment, at least one ultrasound probe includes at least one first transducer mounted on a first ultrasound probe support that is linked at the container, and at least one second transducer also first ultrasound probe support or fixed to a second ultrasound probe support (not shown). Elastomeric sheath (as described above) seals the biological body segment from the remainder of the container volume. Device further includes a processing unit to which at least a portion of the ultrasound probes are linked, whereby a three-dimensional image of the biological body segment at different pressures can be generated. In one such an embodiment, the processing unit is linked to the pressurizing device. In another embodiment, at least a portion of the second transducers are single element transducers. In still another embodiment, as the portion of the second transducers can perform echo ultrasound to thereby generate an image of the musculoskeletal tissue of the biological body segment.

In another embodiment, the invention is a method of three-dimensional imaging of musculoskeletal tissue of a biological body segment under compressive loads that includes the step of immersing a biological body segment into a container of fluid, the container defining a volume that is pressurizable while the biological body segment is immersed in the fluid and traverses a boundary between the container volume and an ambient volume beyond the container volume. A plurality of ultrasound images of the biological body segment is generated by at least one ultrasound probe within the container volume. The images are generated while the biological body segment is subjected to a plurality of discrete pressures within the container. A three-dimensional image of the biological body segment can then be constructed from the plurality of ultrasound images. In one specific embodiment of the method, a plurality of ultrasound images are generated by a plurality of ultrasound probes. In another embodiment, generation of the biological body segment includes finite element analysis of the plurality of ultrasound images. In still another embodiment, the ultrasound probes image the biological body segment while circumscribing the biological body segment.

In yet another embodiment of the method of the invention, the ultrasound probes are supported at different distances from a common center of rotation or from a point bisecting a line between distinct centers of rotation about which the ultrasound probes move, while generating images of musculoskeletal tissue of the biological body segment, whereby the plurality of ultrasound probes image the musculoskeletal tissue of the biological body segment at different distances from the biological body segment.

In one such embodiment, a plurality of nodes are fixed to an elastomeric sheath that seals the biological body segment from the remainder of the volume of the container and transmits signals to a processing unit that, in combination with the ultrasound probes, forms the three-dimensional image of the musculoskeletal tissue of the biological body segment. In one embodiment, the processing unit forms a three-dimensional image of the biological body segment that includes internal bone geometries of the biological body segment.

In one embodiment, the ultrasound transducers may be fabricated as MEMS-based Piezoelectric Micromachined Ultrasonic Transducers (PMUT). Unlike standard piezoelectric sensors, PMUT are based on flexural motion coupled with a thin piezoelectric film, this offers advantages such as more flexible geometries, increased bandwidth, and reduced voltage requirements.

In one embodiment, the ultrasound transducers may be fabricated as Capacitive micromachined ultrasonic transducers (CMUT). Whereas most commercial ultrasonic transducers on the market today use piezoelectricity, CMUTs utilize energy transduction due to change in capacitance, and are manufactured on silicon using micromachining methods. Since these are micromachined, it is easier to construct 2D and 3D arrays of transducers and allows for large bandwidth.

In one embodiment, non-contact laser ultrasound (LUS) can be used to acquire tomographic ultrasound data by employing laser sources and laser receivers without using a water tank for coupling.

All embodiments can include one or more cameras for motion compensation or for determining 3D geometry of the imaged body segment.

Ultrasound transducers may operate in one or more modes including pulse-echo and transmission.

The invention will now be described in an embodiment that is not intended to be limiting in any way. Experimentation was performed to assess an example device, a description of which follows.

*Materials and Methods*

For all measurements, a 9 MHz linear array probe and LOGIQ E9 (GE, Niskayuna, NY) system with built-in SWE capabilities was used. This system displayed 2D images of SWV within a user-selected ROI overlaid on top of a larger B-mode image at the same location. The ROI was rectangular-shaped. In this study, a calibrated phantom as well as human limbs, were scanned. Subsequently, a standard gel approach was compared to a non-contact water tank embodiment of the apparatus and method of the invention. A 3D scan was also performed to demonstrate the feasibility of volumetric elastography.

*2D Phantom Scans*

An apparatus for scanning of a calibrated liver phantom (CIRS, Norfolk, VA) of known SWV (4 m/s) is shown in FIG. 60 (prior art) and FIG. 62. Phantom was made of homogenous material and contained no inclusions. Separate experiments were performed with (i) gel layer separating the probe and phantom (i.e., standard clinical approach) (FIG. 60 (prior art)), and (ii) in a water tank (i.e., 'non-contact' approach) (FIG. 62). The expected SWV of the calibrated phantom could be acquired using a water tank in a comparable manner to that of the standard clinical approach. The effect of distance between the transducer surface and the phantom on the resulting measurement was evaluated. For each distance, at least 20 SWV measurements were taken and averaged together to ensure consistency.

For the gel experiment (FIG. 60 (prior art)), the phantom was placed on a flat surface and mounted in place to restrict motion. A copious amount of gel was placed on the surface to acoustically couple the ultrasound transducer to the phantom. To eliminate human and motion error, the transducer was mounted onto a ring stand to hold it in place at the surface of the phantom while data was collected. SWV data was acquired within a specified ROI approximately 0.5 cm below the phantom surface at incremental gel thickness layers. The first measurement was acquired at 0 cm (i.e., direct contact), and measurements were then taken incrementally every 0.5 cm until there was a loss of signal to analyze the measurement accuracy as a function of distance between the transducer and the phantom surface.

Similarly, SWV measurements were acquired with the phantom while submerged in a water tank setup (FIG. 62). Accurate SWV measurements could be achieved while the probe was not making contact with the imaged object by scanning in a water tank. The tank was filled with deionized water to minimize the effect of sound speed gradients. As before, the transducer was clamped to a ring stand and held stationary and data was collected at 0.5 cm incremental distances (starting at 0 cm) to analyze the measurement accuracy as a function of distance between the transducer and the phantom surface.

*2D Human Limb Scans*

Two subjects were recruited following a procedure approved by the MIT Committee on the Use of Humans as Experimental Subjects (COUHES). Both subjects were healthy male adults (Average $\pm$ S.D. age: 27.5 $\pm$ 2.1; Average $\pm$ S.D. BMI: 22.7 $\pm$ 2.4). Analogous to the phantom experiments, 2D measurements were acquired for each subject using both a conventional gel approach and by use of embodiments of the apparatus and method of the invention in order to demonstrate that: (i) accurate SWV measurements can be achieved while the probe was not making contact with the imaged limb by scanning in a water tank employed by the invention, and (ii) SWV measurements can be acquired while an external load (not from probe) is applied to the imaged segment of the limb.

FIG. 61 (prior art) shows the setup for conventional 2D human limb scans, and for 2D human limb scans while the limb is under compression or load. As shown, the probe is positioned mid-way up the subject's calf, and their limb was in the same position for each *(i.e.,* foot flat and resting on the ground with limb perpendicular to the ground). The subject was seated so as to minimize motion and ensure comfort. For measurements using gel, at least 0.5 cm of gel was placed between the probe and the limb to minimize the effect of the transducer making contact with the tissue.

FIG. 63 is a perspective view of the setup employed as an embodiment of the apparatus of the invention suitable for use with an embodiment of a method of the invention. SWV measurements were acquired in an unloaded state and in three different compressive states as embodiments of the method of the invention, without the transducer making contact with the limb. Compressive states were achieved by using sheer support compression garments (BrightLife Direct, Washington, DC) at grades: 8-15 mmHg, 15-20 mmHg, and 20-30 mmHg. An ROI was selected so that measurements were taken at superficial muscle layers (i.e., where the effects of the compression sock are most evident).

For both the gel and tank scans, measurements were acquired in the longitudinal and transverse directions since it has been well documented that transducer orientation along or perpendicular to the muscle fiber can play a role in the accuracy of SWE measurements. At each compressive state, two collections of at least 20 measurements each were taken. Two separate trials were completed to eliminate sources of error related to repeatability. Measurements over both trials were averaged together and plotted for comparison.

*3D Human Limb Scans*

Three-dimensional SWE imagery of a subject's limb in a water tank setup were acquired, as shown in FIG. 64. This setup was described in detail in previous publications from, for example, Ranger BJ, Feigin M, Herr H, Anthony BW. Image registration in a tomographic ultrasound system: comparison between camera-tracking and image-based motion compensation. IEEE Int Ultrason Symp 2017. pp. 1-4; Ranger BJ, Feigin M, Pestrov N, Zhang X, Lempitsky V, Herr HM, Anthony BW. Motion compensation in a tomographic ultrasound imaging system: Toward volumetric scans of a limb for prosthetic socket design. Proc Annu Int Conf IEEE Eng Med Biol Soc EMBS 2015. pp. 7204-7207; and, Ranger BJ, Feigin M, Zhang X, Mireault A, Raskar R, Herr HM, Anthony BW. 3D optical imagery for motion compensation in a limb ultrasound system. Proc SPIE 2016. pp. 9-12, the entire contents of which are incorporated herein by reference.

In summary, the setup consisted of a water tank with a ring bearing mounted on top. A custom 3D-printed mount secured the ultrasound probe to the rotating portion of the ring bearing, thus allowing for circumferential rotation of the probe around the limb at a fixed radius. To complete these scans, SWE images were collected at 10-degree increments around the limb in the longitudinal directions. As with the 2D image data collection, compressive states were achieved by using 8-15 mmHg, 15-20 mmHg, and 20-30 mmHg sheer support compression garments, and an ROI was selected so that measurements were taken at superficial muscle layers.

*Data Analysis*

A custom script was created in order to extract the SWV map from the DICOM data and into Matlab for analysis. Once in Matlab, a mean and standard deviation value was calculated for all the measurements taken in a particular acquisition and then plotted. For volumetric analysis of data collected in 3D, images were first placed in space in the orientation in which they were collected, turned into scattered data, and sampled to a grid. In-plane density was reduced by a factor of 20 - since the pixel size was originally 0.12 mm x 0.12 mm, this adjusted the voxel size to approximately 2 mm. This size is close to what is commonly seen in MRI, and also kept the overall computational time reasonable. Natural-neighbor interpolation, as implemented using the *scatteredInterpolant* class in Matlab, was used to process both the B-mode data as well as the SWE data. The GIBBON Toolbox in Matlab, was used to visualize the image volumes and segment out the water.

*Results*

FIGs. 67 and 68 show examples of ultrasound SWE images of the calibrated phantom taken at incremental transducer distances away from the phantom for both a gel method (FIG. 67) and a water tank method (FIG. 68), wherein the heat map represents SWV velocity values. The plot in FIG. 69 represents mean and standard deviation values for the SWV maps shown in FIGs. 67 and 68. As shown, the SWV output agrees with the expected calibrated phantom values for gel layers that are 2.5 cm or less, and water layers for up to at least 6 cm. These results demonstrate that accurate measurements of SWV may be acquired in a water tank setup. Additionally, measurements in the water tank appear more stable than when using gel, allowing for accurate measurements to be taken at a larger range.

FIG. 70 depicts example ultrasound images, in both longitudinal and transverse orientations, acquired of a subject's leg under the four compressive states. Qualitatively, it is noted that the heat map of SWV measurements "increases with" increasing compression, as expected. As shown, the heat map of SWV measurements increases with increasing compressing in both orientations. FIGs. 71A and 71B, and FIGs. 72A and 72B include plots of mean and standard deviation values for the SWV maps exemplified in FIG. 70 for the two research subjects, dubbed subjects 1 and 2, respectively. Each plot included gel and water tank measurements for comparison. Two primary conclusions were drawn from these plots: (i) measurable changes in mean SWV at superficial muscle layers could be detected when applying an external compressive load to the limb, and (ii) measurements taken from the standard gel approach were comparable to those acquired in the water tank.

Volumetric data taken from the ultrasound system is shown placed in 3D space in FIGs. 73 and 74. FIGs. 75A-D depict the interpolated SWV data as a volume of a subject's limb at the four compressive states. Similar to the measurements in FIGs. 71A and 71B, and FIG. 72A and 72B, upon qualitative evaluation of the SWV heat map, measurements of FIGs. 75A-D increased as a function of applied compressive load (plotted in FIG. 76). Similar to the 2D data, measurable changes in mean SWV at superficial muscle layers may be detected when applying an external compressive load to the limb. Further, measurements taken from the standard gel approach are comparable to those acquired in the water tank.

The foregoing was a demonstration of volumetric SWV measurements collected of a limb under varying loads by a method of the invention in a non-contact water tank embodiment of the invention, thereby providing a full volumetric SWE scan of a limb under various loading conditions in an apparatus of the invention.

*Discussion*

SWE in a water tank, by imaging a limb in a near-unperturbed state, as well as under various compressive states, was demonstrated, without the ultrasound transducer making contact with the imaged body segment. Therefore, a 3D ultrasound SWE data set was achieved that contained a combined illustration of tissue morphology and biomechanics in multiple dimensions thereby providing a cost-effective apparatus and method of obtaining detailed spatial understanding of limb tissue structures for diagnosis and intervention. Moreover, the resulting SWE data can be combined with finite element analysis (FEA) which informs even more realistic ultrasound-based constitutive modeling of biological soft tissue.

This work demonstrated that: (i) accurate SWV measurements can be acquired while the probe is not making contact with the limb by performing the scan in a water tank; and (ii) three-dimensional SWV measurements can be acquired while an external load (not from the transducer) is applied to the imaged segment of a limb. To do so, we imaged both a calibrated shear wave phantom and human limbs submerged in a water tank. Since the water acted as the acoustic coupling medium, no gel was needed, and the transducer did not need to make direct contact with the limb.

To demonstrate the accuracy of using SWV as a measurement tool in a water tank, 2D images were acquired of a calibrated phantom at varying distances from the transducer, both using ultrasound gel (the clinical standard) and a water tank (non-contact). Shown in FIGs. 67, 68 and 69, accurate measurements of the calibrated liver phantom were obtained for both the gel (conventional) and water tank (as an embodiment of the invention) methods. An important observation from these results was that the water tank setup of the invention performed more consistently at increasing spacing between transducer and phantom surface, and also allowed for measurements to be taken at more than twice the distance of the gel layer. This was likely due to air bubbles in the gel that may cause attenuation of the ultrasound signal. Overall, these results demonstrated successful acquisition of SWE data in a water tank by the method of the invention without the probe making contact with the imaged body.

Extending on the results of the calibrated phantom, 2D images of human limbs (FIG. 70) with associated plots (FIG. 71A, 71B, 72A, and 72B) under four different compressive loads are presented. For each limb, and in both the transverse and longitudinal orientations, SWV measurements increased as a function of applied external load. Measurements for mean SWV fell within the range reported in other studies who have acquired data on human limbs. As expected, measurements in the longitudinal direction appeared more consistent. However, we note similar trends in the transverse direction; this may be partially explained in that our study only imaged muscle in a passive state, and not at varying levels of muscle contraction. In sum, the 2D image results demonstrate the feasibility of collecting *in vivo* data of tissue biomechanics without the transducer making contact with the limb in a water tank.

Volumetric results of SWE measurements collected of a limb section at various compression states (FIGs. 75A-D) were also obtained. Similar to the 2D results, a measurable increase in SWV is noted as load increases (FIG. 76). Volumetric SWV measurements were successfully collected in a water tank system while varying the compressive load on the limb, demonstrating a full volumetric SWE scan of a limb in a non-contact water tank system of the invention under various loading conditions.

The apparatus and method of the invention have broad implications for clinical musculoskeletal imaging. For example, this data can be used for 3D constitutive modeling of limb biomechanics, and could have far-reaching applications in impact biomechanics, rehabilitation engineering, and surgical simulation. Further, since the approach allows for volumetric elastography, it paves the way for research such as longitudinal studies of disease progression, more accurate analysis of muscle state and contraction ability, large-scale multidimensional elastography, and detailed comparative studies between patients.

*Conclusion*

The results represent a significant advancement for SWE and precision medicine as it pertains to musculoskeletal health, in that volumetric *in vivo* elastographic data of the limb can be successfully collected under different loading conditions in a water tank setup by the method and apparatus of the invention.

9. Camera-Based Motion Compensation for Three-Dimensional Imaging Under Compressive Loads

Devices and methods for three-dimensional imaging under compressive loads are described in the preceding section, Section 8. In particular, a multi-modal imaging system that is able to acquire a volumetric image by scanning the limb with the transducer array oriented vertically, effectively collecting an array of single element images circumferentially around the limb to capture an entire volume is described. If no spatially overlapping image frames are obtained, image-based motion compensation may not be not possible. To address this, a 3D camera, mounted in the bottom of the tank, to track motion of the body segment and appropriately position the imagery in space can be included in such devices.

CAD renderings depicting the mechanical design of an ultrasound imaging system 1100 are shown in FIGS. 77A-C. The system 1100 includes a friction pinion 1102 with a DC motor to drive rotation of a ring bearing 1104. A mount 1106 is included that allows for radial adjustment of a transducer 1108 to account for different limb sizes. A magnetic encoder 1110 is also included to track angular position of the transducer 1108. A stepper motor 1112 controls motion of the transducer 1108 in the z-direction. The system includes a tank 1114, at the bottom of which is disposed a 3D camera 1116.

A study was performed in which 2D image results *(i.e.,* ultrasound transducer oriented horizontally) of both a tissue-mimicking phantom and residual limbs are presented, thereby demonstrating that motion compensation is effective at creating tomographic slices. The 3D results *(i.e.,* ultrasound transducer oriented vertically) are also presented, which demonstrate that external limb and bone shape may be obtained by segmenting the volumetric image data. External limb and bone shape may further be incorporated directly into automated prosthetic design methods. Though this work focuses on imaging human residual limbs, the concepts could reasonably transfer to other imaging applications, such as acoustic tomography, where volumetric imaging may provide additional clinical insight, and where motion artifacts have been shown to distort image reconstruction. Furthermore, the methods and devices described herein can be applied to other musculoskeletal (MSK) US clinical applications, such as monitoring muscular dystrophy progression, bone density monitoring, and orthopedic surgery planning.

*System Overview and Mechanical Design*

A mechanical system allowing for controlled circumferential US scanning of an unloaded limb inside of a water tank was built. To complete a scan, a subject placed their limb in the tank, and the US transducer (LOGIQ E9, 9L Transducer, General Electric, Niskayuna, NY) rotated circumferentially around the limb collecting images at set angular increments. A combination 3D, IR, and color camera (SR300 RealSense Camera, Intel Corp., Santa Clara, CA) was secured below the tank, pointed upward, to track limb position during the scan by imaging the 3D surface geometry of the limb (see FIG. 64). The camera produced images at a standard resolution of 640x480.

A prototype tank was constructed in accordance with CAD renderings shown in FIGS. 77A-C. The prototype was constructed from a 24x24x18 inch clear acrylic tank with a 600 mm-diameter ring bearing mounted on top. A custom 3D-printed mount secured the ultrasound transducer to the z-axis linear rail on the ring bearing; the 3D printed mount was designed to allow transducer rotations relative to the horizontal at pre-determined angles.

Circumferential rotation of the ring bearing was driven by a DC motor (2826 100:1 metal gear motor, Pololu Electonics, Las Vegas, NV) with a custom friction pinion. A NEMA-17 stepper motor (Schneider Electric, Cambridge, MA) enabled controlled vertical z-translation of the ultrasound transducer. Additionally, the radial position of the ultrasound transducer can be adjusted manually at set circumferential positions, enabling adaption of the scanning apparatus for a range of limb sizes.

The angular position ($\theta n$) of the transducer was tracked using a linear magnetic encoder system (LM10, Renishaw Inc., Gloucestershire, UK). The encoder consisted of an adhesive-backed magnetic scale taped around the circumference of the ring bearing, and a non-contact magnetic encoder head to detect quadrature encoding on the magnetic scale. The angular position ($\theta n$) of the transducer was calculated via quadrature decoding and basic geometry on the controller.

A diagram depicting the electronic control system is shown in FIG. 78. An Arduino UNO microcontroller read trigger signals from the GE ultrasound system and calculated the angular position data from the magnetic encoder. It also controlled the motors, image trigger of the 3D camera, and time synchronized data collections to the PC.

*3D Camera-based Motion Compensation*

Motion can present a significant challenge for limb imaging in an US water tank setup for several reasons. First, acquiring unloaded limb geometry, which is important for applications to prosthetic socket design as well as for accurate biomechanical tissue modeling, necessitates that the limb be in an unperturbed state. Therefore, a harness or mount that makes contact with the imaged body segment may not be used to provide structural support and stabilize limb motion during a scan. In addition, for US images collected circumferentially around a limb, acoustically reflective surfaces (e.g., bone) change appearance depending on the orientation of the imaging array. Therefore, traditional image registration and stitching based on image features (e.g., random sample consensus (RANSAC) methods) is not always effective. To solve this, a structured light-based 3D camera was utilized to track residual limb surface structure during a scan.

Two-stage tracking was performed on the 3D camera output: first, each camera frame was referenced to the first frame in the circumferential scan, and then the first frame was referenced to the first frame of each scan in the series. This approach results in better per-scan alignment while allowing (i) creation of an aggregate volume using multiple scans, and (ii) acquisition of multiple scans of the same section, which may be combined to produce a higher resolution volume. Since the 3D camera has a different frame rate than the ultrasound system, and no input/output trigger, each received image was time stamped. Based on the limb tracking, a time dependent position vector was generated, from which the ultrasound frame position was interpolated.

To perform limb tracking, the point cloud 3D structure produced by the 3D camera was used, where each 3D frame, with index *n,* provides a set $P_n$ consisting of *m* points:

$$\mathcal{P}_n = \{p_n^1, p_n^2, p_n^m\} \in \mathcal{R}^3 \qquad \textbf{50}$$

where $p_n^i$ represents the position vector of the i-th point of time step index n. In the experimental setup, since the subject was seated and asked to fully extend their limb *(i.e.,* knee was not bent) into the scanning tank, it was assumed that rotation at the knee and out-of-plane motion was negligible; therefore, motion was approximated as translation-only. An initial estimation of the limb displacement was obtained from the location of the overall mean of the point cloud compared to the mean value of the reference frame point cloud.

The iterative closest point (ICP) algorithm was then applied to improve alignment between the point clouds. For this study, the generalized ICP algorithm (GICP) was utilized, as implemented in the Point Cloud Library (pointclouds.org). The ICP algorithm computed the affine transformation between two point clouds that minimized the Euclidean distance between the clouds. There are several approaches to using the ICP algorithm, but in its simplest formulation, the ICP algorithm looks at point-pair matches between the point clouds and minimizes the mean of the sum of squared distances between these point pairs. This is a greedy optimization-based algorithm that significantly benefits from a good initial guess (in this case, the mean approximation).

Assuming translation-only motion, since the subject held their limb straight without bending at the knee, we ignored the rotation/scaling factors produced by the ICP algorithm and utilized the translation offset only ($\Delta x_n$; $\Delta y_n$; $\Delta z_n$). This is another factor that benefited from the mean approximation, as with non-centered objects, there can be an ambiguity between rotational and translational motion. An example of the 3D surfaces acquired from the 3D camera are shown in FIGS. 79A-B, projected on to the x-z and x-y plane. Two of the surfaces 1140, 1142 were acquired from the 3D camera at two different time points during the scan. The second surface 1140 is shifted to its motion compensated position 1144.

As depicted in FIG. 80, to create a 2D ultrasound image, U(i, j), the $\Delta x_n$, $\Delta y_n$, $\Delta z_n$ positions as calculated by the ICP algorithm were used to spatially place the individual ultrasound images (collected at each $\theta_n$) in the compound image space. Each individual B-mode capture $u_n$(i, j), which has pixel size of 0:234 1:593 mm, is rotated in-plane based on its associated $\theta_n$. Images were stitched together into the 2D image slice U(i, j) by choosing the maximum value at each overlapping pixel, where (i, j) are the grayscale values in the images. Maximum values were chosen for this study instead of mean or median values because, for this particular application to computational prosthetic socket design, a focud was on acquiring bone and skin surfaces. In ultrasound imagery collected in a water tank system, reflective surfaces (e.g., bone and skin) present as elevated pixel values. In order to retain this information in the final imagery, rather than blur out these features, a maximum value was chosen.

To create a 3D image of the limb, ultrasound data collected from the scan in which the transducer was in the longitudinal position were pieced into a volume and interpolated. In this case, there was no overlapping imagery, so placement of the images in space was accomplished using only 3D camera data, as described above. The image volume can then be resliced into a 2D image for direct comparison to the 2D images created above.

*System Calibration*

In order to reconstruct a tomographic image, each ultrasound sample was positioned in 3D space. This entailed two challenges: (i) distinguishing the physical position of the transducer in each frame, and (ii) correcting for relative subject motion with respect to the transducer. This translated into two calibration tasks. First, the ultrasound system parameters were calibrated; this included rotation radius and transducer offset with respect to the center line. Second, camera tracking coordinates were translated to the ultrasound image space *(i.e.,* translation scaling and relative rotation). To this end, an image of a calibration phantom was constructed, with the requirements of being able to track the phantom in both ultrasound and camera image spaces. The phantom was custom-made into a cylinder (10 cm diameter) and composed of a mixture of copolymer, mineral oil, and graphite powder. The calibration device (see, for example, FIGS. 81A-D) consisted of a PVC rod mounted to a linear rail and connected to the phantom. A fiducial marker, in this case half of a standard ping pong ball, was mounted to the bottom of the rod for camera tracking.

*Ultrasound System Calibration*

To calibrate the ultrasound system scanning hardware, a full tomographic scan with the transducer oriented in a transverse *(i.e.,* horizontal) position was performed to acquire in-plane images. Since the scan follows a fixed circular path, the distance and relative orientation of the center of rotation to the transducer face was fixed. Thus, all images in the scan can be rotated and translated in-plane (FIG. 81B) so that they were aligned into a joint coordinate system according to the

following transformation:

$$\widehat{\mathbf{p}} = \mathbf{R}\mathbf{p} + \mathbf{b} \qquad \mathbf{51}$$

where R was the rotation matrix and *b* a translation vector, the center point was the stationary point of this transformation. The center point of rotation $\hat{r}$, in the ultrasound image coordinate system, was given by:

$$\widehat{\mathbf{r}} = \mathbf{R}\mathbf{r} + \mathbf{b} \qquad \mathbf{52}$$

$$\mathbf{r} = (\mathbf{1} - \mathbf{R})^{-1}\mathbf{b} \qquad \mathbf{53}$$

All images collected in the calibration scan were aligned in pairs to each other using the algorithm described in [29] M. Feigin, B. J. Ranger, and B. W. Anthony, "Statistical consensus matching framework for image registration," In Proceedings of the 23rd IEEE International Conference on Pattern Recognition (ICPR), pp. 1827-1832, Dec. 2016, the entire contents of which are incorporated herein by reference. The above formula was then applied to recover r for each image pair, and finally, all the results were averaged after outlier removal and the transducer position in ultrasound image space (in pixels) was computed with respect to the center of rotation. Outliers were removed by calculating the median point and filtering out points that were outside of 50% of the closest points.

*Camera Calibration*

To transform from 3D camera image space into ultrasound image space, two parameters were recovered: (i) rotation and (ii) scaling. As all frames are referenced to the same reference frame, the transformation is invariant to the joint translation. For this purpose, it was possible to take an image with both ultrasound and 3D camera at two different arbitrary positions (with in-plane motion between them) and having a translation vector (two points) in each domain uniquely defines both scaling and rotation. In practice, a full scan of the phantom was taken at two different positions and the transforms were averaged over all matching image pairs in order to calibrate the system.

*Scanning Protocol*

Subjects were recruited following a procedure approved by the MIT Committee on the Use of Humans as Experimental Subjects (COUHES). Two male transtibial amputee subjects were recruited for this study: one bilateral and one unilateral, allowing for a total of three limbs to be imaged.

To avoid sound speed gradients, the imaging tank was filled with deionized water that was approximately body temperature. To complete the ultrasound scan, the subject was seated above the imaging tank and asked to submerge their limb into the water. Once the subject was comfortably situated, the ultrasound transducer completed a 360-degree pass circumferentially around the limb; during this period, the ultrasound transducer and 3D camera collected data simultaneously. One 360-deg pass took approximately 15 seconds to complete. For each subject, the following scans were collected: (1) 3 separate image slices in which the ultrasound transducer is situated in the transverse (horizontal) orientation and (2) a full volume scan in which the transducer was placed in the longitudinal (vertical) orientation.

MRI was performed for comparison to the ultrasound images produced from our system. To complete the scan, the subject was situated prone and feet-first inside a 3 Tesla MRI scanner (Siemens Magnetom Tim Trio 3T, Siemens Medical Systems, Erlangen, Germany). All imaging was performed with a RF body coil wrapped around the limb; no contact was made between the coil and limb so as to prevent tissue deformation. A T1 MPRAGE sequence was used (for patient 1:TR = 2530, TE = 3:9, acquisition matrix 176x 256, 176 slices, voxel size 1.0x1.0x1.0 mm; for patient 2: TR = 633, TE = 8:7, acquisition matrix 320x320, 90 slices, voxel size 1.0x1.0x1.0 mm) for image data acquisition. Slight differences in the sequences between the two patients were due to real-time optimizations by the radiographer for the given scan.

*2D Image Analysis and Comparison to MRI*

To compare the ultrasound to MRI images, qualitative and quantitative evaluations were performed. Data outside the skin contours (e.g., water visible in the ultrasound scans) was excluded and cut out from the views for this comparison. For qualitative assessment, similarities between anatomical structures such as muscle architecture and bone were analyzed. To quantitatively compare between 2D image sets, ImageJ (v1.46r) was utilized to manually select a region of interest (ROI) around both the skin and tibia for all 9 slices that were collected (see FIGS. 82A-B). A pixel count was performed for each region of interest and used to calculate the area (in $mm^2$) of the tibia and bone. A percent error was calculated to analyze differences between MRI and ultrasound images.

*3D Image Analysis and Comparison to MRI*

To evaluate the ability of the prototype ultrasound system to accurately generate limb geometry, skin and bone shapes segmented from the ultrasound data to its respective MRI were compared. For this purpose, the GIBBON MATLAB Toolbox was used to: (i) view the image data in 3D space, (ii) semi-automatically create contours of the skin and bone surfaces from both MRI and US volume data (GIBBON *imx* function), and (iii) quantitatively compare surface shapes by evaluating closest point-to-point distances.

*Results*

FIGS. 81A-C illustrate a tissue-mimicking cylindrical gel phantom, a prototype of which was created to model a residual limb. A tissue-mimicking phantom 1150 is shown in FIGS. 81A and 81C, which includes a linear rail 1152, a hollow pipe 1154, a tissue-mimicking gel 1156, and a fiducial marker 1158. A calibration procedure with the phantom 1150 is shown in FIG. 81B.

For the experimental phantom, a 3-cm-diameter hollow PVC pipe, which acts as a reflective surface to mimic bone, was used as the hollow pipe 1154. In FIG. 81D, the reconstructed ultrasound image accurately represents the expected cross-section (FIG. 81C).

Results to demonstrate the 3D camera approach for motion compensation are shown in FIGS. 82A-D. In FIG. 82A, two ultrasound images that were collected at distinct circumferential positions of the same limb are shown overlaid; there is clear motion between images as evidenced by the discontinuity seen in the skin boundary. In FIG. 82B, the same images are shown with having been shifted into the correct spatial position. FIGS. 82C and 82D show the corresponding full ultrasound image of the same subject, both before motion compensation and after motion compensation. This demonstrates that motion correction with the 3D camera is effective and does not require further registration using image features.

2D tomographic image results, in which imagery was collected with the transducer oriented in the transverse direction, are shown in In FIGS. 83A-D for the two research subjects. FIG. 83A and FIG. 83C are MRI images, while FIG. 83B and FIG. 83D are the two corresponding ultrasound image slices of FIGS. 83A and 83C, respectively. The tibia and fibula are clearly seen in both the MRI and ultrasound data sets and have similar morphology, and general muscle structures including the gastrocnemius and tibialis anterior are comparable.

Quantitative results comparing the size of bones and limb in 2D images are shown in Table 13. The average percent error of tibia size between all three limbs was 1:61 ± 1:64, and the average percent error of limb size as measured by the skin boundary was 2:98 ± 1:44.

**Table 13. 2D Size Comparison Between MRI and Ultrasound**

| Limb | Slice | Part | Area MRI ($mm^2$) | Area US ($mm^2$) | % Error |
|---|---|---|---|---|---|
| 1 | 1 | Tibia | 1519 | 1530 | 0.70 |
| | | Skin | 8080 | 8482 | 4.98 |
| | 2 | Tibia | 2830 | 2829 | 0.02 |
| | | Skin | 8415 | 8489 | 0.88 |
| | 3 | Tibia | 641 | 628 | 2.05 |
| | | Skin | 6612 | 6488 | 1.87 |
| 2 | 1 | Tibia | 708 | 702 | 0.82 |
| | | Skin | 6553 | 6243 | 4.74 |
| | 2 | Tibia | 626 | 627 | 0.13 |
| | | Skin | 5472 | 5337 | 2.47 |
| | 3 | Tibia | 1118 | 1151 | 2.91 |
| | | Skin | 7473 | 7619 | 1.95 |
| 3 | 1 | Tibia | 544 | 552 | 1.51 |
| | | Skin | 5452 | 5205 | 4.53 |
| | 2 | Tibia | 837 | 828 | 1.09 |
| | | Skin | 6231 | 6076 | 2.49 |
| | 3 | Tibia | 3807 | 4007 | 5.25 |
| | | Skin | 8208 | 8450 | 2.95 |

(continued)

| Limb | Slice | Part | Area MRI ($mm^2$) | Area US ($mm^2$) | % Error | |
|---|---|---|---|---|---|---|
| | | | | Avg. % error (tibia) | 1.61 | 1.64 |
| | | | | Avg. % error (skin) | 2.98 | 1.44 |

A volume result of a residual limb, in which ultrasound imagery was collected with the transducer oriented in the longitudinal direction, is shown in FIG. 85, as compared with MRI shown in FIG. 84. The vertical array did not cover the entirety of the imaged body segment, therefore five acquisitions at set z increments were collected and then stitched together. Images were pieced together into the volume using 3D camera tracking without overlapping imagery. The full volume with cross-sectional views is shown in the top left. 2D cross-sectional slices of the image volume are also shown in the XY, XZ, and YZ planes. Limb and bone shape, as well as other anatomical soft tissue structures, are discernible. Though some penetration issues are evident in the XY slice for US, it is evident that a 3D-camera-based approach that allows for rapid volumetric imaging is feasible for this application.

Examples of obtained surface contours of the skin 1162, tibia 1166, and fibula 1164 that were segmented from US and MRI volume imagery for one limb are shown in FIGS. 86A-F. User-selected contours 1162, 1164, 1166 are shown in FIG. 86A, with the resulting 3D surfaces (skin 1162a, tibia 1166a, and fibula 1164a) from US and MRI shown in FIG. 86B and FIG. 86C. FIGS. 86D-F show difference maps that depict shape differences between skin, tibia, and fibula, respectively. Average differences with associated standard deviations are recorded in Table 14; results for all are between 2-3 mm.

**Table 14. 3D Shape Comparison Between MRI and US**

| Part | Avg. difference between MRI and US (mm) |
|---|---|
| Skin | 2.75 ± 4.11 |
| Tibia | 2.38 ± 5.18 |
| Fibula | 2.25 ± 2.72 |

*Discussion*

The multi-modal imaging approach described incorporates both a clinical US transducer and 3D optical imagery for motion compensation. The integrated imaging system produces fast, safe, and adequately accurate 3D ultrasound imagery of the salient volumetric features of a residual limb. One rotation of the transducer around the limb takes approximately 15 seconds; thus, the entire volume of the limb may be scanned in approximately 2-3 minutes depending on the size of the limb. Compared to a standard MRI scan, which can take approximately 10-15 minutes, this rapid collection of 3D imagery addresses several key limitations for accurate and quick lower extremity US imaging.

Computed tomography (CT) imaging is, at present, the method of choice for the generation of subject-specific finite-element models of bone. It is, however, not ideal for multi-scan applications because CT exposes the subject to ionizing radiation, and thus is not ideal for applications that may require multiple scans. Though MRI has its own limitations related to cost and requirements for a specialized imaging facility, it was chosen for our preliminary comparative studies here since it has been effective for the purposes of modeling the residuum for prosthetic socket design and does not present the same radiation concerns.

When comparing the 2D ultrasound image results (FIGS. 83A-D) to corresponding MRI images, similar anatomical structures, including shape of the tibia and fibula bones and general muscle architecture of the gastrocnemius and tibialis anterior, are noted. Differences in overall limb morphology are expected since imagery was collected while the patient is situated in different orientations: during an MRI scan the subject is situated in the prone position with leg elevated, while during the ultrasound scan the subject is situated upright with their limb extended downward and submerged in water. Imagery may also be different since residual limb volume is expected to fluctuate significantly, even throughout the day. Residual limb cross-sectional area changes over the course of a day can vary from -2.4% to +2.2%. Therefore, the percent error values as reported in Table 13 are sufficiently low when comparing the tibia and the skin across our sample set. This validates, in part, that the 2D imagery is producing accurate representations of limb anatomy. These results demonstrate the ability of the described motion compensation framework to produce accurate images, and opens the door for 3D imaging without using image features or fiducial markers.

Resliced images of the limb volume, as shown in FIGS. 84-85, demonstrate that imaging with the longitudinal transducer orientation in combination with camera-based motion compensation is feasible for rapidly acquiring 3D imagery of a limb. Qualitatively, overall morphology of skin, muscles and bones are comparable to MRI. Imaging with a higher penetration depth or lower frequency may remediate shadowing behind the tibia and fibula bones in the transverse slice.

Anatomical structures, including skin and bone, can be segmented from the imaging data. FIGS. 86A-F, along with Table 14, provide the spatial differences between skin, tibia, and fibula. The average differences fall within approximately 2-3 mm for each. Given that the isotropic voxel size for our MRI images is 1 mm, that previous studies have shown successful MRI segmentations for prosthesis design with voxel depth of 2 mm, and that the MRI sequence used in the study was not optimized for musculoskeletal scanning, the differences in size are reasonable for preliminary results and further demonstrate that the described devices and methods produce adequately accurate volumetric imagery of the residual limb useful for prosthesis design.

Despite recent innovations for computational prosthetic socket design, existing methods often incorporate expensive scanning tools that are only available in specialized clinical facilities. Ultrasound imaging techniques, like those described herein, can acquire detailed morphological information of the bone, soft tissue, and external shape of the limb. As such, the devices and methods described herein can provide innovative and cost-effective means to collect useful data to create biomechanical models and perform FEA of residual limb soft tissue.

Though the imaging residual limbs is described, the devices and methods described herein can also be applied to other clinical applications, such as acoustic tomography, where motion artifact has been shown to distort image quality. The devices and methods can also be applied to other MSK US clinical applications, where there is a need for more standardized assessment of muscle quality. Specifically, muscle elasticity may be measured in the tank system described herein. This may be done either by (1) using commercially available clinical elastography systems, or (2) through transmission systems that can quantitatively measure sound speed. Similarly, bone may be imaged, thus allowing for the potential of bone density monitoring, bone fracture detection, and assessing healing associated with orthopedic surgical procedures.

10. **Inferring internal geometry from multiple mechanical states**

Geometric features of interest which are embedded, either partially or fully, inside another medium or material can be challenging to resolve. For instance, if the medium or material in which the features are embedded is optically non-transparent or otherwise opaque, determination of such features typically requires imaging methods, such as x-ray, CT, MRI or Near-infrared Light Imaging. However, some embedded features may not be compatible with such imaging methods, or the medium or features may be opaque with respect to the physical principle employed in the imaging. In addition, in the case of medical imaging techniques and imaging of biological body segments, it may be undesirable to use methods involving ionizing radiation. Furthermore, medical imaging techniques, such as MRI, can be costly and time consuming, and are often not mobile. Therefore, there is a need for non-invasive determination of internal structures without the use of imaging techniques, such as x-ray and MRI.

Devices and methods for the determination of a shape or a hidden feature embedded inside a deformable media based on mechanical perturbation of the exterior of the media are provided. An example of a use of this method is to infer bone geometries inside a biological body segment through an analysis of a loading experiment performed on the skin surface of the body segment. The loading experiment can be, for example, applying a pressure, such as a homogeneous pressure, to the body segment and obtaining images of the body segment under a pressurized state (see also Sections 1, 7, 8, and 9 herein). Images of the body segment under a pressurized state can provide for the deduction of an internal bone structure, potentially obviating a need for medical imaging techniques such as MRI, CT, and X-ray to form a 3D model of the body segment.

The method can include first determining an initial exterior geometry and shape of the body segment, which may include imaging of the exterior boundary of the entire object or may focus on a local region of interest. The measurement of geometry and shape can be performed using any suitable method, including, for example, 3D scanning methods or optical techniques such as DIC. Next, a geometry and shape of the object can be altered using a mechanical perturbation, with the geometry and shape in the deformed state being recorded. Multiple mechanical perturbations and multiple associated deformed states may be recorded. The mechanical perturbations may vary in nature and type (e.g., pressure, indentation, vibration, frequency) and intensity (e.g., pressure magnitude, indentation depth, vibration amplitude).

Hidden embedded features can be estimated/predicted from the loaded state. In the case of a homogeneous pressure, the deformation (e.g., the difference between the initial and a loaded state) can be exaggerated to provide an estimation of the embedded features. Alternatively, the embedded features can be instead estimated based on assumptions of local depth and thickness. Alternatively, the estimation may rely on prior knowledge (e.g., surface anatomy informing assumed bone locations), statistical shape modeling, and machine learning approaches. If the estimate of the embedded features created is deemed sufficient, no further steps are required. If refinement is needed, additional modeling can be performed.

A mechanical computational model, such as a finite element model, can be constructed of the initial geometry and the embedded features. In a computational model, the deformable medium surrounding the embedded features and the embedded features are each assigned an appropriate mechanical behavior (such as hyperelastic and poroelastic formulations in the case of rubbers and soft tissues). The mechanical behavior can be spatially varying and/or anisotropic. Model boundary conditions can next be prescribed, allowing for the simulation of the mechanical perturbation events. The simulated deformed state following each mechanical perturbation can be exported. Analysis of a difference between the experimentally measured deformation and the simulated deformation allows for adjustment/refinement of the estimated embedded geometry. This process can then be repeated in an iterative optimization scheme, whereby the embedded features in the model are iteratively adjusted to minimize the difference between the experimental and simulated boundary conditions (e.g., deformation).

FIGS. 87 and 88 illustrate examples of the above process for inferring hidden features embedded in deformable media.

In FIG. 87, an initially spherical region of a deformable solid 1210 containing an inclusion 1220 is imaged in an unloaded state 1200. The inclusion 1220 within the spherical region is, as illustrated, a rigid *(e.g.,* rigid with respect to the deformable medium) 3D star shape. The deformable solid 1210 is then imaged in at least one loaded state 1230. Features 1212 pertaining to the internal structure can become visible. Through analysis of the mechanical perturbation(s) applied by the increase in pressure, in this case a homogeneous pressure, a predicted shape 1240 of the inclusion 1220 can be obtained. As illustrated, the prediction is based on exaggeration of the experimentally assessed deformation (e.g., state 1230) by a constant factor.

In FIG. 88, an initially ellipsoidal region of a deformable solid 1250 containing an inclusion 1260 is imaged in an unloaded state 1200. The inclusion 1260 within the ellipsoidal region is in the shape of a human femur bone. The deformable solid 1250, which can represent soft tissues of a body segment, can undergo mechanical perturbation, such as through application of a homogeneous pressure, and be imaged in at least one deformed state 1230. Through analysis of the mechanical perturbation and the deformed state, features 1252 can be used to predict a shape 1270 of the inclusion. In particular, the experimentally assessed deformation (e.g., state 1230) can be exaggerated by a constant factor, to infer a geometry of the inclusion.

To understand the principle of the above predictive method, one may imagine the application of an infinite pressure to a deformable medium containing a rigid inclusion. In this hypothetical scenario, the infinite pressure can cause the compressible/deformable medium to shrink around the inclusion and, therefore, reveal the rigid inclusion.

If, instead, a physically realistic and lower pressure is used, the exterior surface may not completely reveal the embedded inclusion. Instead, its outer shape presents an intermediate between an initial shape and the true embedded shape. The deformable medium acts as a type of blurring or low pass filter on the embedded shape, causing the predicted shape to present with less detail (e.g., sharpness is reduced, and small features may be missed). However, an initial prediction based on the external deformation can provide a good overall fit to the shape and may be sufficient for some applications. If refinement is needed, additional or higher amplitude mechanical perturbations can be employed, or iterative adjustment and optimization can be employed, as described above.

To improve performance, mechanical properties of the materials involved may first be identified. However, if an iterative scheme is employed (e.g., optimization), the system can simultaneously solve for the mechanical properties of the embedded geometry and the medium.

### 11. Virtual Prototyping and Design Optimization

A data-driven computational procedure for prosthetic socket design is provided. However, while the examples provided herein refer to prosthetic socket design, the devices and methods described can be applied equally well to the digital design, and subsequent digital manufacturing, of any biological segment and biomechanical interface attached thereto, including but not limited to: an ankle-foot in the case of shoe design, breasts in the case of bra design, an amputated-residuum in the case of prosthetic socket design, buttocks in the case of seat design, and a limb or a torso, or section thereof, in the case of an exoskeletal or orthotic design.

FIG. 89 provides an overview of a data-driven prosthetic socket design process. In particular, imaging of the biological body segment and segmentation of tissue contours can be performed (step A). As shown, the residual limb is imaged with MRI; however, any other imaging modality or combination of imaging modalities that provides for external and internal patient-specific geometries can alternatively be performed. Examples include US, CT, optical cameras with DIC techniques, near-infrared imaging. Devices and methods that can provide for patient-specific geometries are also described in Sections 1-10 herein.

Based on the obtaining imaging and, optionally, segmentation of tissue contours (step A), subject-specific surface geometry can be reconstructed (Step B). The reconstructed geometries can be used for Finite Element Analysis (FEA) and indentation-based subject-specific mechanical property evaluation (Step C). Devices and methods that can provide for mechanical property evaluation are described in Sections 1 and 3-10 herein.

Further, from the derived geometry, anatomical landmarks can be identified (Step D), allowing for the automated creation of socket source geometries and combined FEA models (Step E). In particular, using the anatomical markers, the socket cut-lines can be automatically created (Step D). The liner and socket source geometries can be offset from the skin surface and can be meshed with the soft tissue to form a single FEA model (Step E). The socket source geometry can be directly derived from the subject's unloaded geometry and, therefore, does not pre-load the tissue. Hence, to formulate a final socket design, the socket geometry can be altered to enhance loading at safe sites and prevent loading at vulnerable sites. Furthermore, compliant socket materials can be used to relieve loading. The design process can include defining spatially-varying socket stiffness and local fitting pressures for the socket (Step F), which can morph the socket into its desired, fitted shape (shown schematically as Step G by the arrows acting on the tissue).

Further, spatially varying mechanical properties can be assigned. By using multi-generational material theory, the socket design can be freely morphed (without developing socket material stresses, hence the socket is shown as transparent in Step G) while donning-induced pre-loads develop in the soft tissue. Once the desired socket equilibrium design is obtained, the socket materials are defined, or "solidified," in a stress-free state while the soft tissues have developed stresses due to the simulated fitting or donning of the socket. Using this process, the designs are therefore initiated, fitted, and donned onto the patient (Step H).

Next the designs can be are evaluated using FEA by applying body weight loading (illustrated schematically by an upward arrow in Step H) allowing for evaluation of skin surface pressures and soft tissue strains (Step I). Based on the simulated tissue loading conditions, the spatially varying socket materials and the fitting pressures can be iteratively optimized as indicated by the circularity of the process in Steps F-I.

Once the iterative virtual test socket optimization process has converged on an optimal design, the socket design can be exported for 3D printing based manufacture (Step J), creating a fully data-driven process to obtain the final socket (Step K).

Steps F-I of FIG. 89 can be realized in different ways and can be expanded. FIG. 90 shows a schematic illustration of an expanded virtual prototyping and optimization routine. The process starts with subject states 1300, including for example, a normal state 1300a, a compressed state 1300b, and an expanded state 1300c. Such subject states can include both geometric and mechanical property data. A subject state defines all information and data required to generate an initial design. The subject state may therefore include subject geometry (e.g., internal and external geometry and anatomical features) and mechanical properties (e.g., stiffness, hyperelastic parameters, viscoelastic parameters, and poroelastic parameters).

Multiple states can be used in the design and design optimization process. In FIG. 90, an example for a potential multitude of states 1300a, 1300b, 1300c is denoted. For lower limb amputees, it is well known that a limb can undergo shape and volume changes due to loading, and with use of sockets, across different time scales. Therefore, three subject states may, for instance, describe a subject's nominal state and several altered (e.g., shrunk, swollen) states. The states may be altered in terms of any aspect of the information and data contained in them, including mechanical and geometrical features.

The next step is the design formulation process. The word design here encapsulates all physical properties of relevance to the socket performance and includes the shape, geometry, and the (global or spatially varying) mechanical properties. Once an initial design 1310 is formulated, such as with initial fitting pressures, it is tested in a virtual prototyping environment. This environment consists of a computational representation of the subject 1312 as a computational model, a computational model of the socket 1314 (or other biomechanical interface device), and a computational biophysical analysis framework (which may include finite element analysis), allowing for the computation and analysis of the interaction of the proposed devices with the subject. Within the virtual environment, the socket 1314 can be fitted onto the virtual subject 1312 for evaluation of socket performance.

The evaluation procedure can include simulation of the donning of the prosthetic liner and/or socket (*e.g.*, with initial fitting pressures) as well as the simulation of use cases 1320 *(e.g.,* with loading pressures during use). What is termed "use case" is information and data (and boundary conditions) for simulating a particular event and/or a particular interaction between the socket and the user. These events may be static or dynamic in nature. The use cases may for instance represent standing, walking up stairs, and running, as denoted by the schematic illustrations in FIG. 90. However, the use cases may be any task that is relevant for the subject. The use cases may be generic or default use cases (*e.g.*, based on a library of use cases) or may be subject specific. Subject specific use cases may stem from dedicated quantitative measurements. For instance, simulation of walking may be based on subject motion capture data. Furthermore, joint bending exercises may be conducted and combined with non-invasive imaging, such as digital image correlation, MRI or Near-infrared Light Imaging, thereby providing subject specific joint motion data. The use cases may be physical in that the subject is likely to experience such an event, or may be unlikely, non-physical, or "superhuman." The latter may be useful to push the optimization beyond what is expected. An example of this would be the desire to allow for a certain safety margin for a particular biophysical finding, for instance maximum stress in the socket material, or maximum pressure on the tissue.

For evaluation, one or more use cases 1320 are simulated and subject specific biophysical conditions relevant to optimization can be computed. Any relevant biophysical metric can be simulated and studied and this may include tissue loading (*e.g.*, stresses and pressures) and tissue motions and deformations (*e.g.*, tissue strains). As an example, computed surface loading pressures 1322, 1324, and 1326 for each use case are shown in the contoured data in FIG. 90. A chosen set of simulated biophysical data can be processed to provide an objective function for iterative socket improvement or optimization. For instance, if socket comfort is deemed related to external skin pressure and internal tissue strain, these metrics can be used to inform an objective function (which may be a single scaler, a matrix, an array, a vector, or any general tensor quantity). The process of optimization then aims to minimize this objective function. The objective function can be formulated for a single use case or may be formulated to represent analysis for multiple use cases. The latter allows one to optimize the device for a multitude of use cases simultaneously. For instance, the comfort of a prosthetic socket can be made optimal for standing *and* walking rather than standing *or* walking. Any combination of use cases can be utilized for optimization. Besides the analysis of different use cases, the virtual design evaluation process can incorporate all subject states. As such, an optimal device design 1330 can be achieved given a set of subject states (*e.g.,* nominal, swollen, shrunk) and use cases (e.g., standing, stair climbing, running). Again, this may be used to inform a multi-parameter objective function or one that is captured by a single scalar objective.

The use cases may be established prior to analysis (*i.e.* such that the virtual prototyping takes place after all data are collected) or may take place in real time. For instance, a subject may be instrumented with fiducial markers for body motion capture and skin pressure sensors. This real-time experimental data can be coupled with real-time virtual prototyping by continuously simulating the current motion in the evaluation step. The socket can then be adjusted in real time in an iterative fashion to provide an optimal design given the custom real-time activities performed.

Once the objective function is evaluated, it can be used to inform the design for the next iteration. The particular method of driving this design process based on the objective function is what forms the optimization method. The term "optimization method" is here not used in its strict mathematical form. Instead, it stands for any method which employs the objective function data to form a new design, with the aim of minimizing properties or numerical values of the objective function. Therefore, the optimization method may include gradient decent methods, stochastic methods, evolutionary algorithms, genetic algorithms, topology optimization, and generative and rule based design algorithms, or any combination thereof. Any hybrid methodology may also be proposed (e.g., a staggered approach whereby first a stochastic regime is used, which is then followed by a second step based on gradient decent methods).

The generative or rule based design algorithms do not seek a minimum per se, but are formulated such that iterative changes lead to iterative improvement. Generative methods may rely on mechanobiological methods. For instance, mechanobiological processes have been described where cartilage and bone tissue adjust *(e.g.,* to achieve some improvement in conditions) in response to biophysical and mechanical stimuli (e.g., loading). Such theories may also be employed to update the biomechanical interface device. For instance, local biophysical stimuli (e.g., local material stresses and strains) may lead to local changes in design parameters (e.g., local shape changes and mechanical property alterations). The device material can be conceptualized as a virtual living material that responds to biophysical stimuli *(e.g.,* by locally resorbing or depositing materials to thereby influence its compliance).

The initial modeled biomechanical interface can be rigid and apply a homogeneous pressure upon donning (e.g., an initial fitting pressure). Next, after a particular use case is simulated, such as static standing in the case of a leg device, the effective local compliance of the socket wall can be increased in regions where loading pressures are deemed too high. Once the compliance is increased within the relevant region of the modeled interface, the use case is simulated once again, and the interface-induced loading pressures are once again determined. If the loading pressures are still too high across the relevant anatomical region, the compliance can once again be increased. In this iterative design framework, the shape of the interface remains unchanged and is equal to the shape that the biological segment assumes at the applied donning homogeneous pressure. At each interface location that exerts pressures on the biological residuum, compliance can be minimized with the constraint that the interface-induced loading pressures fall below a maximal, physiologically-acceptable level for a simulated use case, such as standing. Through this approach, the interface can be maximally stiff while satisfying loading pressure constraints at each anatomical point during a particular use case, such as standing. In the case of a prosthetic limb, the socket interface can be iteratively designed using this approach where socket wall compliance can be optimized computationally at each anatomical loading point, resulting in a final socket design with a spatially varying wall compliance optimized for ideal loading pressures.

The physiologically-acceptable pressure levels may vary spatially across the biological residuum, as different anatomical regions may have a distinct capacity for load bearing. For example, the transtibial residual limb area can be divided into four distinct biomechanical regions, as shown in FIG. 120A. Three of the four illustrated regions are considered in the following example, including: the patellar bar (region no. 2 in FIG. 120A), the distal end (region no. 3 in FIG. 120A), and the remaining, residual area (region no. 4 in FIG. 120A). These areas are distinct because each has a different load-tolerance; of the three regions, the patellar bar and distal end are the best and the worst load-tolerant regions, respectively, whereas the residual area has an intermediate load-tolerance. Such load tolerances may vary from anatomical point to anatomical point, as well as from person to person, and can be empirically determined (*See* Lee, W. C., Zhang, M. & Mak, A. F. (2005), Regional differences in pain threshold and tolerance of the transtibial residual limb: including the effects of age and interface material. Archives of Physical Medicine and Rehabilitation, 86 (4), 641-649, the entire contents of which are incorporated herein by reference).

Once the design evaluation and compliance updating procedure has iterated to a spatially-varying, compliant design where all pressures at each anatomical point are below the maximal physiologically-acceptable levels, further compliance adjustments can be made to maximize the uniformity of the pressure differential at each anatomical point between the predicted pressure applied on the body by the interface during a use case (e.g., standing), and the maximal physiological pressure at that same point. For example, if all anatomical regions have a socket-induced loading pressure that falls below the physiologically-acceptable pressure level, but there exists one anatomical region where the interface-induced loading pressure is near the maximal physiological pressure level, interface compliance can be increased in that region in order to lower the applied pressure and thereby increase the differential between the applied pressure and the physiologically-acceptable pressure level for the particular use case (e.g., standing). By maximizing the pressure differential and/or the uniformity between maximal acceptable levels and the load bearing pressures at each anatomical point across the surface of the biological residuum during a simulated use case such as standing, socket interface comfort can be optimized. This particular example provides for a rule-based socket improvement/optimization framework. Although the example describes the design of a compliant prosthetic socket, the framework can be applied to other biomechanical interfaces such as, but not limited to, shoes, seats, braces, and bras.

The maximal physiologically-acceptable pressure level estimate can be in terms of pain threshold or pain tolerance. The pain threshold at an anatomical location is defined as the minimum pressure required to induce pain at that point. In distinction, the pain tolerance is defined as the maximum tolerable pressure at an anatomical point. Both the pain threshold and pain tolerance can be quantified for a biological body segment. For example, pain threshold and pain tolerance have been quantified for a transtibial residual limb at distinct anatomical locations, as described in Lee, W. C., *et al.*. Alternatively, such data can be obtained by testing of a biological body segment, such as by indentation tests.

Biomechanical interface geometry can be iterated to control for interface-induced loading pressures for a particular use case, such as standing. In this approach, different initial values of uniform fitting pressures, representing the loads applied on the residuum after donning the interface, but before load bearing, are applied to distinct anatomical regions. For example, the transtibial residual limb area can be divided into three distinct biomechanical regions, as shown in FIG. 120A: the patellar bar (region no. 2), the distal end (region no. 3), and the remaining, residual area (region no. 4). The soft tissue deformations caused by these donning pressures define socket geometry. FEA can then be performed to compute the loading pressures applied on the skin surface as a consequence of a use case, such as standing *(i.e.,* where body weight would be applied). Subsequently, the average loading pressure can be computed within each anatomical element, and the fitting pressures in anatomical elements that result in higher than average interface-induced loading pressures are reduced, while the fitting pressures in anatomical elements that result in lower than average loading pressures are increased. Following this numerical step, FEA can be performed once again to re-calculate the loading pressures resulting from a particular use case, such as standing. This numerical process can be repeated until the loading pressures are below the physiological tolerance at each anatomical point and/or region. Once below the physiological tolerance within an anatomical region with a distinct physiological tolerance, the variance among loading pressures can be minimized. Once below, the numerical process can be repeated until the variance of the interface-induced loading pressure distribution within each region is no longer decreased with additional numerical iterations. Through this methodology, an optimized level of pressure uniformity within each anatomical region upon loading onto the interface can be obtained. Still further, the differential between the loading pressure and the physiological tolerance at each anatomical point and/or each anatomical region can be maximized, and/or the variance of the differentials among anatomical points or anatomical regions can be minimized.

This iterative interface design optimization process is illustrated in FIGS. 120B-D for the particular example of a transtibial socket. For a pilot study employing this design methodology, FIG. 120B shows final fitting pressures from a representative socket after this iterative process had been conducted, and FIG. 120C depicts corresponding loading pressures estimated using FEA for a standing use case. FIG. 120D shows the final socket design that, upon donning, results in the fitting pressures shown in FIG. 120B, and upon body weight loading, results in the loading pressures shown in FIG. 120C.

Given a set of subject states and a set of use cases, the virtual prototyping and optimization procedure may be defined to terminate with a single proposed optimal design. However, branching in the optimization process can also occur. For instance, in the case that the optimal state for a given use case departs too much from the optimal state of another, as computable from some distance metric defined over the objective function data, two optimal designs can be generated. An example of such a case is that an optimal socket for standing does not agree with an optimal socket for running. As such, the optimal socket for running may not be suitable for standing and *vice versa.* The system can be defined to allow for the branching of the optimization (after some definable cost is exceeded) such that more than one design is proposed. It may be that an all-round average design is still proposed, but that use case specific designs are also provided. The same branching procedure may be chosen to apply to subject states, *e.g.*, it may not be possible to define a single sufficiently optimal socket for all subject states. In this case, multiple designs may again be proposed.

*Quantitative Alignment of Lower-extremity Prosthetic Interfaces*

An algorithm to define a mechanical axis, load line, and alignment of a quantitatively designed prosthetic interface is provided. A prosthetic leg socket can be aligned to prosthetic alignment componentry using a quantitative algorithm. As shown in FIGS. 119A-B, the mechanical axis and load line within the coronal plane are defined for the lower-extremity. The load line can be used to define the location and orientation of prosthetic socket alignment components relative to the prosthetic socket interface. As illustrated in FIG. 119A, the coronal plane mechanical axis is approximately vertical during quiet standing for a person without leg amputation. Therefore, the load line for a person with leg amputation wearing a socket can be parallel to the mechanical axis so as to reduce misalignment.

The lower-extremity, coronal mechanical axis is defined as a straight line connecting the coronal plane center of the head of the femur to the coronal plane center of the ankle joint (FIG. 119A). This mechanical axis passes approximately 8mm lateral of the apex of the tibia (FIG. 119B). For both the transtibial and transfemoral socket interfaces, the coronal plane load line is defined as being parallel to the mechanical axis.

To find the mechanical axis for a transtibial amputation case, the transtibial limb can first be imaged using a non-invasive imaging modality (e.g., CT, MRI, or Ultrasound). Typically, the imaging volume comprises the amputated residuum (amputated fibula, tibia), the patella, and the distal aspect of the femur. To estimate the mechanical axis orientation, an intact femur model, scaled to the length of the patient's unaffected-side femoral length, is aligned with the imaged distal femur segment of the person with transtibial amputation. An iterative closest point (ICP) algorithm can be used to register the intact femur model to the distal femur portion estimated from non-invasive imaging (FIG. 119C shows the registered state of the two models). Following this analysis, the mechanical axis can be defined as passing from the coronal plane center of the femoral head to a point 8mm lateral of the apex of the tibia (FIG. 119B). The composite bones of the femur, patella, tibia, and fibula can then be rotated until the mechanical axis is aligned vertically within the simulation space. Alternatively, a mechanical axis can also be estimated fora transtibial amputation by fitting both an intact femur bone model and an intact tibia bone model to, respectively, the distal femoral portion and the proximal tibial portion of the residual limb, as derived from the imaging data. In this approach, an iterative closest point (ICP) algorithm can be used to register the intact femur bone model to the distal femur portion estimated from non-invasive imaging, and to register the intact tibia bone model to the proximal tibia portion estimated from non-invasive imaging. The lower-extremity, coronal-plane mechanical axis can then be defined as a straight line connecting the coronal plane center of the head of the femur to the coronal plane center of the ankle joint (FIG. 119A).

This mechanical axis analysis procedure estimates the bones' spatial orientation during quiet standing if the leg had not undergone amputation. The load line is then defined as being parallel to the mechanical axis while passing through the lowest point of the amputated transtibial residuum. The lowest point of the residuum is defined as its most distal anatomical point on the skin surface when the mechanical axis is made vertical in the simulation space. The load line then defines the position and orientation of the distal socket alignment componentry. For example, if a prosthetic pyramid is placed at the base of the transtibial socket, the coronal plane load line would pass through the coronal plane pyramid center point, and the longitudinal axis of the pyramid would be parallel with the load line. The red point at O in FIG 119C illustrates a pyramid center point obtained using the above algorithm.

Fora transfemoral amputation case, the mechanical axis can be defined as passing through the coronal plane center of the head of the femur where the angle between mechanical axis and the amputated femoral anatomic axis is 6 degrees (or within a range of about 10 to about 2 degrees). The amputated femoral bone can then be rotated until the mechanical axis is vertically aligned in simulation space. After this rotation, the femur can be obliquely aligned inwardly with the approximate knee location (e.g., phantom knee) being medial to the femoral head. To define the femoral anatomic axis, the amputated femoral point cloud can be processed by using singular value decomposition and then selecting the principal axis. Following this analysis, the load line used to align the transfemoral socket is made parallel to the mechanical axis while also passing through the lowest point of the amputated residuum. The lowest point of the transfemoral residuum is defined as its most distal anatomical point on the external skin surface when the amputated femur's mechanical axis is vertically aligned in simulation space. Similar to the transtibial case, the coronal plane load line defines the position and orientation of the distal socket alignment componentry. For example, if a prosthetic pyramid is placed at the base of the transfemoral socket, the coronal plane load line would pass through the coronal plane pyramid center point, and the longitudinal axis of the pyramid would be parallel with the load line.

For the sagittal plane alignment of transtibial and transfemoral sockets, the sagittal-plane anatomic axis of the amputated tibia and femoral bones can be used to define the sagittal plane pyramid center, respectively. Further, the sagittal-plane, longitudinal pyramid axis for the transtibial and tranfemoral sockets can be made parallel to the sagittal-plane anatomic axis of the tibia and femur bones, respectively. Here again, to define the anatomic axis, the amputated bone point cloud can be processed using singular value decomposition and then selecting the principal axis.

*Device Aesthetics*

Besides metrics of biophysical importance (e.g., local shape and compliance influencing comfort), the aesthetic of a device may be critical to the acceptance of the design by a particular subject. As such, aesthetic desires and requirements can be incorporated into the virtual design and design optimization process. Representing the aesthetic features in the optimization can also be included if the aesthetic parameters influence the biophysical performance, or if the features optimizing the biophysically motivated objective function influence the aesthetic qualities of the design. Particular cosmetic/aesthetic metrics can also be computed and can be added to the objective function formulation for inclusion in optimization.

If, on the other hand, the aesthetic design features do not influence the biophysical performance that the on which the optimization is focused, the aesthetic design features can be directly coded to be added after the optimization scheme has terminated (e.g., extrusions, cuts, extensions, lofts, rounds, can be directly coded). Exterior surface of a proposed design may also be subject to generative art (e.g., organically grown features). A particular cosmetic or aesthetic set of features may be formulated and/or parameterized freely in agreement with the subject. Alternatively, the aesthetic nature of the device may be linked to subject specific measurements. In FIG. 91, an example process is illustrated whereby a design 1340 is updated to include a set of cosmetic features 1342 such that a resulting prosthesis 1350 and cosmesis 1352 resembles a mirrored form of an unaffected limb 1344 of a subject.

**12. Generation of Functionally Graded Porous Materials**

Biomechanical compatibility is an important factor for a wearer of a biomechanical interface. An interface that is biomechanically compatible transfers loads to the underlying tissue appropriately and is able to do so without causing discomfort or injury. For prosthetic sockets, biomechanical compatibility can be obtained through an appropriate spatially varying fit or tightness of the socket. Furthermore, since loading conditions and the patient's limb geometry can vary with time, it is beneficial to incorporate compliance in the biomechanical interface, such as in the form of a deformable socket. Besides biomechanics, thermal conditions in the socket can also be considered and optimized. For example, breathability (e.g., to avoid heating, sweating) is desirable. Realizing breathable and compliant prosthetic sockets has been challenging due to the lack of suitable materials available.

A framework for the design of compliant and breathable prosthetic sockets based on porous lattice structures is provided. While application to prosthetic sockets is described, the described framework can apply equally well to other types of biomechanical interfaces, such as shoes, bras, braces, orthoses, seats and exoskeletons.

Spatially varying compliance of a socket can be achieved by locally varying a geometric structure of a lattice material that forms a biomechanical interface of the socket. Porosity and strut thickness can also be varied. The biomechanical interface can be designed and manufactured to include spatially-varying structures (e.g., structures in which internal geometry is varied). Such structures can also controllable; for example, the internal geometry of such structures can be defined and can provide a desired compliance. Such controllable and spatially-varying structures can be manufactured, such as by 3D printing.

Examples of structures that can form a material for a socket are shown in FIG. 92A. For example, changes in effective stiffness can be achieved by altering a type of structure within the lattice, such as with straight struts 1410 and coiled struts 1420, and/or by altering the lattice structure itself, such as face-based lattices 1430a (shown in a compressed state as 1430b) and edge-based lattices 1432a (shown in a compressed state as 1432b). For FEA-based prosthetic socket design, porous structures, exhibiting fine detail and many elements, are undesirable. To overcome this problem, a solid formulation 1434a (shown in a compressed state as 1434b) can be fitted to the FEA design, the solid formulation capturing the mechanical behavior of the lattices 1430a, 1432a.

An example of an FEA-based socket design 1440 is shown in FIG. 92B. Desired elastic behavior for the socket can be modeled with solid formulations, as shown in the socket design 1442 of FIG. 92C. Such formulations can be used in FEA-based socket design optimization. Desired elastic behavior can then be converted to lattice structures, such as structure 1444, shown in FIG. 92C, which can be manufactured using additive manufacturing (e.g., stainless steel, as shown in FIG. 92C). Through the use of coiled features, the stiffness of the metal lattice structures can be reduced to enhance local compliance.

Spatially varying the functional properties of a material, such as stiffness, density, porosity, and conductivity (e.g., thermal conductivity), can be realized using structural variations in the material. Such structural variations can be incorporated in porous materials. Two families of porous materials are defined here: 1) cellular solids, and 2) lattice structures. Methods for the generation of these material types are provided. In particular, two general lattice structure types are presented: 1) edge based lattice structures, and 2) face center based lattice structures.

*Cellular solids*

One means of creating a spatially varying porous material configuration is with the use of cells or compartments. Such materials are termed cellular solids. Regions in space are tessellated using a particular distribution of polyhedral elements. The walls of the cellular regions, and the interior of the cellular regions can be realized in two different materials. Alternatively, the cell interiors can be realized as voids leading to a hollow cellular material. An example of a cellular solid is provided in FIG. 93 whereby a regular rhombic-dodecahedron 1510 is used to tessellate 3D space to produce a cellular tessellation 1512 (shown in perspective and cross-sectional views). All elements 1514 in the structured cellular solid 1512 are of equal volume.

A method for the creation of cellular solid structures is provided. A tessellation can be realized with cells of spatially varying volume. An example of this is provided in FIG. 94, whereby a space is tessellated using tetrahedral elements 1520. Here, a general tetrahedral meshing method was used. The left side 1526 of a rectangular object 1512 was meshed using highly refined elements while larger elements where used on the right side 1524 of the object 1522 (shown in perspective and cross-sectional views). This way, functionally graded materials can be realized with spatially varying mechanical properties and porosity. Properties such as anisotropy, thermal conductivity, and breathability, and other physical properties influenced by the cellular nature of the material, can also be realized with functionally graded materials.

To create structural variations of cellular meshes (either locally or globally) the cells can be converted to conforming cellular structures of a different type. For example, as shown in FIG. 95, a tetrahedral element 1520 can be converted to an element 1530 comprising a set of 4 hexahedral elements.

Another method for obtaining structural variations of a cellular solid mesh is to locally or globally use a dual of the cell. In FIG. 96 an example is shown for the icosahedron 1540 whose dual is a dodecahedron 1510. Such a dual description can be obtained by connecting local face centres to form new faces. Alternative dual descriptions can be obtained when new faces are generated from edge centres instead. Since the dual may contain altered connectivity (e.g. the example in FIG.96 shows the ability to switch between cells with 5 sided faces and 3 sided faces), properties such as the mechanical properties are altered as well.

*Lattice structures from triply-periodic functions*

Methods for creating conforming 3D lattice structures in rectangular domains (which can be a single hexahedral element) are presented here through the use of iso-surfaces on scalar valued 3D functions. Triply-periodic functions can be used, such as:

$$M(x,y,z,f) = \cos(fx) + \cos(fy) + \cos(fz) \qquad \textbf{54}$$

where *x, y, z* are Cartesian coordinates and f is the frequency. This formulation can be used to retrieve the so-called Schwarz p-surface, a triply-periodic minimal surface, since it exists where:

$$M(x,y,z,f) = 0 \qquad \textbf{55}$$

Six examples of triply periodic functions are shown in FIGS. 97A-F.

The frequency can be altered locally or globally to create lattice pattern densities spatially. To create a closed surface description with a certain thickness (or porosity) two threshold levels can be used:

$$m_1 < M(x,y,z) < m_2 \qquad \textbf{56}$$

By altering the values of the thresholds $m_i$ the structure and the porosity can be adjusted. To refine the structure in a particular domain, multiple periods can be used. Lattice structures can thereby derived. Lattices can be manufactured (e.g., 3D printed) from, for example, any of the structures shown in FIGS. 97A-F.

*Mapping based on iso-parametric formulations*

When simplices are meshed using volumetric elements, and it is of interest to define a common type of geometric feature *(e.g.,* a lattice structure) in each of the elements, it can be convenient to use iso-parametric formulations. Essentially, iso-parametric formulations allow one to map a lattice structure defined in a single chosen element to a whole set of elements of the same type. Below, an example is given for tetrahedral elements, but similar iso-parametric formulations can be defined for other volumetric element types (such as hexahedral elements).

For a tetrahedron, the 4 element nodes can be expressed in Cartesian coordinates or using an iso-parametric coordinates. Iso-parametric coordinates are sometimes termed natural coordinates, quadray coordinates, simplicial coordinates, and in the case of tetrahedrons, tetrahedral or barycentric coordinates. The natural coordinates are denoted $\xi$ = $[\xi_1\ \xi_2\ \xi_3\ \xi_4]^T$, the component $\xi_i$ is 1 at node i, is zero at all other nodes and the opposing face, and varies linearly in between as a function of the distance to the opposing face. Since only three coordinates are independent in 3D space, the natural coordinates sum to 1, leading to the constraint:

$$\xi_1 + \xi_2 + \xi_3 + \xi_4 = 1 \qquad \mathbf{57}$$

Following these definitions, the natural coordinate system can be used to specify interpolation functions. For a tetrahedron, where a function *F(x, y, z)* has the values $F_i$ at its nodes, the following interpolation function can be specified:

$$F(x,y,z) = F(\xi_1,\xi_2,\xi_3,\xi_4) = F_1\xi_1 + F_2\xi_2 + F_3\xi_3 + F_4\xi_4 = F_i\xi_i \qquad \mathbf{58}$$

Here, Einstein summation convention over repeated indices is implied. The interpolation can also be used for Cartesian coordinates allowing for the derivation of a particular point's position vector p based on its natural coordinates $\xi$:

$$\mathbf{p} = \begin{bmatrix} x \\ y \\ z \end{bmatrix} = \begin{bmatrix} x_i\xi_i \\ y_i\xi_i \\ z_i\xi_i \end{bmatrix} \qquad \mathbf{59}$$

This leads to the matrix expression:

$$\begin{bmatrix} 1 \\ x \\ y \\ z \end{bmatrix} = \begin{bmatrix} 1 & 1 & 1 & 1 \\ x_1 & x_2 & x_3 & x_4 \\ y_1 & y_2 & y_3 & y_4 \\ z_1 & z_2 & z_3 & z_4 \end{bmatrix} \boldsymbol{\xi} \qquad \mathbf{60}$$

Inversion of this expression allows for the derivation of a points natural coordinates $\xi$ based on its Cartesian position vector **p** and the Cartesian coordinates of the local element nodes.

The above shows how when a desired structure pattern is specified for a single template element *(e.g.,* a tetrahedron or a hexahedron) this structure can be mapped into arbitrary elements of this type *(e.g.,* a 3D volumetric mesh consisting of many tetrahedral elements). FIGS. 98A-B illustrate a mapping of a simple lattice structure 1550, in particular, a structure with struts going from the center of each face to the center of each element, to form a continuous lattice structure 1552.

*Direct conversion from volumetric elements*

A method is provided for the creation of lattice structures from general volumetric mesh descriptions. FIG. 99 shows how arbitrary surface meshes 1560 can be meshed using volumetric elements 1562, which can be converted to lattice structure descriptions 1564.

The example in FIG. 99 presents tetrahedral element conversion which can be implemented from using established tetrahedral meshing methods (e.g., as described in Si H (2015) TetGen, a Delaunay-Based Quality Tetrahedral Mesh Generator. ACM Trans Math Softw 41:1-36. doi: 10.1145/2629697, the entire contents of which are incorporated herein by reference). However, mixed meshes are also possible. For instance, mixed hexahedral and tetrahedral meshing can be generated, as shown in FIG. 100. Mixed meshing can be generated by first converting the input surface description to a 3D image, where internal voxels form hexahedral elements. Next, the remaining space can be tessellated using tetrahedral elements. While the method is described with respect to hexahedral and tetrahedral meshing, other combinations of shapes can also be generated.

For tetrahedral meshing, the local element volumes can be controlled to be constant or spatially varying. An example of a spatially varying mesh density is shown in FIG. 101.

Furthermore, to create mesh variations, these elements can be subdivided or converted into different element types. For instance, a hexahedral element 1570 can be converted to various tetrahedral elements 1572, 1574, 1576, 1580, and 1582, as shown in FIG. 102. Also, a tetrahedral element 1520 can be converted to a hexahedral element 1530, as shown in FIG. 95.

Through these operations, the volumetric element descriptions can be converted to different structural variations with varying physical properties (e.g., mechanical properties).

Two types of direct conversion methods are described to obtain lattice structures form the above volumetric mesh descriptions. One method includes shrinking all elements and all faces and connecting the original and shrunk node sets. This yields the so-called element edge lattice. FIG. 103 illustrates examples of how the edges of a hexahedral element can be converted to various edge lattice structures. The density of the lattice can be increased by subdividing the element, as shown, for example, with lattice 1584 being converted to lattice 1586. The structure of the lattice can be altered by converting the mesh, for example, from a hexahedral mesh lattice 1584 to a tetrahedral mesh lattice 1588. The process of conversion and subdivision can be repeated, as shown with lattices 1590, 1592, 1594, to obtain numerous structural variations, all of which conform to the original input boundary.

The effective porosity can be varied, as shown in FIG. 104, by altering an amount of shrinkage used during lattice structure creation. A shrinkage factor, which may be spatially varying, can therefore provide the lattice strut thickness and porosity. For example, a lattice 1596 having a relatively thin strut thickness can be converted to lattice 1598 or lattice 1600 having relatively thicker strut thicknesses, or vice versa.

As an alternative to edge-based lattices, complementary face lattices can be created. Similar to an edge based lattice, the elements and the faces of the lattice can be shrunk. However, for a face lattice, the original coordinates are omitted and a novel lattice structure is instead formed by connecting the nodes formed by shrinking the faces and elements. FIG. 105 shows three types of edge lattices 1602, 1604, 1606, and their complimentary face lattices 1612, 1614, 1616, respectively. The edge-based and face structures can also be combined or interleaved together, as shown with lattices 1622, 1624, and 1626. The lattices can be made to touch by altering the shrink factor or the lattice structure type. For example, the edge lattice 1604 touches the face lattice 1614 to form the combination lattice 1624.

FIG. 106 illustrates edge-based lattices 1632, 1634, 1636 and complementary face lattices 1642, 1644, 1646 for tetrahedral element descriptions. Combination lattice structures 1652, 1654, 1656 are also shown.

The method of obtaining lattice structures from volumetric element descriptions can be convenient, as many volumetric meshing methods exist for arbitrary input surfaces. FIG. 107 illustrates volumetric tetrahedral meshes 1662, here on a prosthetic socket 1660, being directly converted to a lattice structure 1664 with spatially varying properties for the same socket 1660.

Another method of varying the effective properties of a lattice structure includes forming hierarchical structures. A direct volumetric-to-edge lattice conversion method can also be used to export element descriptions, *i.e.,* the lattice structure can be generated using volumetric elements (e.g. tetrahedral or hexahedral elements) that are generated on the lattice structure. As such, the volumetric elements on the lattice can again be converted to a lattice structure. This process can be repeated to produce hierarchical (or fractal) lattices of a desired degree. For example, as shown in FIG. 108, a tetrahedral element 1670 was converted to an edge lattice structure 1672 consisting of hexahedral elements. The hexahedral elements of lattice structure 1672 were then converted to an edge lattice to form hierarchal structure 1674. Similarly, a tetrahedral element 1680 was converted to an edge lattice structure 1682 consisting of hexahedral elements. The hexahedral elements of the edge lattice structure 1682 were first converted to tetrahedral elements prior to a second edge lattice iteration to form hierarchal structure 1684.

*Coiled lattice structures*

To modulate an effective stiffness of a lattice structure, coiled struts can be included within a lattice structure. Rather than linearly connecting two points to form a straight strut, a helical strut can be included. To allow for the helical feature to arrive from the first point to the second point in a smooth sense, Gabor coils can be included. A Gabor function is a sinusoidal function whose amplitude is modulated by a Gaussian function. Similarly, the proposed Gabor coils are helical functions (whose coordinates orthogonal to the helix axis are expressed using sinusoidal functions), which are modulated by a Gaussian function. The equation below shows a parameterization of a Gabor coil using the parameter t and the coordinates *x, y, z:*

$$\begin{gathered} x = \mathrm{G} \cdot \mathrm{A} \cdot \cos(t) \\ y = \mathrm{G} \cdot \mathrm{A} \cdot \sin(t) \\ z = \mathrm{t} \\ G(\sigma) = e^{-\frac{1}{2}\left(\frac{t}{\sigma}\right)} \end{gathered} \qquad \mathbf{61}$$

Here A is the coil amplitude, and G is the Gaussian function with standard deviation $\sigma$. This form can be used for any edge set to generate coiled features. The coiled struts can be obtained by sweeping a strut section along a coil path. FIG. 109A shows an example of a linear strut 1692 and examples of coiled struts 1694, 1696, 1698, each with a varying amplitude. FIG., 109B illustrates 3D lattice structures 1702, 1704 formed with coiled struts, including an edge-based lattice structure 1702 and a face lattice structure1704. The inclusion of coiled struts can allow for creation of highly compliant lattice structures.

*Multi-phasic structures*

Complementary lattice structures, such as complementary edge-based lattice 1602 and face lattice 1612 as shown in FIG. 105, can be combined in a space filling sense. For example, as shown in FIG. 110, for a hexahedral input mesh 1710, an edge-based lattice 1712 and complementary face lattice 1714 can be combined to form a multiphasic structure 1720.

The two structures 1712, 1714 can be of different materials. For instance, the edge-based lattice 1712 can be fabricated from a relatively stiff material while the face lattice 1714 can be fabricated from a relatively compliant material, or vice versa. Furthermore, one of the structures can be of a gel-like nature. For example, a waterproof structure can be created. The gel phase may also be a hydrogel capable of absorbing moisture at a biomechanical interface while the other complimentary phase provides structural support.

Additionally, one phase can be generated in a solid material, while the other phase is generated in a porous materia. For example, the face lattice 1714 can be of a hierarchical lattice structure, such as the structures 1674, 1684 shown in FIG. 108.

*Smoothing of lattice structures*

Smoothing of surface meshes suppresses sharp features and can be useful for suppressing noise. An example of a smoothing process is shown in FIG. 111. As illustrated, a structure 1720 comprises a noisy, or angular, mesh. The structure 1720 can undergo Laplacian smoothing to produce the smoothed structure 1722, or HC-Classes smoothing to produce the structure 1724. The semitransparent overlays 1732, 1734 illustrate a true, un-eroded geometry of the structure.

In particular, the noisy surface of the structure 1720 can be improved through smoothing techniques. A mesh with node set q can be iteratively smoothed. A simple smoothing method is Laplacian smoothing. For Laplacian smoothing the current point $\mathbf{q}_i$ is replaced by the modified point set $\mathbf{p}_i$:

$$\mathbf{p}_i = \frac{1}{|\mathrm{Adj}(i)|} \sum_{j \in \mathrm{Adj}(i)} \mathbf{q}_i \qquad \mathbf{62}$$

As such, for Laplacian smoothing, for each iteration, $\mathbf{p}_i$ is simply the mean of the coordinates of the local environment. The local environment is the adjacent point set to point i denoted by Adj(*i*). This type of smoothing is effective to reduce sharp features and noise, but can also accompanied by general shape changes visible as local shape shrinkage or swelling at convex and concave regions, respectively. Some methods aim to reduce these undesirable shape changes such as HC-classes smoothing. Structures 1722 and 1724 illustrate a comparison of Laplacian and HC-classes smoothing.

When smoothing is applied to meshes describing lattice structures, sharp edges can be suppressed and rectangular struts can become rounded. For instance, the straight struts featuring rectangular sections of, for instance, structure 1646 of FIG. 106 can be smoothed yielding rounded and smooth lattices, such as that of the structure 1740 in FIG. 112. However, as the series of images in FIG. 112 demonstrates, iterative smoothening also induces shape changes. In the case of the geometry in structures 1740, 1742, 1744, 1746, 1748, 1750, 1752, 1754, each structure progressively undergoing additional smoothing iterations, the face based lattice is seen to shrink.

FIG. 113 illustrates smoothing on an edge based lattice. The nodes for boundary faces were not altered during smoothing and were constrained to remain constant. Contrary to the structures of FIG. 112, the porosity of the lattices 1756, 1758, 1760, 1762, 1764, 1766, 1768, 1770 in FIG. 113 decreases with increased amounts of smoothing. For this geometry, increasing amounts of smoothing creates increasing amounts of "closure," with the appearance of an aperture closing itself.

These examples demonstrate that smoothing of the geometry offers a general method to alter strut thickness and porosity. The smoothing-induced shape and porosity variations may be global or local and can vary spatially. The tetrahedral domains in FIGS. 112 and 113 can be viewed as a region of space where a particular smoothing regime (smoothing parameters and iteration settings) was applied. However, the smoothing regime may be spatially varying such that varying degrees of smoothing are used across a larger lattice structure. Smoothing can be used to spatially vary a lattice structure shape and porosity.

*Mechanical behavior analysis*

Understanding the mechanical behavior of lattice structures can include performing experimental investigation, such as tensile, compressive, and shear loading, and computational modeling of the lattice structure, or a solid formulation representing the lattice structure.

To investigate compliance introduced through the use of a lattice structure, computational models of a cubic domain (36x36x36 mm) were created, which were subjected to tensile, compressive, and shear dominated loading (20% strain for each mode). FIG. 114 illustrates a solid cube and FIGS. 114B-D illustrate the solid cube subjected to the noted deformation modes. FIG. 115A illustrates a lattice structure and FIGS. 115B-D illustrate the lattice structure simulated to undergo the same deformations. Constitutive behavior for all structures was represented by Eqn. 44. Since only the relative reduction in stiffness was of interest, arbitrary material constants were assigned (c = 1 kPa, m = 6,$\kappa$ = 20 kPa).

FIGS. 116A-O illustrate the force responses as a function of time (all simulations were for 1 s) for the geometry types shown in FIGS. 114A and 115A-D. The solid model presents with maximum tensile, compressive and shear forces in the order of 0.85, -1.75, and 0.2 respectively. Comparison of these forces with the equivalent for the other geometry types allows for the estimation of a force reduction factor for each geometry (e.g., force of solid model divided by the force of the particular geometry case). Table 13 lists such factor estimates for tensile loading. As can be observed, through variations in porosity and structure, the maximum force generated under 20% tension can be reduced by a factor of up to 945. This means that the effective compliance can here be controlled and reduced to up to 3 orders of magnitude. Higher reduction factors may be possible with altered porosity, and altered structural and coil configurations. Note that the structures proposed can be made to conform to each other and connect in space such that a functionally graded material can be created.

Here, analysis focussed on maximum force for several types of geometries and loading regimes. However, this can easily be expanded to the analysis of other mechanical properties (*e.g.*, stiffness, non-linearity, viscoelasticity etc.) and other loading regimes.

**Table 13: The force reduction factors with respect to a solid material**

| Geometry type | Force reduction factor |
|---|---|
| Solid material (FIG. 114A) | 1 |
| An edge based lattice with porosity 0.8 (FIG. 115A) | 15 |
| An edge based lattice with porosity 0.95 (FIG. 115B) | 95 |
| A face based lattice with porosity 0.95 (FIG. 115C) | 212 |
| A coiled lattice with porosity 0.91 (FIG. 115D) | 945 |

The teachings of all patents, published applications and references cited herein are incorporated by reference in their entirety. While example embodiments have been particularly shown and described, it will be understood by those skilled in the art that various changes in form and details may be made therein without departing from the scope of the embodiments encompassed by the appended claims.

CLAUSES

1. A device for three-dimensional imaging of a biological body segment, comprising:

a structure configured to receive the biological body segment, the structure including a first array of imaging devices disposed about a perimeter of the device to capture side images of the biological body segment and a second array of imaging devices disposed at an end of the device to capture images of a distal portion of the biological body segment, the second array having a generally axial viewing angle relative to the perimeter; and
a controller configured to:

receive images captured from the first and second arrays, and
generate a three-dimensional reconstruction of the biological body segment based on cross-referencing of the captured images.

2. The device of clause 1, wherein the imaging devices are cameras and cross-referencing includes three-dimensional digital image correlation (DIC).

3. The device of clause 2, wherein the DIC is based upon a pattern printed on the biological body segment.

4. The device of clause 3, wherein the pattern is a speckle pattern.

5. The device of any one of clauses 2-4, wherein the controller is further configured to transform a two-dimensional image point visible in at least two captured images to a three-dimensional image point by direct linear transformation, with or without distortion correction.

6. The device of clause 1, wherein the imaging devices are ultrasound sensors.

7. The device of clause 6, wherein the structure includes a tank containing a fluid.

8. The device of any one of clauses 1-7, wherein the imaging devices of the first array are disposed to fully surround the perimeter of the biological body segment.

9. The device of any one of clauses 1-8, wherein the first array is moveable relative to the structure.

10. The device of any one of clauses 1-8, wherein the first array is stationary relative to the structure.

11. The device of any one of clauses 1-10, further comprising a mechanical perturbator.

12. The device of clause 11, wherein the mechanical perturbator includes a force sensor.

13. The device of clause 11 or clause 12, wherein the mechanical perturbator includes an ultrasound sensor.

14. The device of clause 11, wherein the mechanical perturbator is a flow-based perturbator comprising a nozzle configured to eject a fluid.

15. The device of any one of clauses 11-14, wherein the controller is further configured to determine a mechanical property of the biological body segment based on an inverse finite element analysis of a three-dimensional model of the body segment reconstructed from the captured images, the captured images including images of a deformation of the biological body segment by the mechanical perturbator.

16. The device of any one of clauses 11-15, wherein the controller is further configured to determine a mechanical property of the biological body segment based on a hyperelastography analysis of the captured images, the captured images including images of a deformation of the biological body segment by the mechanical perturbator.

17. A method of generating a three-dimensional reconstruction of a biological body segment, comprising:

capturing side images of the biological body segment with a first array of imaging devices disposed about a perimeter of the biological body segment;
capturing images of a distal portion of the biological body segment with a second array of imaging devices, the second array of imaging devices having a generally axial viewing angle relative to the perimeter; and
generating a three-dimensional reconstruction of the biological body segment based on cross-correlation of the captured images.

18. The method of clause 17, wherein the imaging devices are cameras and cross-correlation includes performing a three-dimensional digital image correlation (DIC) of the captured images.

19. The method of clause 18, further comprising transforming a two-dimensional image point visible in at least two captured images to a three-dimensional image point by direct linear transformation, with or without distortion correction.

20. The method of clause 18 or clause 19, further comprising applying a speckle pattern to the biological body segment.

21. The method of clause 17, wherein the imaging devices are ultrasound sensors.

22. The method of any one of clauses 17-21, wherein the imaging devices of the first array are disposed to fully surround the perimeter of the biological body segment.

23. The method of any one of clauses 17-22, further comprising mechanical perturbating the biological body segment.

24. The method of clause 23, wherein the mechanical perturbation is performed with an ultrasound probe, an ultrasound probe including a force sensor, a flow-based perturbator including a nozzle configured to eject a fluid, or any combination thereof.

25. The method of clause 23 or clause 24, further comprising determining a mechanical property of the biological body segment based on an inverse finite element analysis of a three-dimensional model of the body segment reconstructed from the captured images, the captured images including images of a deformation of the biological body segment by the mechanical perturbation.

26. The method of clause 23 or clause 24, further comprising determining a mechanical property of the biological body segment based on a hyperelastography analysis of the captured images, the captured images including images of a deformation of the biological body segment by the mechanical perturbation.

27. A method of modeling a biological body segment; comprising:

obtaining images of an internal structure of the biological body segment;
capturing images of an external surface of the biological body segment with a photogrammetric imaging device;
generating a three-dimensional model of external features of the biological body segment based on the captured images from the photogrammetric imaging device; and
inter-digitizing the images of the internal structure of the biological body segment with the three-dimensional model to thereby generate a compound model of internal and external features of the biological body segment.

28. The method of clause 27, wherein the inter-digitizing includes performing a shape registration of alignment points of the biological body segment.

29. The method of clause 27 or clause 28, wherein the images of the internal structure are obtained from computed tomography (CT) imaging, magnetic resonance (MR) imaging, ultrasound (US) imaging, or any combination thereof.

30. The method of clause 29, wherein the images are obtained from a medical image repository.

31. The method of clause 29, further comprising imaging the biological body segment with at least one of CT, MR, and US to obtain the images of the internal structure.

32. A method of manufacturing a biomechanical interface, comprising:

with the compound model generated in the method of clause 27, generating a complementary biomechanical interface; and
fabricating the generated biomechanical interface.

33. A device for three-dimensional imaging of a biological body segment, comprising:

an object including a plurality of inertial measurement units, the object configured to trace a surface of the biological body segment; and
a controller configured to:

receive motion data from each of the plurality of inertial measurement units,
determine trajectories of the object in a three-dimensional space based on the received motion data, and
generate a three-dimensional reconstruction of the biological body segment based on the determined trajectories.

34. The device of clause 33, wherein each of the plurality of inertial measurement units is a six-degree of freedom inertial measurement unit.

35. The device of clause 33 or clause 34, wherein the object is a sphere.

36. A three-dimensional measurement device for a biological body segment, comprising:

a) an elastomeric sheath conformable to the biological body segment;
b) a plurality of nodes affixed to the elastomeric sheath;
c) a grid of electrically-conductive conduits connecting the nodes; and
d) a plurality of first transducers at at least a portion of either the electrically-conductive conduits or the nodes, whereby data collected by the first transducers can be employed to generate a three-dimensional representation of at least a portion of the biological body segment.

37. The device of clause 36, further including a plurality of second transducers at at least a portion of the other of the electrically-conductive conduits or the nodes at which the first transducers are located.

38. The device of clause 36 or clause 37, wherein the first transducers are at the electrically-conductive conduits and include at least one member of a stretch sensor and a curvature sensor.

39. The device of any one of clauses 36-38, wherein the first transducers include stretch sensors.

40. The device of clause 39, wherein the stretch transducers include at least one parallel plate capacitive stretch sensor.

41. The device of any one of clauses 36-40, wherein the first transducers include curvature sensors.

42. The device of clause 41, wherein the curvature sensors include at least one member of the group consisting of a unipolar resistive curvature sensor and a bipolar resistive curvature sensor.

43. The device of any one of clauses 36-40, wherein the first transducers include at least one curvature sensor and at least one stretch sensor.

44. The device of any one of clauses 36-43, wherein the second transducers include at least one member of the group consisting of a biomechanical sensor, biometrical sensor, an ultrasound sensor, an ultrasonomicrometry crystal sensor and a sensory feedback component.

45. The device of clause 44, wherein the second transducers include biomechanical transducers.

46. The device of clause 45, wherein the biomechanical transducers include at least one member selected from the group consisting of skin modulus sensors, pressure sensors, shear force sensors, electromyographic sensors, and inertial measurement units.

47. The device of clause 44, wherein the biomechanical sensors include a piezoelectric material.

48. The device of clause 44, wherein the second transducers include biometrical sensors that sense at least one member of the group consisting of temperature, moisture, tissue depth, hydration, vascularization and peripheral nerve anatomy.

49. The device of clause 44, wherein the second transducers sense a plurality of biomechanical features of the biological body segment.

50. The device of any one of clauses 36-49, wherein at least a portion of the second transducers include a plurality of different types of transducers.

51. The device of any one of clauses 36-50, wherein at least a portion of the electrically-conductive conduits include an elastomeric component, whereby the electrically conductive conduits are essentially elastic.

52. The device of clause 51, wherein the electrically-conductive conduits further include conductive particles.

53. The device of any one of clauses 36-52, wherein the electrically-conductive conduits connecting the nodes have a serpentine shape.

54. The device of any one of clauses 36-53, wherein the biological body segment includes at least one member of the group consisting of a human chest, an abdomen, an arm, a leg, and a foot.

55. The device of clause 54, wherein the biological body segment is the human chest and the elastomeric sheath is a component of a bra, wherein the nodes include at least one member selected from the group consisting of skin modulus sensors and ultrasound transducers.

56. The device of clause 55, wherein the bra further includes at least one member selected from the group consisting of an electrocardiographic sensor, a respiration rate sensor, a pressure sensor, a shear force sensor, a temperature sensor, a heart rate sensor, a blood oxygenation sensor, a electrodermal response sensor, a molecular composition of sweat sensor, a moisture sensor, a tissue depth sensor, a hydration sensor, a vascularization sensor, a peripheral nerve anatomy sensor, an initial measurement unit, a light-emitting diode, and a vibration motor.

57. A system for generating a three-dimensional representation of a biological body segment, comprising:

a) a synthetic skin component, including:

i) an elastomeric sheath conformable to the biological body segment,
ii) a plurality of nodes affixed to the elastomeric sheath,
iii) a grid of electrically-conductive conduits connecting the nodes, and
iv) a plurality of first transducers at at least a portion of either the electrically-conductive conduits or the nodes; and

b) a handheld probe that includes at least one probe transducer selected from the group consisting of an ultrasound transducer, a pressure sensor, a sheer force sensor, a temperature sensor and an inertial measurement unit (IMU), whereby data collected by at least one of the first transducers and the probe can be employed to generate a three-dimensional representation of the biological body segment.

58. The system of clause 57, further including a plurality of second transducers at at least a portion of the other of the electrically-conductive conduits and the nodes at which the first transducers are located.

59. The system of clause 58, wherein the nodes are configured to interact with the probe to provide feedback to a user of the system.

60. A method of forming a biological body segment modeling device, comprising the steps of:

a) forming an elastomeric sheath that is conformable to the biological body segment;
b) applying a plurality of nodes to the elastomeric sheath; and
c) forming electrically-conductive interconnects between at least a portion of the nodes, wherein at least a portion of at least one of the nodes and the interconnects includes a first transducer.

61. The method of clause 60, wherein at least a portion of the other of the nodes and the interconnects includes a second transducer.

62. The method of clause 60 or clause 61, wherein the interconnects include an elastomeric component.

63. The method of any one of clauses 60-62, wherein the interconnects further include at least one electrically-conductive component selected from the group consisting of carbon black, silver, eutectic gallium-indium (EGaIn), and carbon nanotubes.

64. The method of any one of clauses 60-63, wherein the interconnects are serpentine.

65. The method of clause 64, wherein the serpentine interconnects are formed between the nodes by:

a) forming the serpentine interconnects on a silicon wafer;

b) transferring the serpentine interconnects to the elastomeric sheath by transfer printing;
c) forming islands at intersections of the serpentine interconnects; and
d) applying transducers at at least a portion of the islands, whereby the transducers can measure strain at the interconnects during flexing of the elastomeric sheath and associated movement of the serpentine interconnects.

66. A device for assessing tissue geometry and mechanical properties of a biological body segment, comprising:

a probe including an ultrasound transducer and a force sensor; and
a controller configured to:

receive data from the ultrasound transducer and the force sensor;
detect a tissue boundary within the biological body segment based on the received data, and
detect a mechanical property of soft tissue of the biological body segment based on the received data.

67. The device of clause 66, wherein the tissue boundary includes bone and the controller is further configured to determine a skin-to-bone depth.

68. The device of clause 66 or clause 67, wherein the mechanical property is based upon a detected indentation depth of the probe into the soft tissue.

69. The device of any one of clauses 66-68, further comprising an accelerometer.

70. The device of any one of clauses 66-69, wherein the probe includes a marker configured to permit tracking of the probe relative to the biological body segment.

71. A device for assessing tissue geometry and mechanical properties of a biological body segment, comprising:

a probe configured to deform soft tissue of the biological body segment, the probe including an ultrasound transducer; and
a controller configured to:

receive shear wave velocity data from the ultrasound transducer of soft tissue in an undeformed state,
receive shear wave velocity data from the ultrasound transducer of soft tissue in a deformed state, and
detect a mechanical property of the soft tissue based on a hyperelastography analysis of the received shear wave velocity data of the soft tissue in the undeformed and deformed states.

72. The device of clause 71 wherein the detected mechanical property is a non-linear elastic behavior of the biological body segment.

73. The device of clause 71 or clause 72 wherein the hyperelastography analysis includes determination of stiffness based on a tissue deformation.

74. A device for detecting a mechanical property of a biological body segment, comprising:

a structure configured to receive the biological body segment, the structure including an array of imaging devices disposed to capture images about a perimeter of the biological body segment;
a pressurization device configured to apply pressure to the biological body segment to deform soft tissue of the biological body segment; and
a controller configured to:

receive images captured by the array of the biological body segment in a plurality of deformed states, and
determine a mechanical property of the biological body segment based on cross-referencing of the captured images.

75. The device of clause 74, wherein the mechanical property is a tissue characteristic.

76. The device of clause 75, wherein the tissue characteristic is modulus, elasticity, stiffness, damping, viscoelastic parameter.

77. The device of clause 74, wherein the mechanical property is a bone-to-tissue depth.

78. The device of clause 74, wherein the mechanical property is a bone structure.

79. The device of any one of clauses 74-78, wherein the imaging devices are cameras and cross-referencing is performed by three-dimensional digital image correlation (DIC).

80. The device of clause 79, wherein the DIC is based upon a pattern printed on the biological body segment.

81. The device of clause 80, wherein the pattern is a speckle pattern.

82. The device of any one of clauses 74-81, wherein the controller is further configured to transform a two-dimensional image point visible in at least two captured images to a three-dimensional image point by direct linear transformation.

83. The device of clause 74, wherein the imaging devices are ultrasound sensors.

84. The device of any one of clauses 74-83, wherein the structure includes a tank containing a fluid.

85. The device of any one of clauses 74-84, wherein the imaging devices of the first array are disposed to fully surround the perimeter of the biological body segment.

86. A device for three-dimensional imaging of musculoskeletal tissue of a biological body segment under compressive loads, comprising:

a) a container defining a volume;
b) at least one ultrasound probe supported within the volume of the container, the ultrasound probe defining an ultrasound transducer surface; and
c) a pressurizing device that applies pressure to a biological body segment that includes musculoskeletal tissue and is placed within the container, the pressurizing device being disposed between the ultrasound transducer surface and the body segment, the ultrasound probe thereby being arranged to image the biological body segment.

87. The device of clause 86, including a single ultrasound probe.

88. The device of clause 86 or clause 87, wherein the ultrasound probe is moveable relative to the container, whereby the ultrasound probe can image the biological body segment from different aspects over time while the biological body segment remains stationary relative to the container.

89. The device of clause 88, including a plurality of ultrasound probes, at least one of which is moveable relative to the container.

90. The device of clause 89, wherein the plurality of the ultrasound probes is moveable relative to the container.

91. The device of clause 89 or clause 90, wherein at least one of the ultrasound probes is independently moveable relative to each of at least one other of the ultrasound probes and to the container.

92. The device of any one of clauses 89-91, further including an ultrasound probe support that includes at least one ring, whereby the ultrasound probes are each independently moveable along the ring to thereby maintain a constant distance from a center of the ring which the body segment will occupy during three-dimensional imaging of the biological body segment.

93. The device of clause 92, wherein at least two of the ultrasound probes are mounted on respective rings of the support at different distances from a common center of the rings, or from a point bisecting a line between distinct centers of the rings on which the ultrasound probes are supported.

94. The device of clause 93, wherein at least one of the ultrasound probes is arranged to form a first image, and at least one of the ultrasound probes is arranged to form a second image transverse to the first image.

95. The device of any one of clauses 86-94, wherein the container defines a boundary between the volume of the container and an atmospheric volume outside the container, and further including a seal at the boundary that partitions the volume of the container from the atmospheric volume when a biological body segment traverses the boundary, whereby the container can be pressurized relative to an ambient pressure of the atmospheric volume.

96. The device of any one of clauses 86-95, further including a processing unit linked to the ultrasound probes, the processing unit employing images obtained by the ultrasound probes to generate a representation of the biological body segment.

97. The device of clause 96, wherein the processing unit employs a finite element analysis to generate a three-dimensional representation of the biological body segment.

98. The device of any one of clauses 86-97, wherein at least one of the ultrasound probes includes a single element transducer.

99. The device of any one of clauses 86-98, wherein at least one of the ultrasound probes generates an echo ultrasound image.

100. The device of any one of clauses 86-99, further including an ultrasound probe support that includes an elastomeric sheath to seal the biological body segment from the remainder of the volume of the container, and wherein the ultrasound probes include nodes at the elastomeric sheath, and further including a processing unit to form a three-dimensional image of the position of the nodes relative to each other, whereby the ultrasound images are obtained of the biological body segment at different pressures applied to the biological body segment by the pressurizing device.

101. The device of clause 100, wherein the at least one ultrasound probe includes,

a) at least one first transducer mounted at a first ultrasound probe support that is linked to the container; and
b) at least one second transducer fixed to a second ultrasound probe support that includes an elastomeric sheath that seals the biological body segment from the remainder of the container volume, and wherein the device further includes a processing unit to which at least a portion of the ultrasound probes is linked, whereby a three-dimensional image of the biological body segment at different pressures is generated.

102. The device of clause 101, wherein the processing unit is linked to the pressurizing device.

103. The device of clause 102, wherein at least a portion of the second transducers includes single element transducers.

104. The device of clause 102 or clause 103, wherein at least a portion of the second transducers perform echo ultrasound.

105. The device of any one of clauses 102-103 wherein at least a portion of the second transducers are configured to receive ultrasound signals from at least a portion of the first transducers.

106. The device of any one of clauses 86-105, wherein the pressurization device is a pressurized fluid contained within the container.

107. The device of any one of clauses 86-105, wherein the pressurization device is a compression garment.

108. The device of any one of clauses 86-107, further comprising a camera disposed within the container and configured to track motion of the biological body segment.

109. A method of three-dimensional imaging of musculoskeletal tissue of a biological body segment under compressive loads, comprising the steps of:

a) immersing a biological body segment into a container of fluid, the container defining a volume that is pressurizable while the biological body segment is immersed in the fluid and traverses a boundary between the container volume and an ambient volume beyond the container volume;

b) generating a plurality of ultrasound images of the biological body segment by at least one ultrasound probe within the container volume, the images being generated while the biological body segment is subjected to a plurality of discreet pressures within the container; and
c) generating a three-dimensional image of musculoskeletal tissue of the biological body segment from the plurality of ultrasound images.

110. The method of clause 109, wherein the plurality of ultrasound images are generated by a plurality of ultrasound probes.

111. The method of clause 110, wherein generating the three-dimensional image of the musculoskeletal tissue of the biological body segment includes employing finite element analysis of the plurality of ultrasound images.

112. The method of clause 111, wherein the ultrasound probes image the musculoskeletal tissue of the biological body segment while circumscribing the biological body segment.

113. The method of clause 112, wherein the ultrasound probes are supported at different distances from a common center of rotation or from a point bisecting a line between distinct centers of rotation about which the ultrasound probes move, while generating the three dimensional image of the musculoskeletal tissue of the biological body segment, whereby the plurality of ultrasound probes image the musculoskeletal tissue of the biological body segment at different distances from the biological body segment.

114. The method of clause 113, wherein a plurality of nodes fixed to an elastomeric sheath sealing the biological body segment from the remainder of the volume of the container transmit signals to a processing unit that, in combination with the ultrasound probes, forms the three-dimensional image of the musculoskeletal tissue of the biological body segment.

115. The method of clause 114, wherein the processing unit forms at least one three-dimensional image of the musculoskeletal tissue of the biological body segment that includes internal bone geometries or internal bone properties of the biological body segment.

116. A device for assessing tissue geometry of a biological body segment, comprising:

a structure configured to receive the biological body segment, the structure including an array of imaging devices disposed to capture images about a perimeter of the biological body segment;
a pressurization device configured to apply pressure to the biological body segment to deform soft tissue of the biological body segment; and
a controller configured to:

receive images captured by the array of the biological body segment in a plurality of deformed states, and infer a geometry of a rigid internal structure of the biological body segment based on cross-referencing of the captured images.

117. The device of clause 115, wherein the applied pressure is homogenous.

118. The device of clause 115 or clause 116, wherein the pressurization device is a container containing a fluid.

119. The device of clause 115 or clause 116, wherein the pressurization device is a compression garment.

120. A method of optimizing a design of a biomechanical interface for a biological body segment, comprising:

generating a three-dimensional model of the biomechanical interface by finite element analysis;
including within the model spatially-varying and controllable internal structures; and
designing the biomechanical interface with the spatially-varying and controllable internal structures.

121. The method of clause 120, wherein the spatially-varying and controllable structures comprise a cellular solid.

122. The method of clause 120, wherein the spatially-varying and controllable structures comprise a lattice.

123. The method of clause 122, wherein the lattice comprises an edge-based lattice, a face-based lattice, or both.

124. A method of manufacturing a biomechanical interface, comprising:
fabricating the spatially-varying structure and controllable structures of the method of clause 120.

125. The method of clause 124, wherein fabricating the spatially-varying and controllable structures includes three-dimensional printing of the structures.

126. The method of clause 124 or clause 125, further comprising fabricating the biomechanical interface with the fabricated spatially-varying and controllable structures.

127. A method of designing a biomechanical interface for a biological body segment, comprising:

generating a three-dimensional model of the biological body segment and the biomechanical interface;
designing the biomechanical interface with an initial fitting pressure;
using the model, determining a loading pressure of the designed biomechanical interface to at least one region of the biological body segment;
comparing the determined loading pressure to a physiological tolerance; and
varying at least one of a compliance or a geometry of the designed biomechanical interface based on a determined loading pressure and the physiological tolerance.

128. The method of clause 127, further comprising iteratively determining the loading pressure of the designed biomechanical interface to at least one region of the biological body segment using the model, comparing the determined loading pressure to a physiological tolerance, and varying at least one of the compliance or the geometry of the biomechanical interface until the determined loading pressure is below the physiological tolerance.

129. The method of clause 127 or clause 128, wherein determining a loading pressure includes determining at least two loading pressures and wherein varying at least one of the compliance or the geometry includes minimizing a variance between the at least two loading pressures.

130. The method of any one of clauses 127-129, further comprising determining a plurality of loading pressures, each loading pressure being of a distinct anatomical point or a distinct anatomical region of the biological body segment; and comparing the plurality of loading pressures to a plurality of physiological tolerances.

131. The method of clause 130, further comprising maximizing a differential between the determined loading pressure and the physiological tolerance for at least two anatomical points or anatomical regions.

132. The method of clause 130 or clause 131, further comprising minimizing a variance of a plurality of differentials between the determined loading pressures and the physiological tolerances.

133. The method of any one of clauses 127-129, wherein the physiological tolerance is a pain threshold or a pain tolerance.

## Claims

**1.** A method of designing with a computer a biomechanical interface for a biological body segment, comprising:
with a computer:

creating a compound model of internal and external features of the biological body segment using a point cloud alignment algorithm between external features detected using photogrammetric imaging and internal features detected using non-invasive imaging;
generating a three-dimensional finite element analysis, FEA, model of the biological body segment and the biomechanical interface based on the compound model;
defining, within the three-dimensional FEA model, an initial configuration of the biomechanical interface with an initial fitting pressure;
using the three-dimensional FEA model, determining a loading pressure applied to at least one region of the biological body segment by the biomechanical interface in the initial configuration;

comparing the determined loading pressure to a physiological tolerance; and
in the three-dimensional FEA model, varying at least one of a compliance or a geometry of the biomechanical interface based on the determined loading pressure and the physiological tolerance to thereby obtain a final configuration of the biomechanical interface;
wherein determining the loading pressure includes determining at least two loading pressures and wherein varying at least one of the compliance or the geometry includes reducing a variance between the at least two loading pressures;
wherein determining the loading pressure includes simulating a dynamic use event.

**2.** The method of claim 1, further comprising iteratively determining the loading pressure of the biomechanical interface to at least one region of the biological body segment using the three-dimensional FEA model, comparing the determined loading pressure to the physiological tolerance, and varying at least one of the compliance or the geometry of the biomechanical interface until the determined loading pressure is below the physiological tolerance.

**3.** The method of claim 1, further comprising determining a plurality of loading pressures, each loading pressure being of a distinct anatomical point or a distinct anatomical region of the biological body segment; and comparing the plurality of loading pressures to a plurality of physiological tolerances.

**4.** The method of claim 1, further comprising maximizing a differential between the determined loading pressure and the physiological tolerance for at least two anatomical points or anatomical regions.

**5.** The method of claim 1, further comprising minimizing a variance of a plurality of differentials between the determined loading pressures and the physiological tolerances and optionally wherein the physiological tolerance is a pain threshold or a pain tolerance.

**6.** The method of claim 1, wherein generating the three-dimensional model includes defining a load line of the biological body segment and the biomechanical interface and wherein the method optionally further comprises defining at least one of a location or an orientation of an alignment component of the biomechanical interface.

**7.** The method of claim 1, further comprising defining at least two subject states of the biological body segment within the three-dimensional FEA model, wherein the at least two subject states optionally include a normal state and at least one of a compressed state or an expanded state.

**8.** The method of claim 7, wherein determining the loading pressure includes determining loading pressures applied to the biological body segment at the at least two subject states.

**9.** The method of claim 1, wherein simulating the dynamic use event includes simulating a motion event performed in real-time by a subject.

**10.** The method of claim 1, wherein the three-dimensional model includes a representation of spatially-varying and controllable internal structures of the biomechanical interface.

**11.** The method of claim 10, wherein the spatially-varying and controllable structures comprise a cellular solid or a lattice, and optionally wherein the lattice comprises an edge-based lattice, a face-based lattice, or both.

**12.** The method of claim 1, further comprising fabricating the biomechanical interface in the final configuration, and optionally wherein fabricating the biomechanical interface includes fabricating spatially-varying and controllable structures comprising the interface.

**13.** The method of claim 1, wherein the non-invasive imaging includes at least one of computed tomography, CT, magnetic resonance imaging, MRI, or ultrasound, US.

**14.** The method of claim 1, wherein the photogrammetric imaging includes at least one of digital image correlation, DIC, or handheld scanning tools.

**15.** The method of claim 1, wherein the dynamic use event includes at least one of standing, walking, walking up stairs, or running.

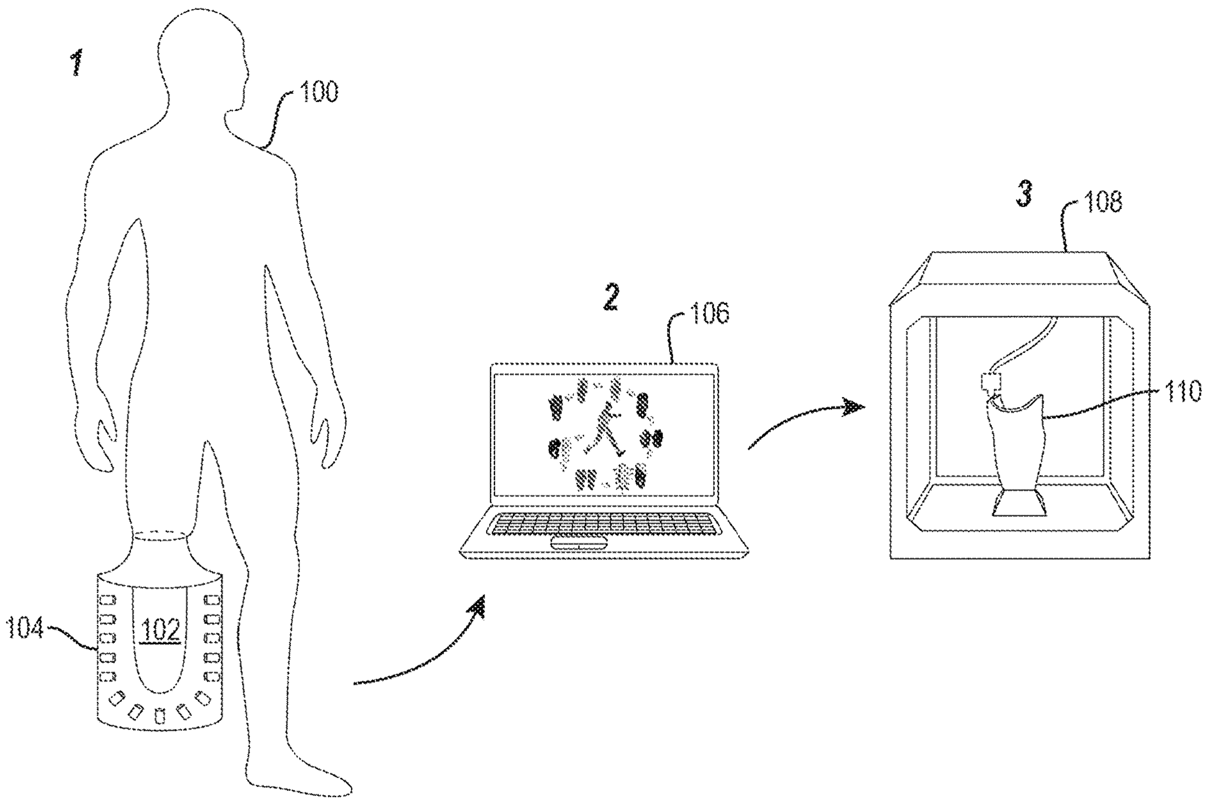


FIG. 1

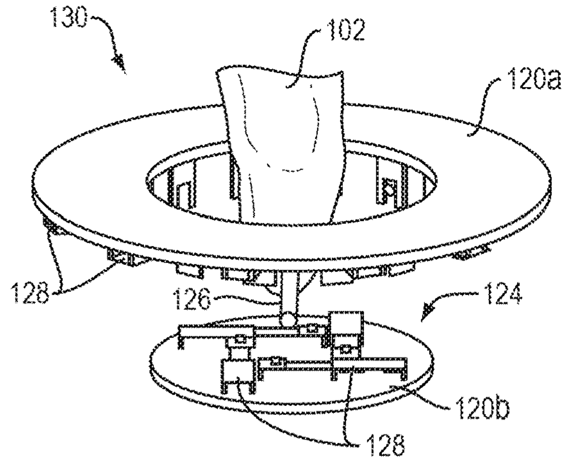


FIG. 2A

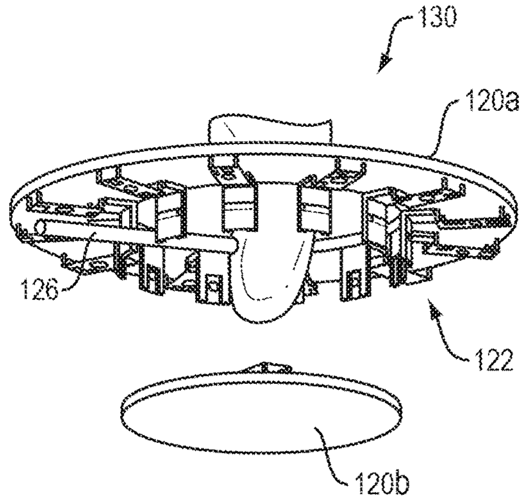


FIG. 2B

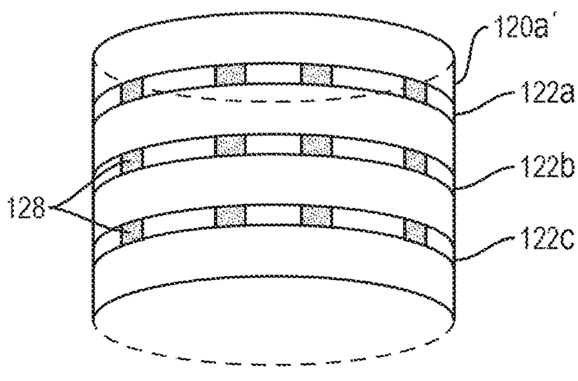


FIG. 3

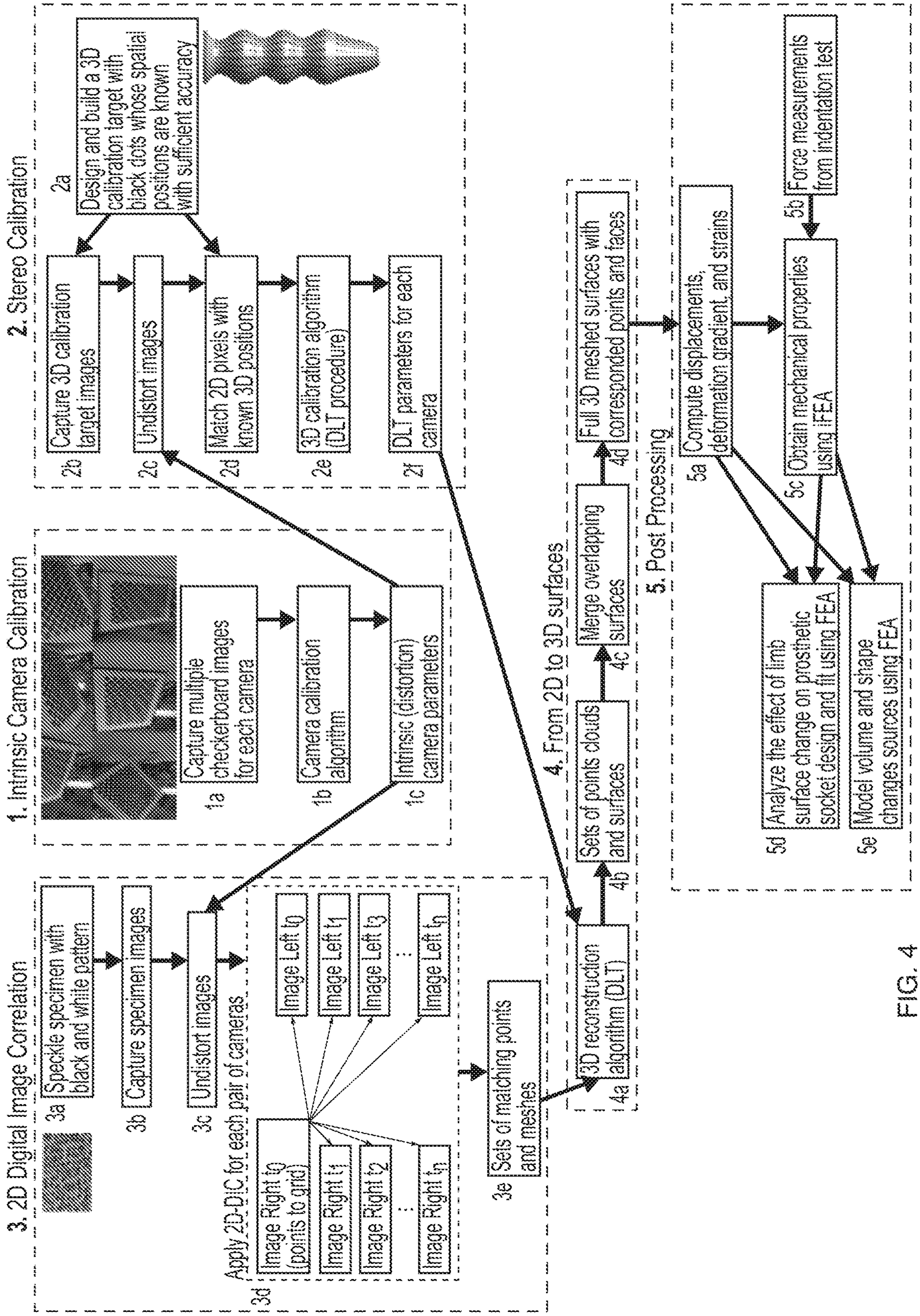
1. Intrinsic Camera Calibration
1a Capture multiple checkerboard images for each camera
1b Camera calibration algorithm
1c Intrinsic (distortion) camera parameters
2. Stereo Calibration
2a Design and build a 3D calibration target with black dots whose spatial positions are known with sufficient accuracy
2b Capture 3D calibration target images
2c Undistort images
2d Match 2D pixels with known 3D positions
2e 3D calibration algorithm (DLT procedure)
2f DLT parameters for each camera
3. 2D Digital Image Correlation
3a Speckle specimen with black and white pattern
3b Capture specimen images
3c Undistort images
3d Apply 2D-DIC for each pair of cameras
Image Right t0 (points to grid)
Image Right t1
Image Right t2
Image Right tn
Image Left t0
Image Left t1
Image Left t3
Image Left tn
3e Sets of matching points and meshes
4. From 2D to 3D surfaces
4a 3D reconstruction algorithm (DLT)
4b Sets of points clouds and surfaces
4c Merge overlapping surfaces
4d Full 3D meshed surfaces with corresponded points and faces
5. Post Processing
5a Compute displacements, deformation gradient, and strains
5b Force measurements from indentation test
5c Obtain mechanical properties using iFEA
5d Analyze the effect of limb surface change on prosthetic socket design and fit using FEA
5e Model volume and shape changes sources using FEA

FIG. 4

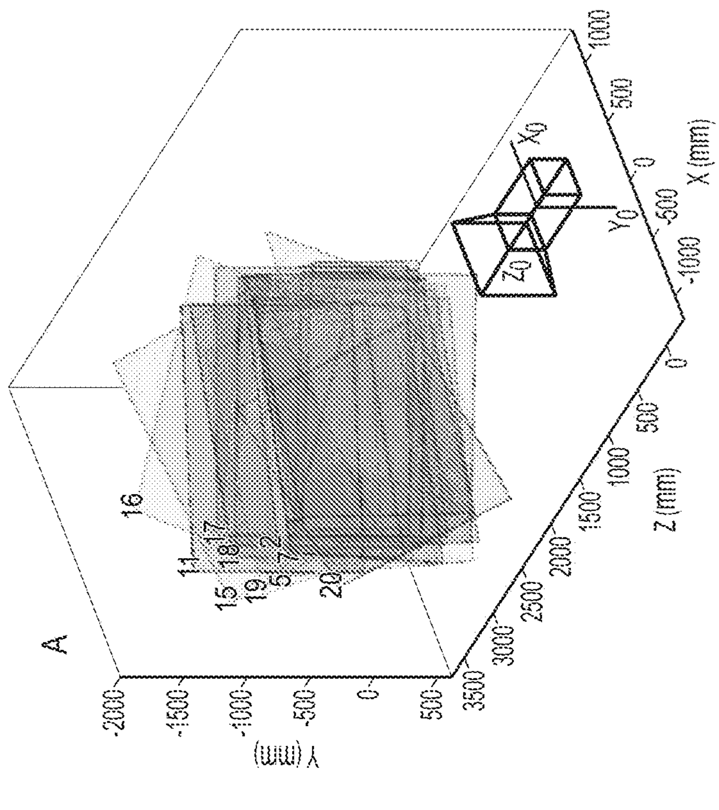


FIG. 5A

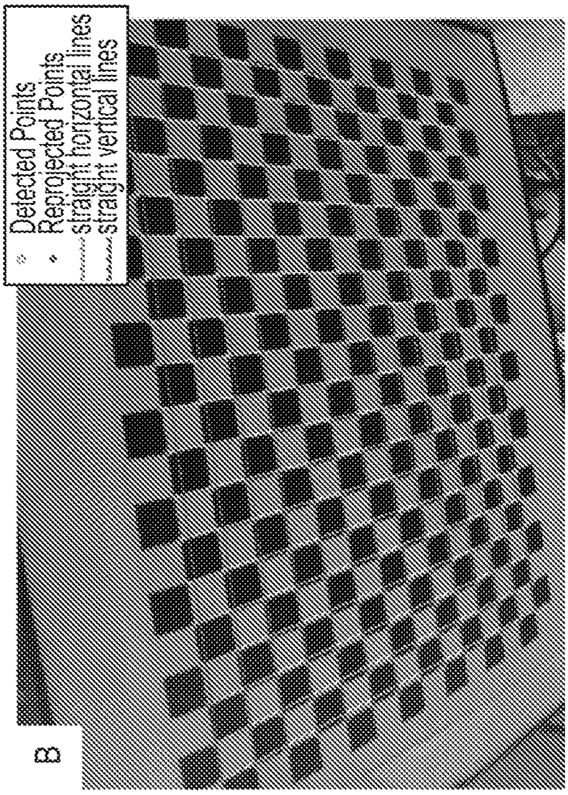


FIG. 5B

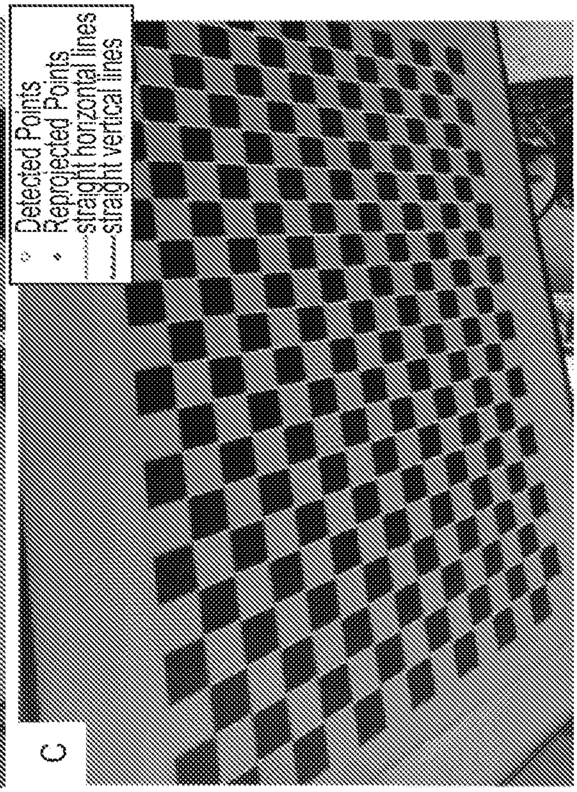


FIG. 5C

A
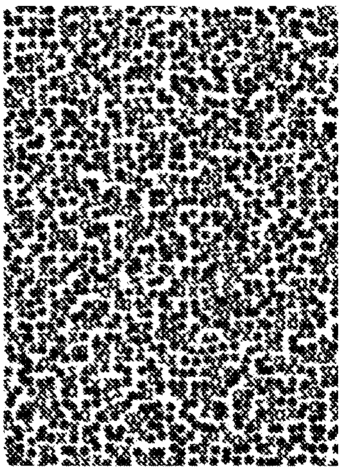

FIG. 6A

B
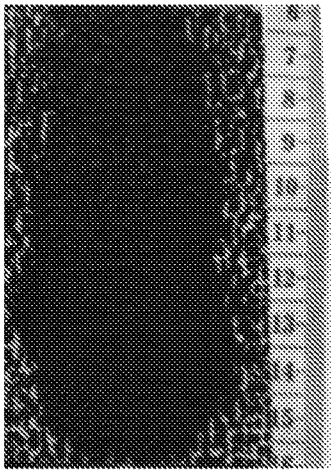

FIG. 6B

C
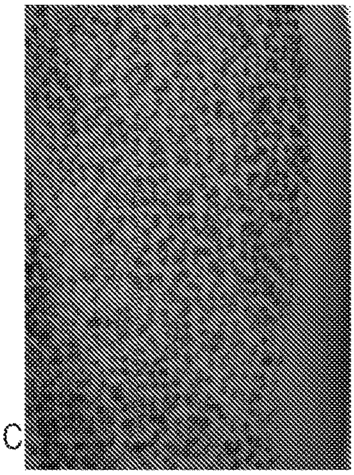

FIG. 6C

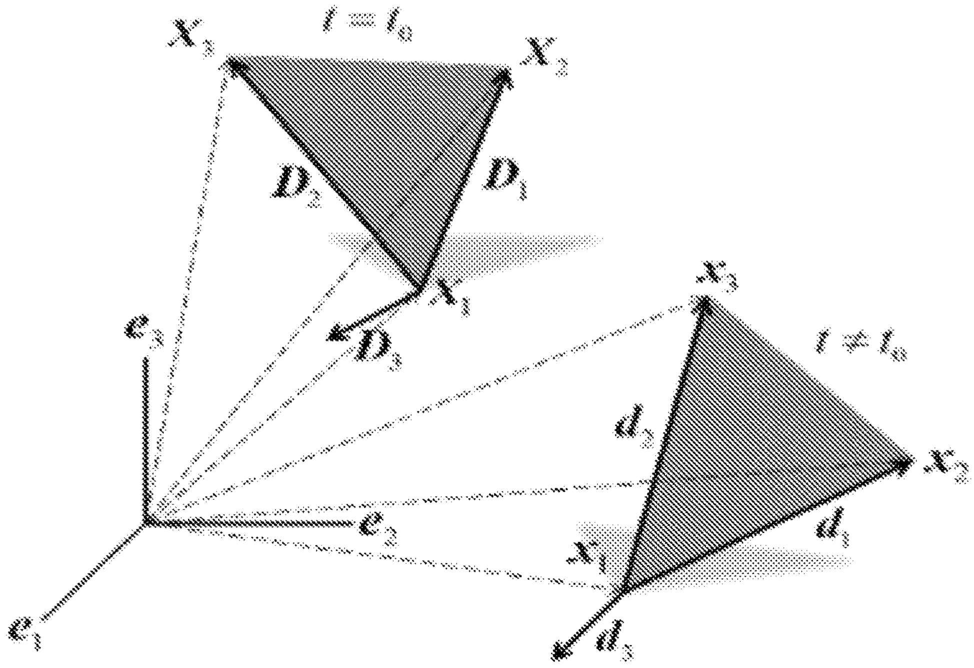


FIG. 7

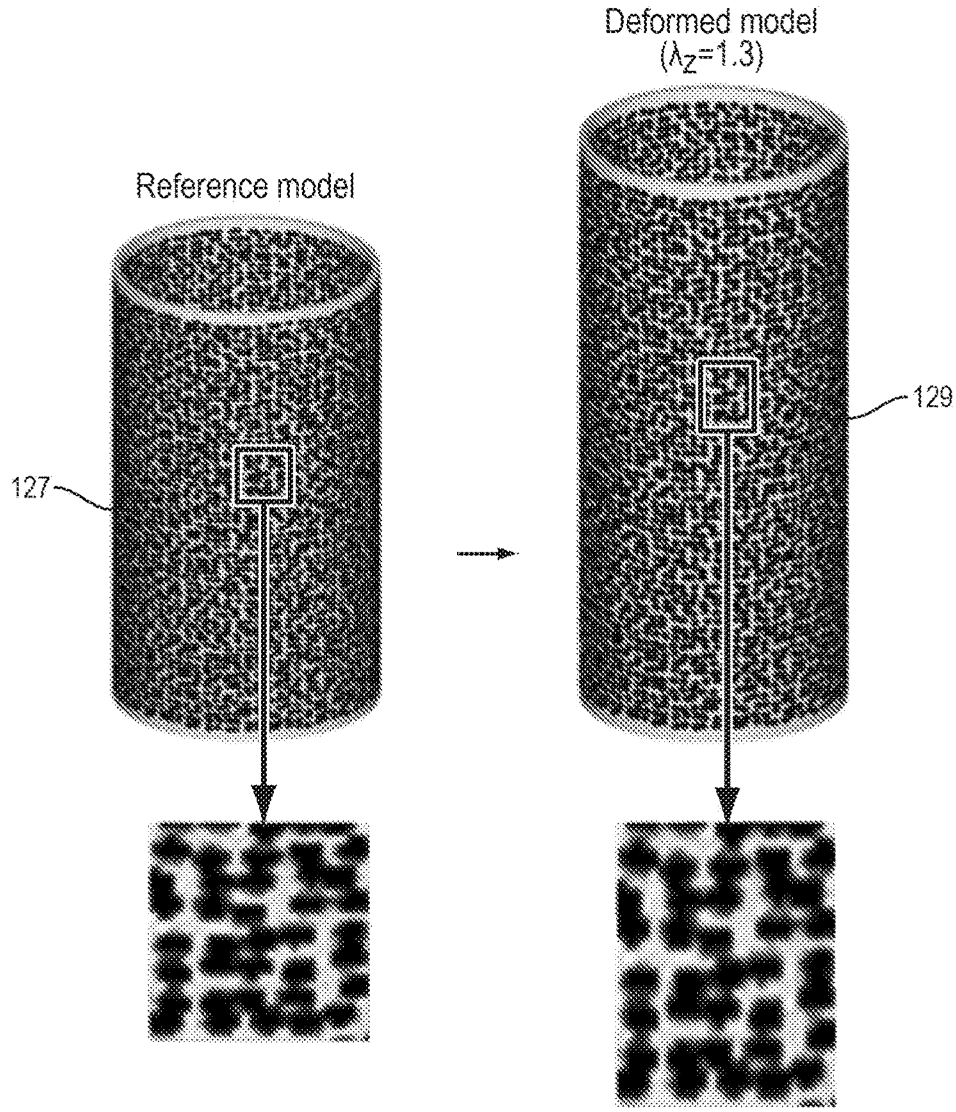


FIG. 8

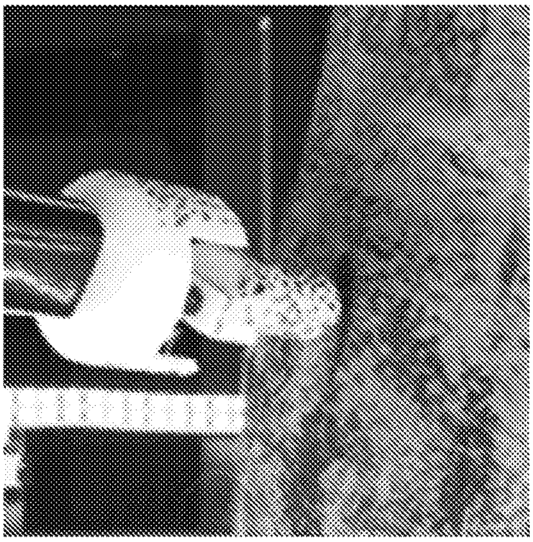

FIG. 9A

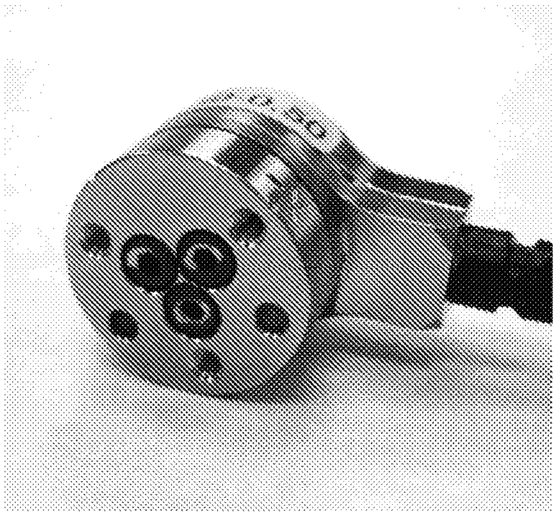

FIG. 9B

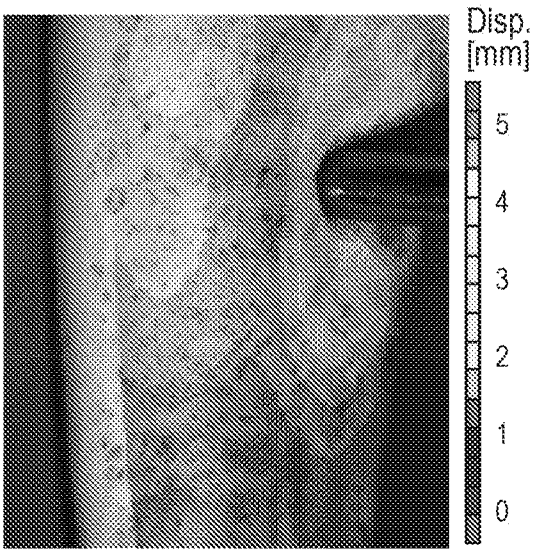


FIG. 9C

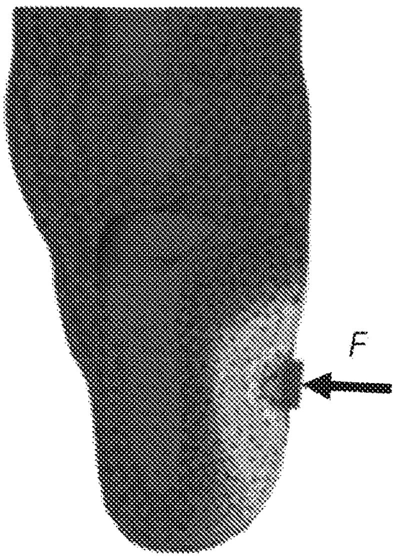


FIG. 9D

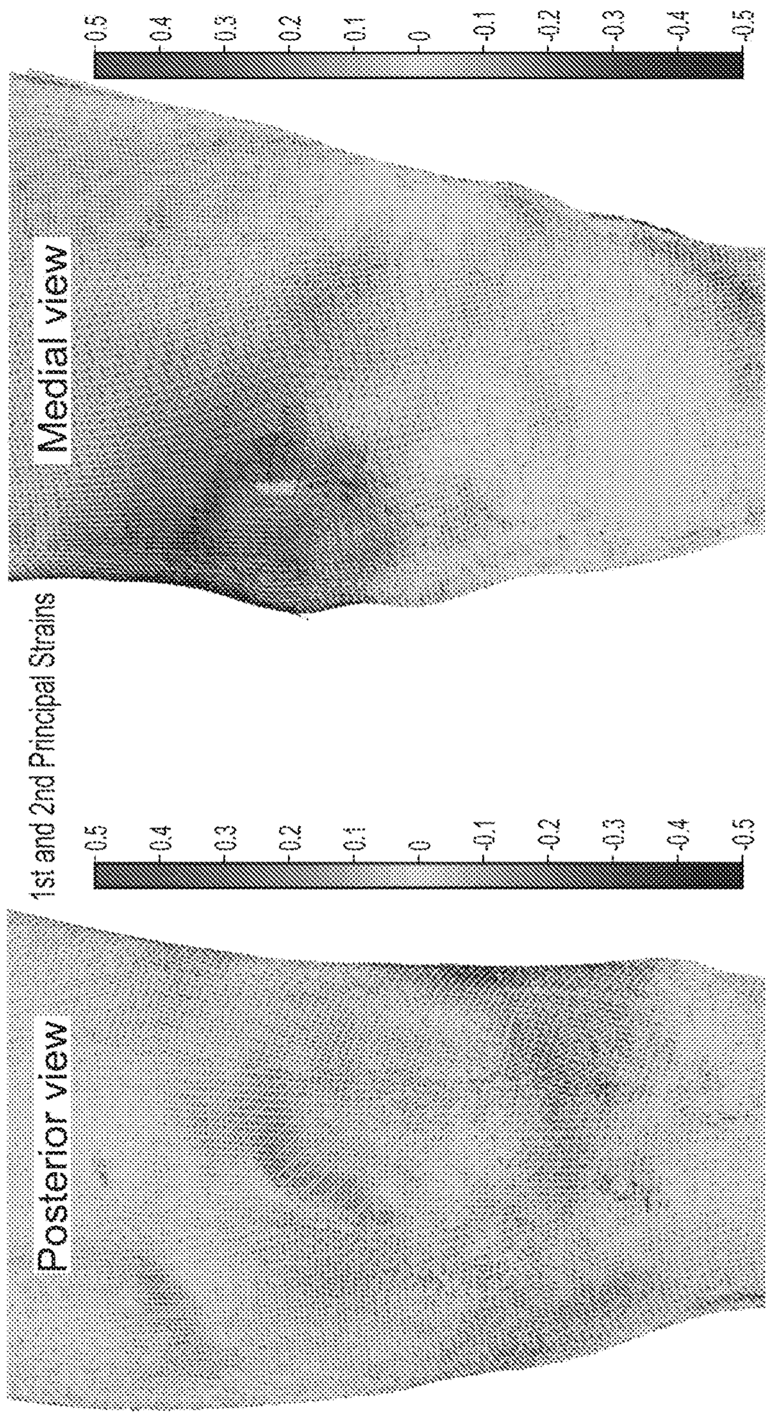
Posterior view
Medial view
1st and 2nd Principal Strains
0.5
0.4
0.3
0.2
0.1
0
-0.1
-0.2
-0.3
-0.4
-0.5

FIG. 10

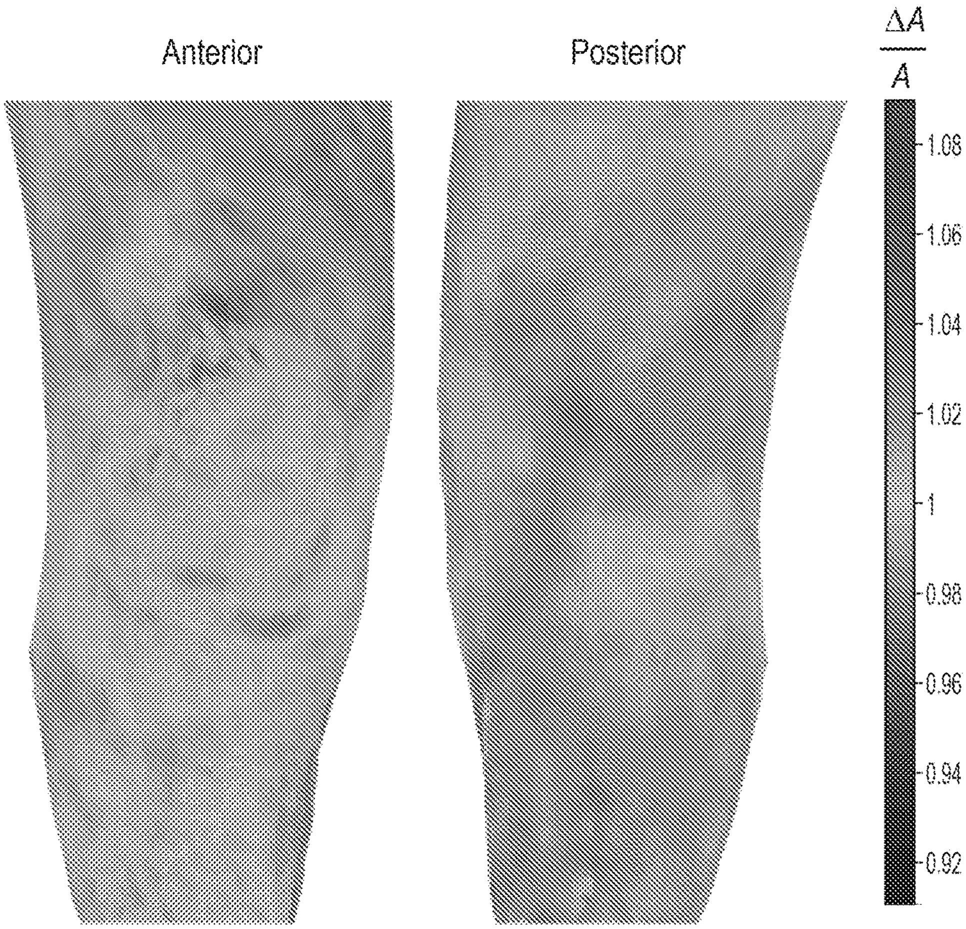


FIG. 11

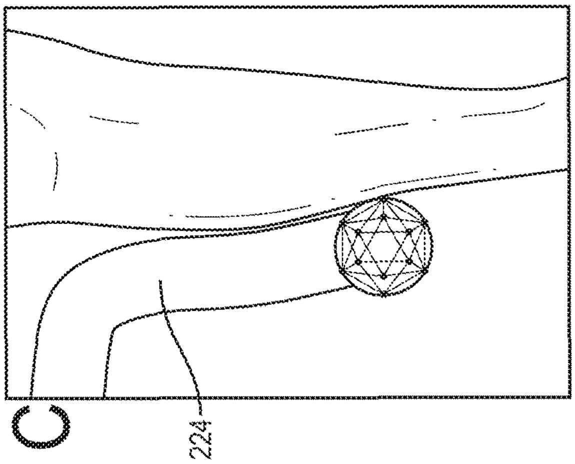


FIG. 12C

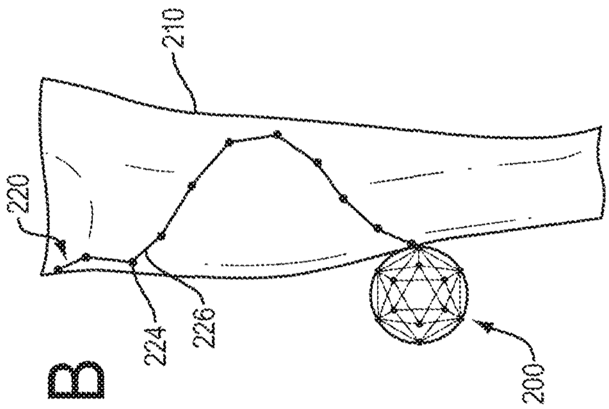


FIG. 12B

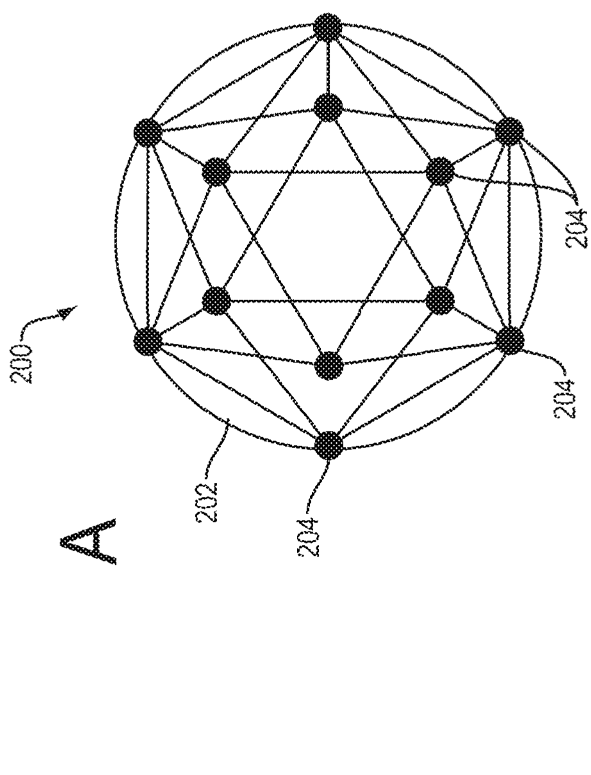


FIG. 12A

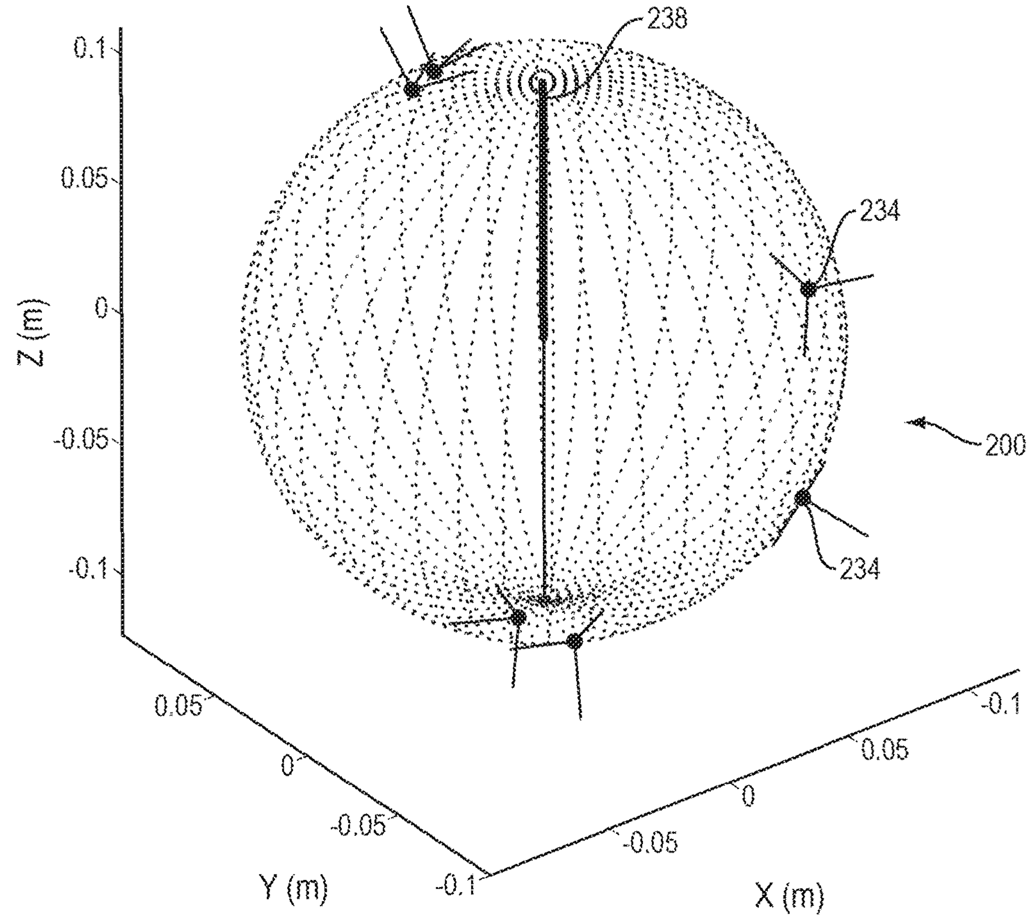


FIG. 13

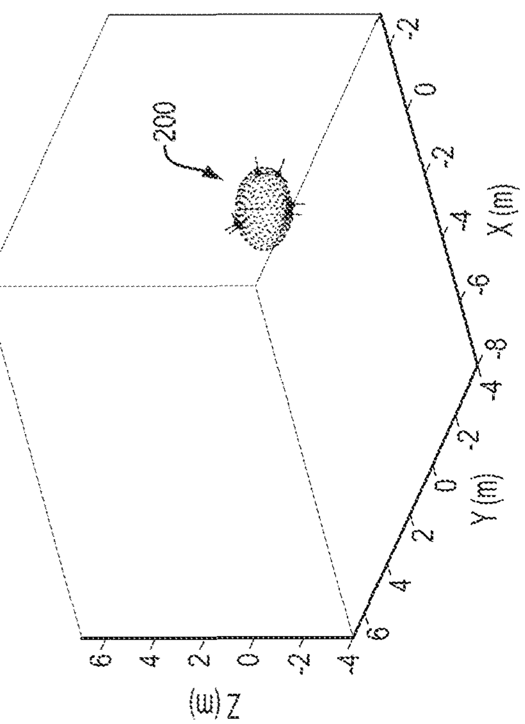


FIG. 14A

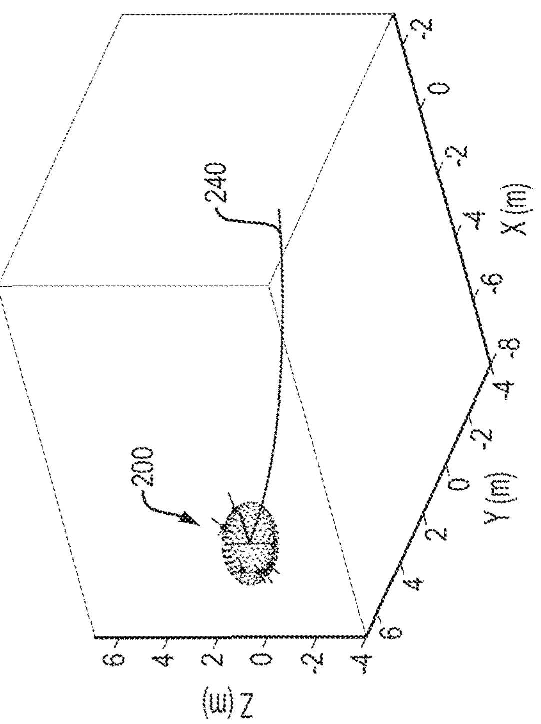


FIG. 14B

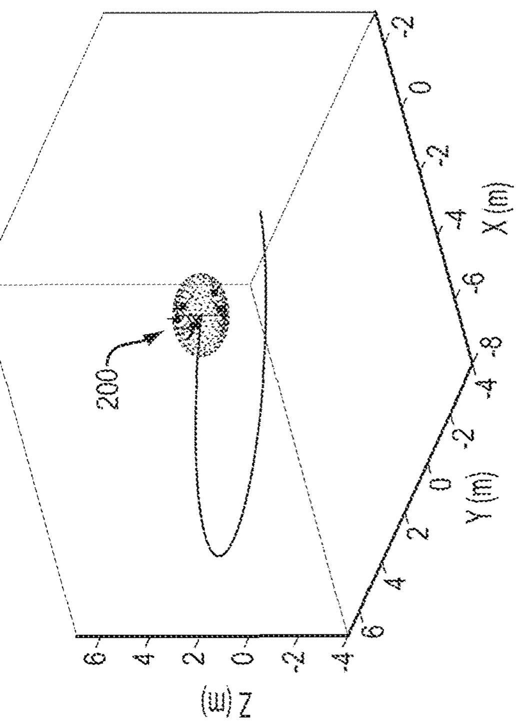


FIG. 14C

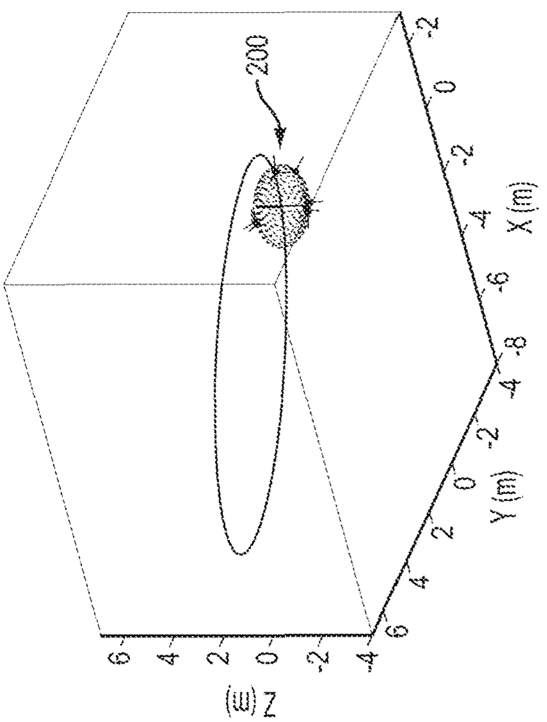


FIG. 14D

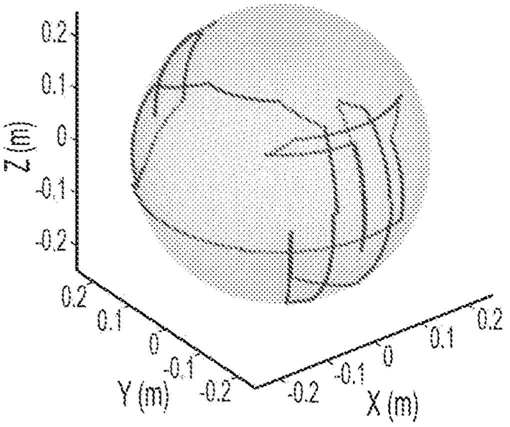


FIG. 15A

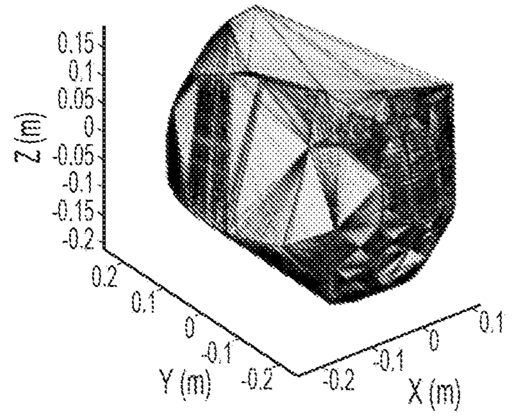


FIG. 15B

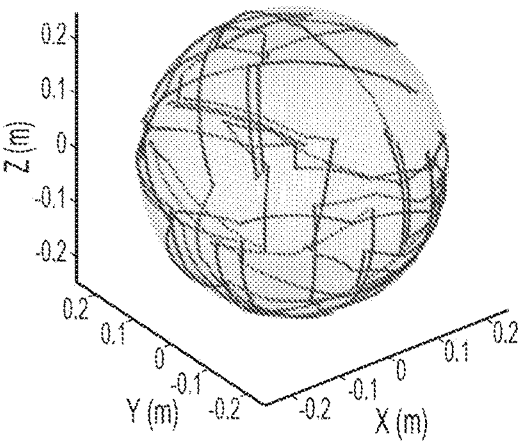


FIG. 15C

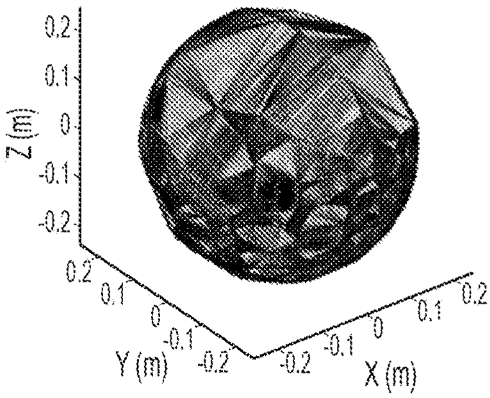


FIG. 15D

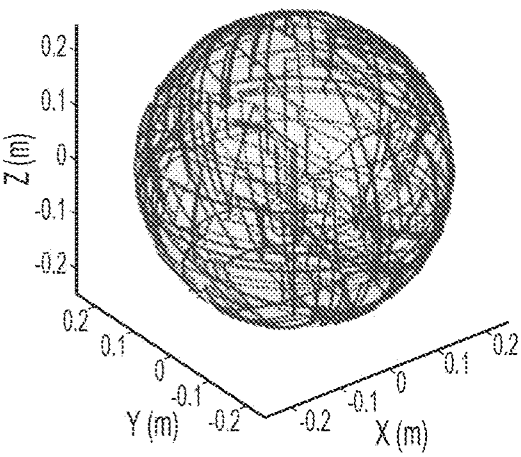


FIG. 15E

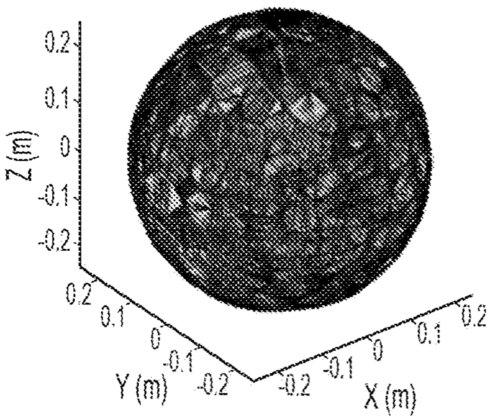


FIG. 15F

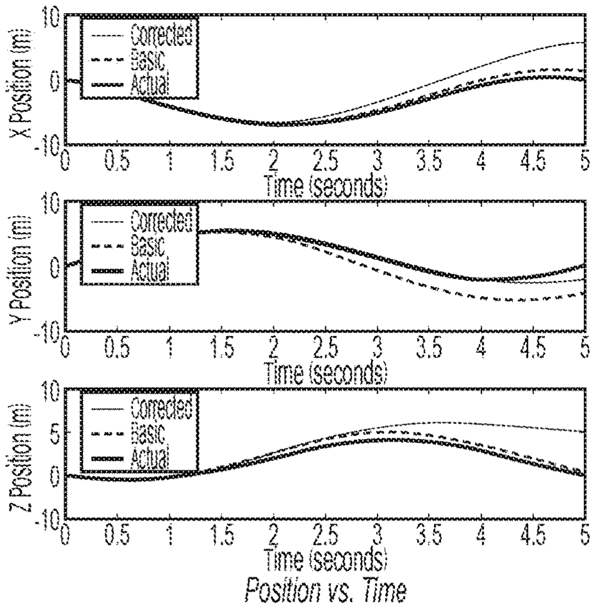


FIG. 16A

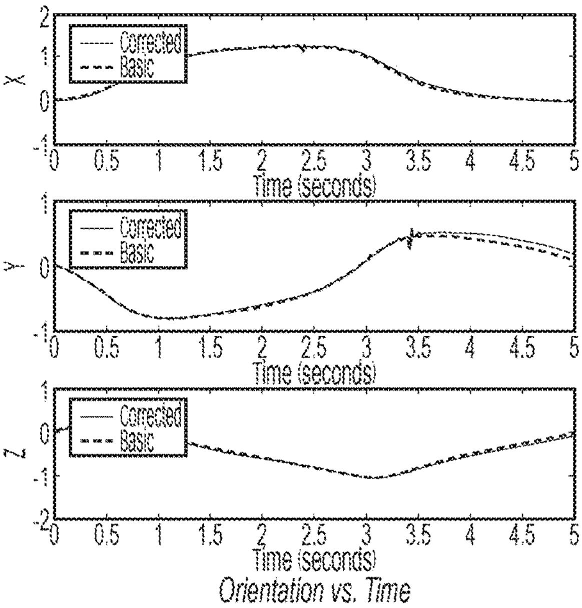


FIG. 16B

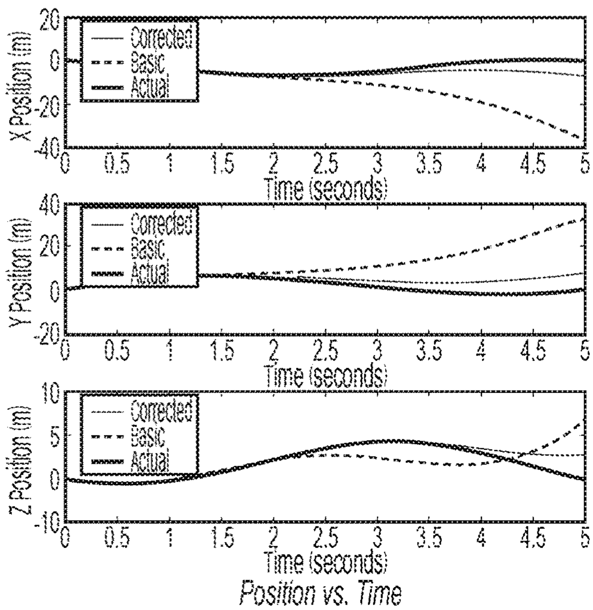


FIG. 16C

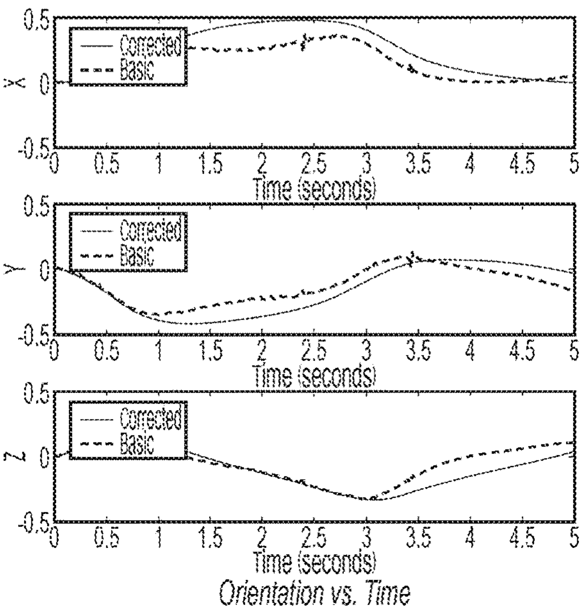


FIG. 16D

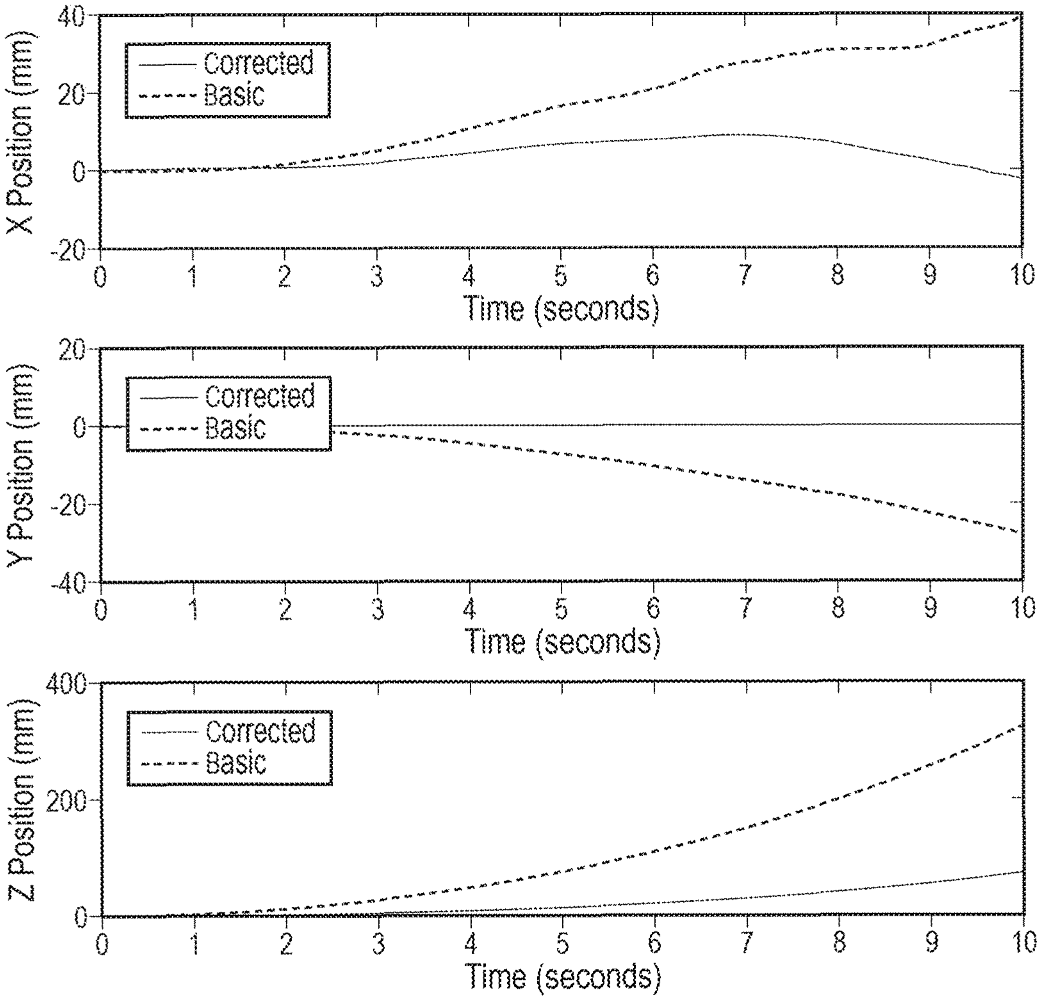
40
20
0
-20
X Position (mm)
Corrected
Basic
0 1 2 3 4 5 6 7 8 9 10
Time (seconds)
20
0
-20
-40
Y Position (mm)
Corrected
Basic
0 1 2 3 4 5 6 7 8 9 10
Time (seconds)
400
200
0
Z Position (mm)
Corrected
Basic
0 1 2 3 4 5 6 7 8 9 10
Time (seconds)

FIG. 17

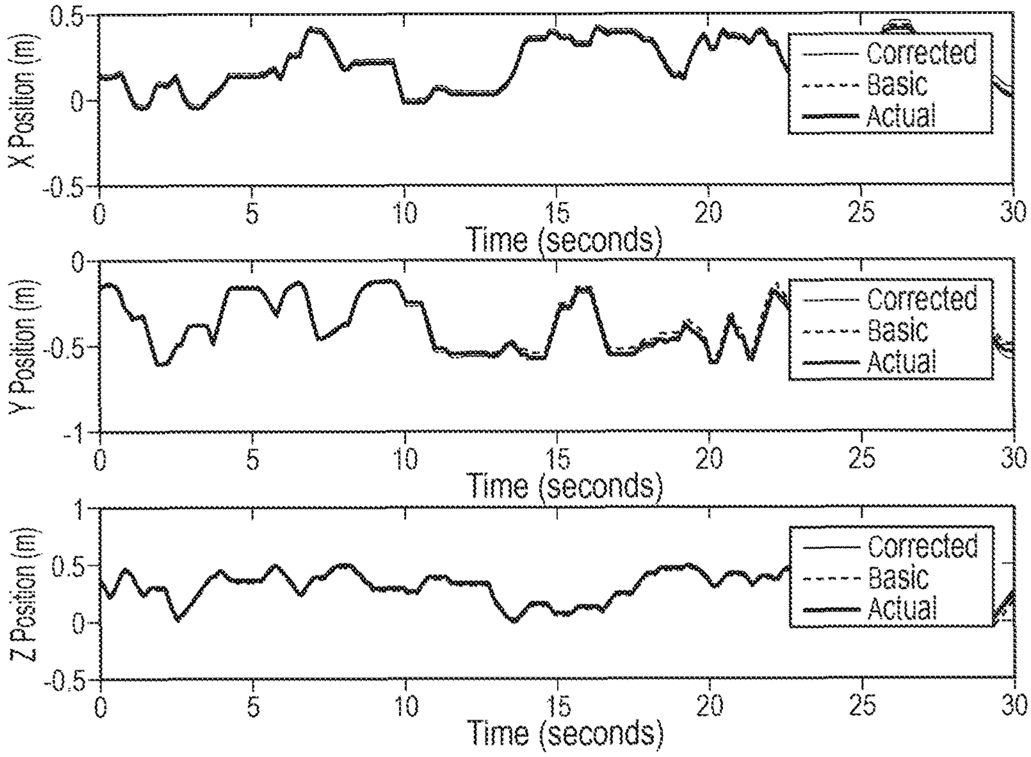


FIG. 18A

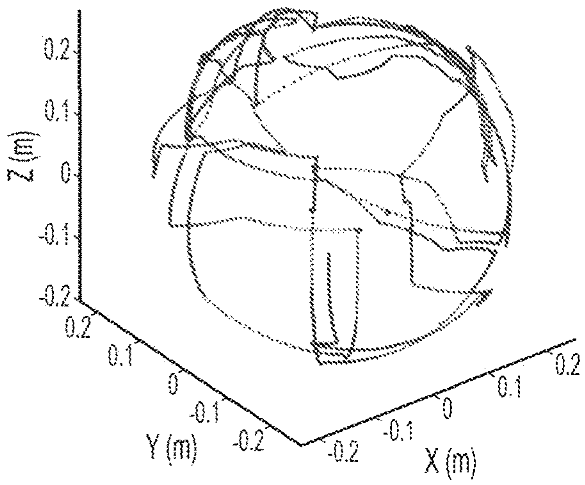


FIG. 18B

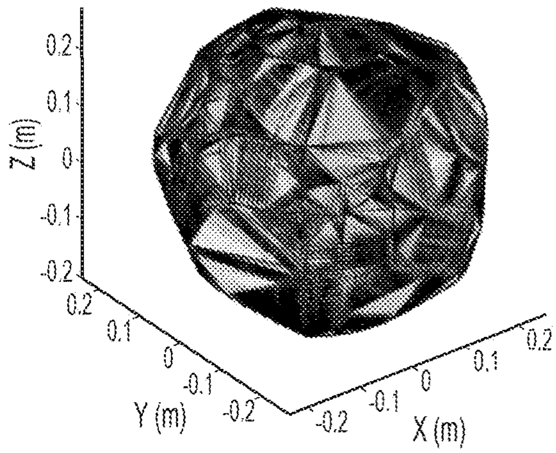


FIG. 18C

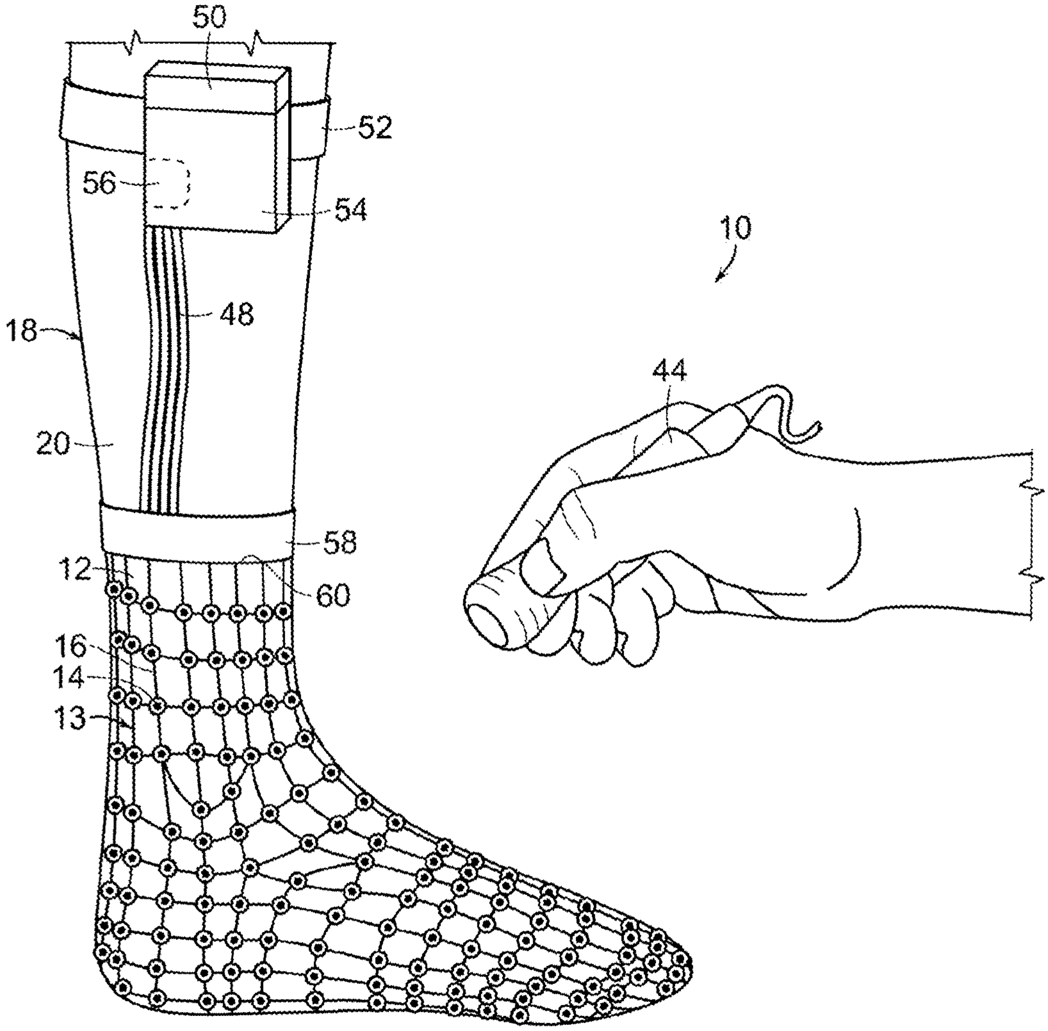


FIG. 19

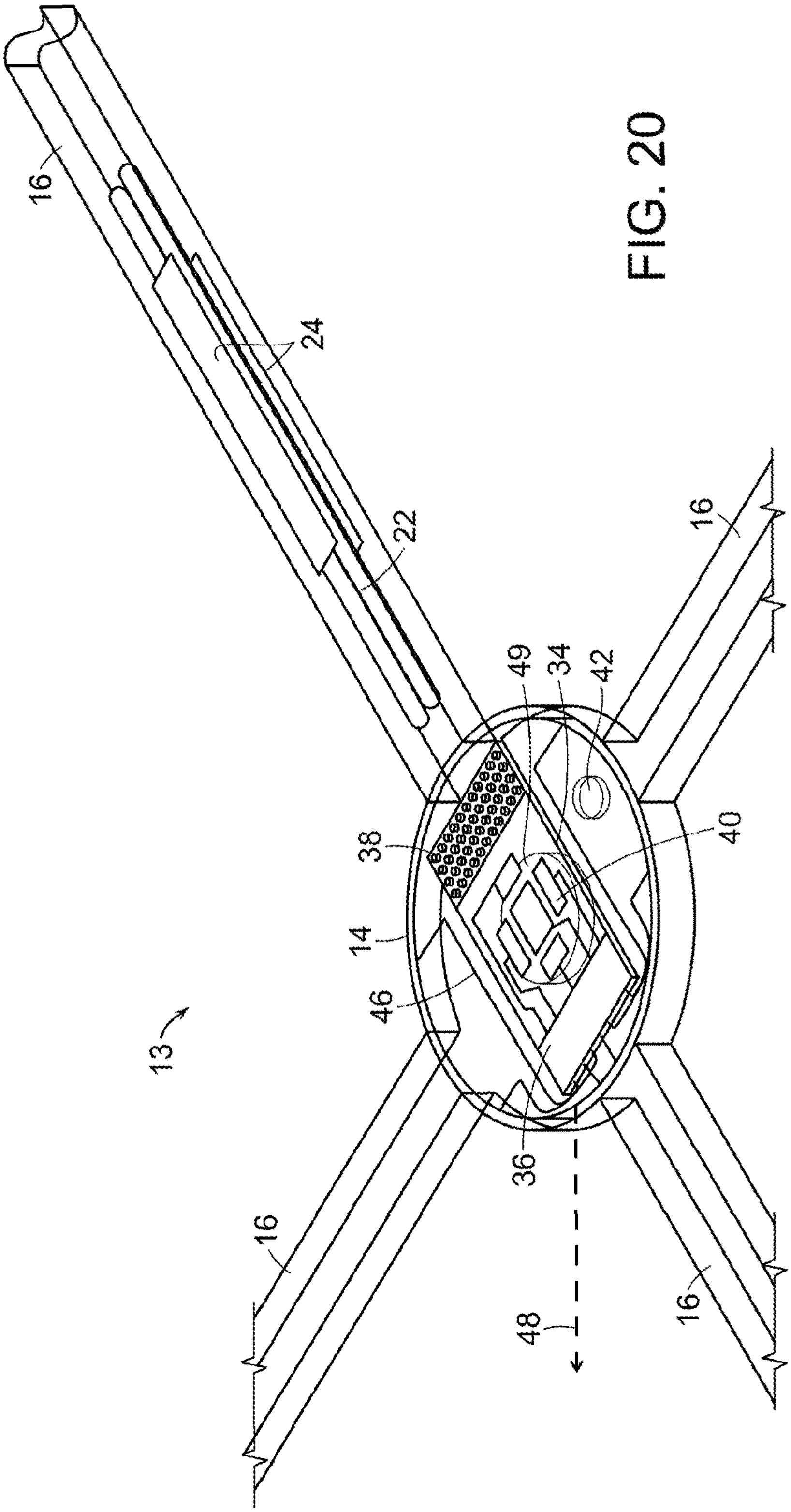


FIG. 20

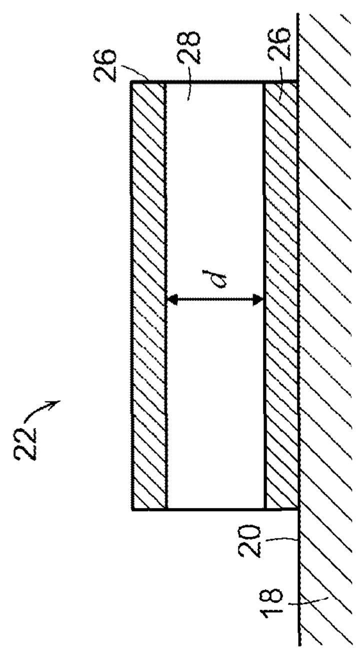


FIG. 21A

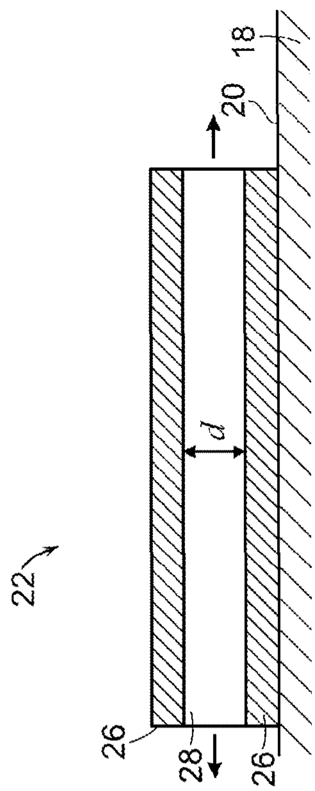


FIG. 21B

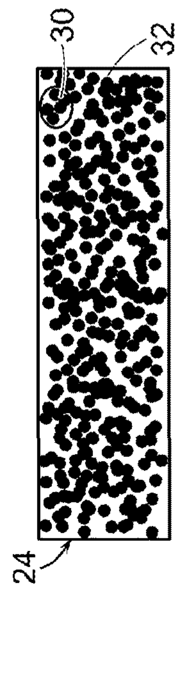


FIG. 22A

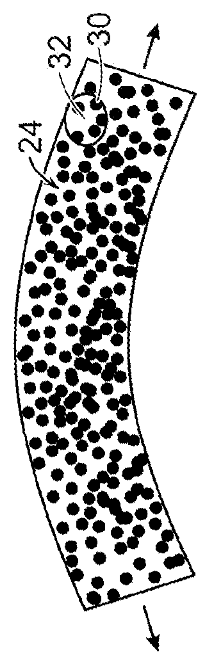


FIG. 22B

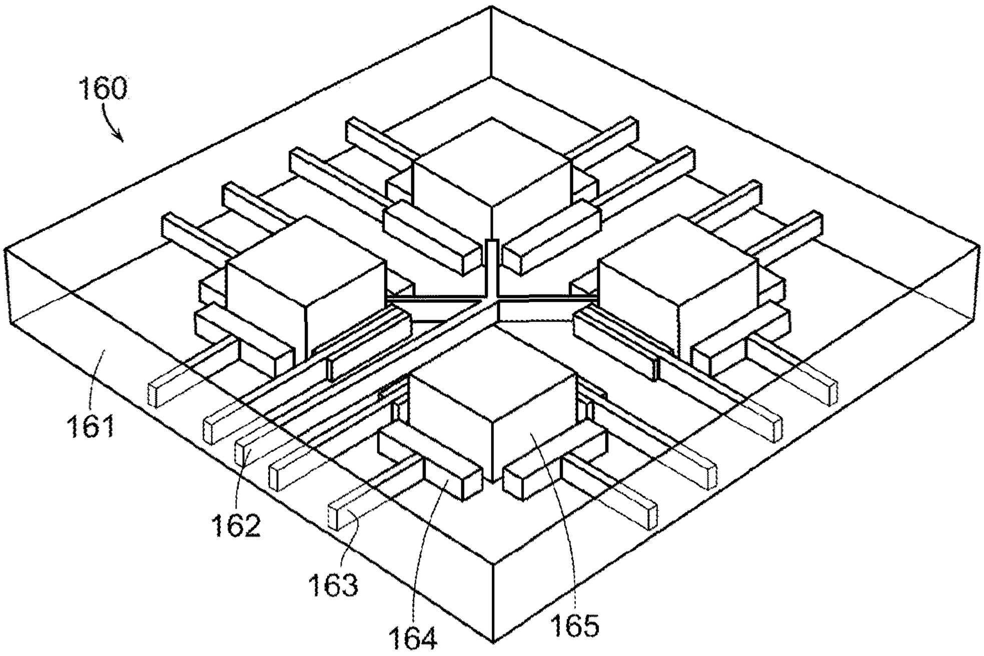


FIG. 23A

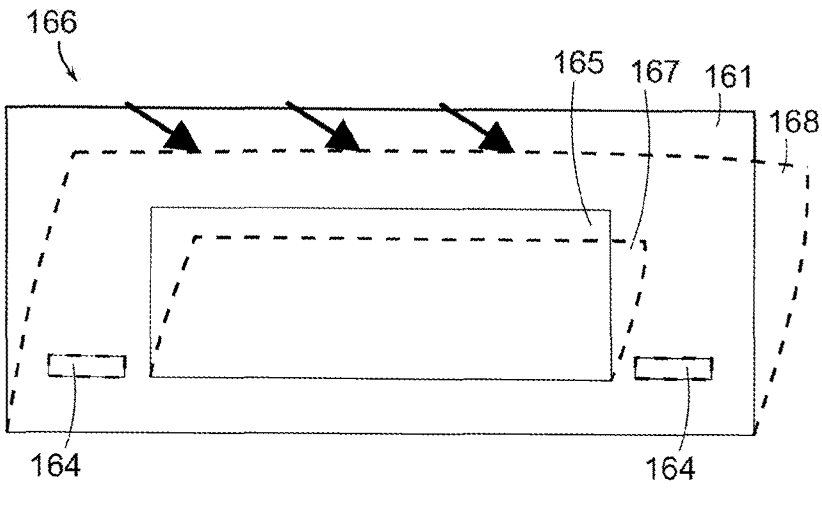


FIG. 23B

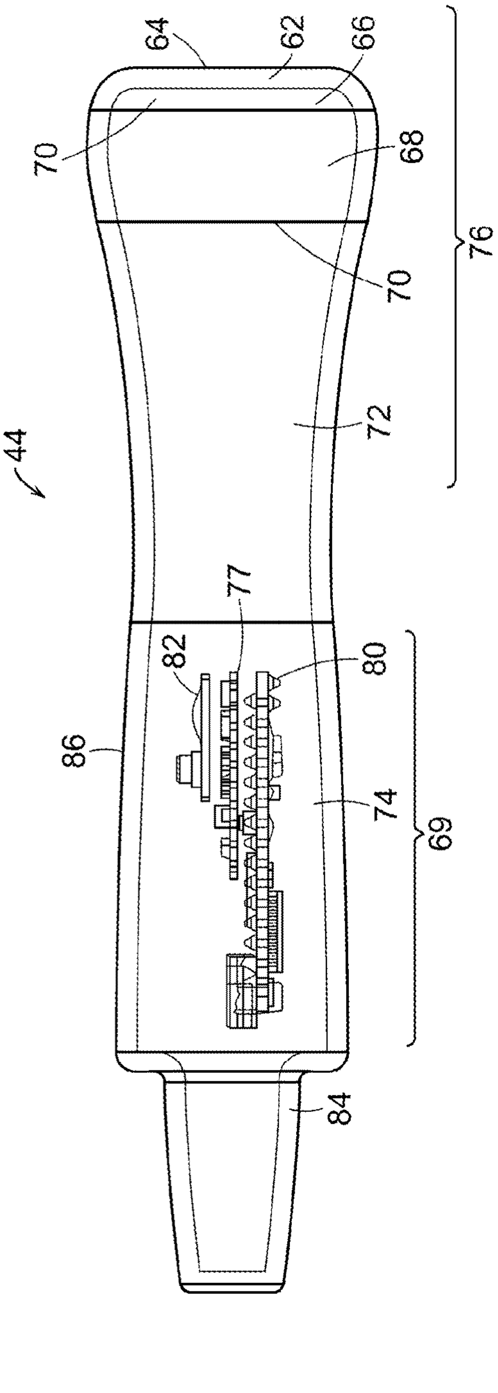


FIG. 24

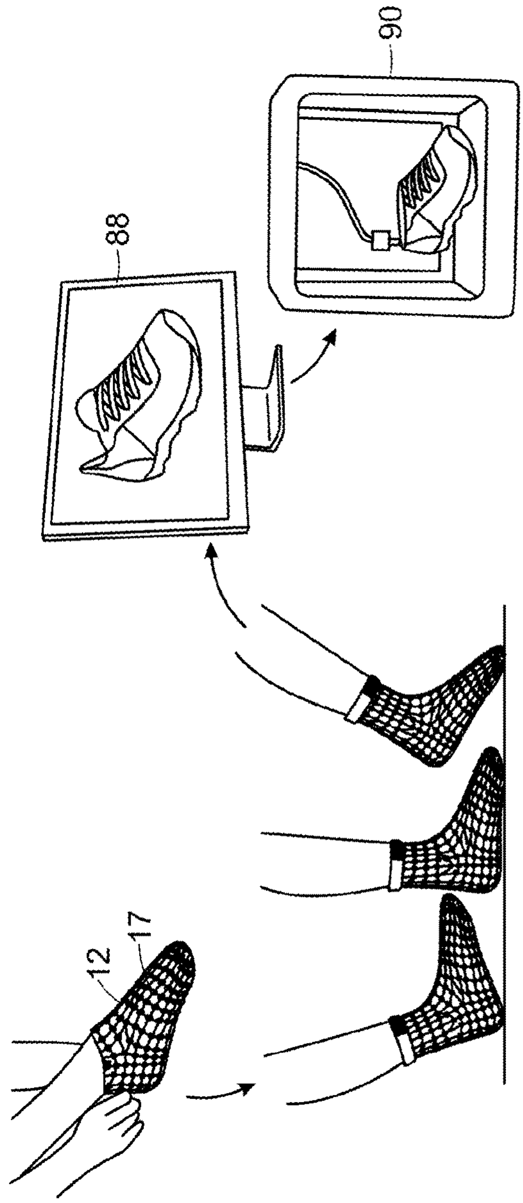


FIG. 25

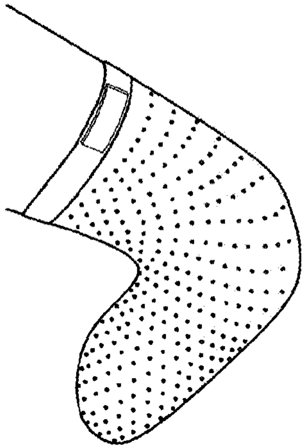

FIG. 26A

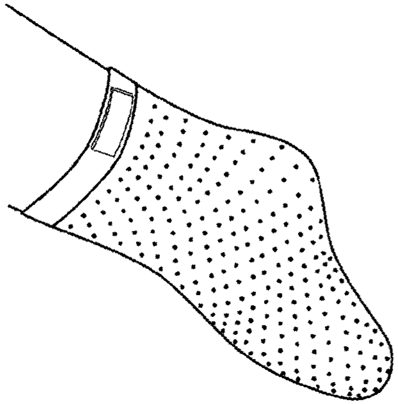

FIG. 26B

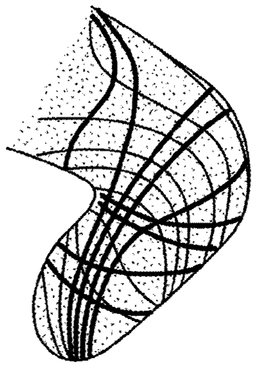

FIG. 26C

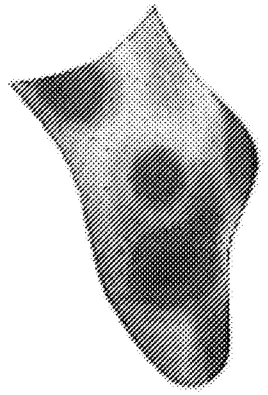

FIG. 26D

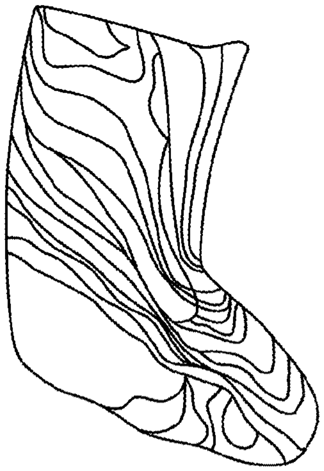

FIG. 27A

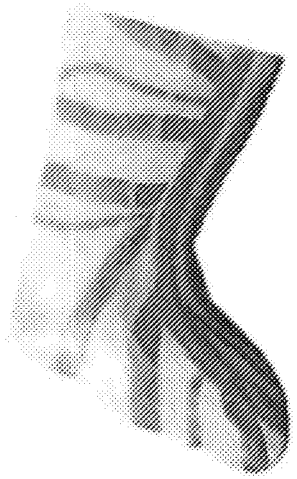

FIG. 27B

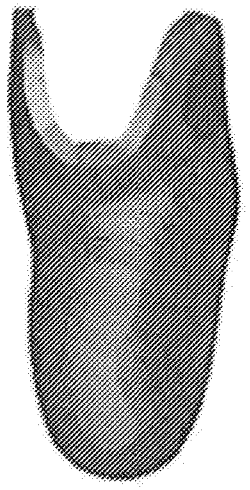

FIG. 27C

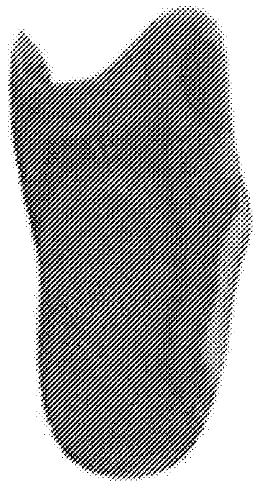

FIG. 27D

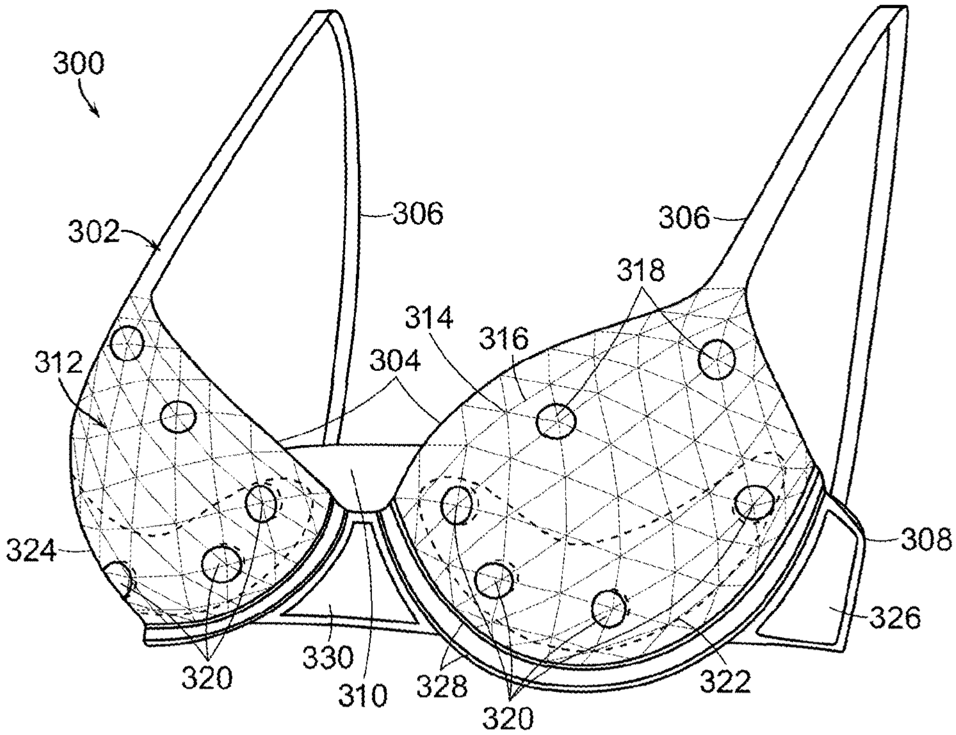


FIG. 28

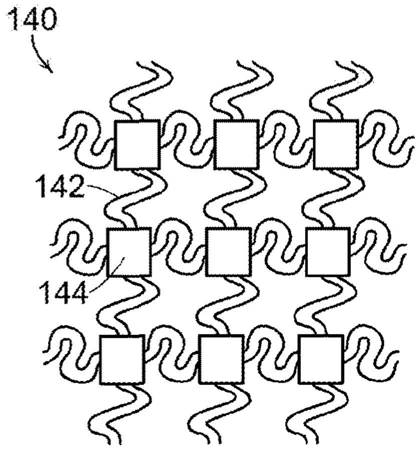


FIG. 29A

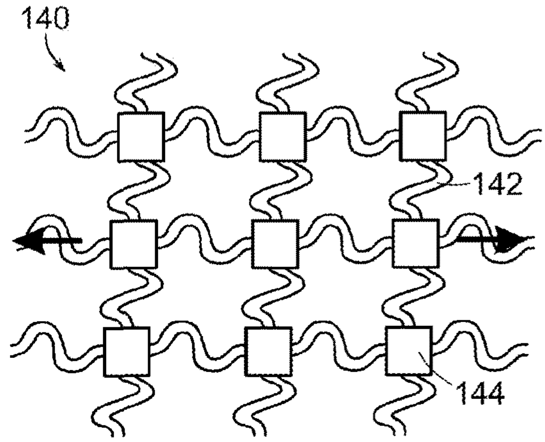


FIG. 29B

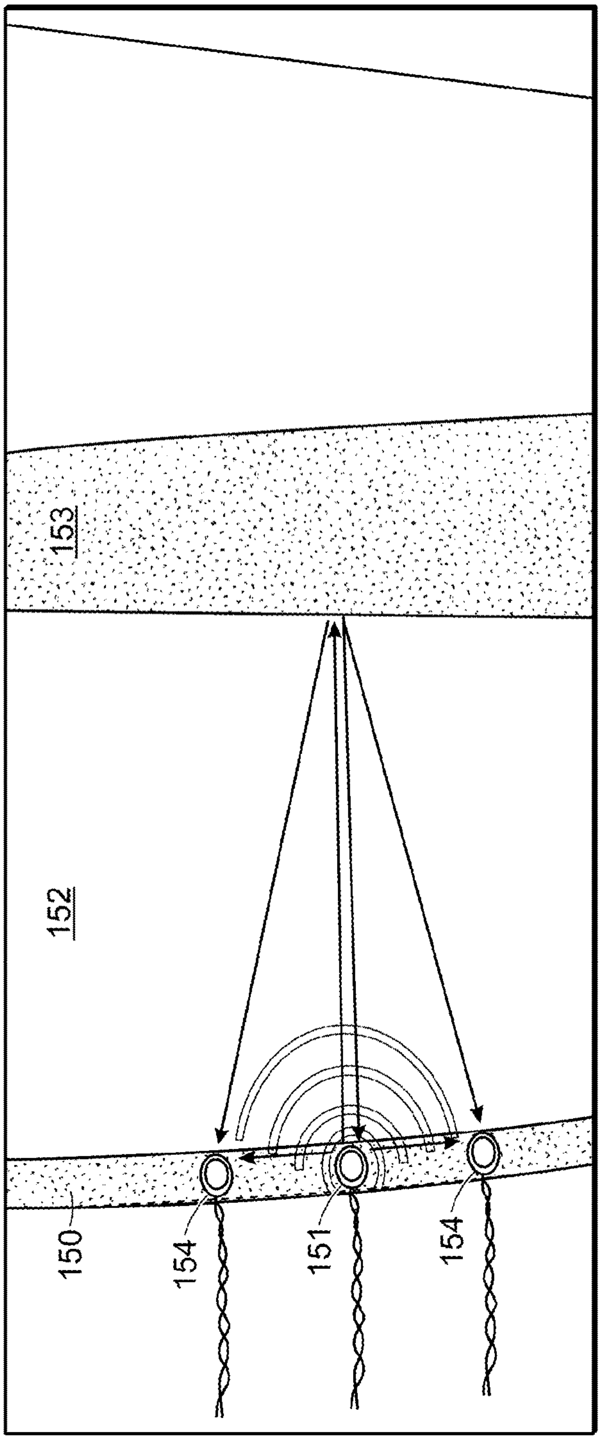


FIG. 30

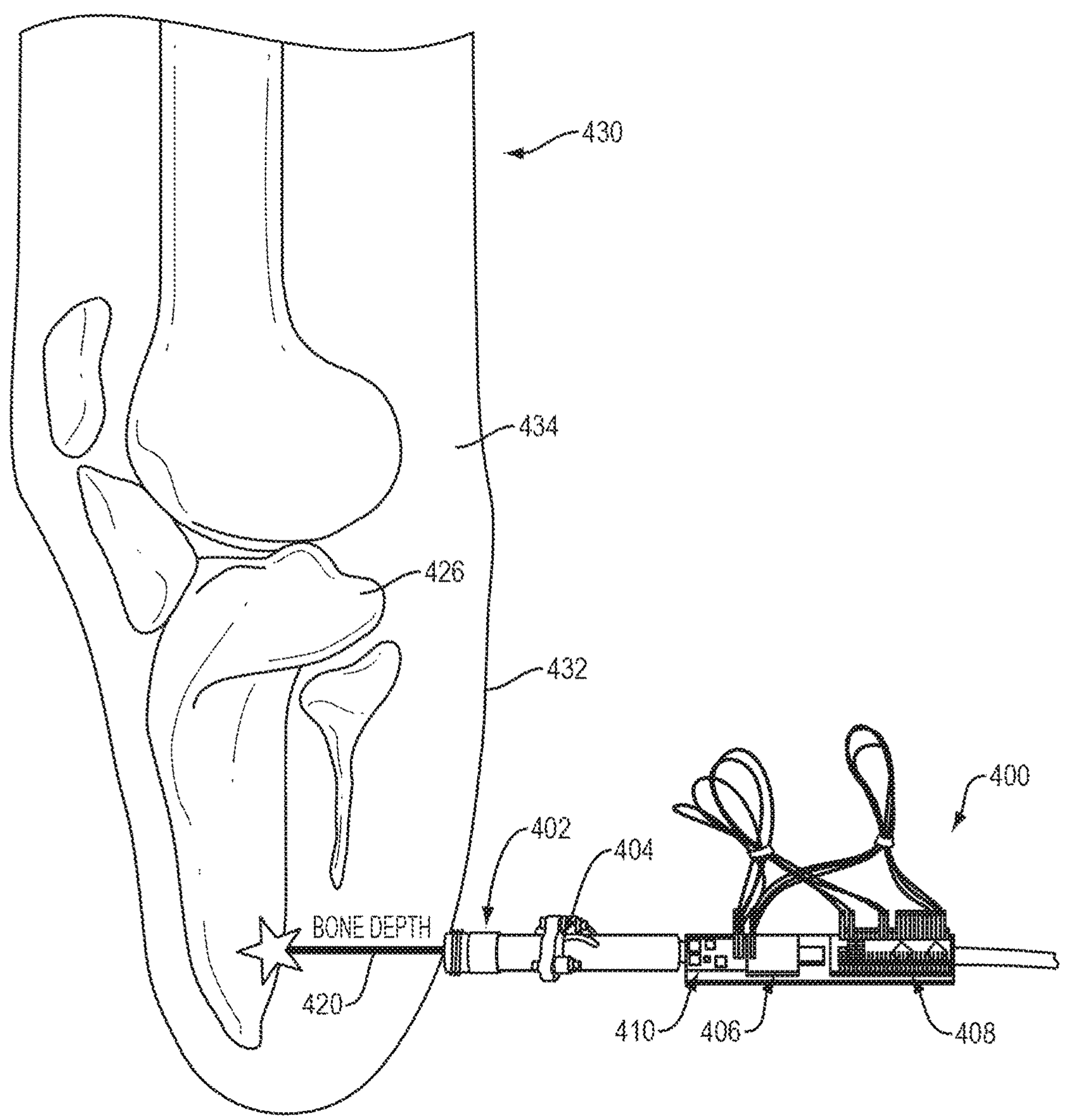


FIG. 31

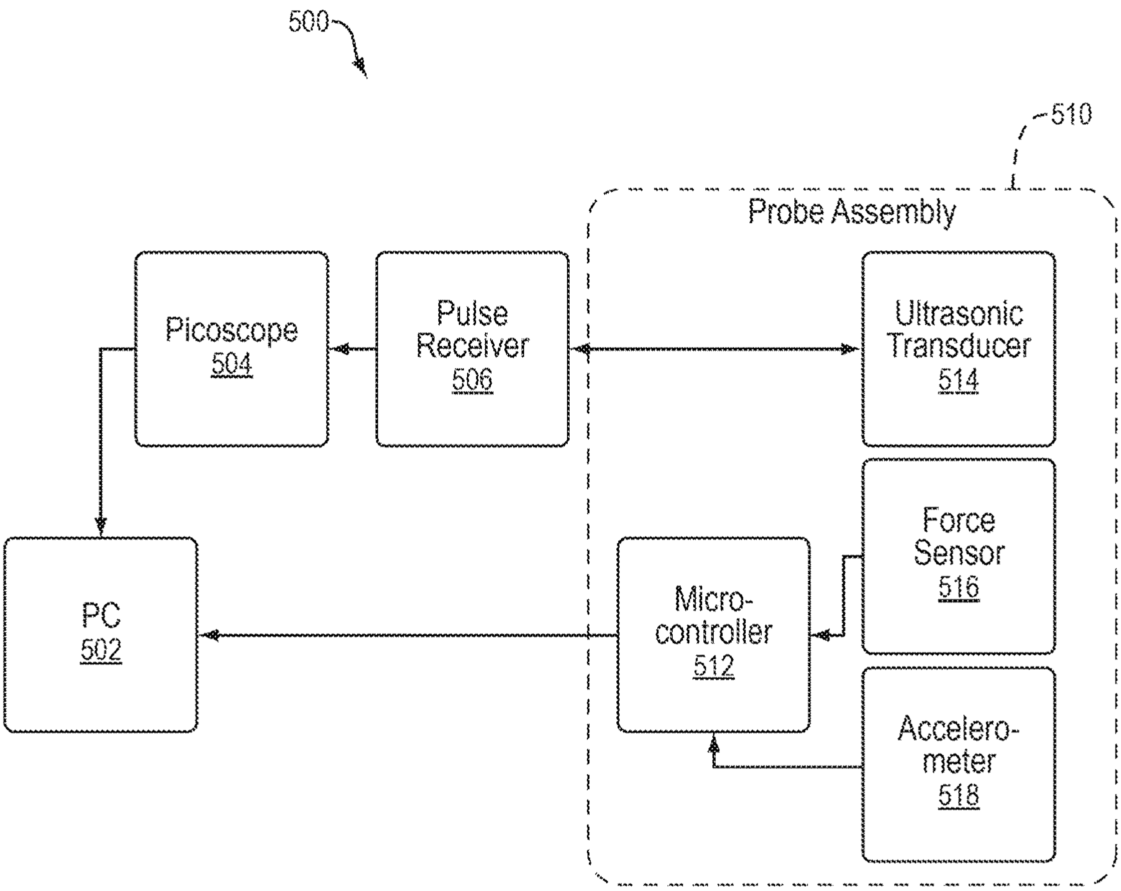
500
510
Probe Assembly
Picoscope
504
Pulse
Receiver
506
Ultrasonic
Transducer
514
Force
Sensor
516
PC
502
Micro-
controller
512
Accelero-
meter
518

FIG. 32

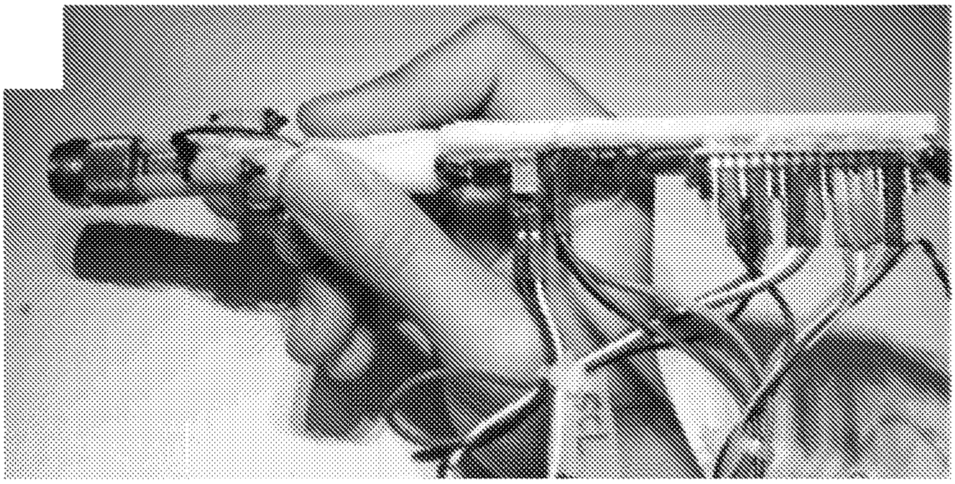

FIG. 33A

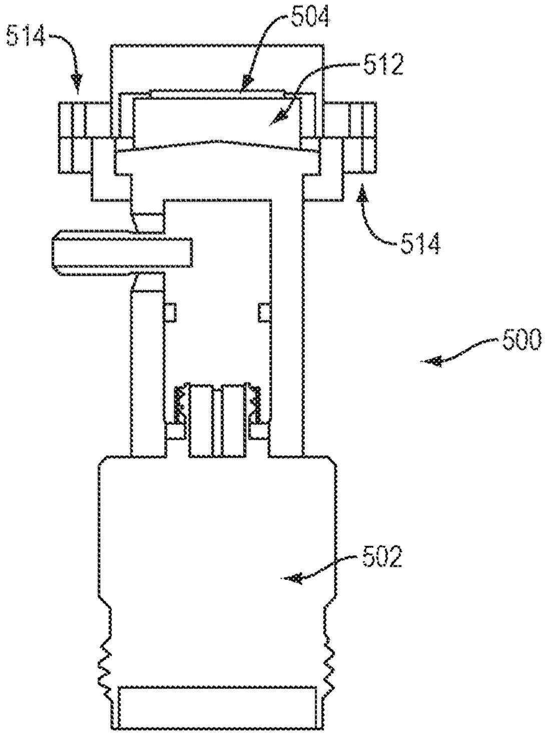


FIG. 33B

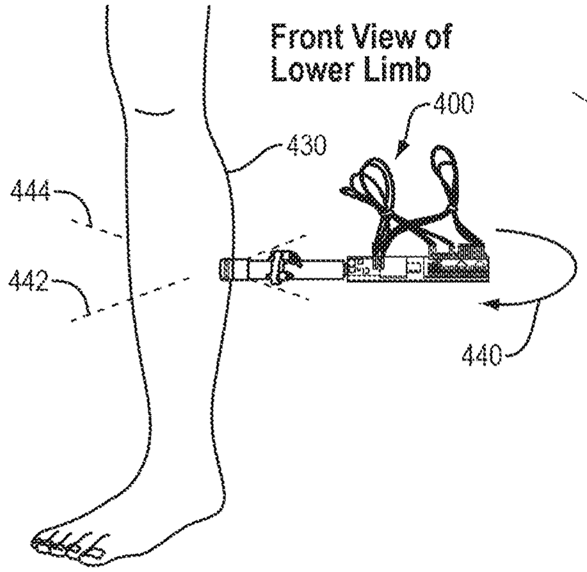


FIG. 34A

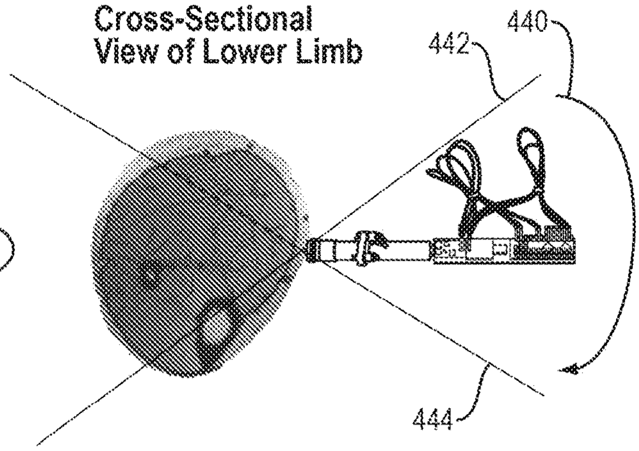


FIG. 34B

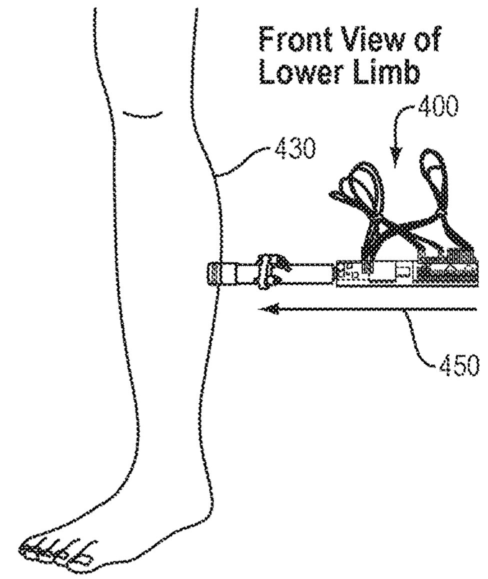


FIG. 35A

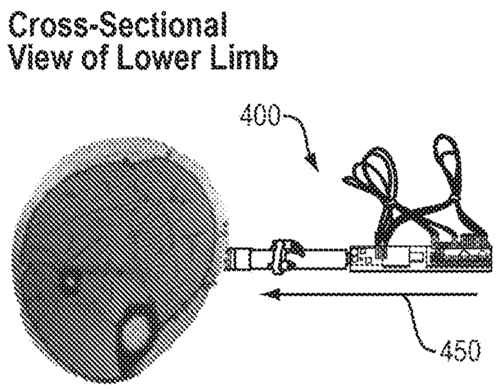


FIG. 35B

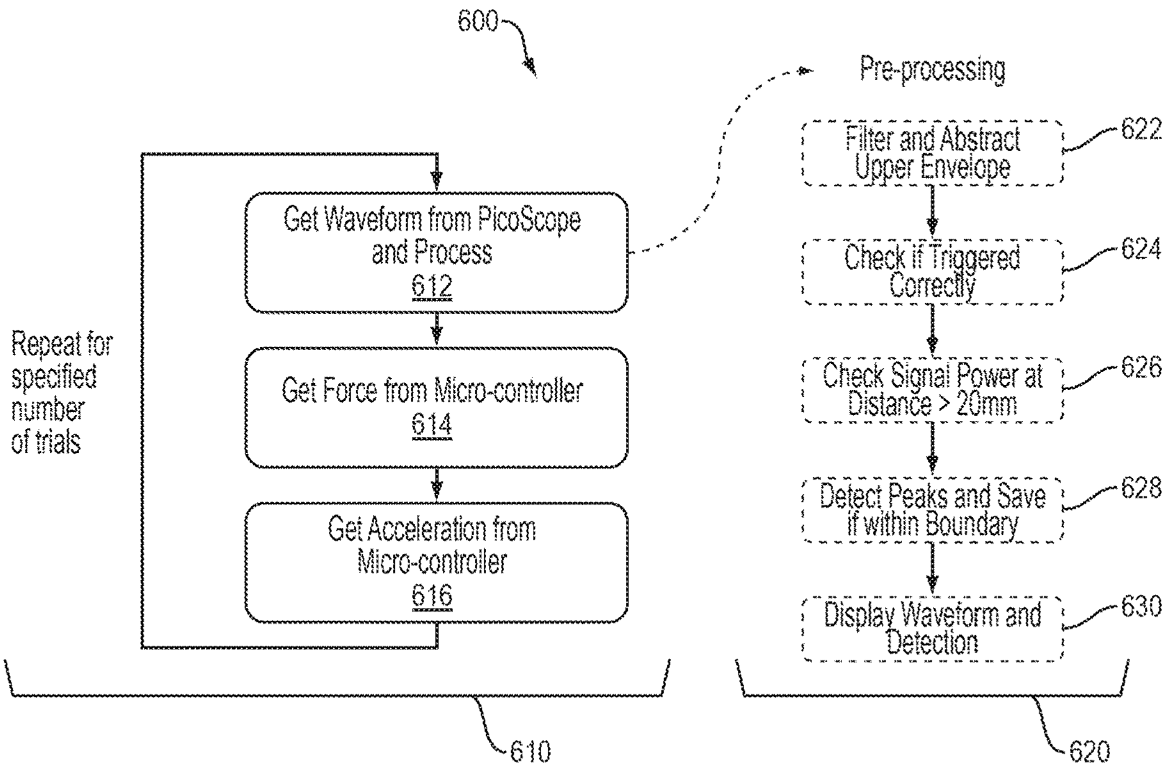
600
Pre-processing
Get Waveform from PicoScope and Process
612
Get Force from Micro-controller
614
Get Acceleration from Micro-controller
616
Repeat for specified number of trials
Filter and Abstract Upper Envelope
622
Check if Triggered Correctly
624
Check Signal Power at Distance > 20mm
626
Detect Peaks and Save if within Boundary
628
Display Waveform and Detection
630
610
620

FIG. 36

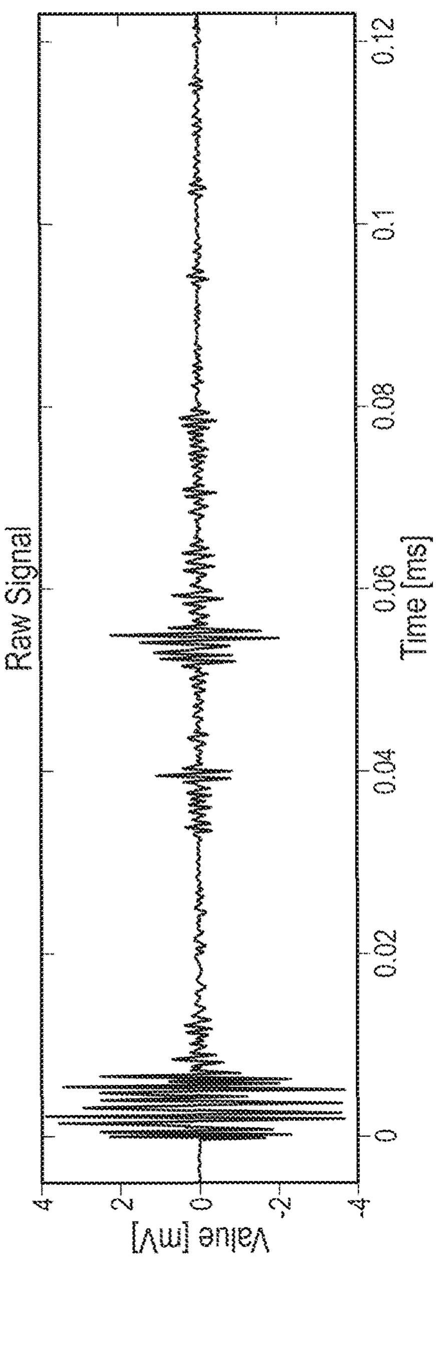


FIG. 37A

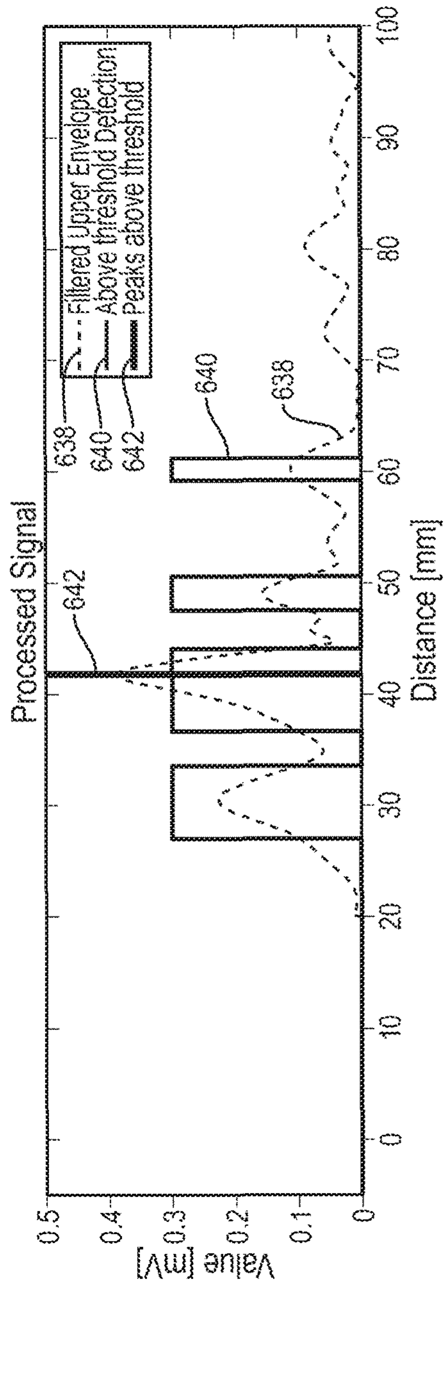


FIG. 37B

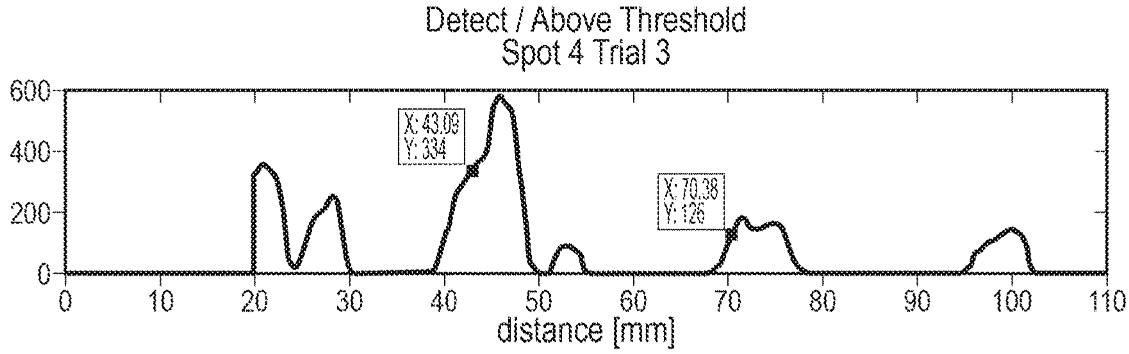


FIG. 38A

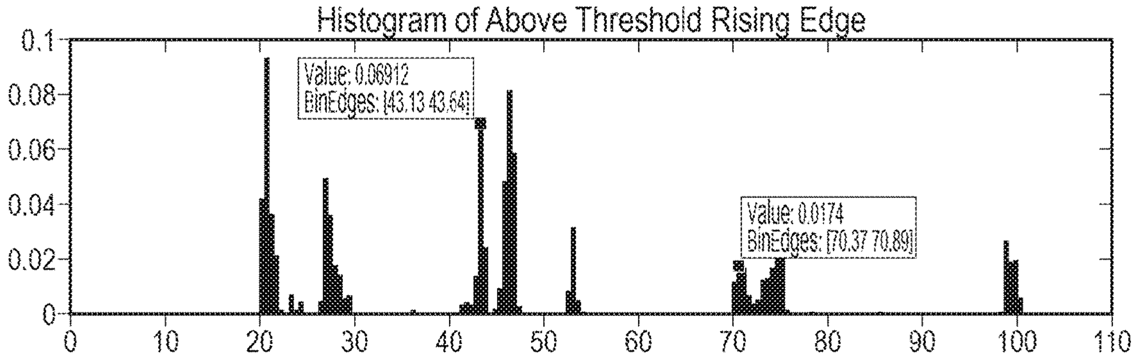


FIG. 38B

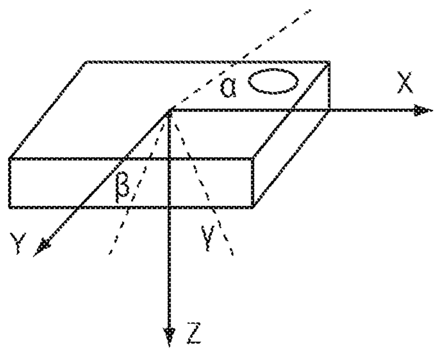


FIG. 39

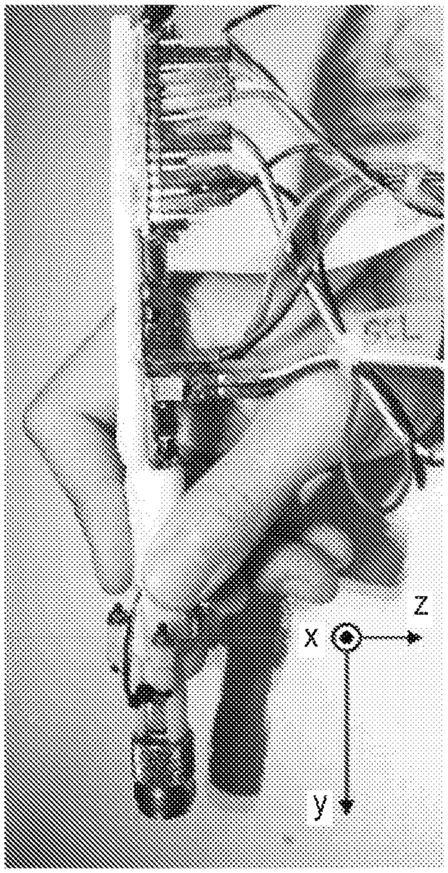


FIG. 40

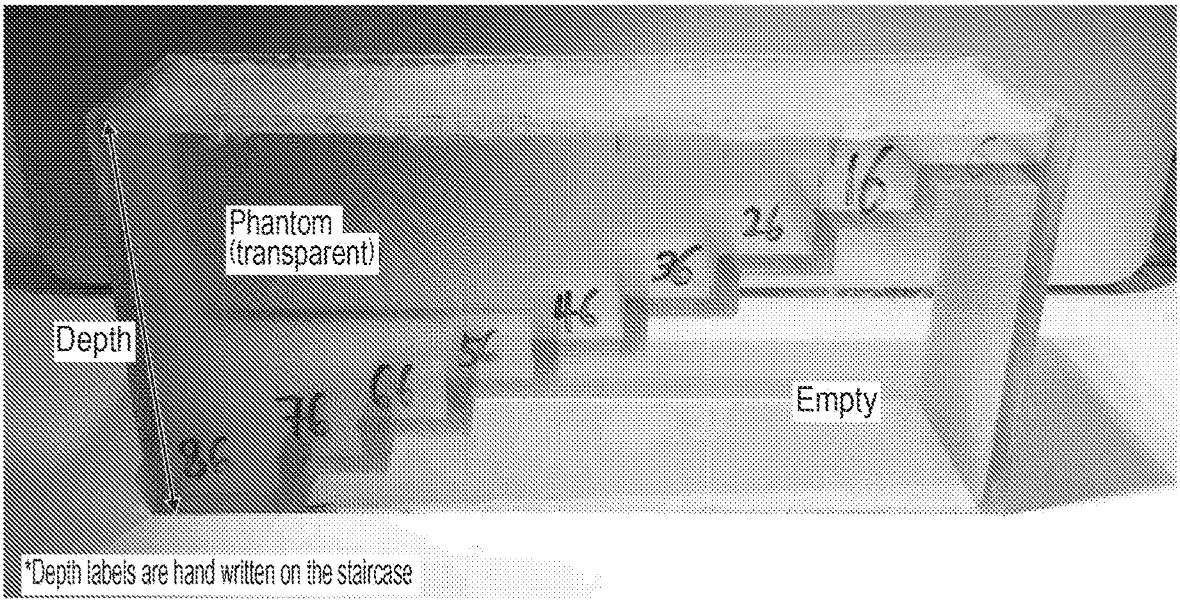
Phantom
(transparent)
Depth
Empty
*Depth labels are hand written on the staircase

FIG. 41

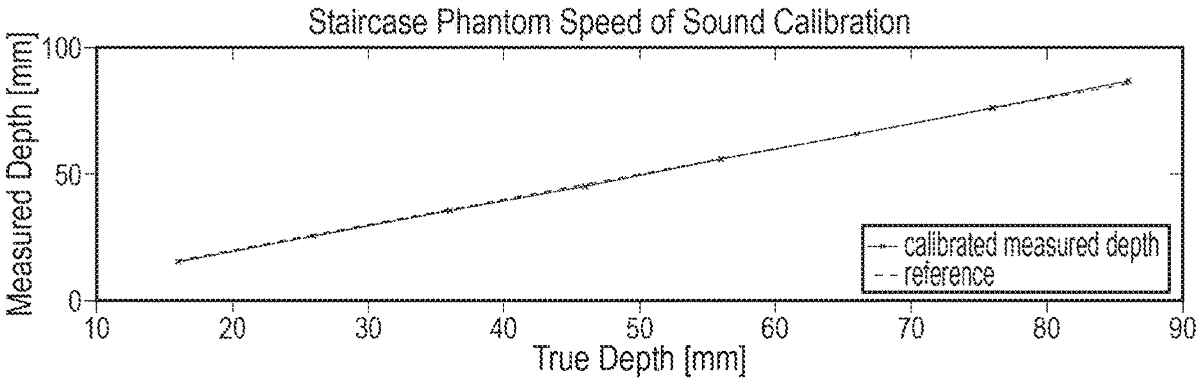
Staircase Phantom Speed of Sound Calibration
Measured Depth [mm]
100
50
0
10
20
30
40
50
60
70
80
90
True Depth [mm]
calibrated measured depth
reference

FIG. 42

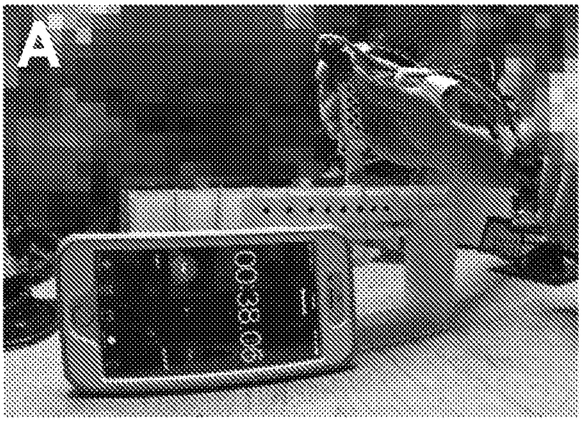


FIG. 43A

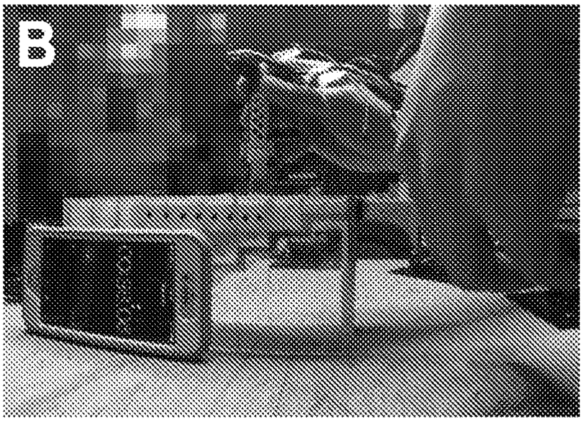


FIG. 43B

A

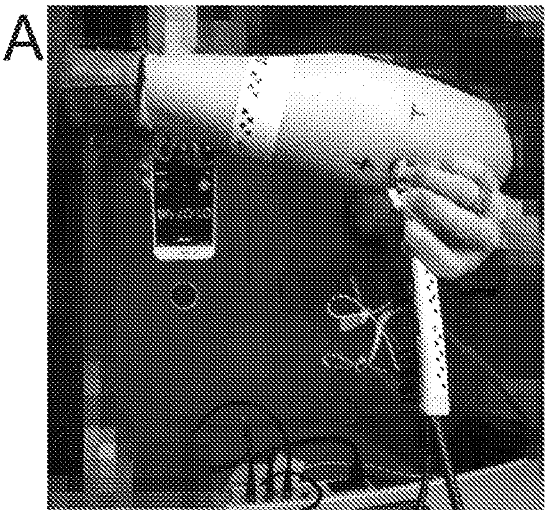

FIG. 44A

B

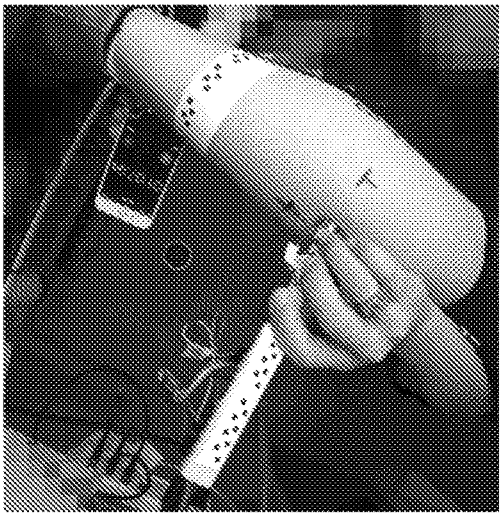

FIG. 44B

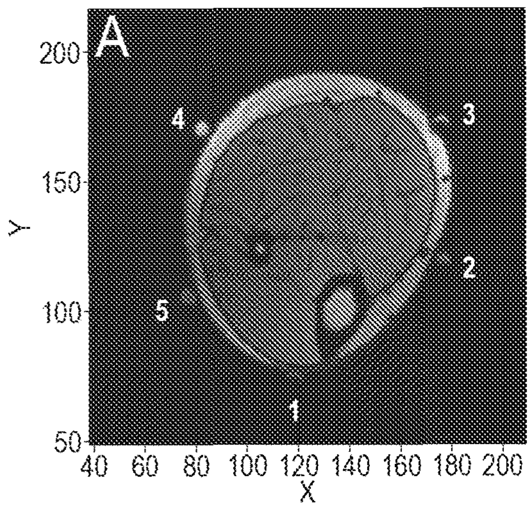


FIG. 45A

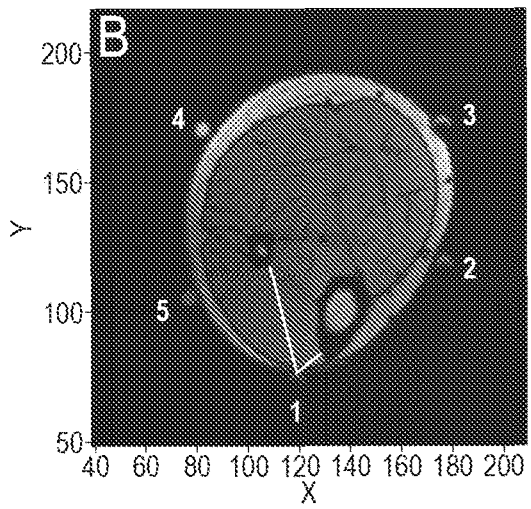


FIG. 45B

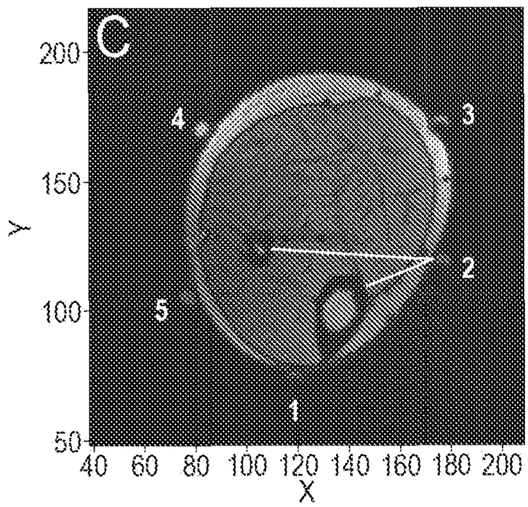


FIG. 45C

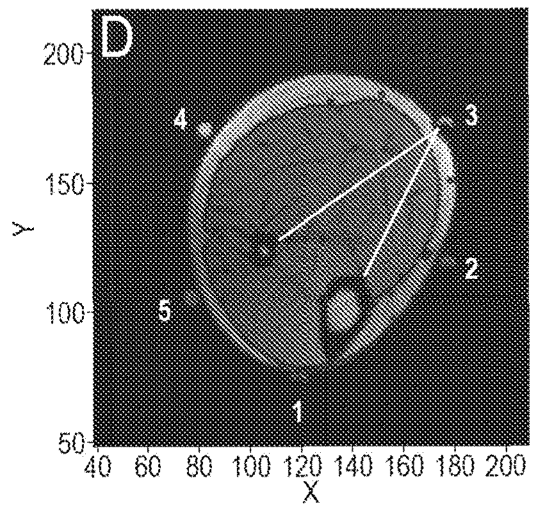


FIG. 45D

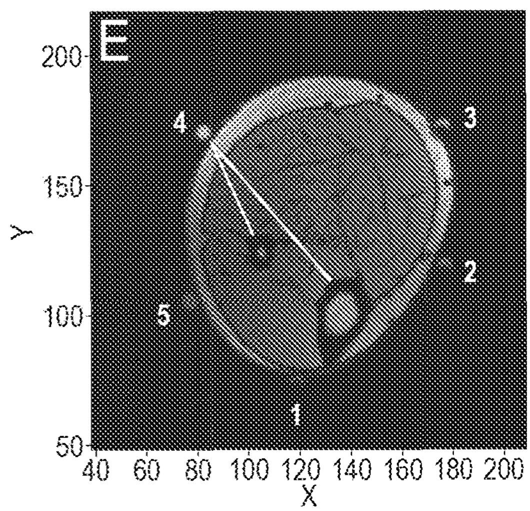


FIG. 45E

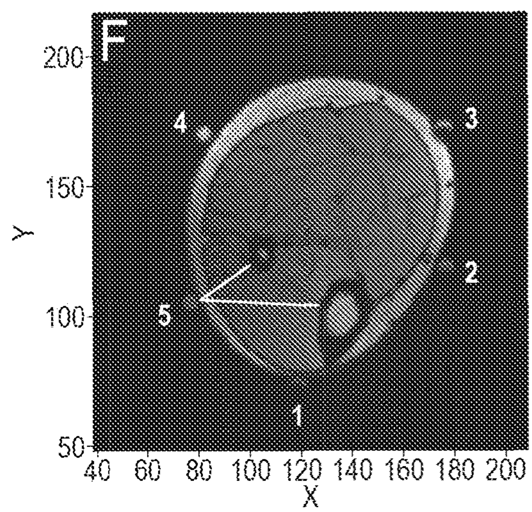


FIG. 45F

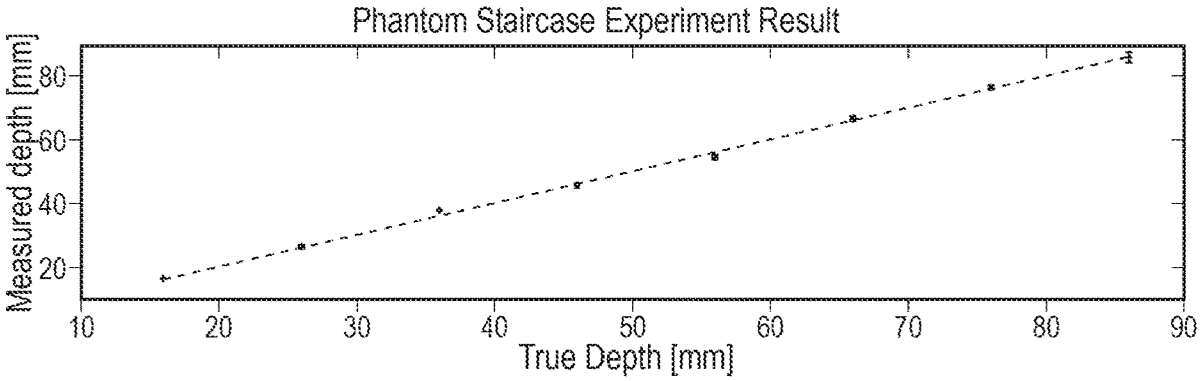


FIG. 46

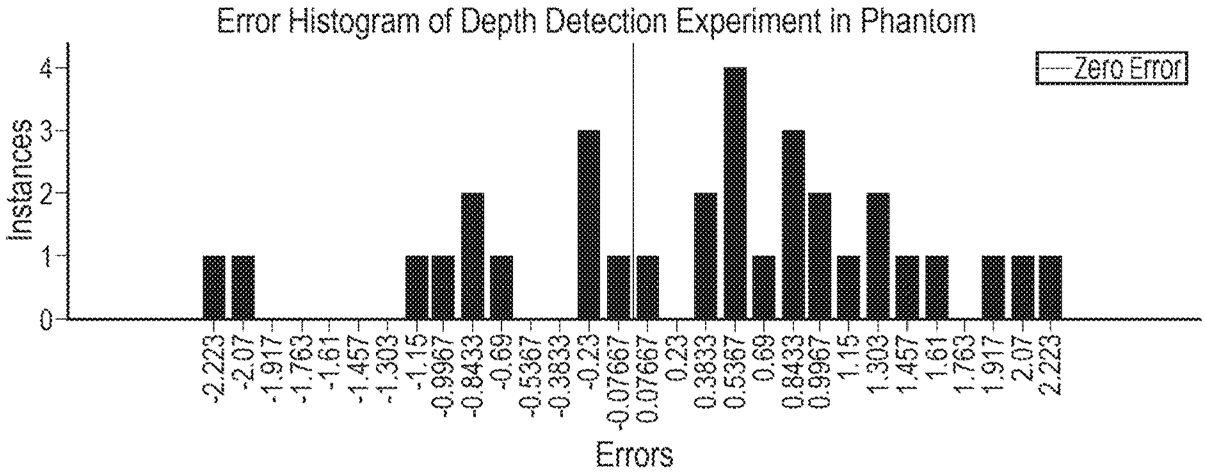


FIG. 47

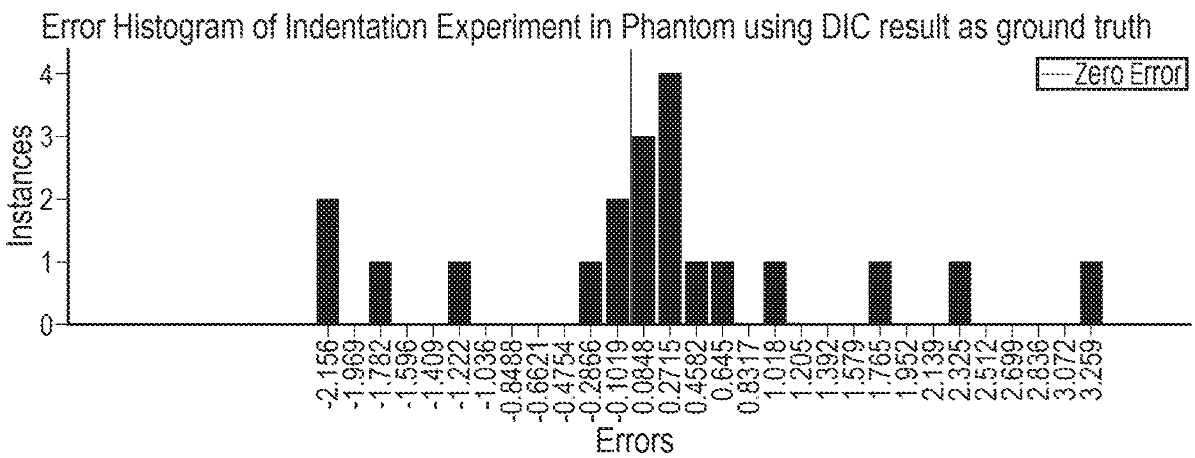


FIG. 48

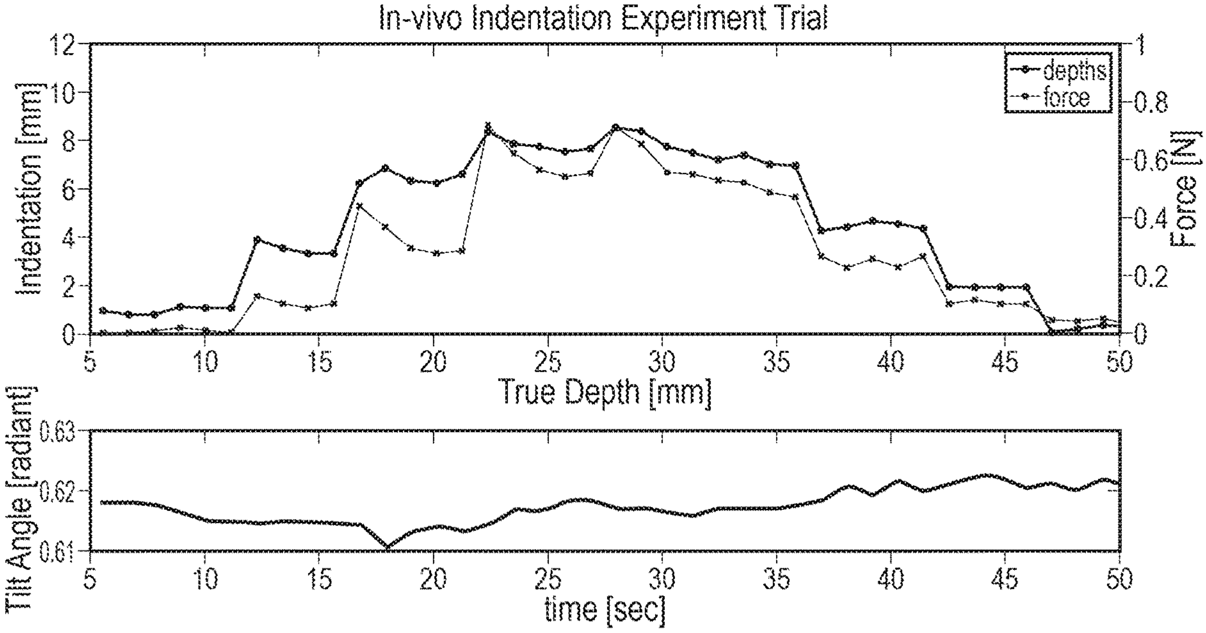
In-vivo Indentation Experiment Trial
Indentation [mm]
Force [N]
depths
force
True Depth [mm]
Tilt Angle [radiant]
time [sec]

FIG. 49

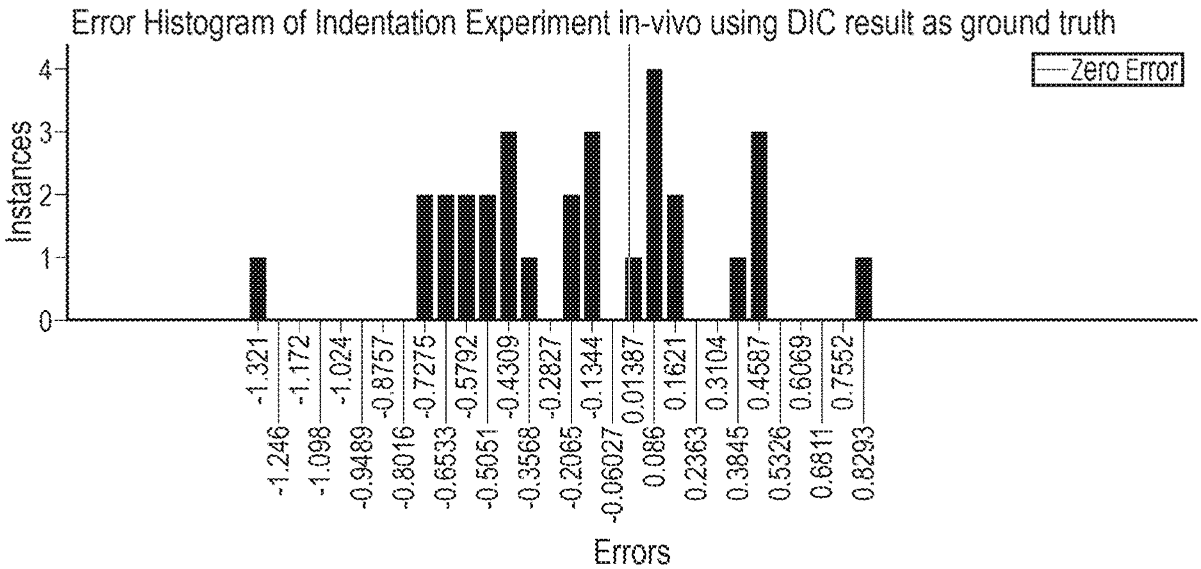
Error Histogram of Indentation Experiment in-vivo using DIC result as ground truth
Instances
Zero Error
-1.321
-1.246
-1.172
-1.098
-1.024
-0.9489
-0.8757
-0.8016
-0.7275
-0.6533
-0.5792
-0.5051
-0.4309
-0.3568
-0.2827
-0.2065
-0.1344
-0.06027
0.01387
0.086
0.1621
0.2363
0.3104
0.3845
0.4587
0.5326
0.6069
0.6811
0.7552
0.8293
Errors

FIG. 50

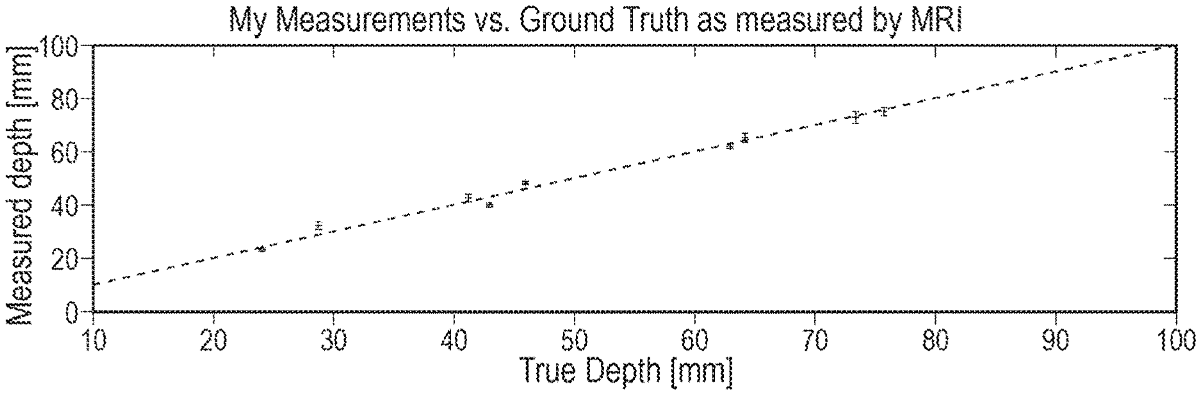


FIG. 51A

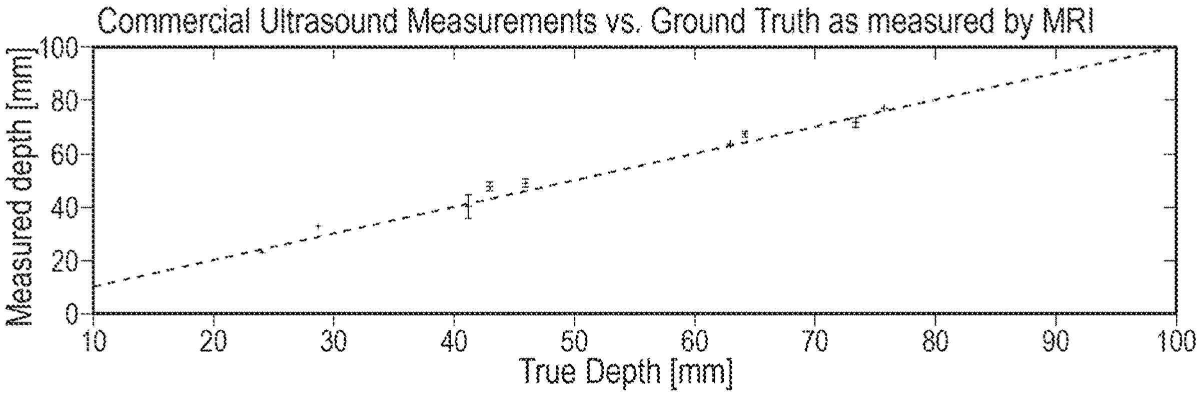


FIG. 51B

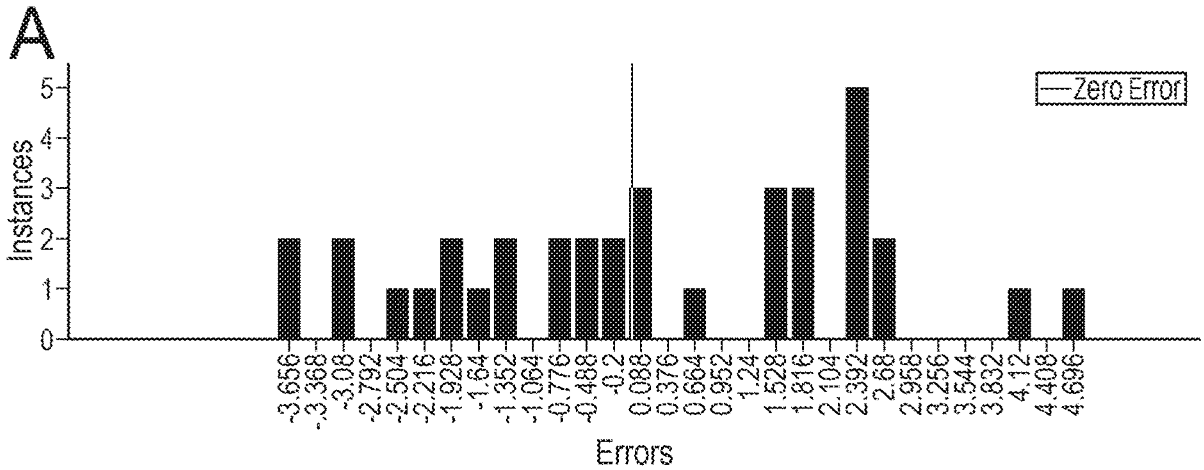


FIG. 52A

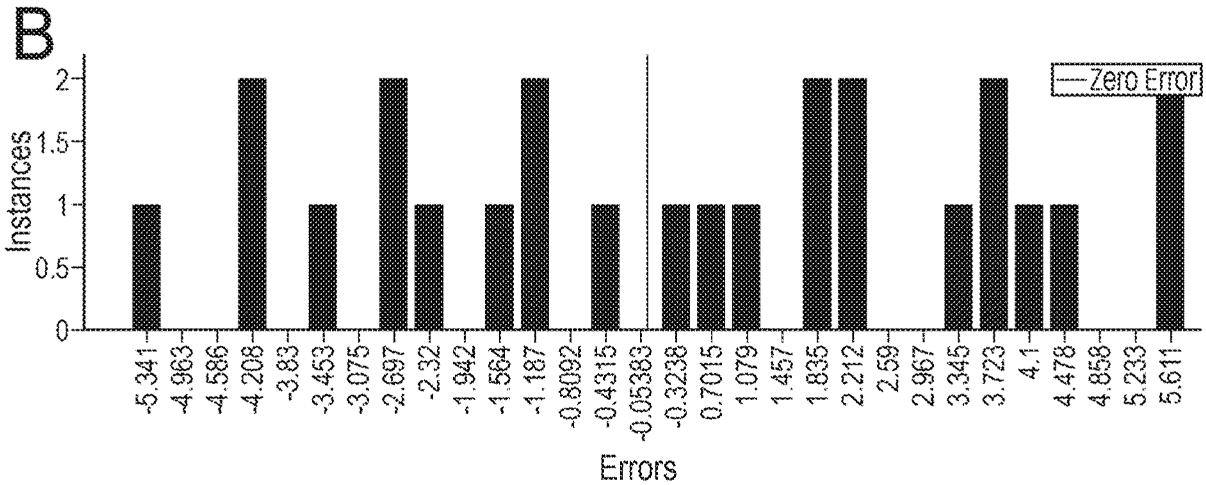


FIG. 52B

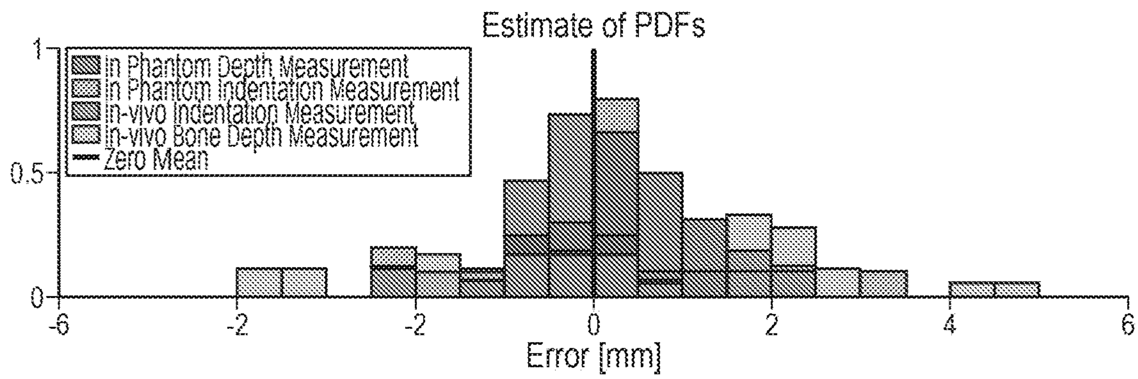


FIG. 53

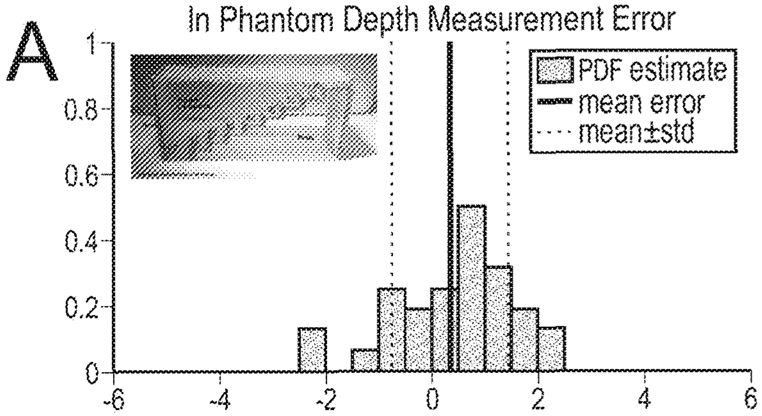


FIG. 54A

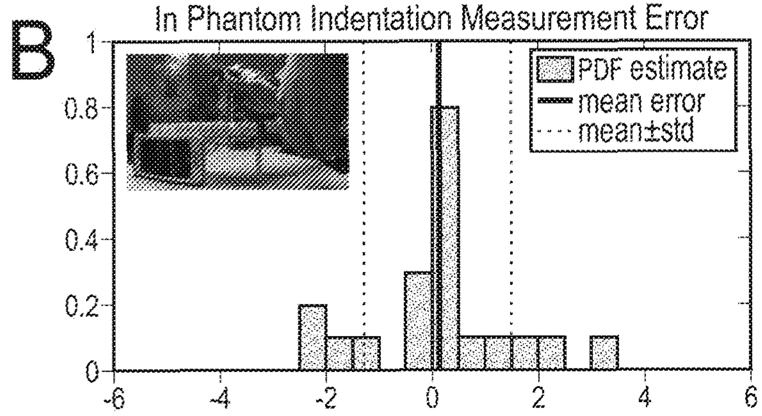


FIG. 54B

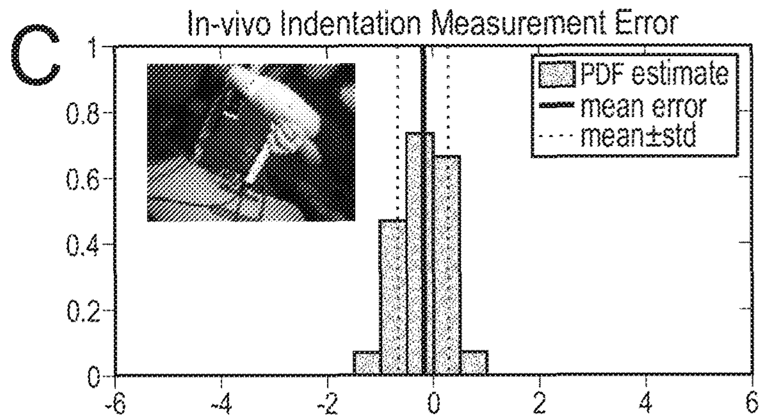


FIG. 54C

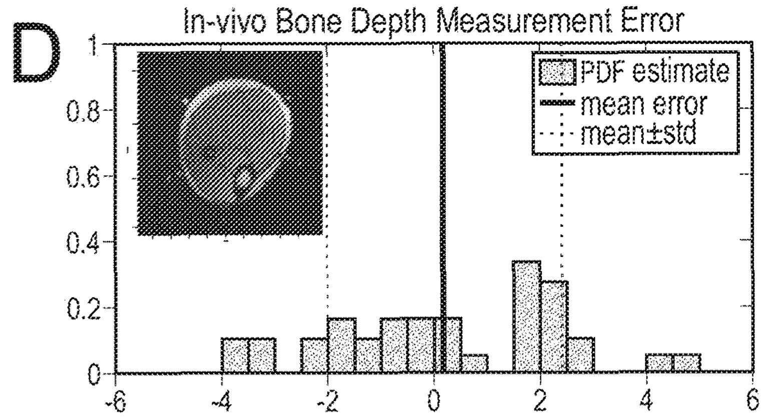


FIG. 54D

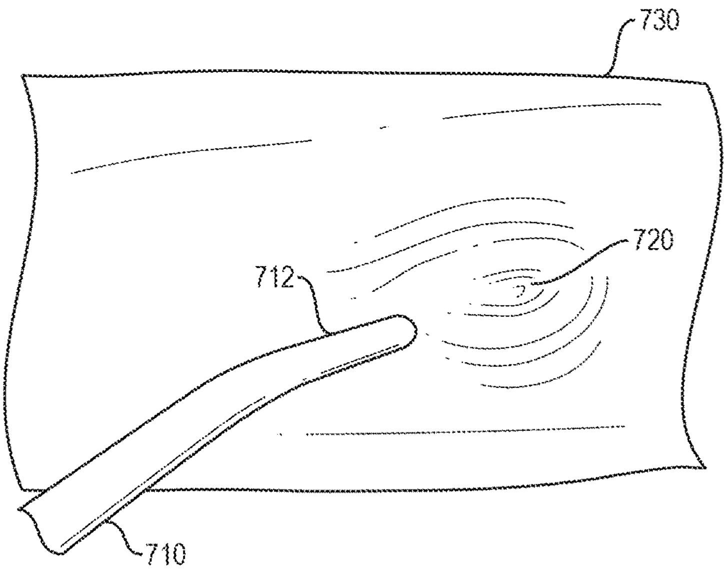
730
720
712
710

FIG. 55

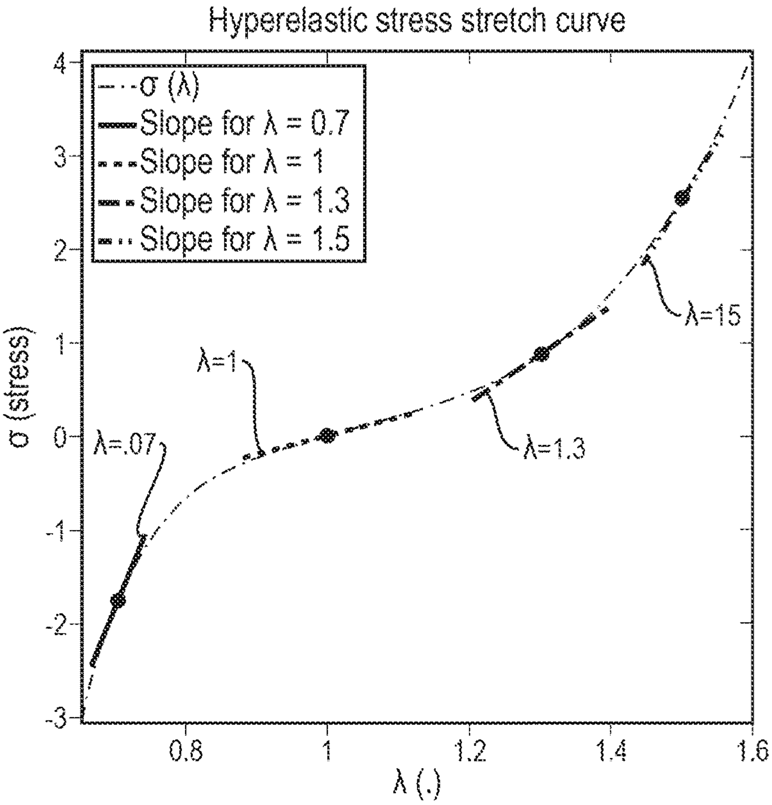
Hyperelastic stress stretch curve
σ (λ)
Slope for λ = 0.7
Slope for λ = 1
Slope for λ = 1.3
Slope for λ = 1.5
λ=15
λ=1
λ=1.3
λ=.07
σ (stress)
λ (.)
4
3
2
1
0
-1
-2
-3
0.8
1
1.2
1.4
1.6

FIG. 56

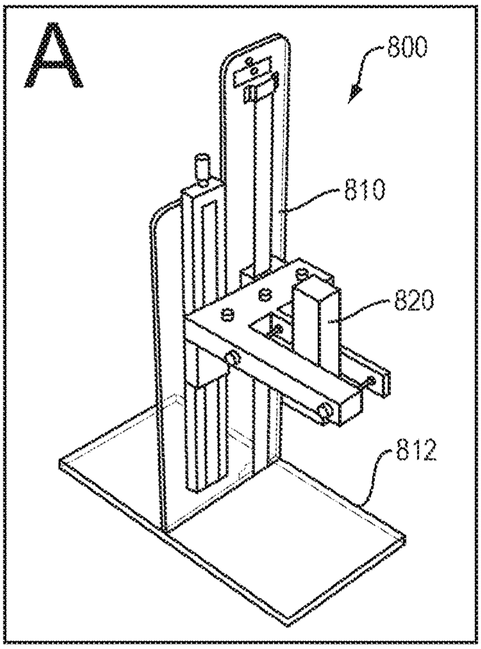


FIG. 57A

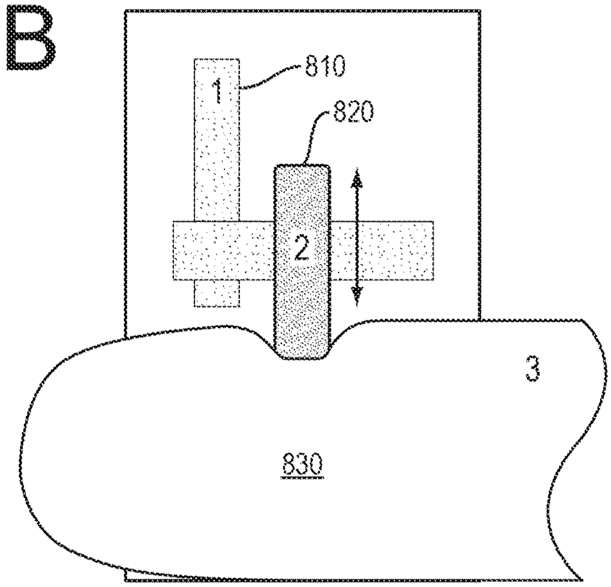


FIG. 57B

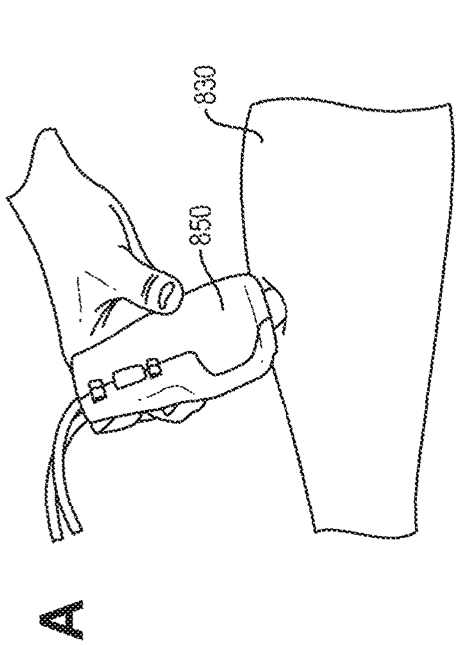


FIG. 58A

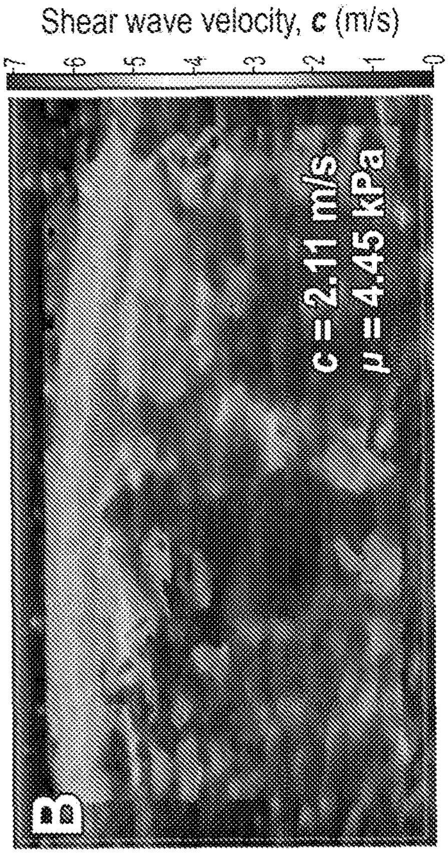


FIG. 58B

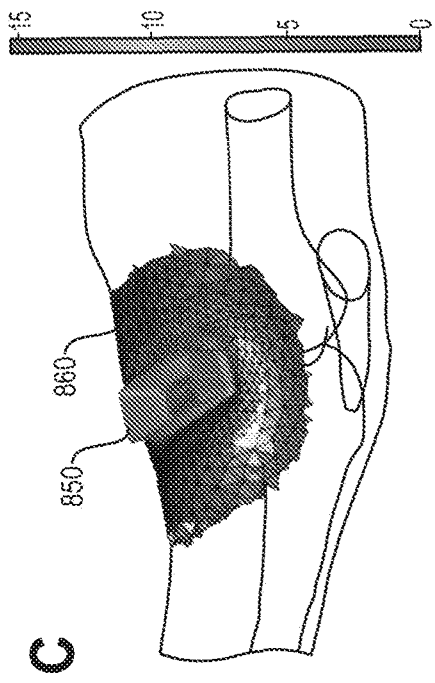


FIG. 58C

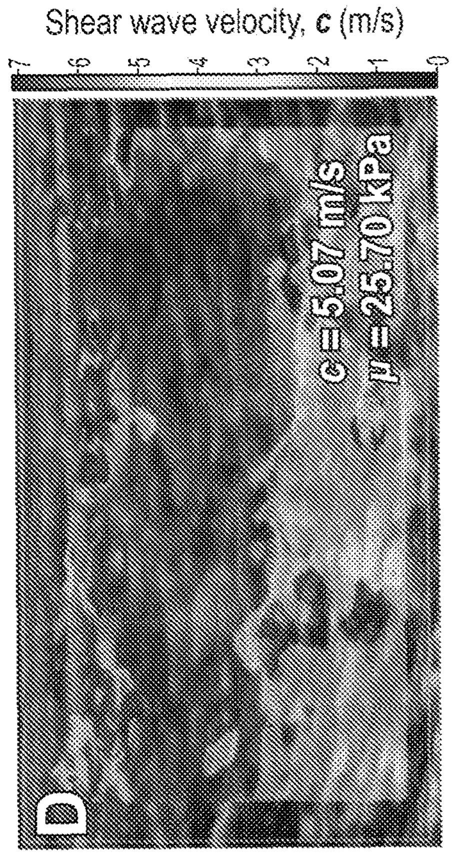


FIG. 58D

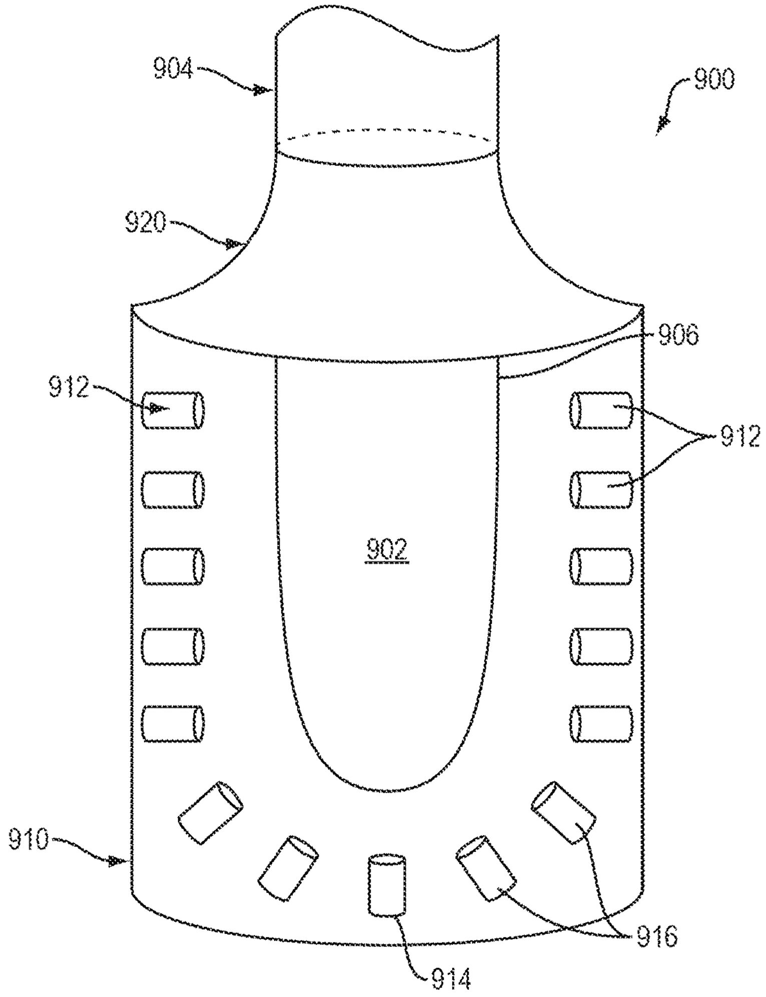


FIG. 59

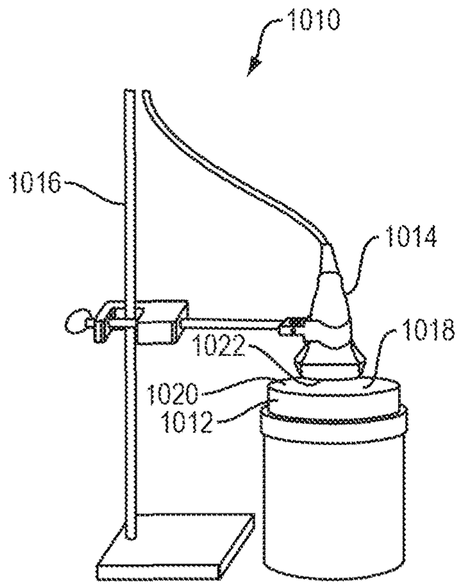


FIG. 60
(PRIOR ART)

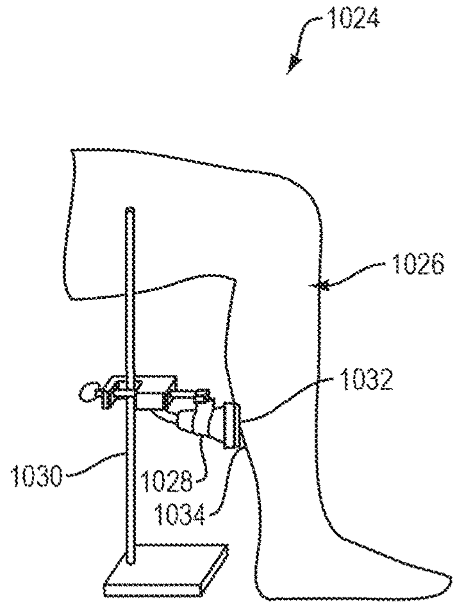


FIG. 61
(PRIOR ART)

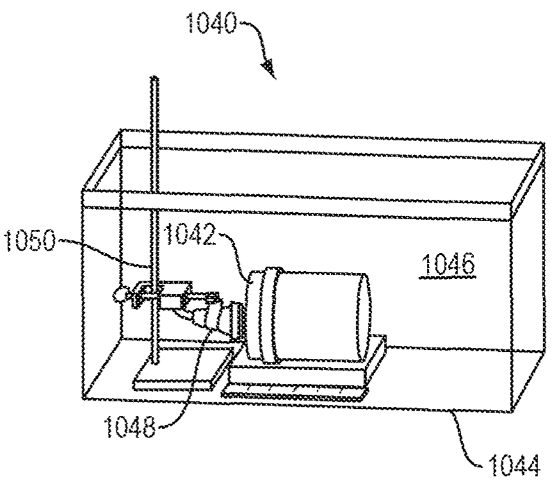


FIG. 62

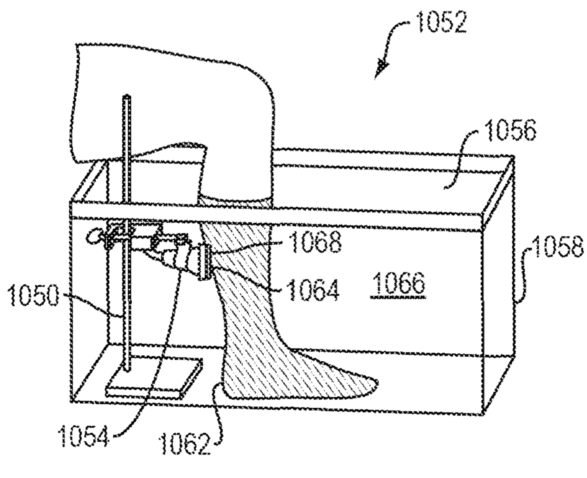


FIG. 63

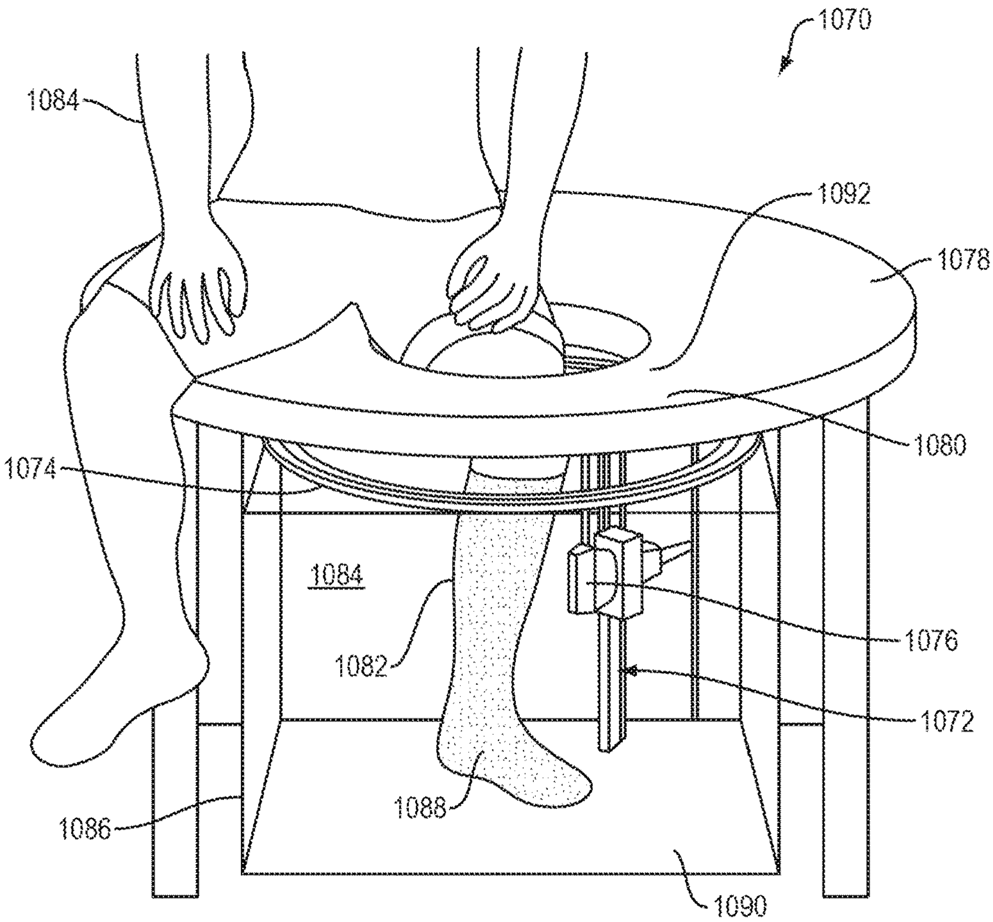
1070
1084
1092
1078
1074
1080
1084
1076
1082
1072
1086
1088
1090

FIG. 64

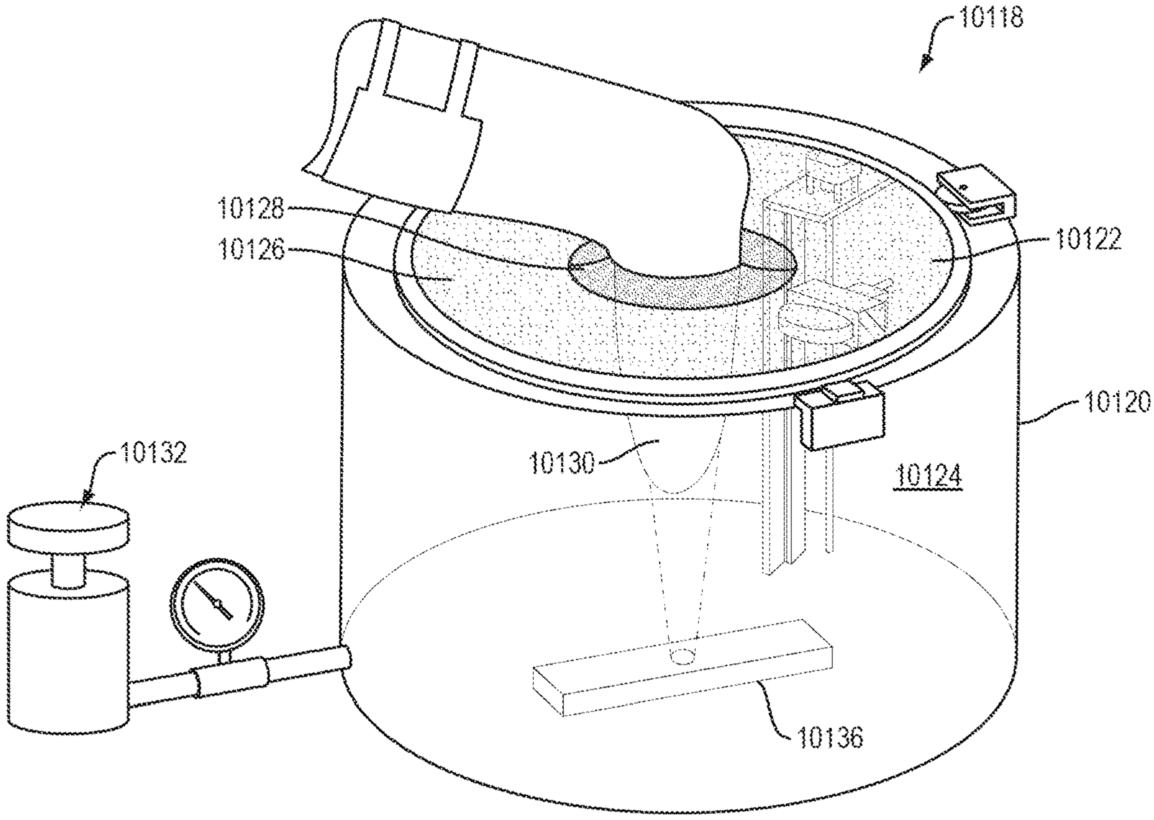


FIG. 65

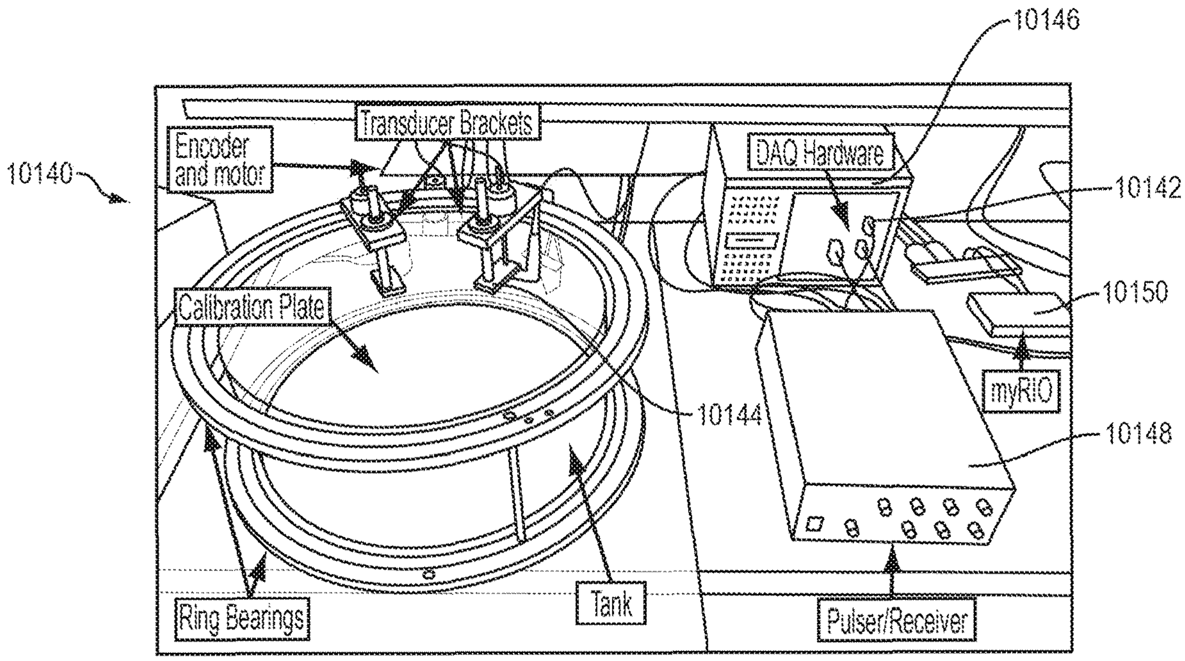
10146
10140
Transducer Brackets
Encoder and motor
DAQ Hardware
10142
10150
Calibration Plate
myRIO
10144
10148
Ring Bearings
Tank
Pulser/Receiver

FIG. 66

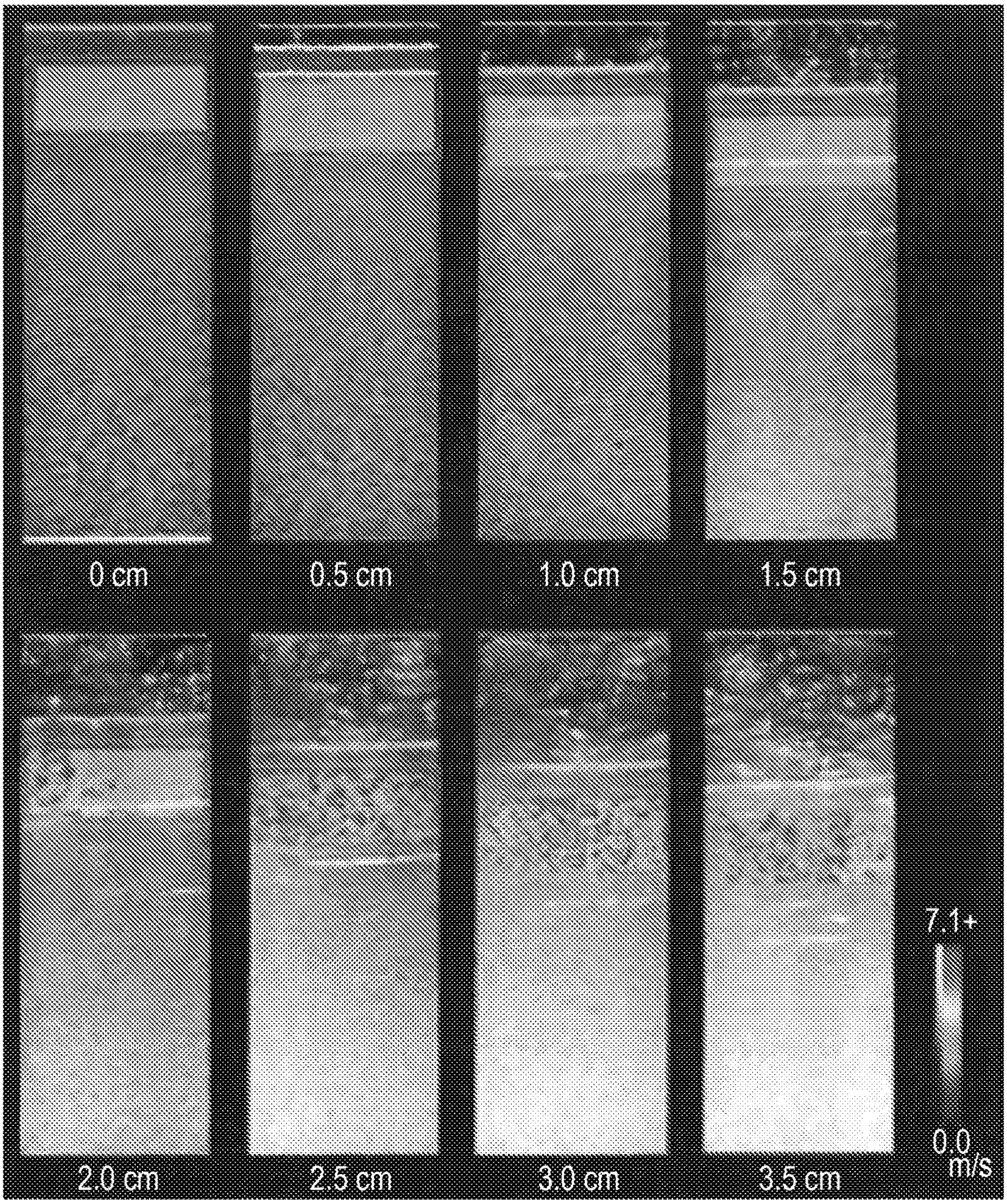


FIG. 67

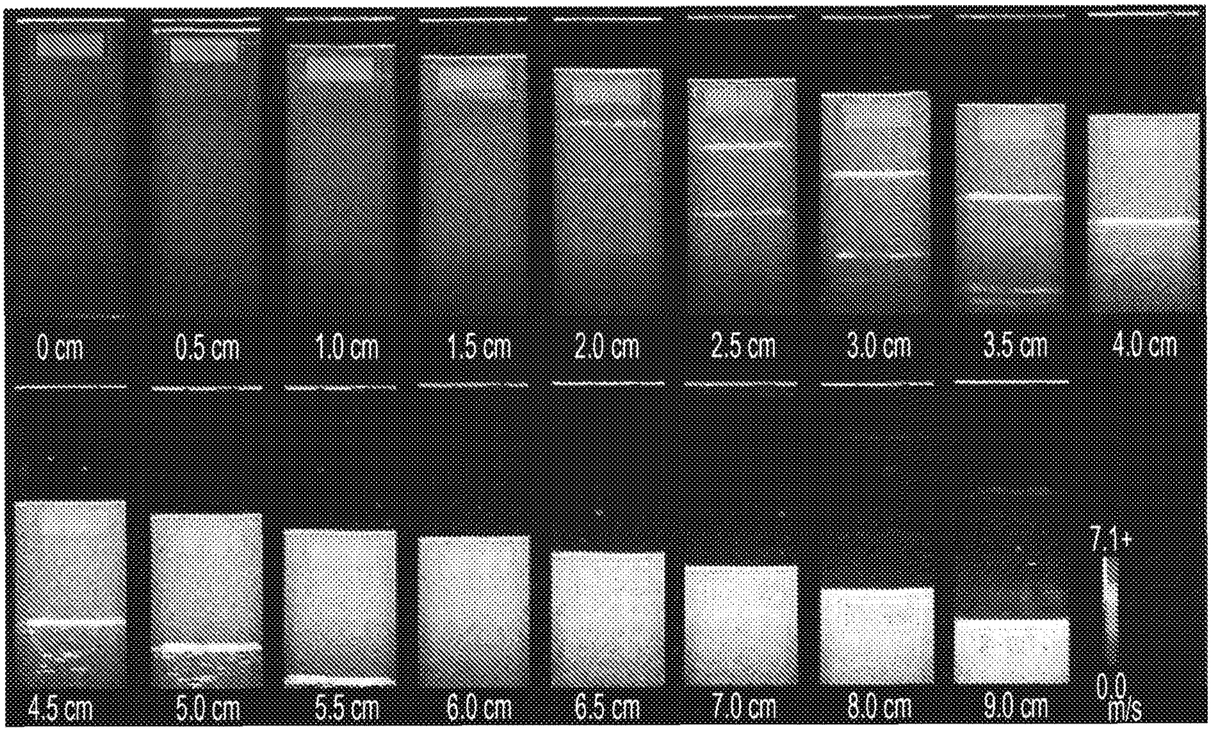
0 cm
0.5 cm
1.0 cm
1.5 cm
2.0 cm
2.5 cm
3.0 cm
3.5 cm
4.0 cm
4.5 cm
5.0 cm
5.5 cm
6.0 cm
6.5 cm
7.0 cm
8.0 cm
9.0 cm
7.1+
0.0
m/s

FIG. 68

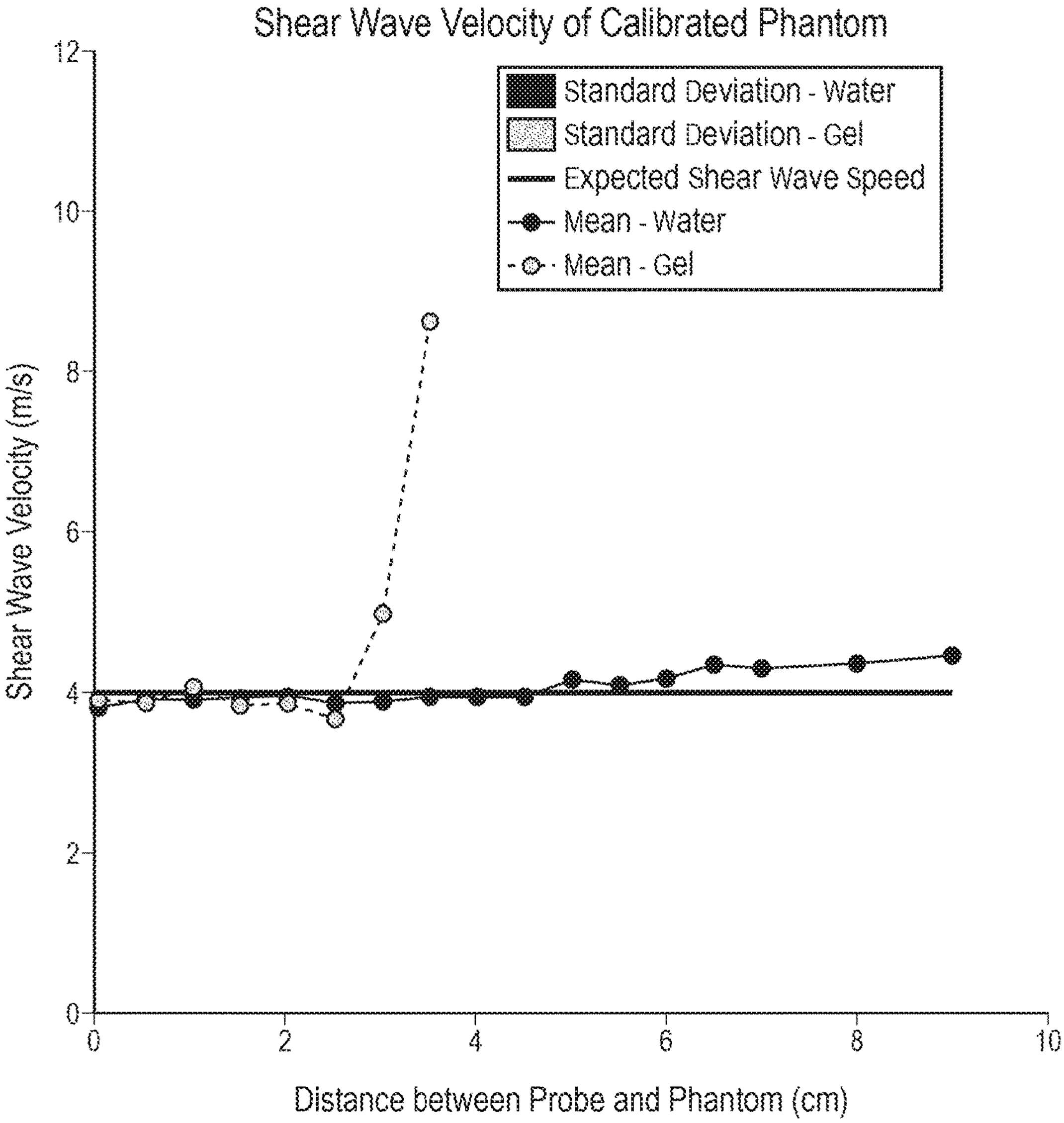
Shear Wave Velocity of Calibrated Phantom
Standard Deviation - Water
Standard Deviation - Gel
Expected Shear Wave Speed
Mean - Water
Mean - Gel
Shear Wave Velocity (m/s)
0
2
4
6
8
10
12
Distance between Probe and Phantom (cm)
0
2
4
6
8
10

FIG. 69

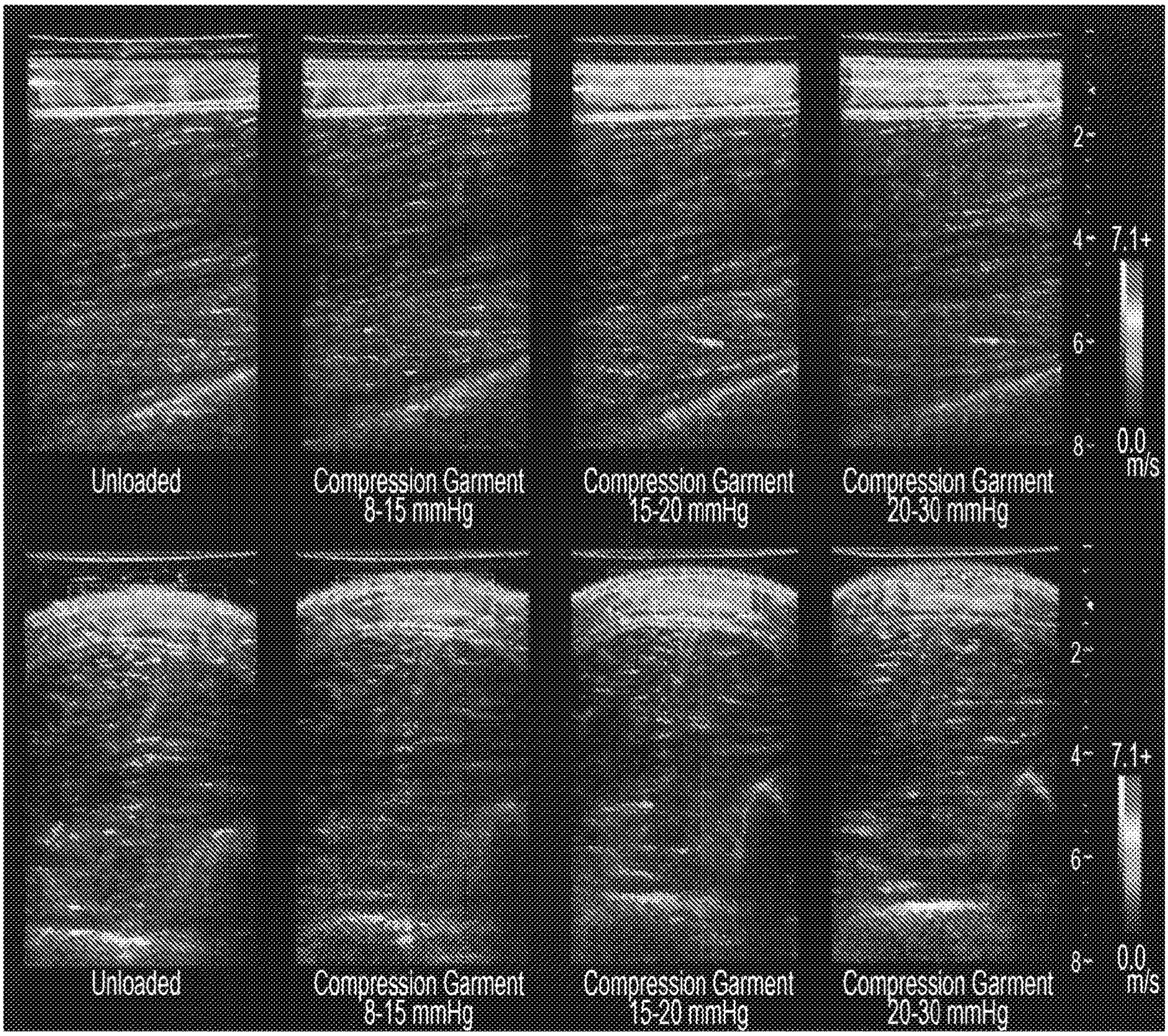
2
4
6
8
7.1+
0.0
m/s
Unloaded
Compression Garment
8-15 mmHg
Compression Garment
15-20 mmHg
Compression Garment
20-30 mmHg
2
4
6
8
7.1+
0.0
m/s
Unloaded
Compression Garment
8-15 mmHg
Compression Garment
15-20 mmHg
Compression Garment
20-30 mmHg

FIG. 70

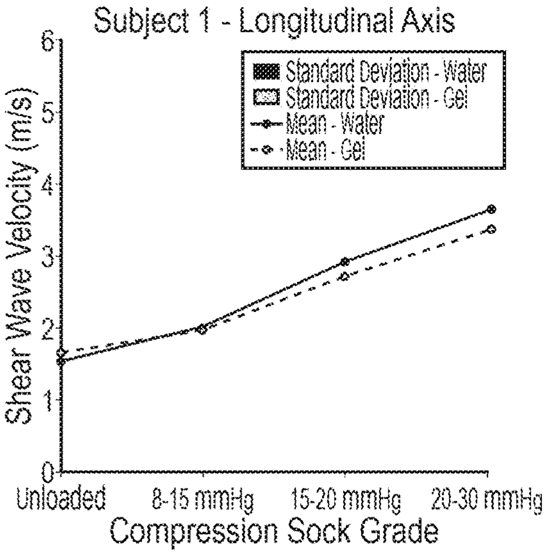


FIG. 71A

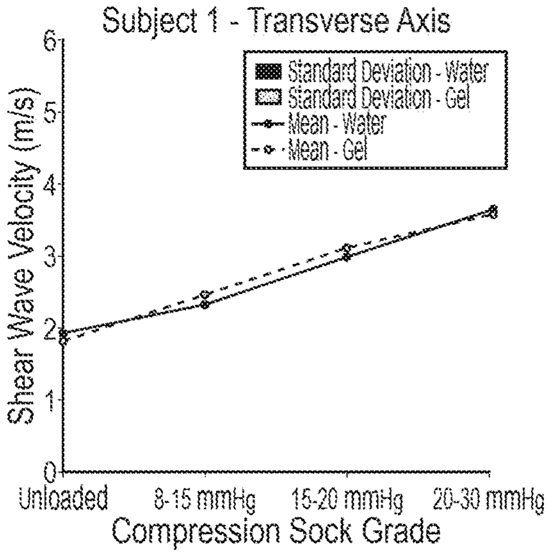


FIG. 71B

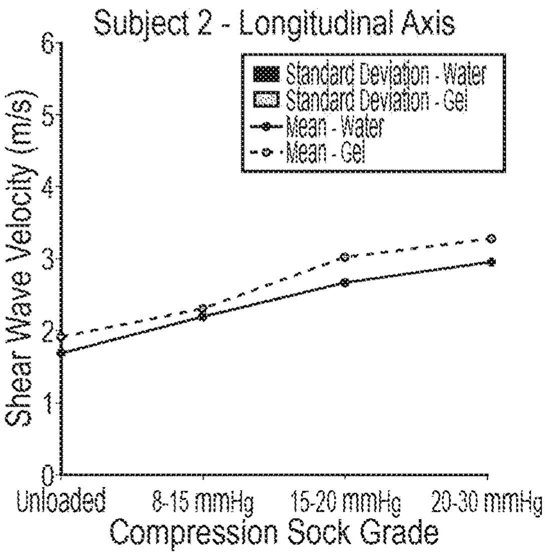


FIG. 72A

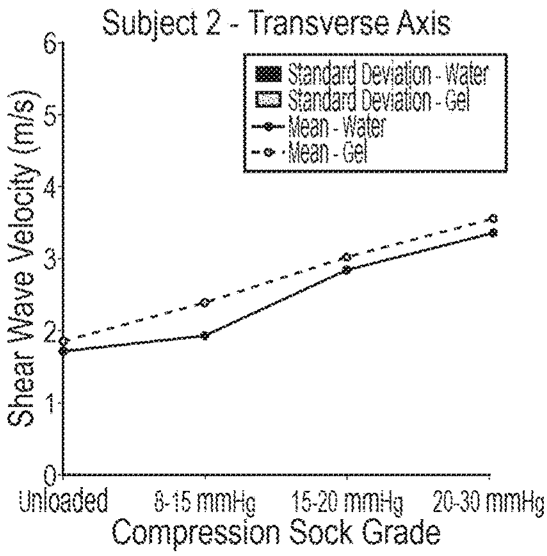


FIG. 72B

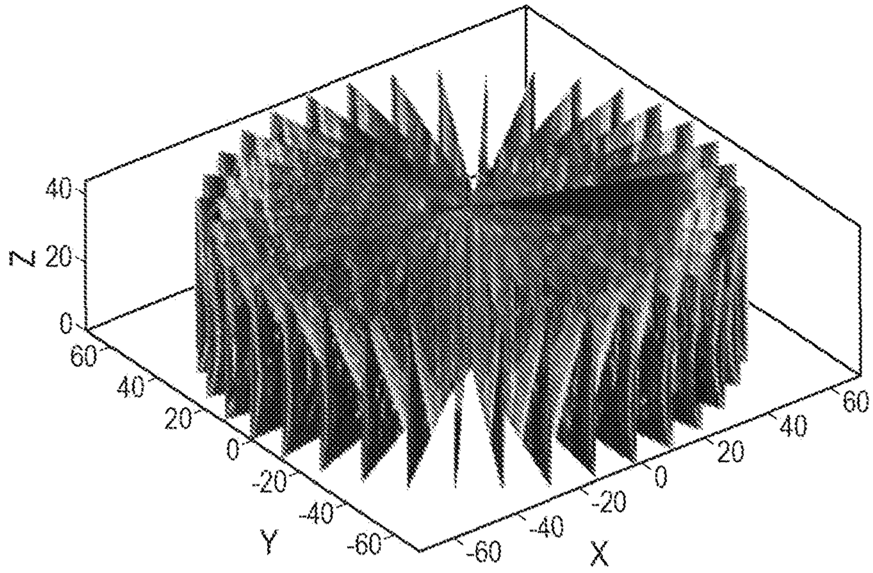
Z
40
20
0
Y
60
40
20
0
-20
-40
-60
X
-60
-40
-20
0
20
40
60

FIG. 73

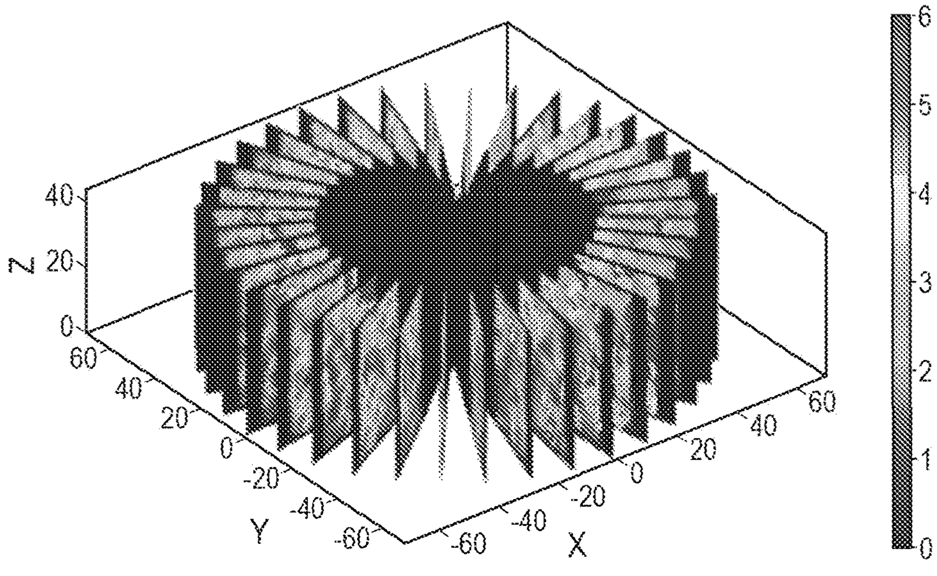
Z
40
20
0
Y
60
40
20
0
-20
-40
-60
X
-60
-40
-20
0
20
40
60
Shear Wave Speed (m/s)
6
5
4
3
2
1
0

FIG. 74

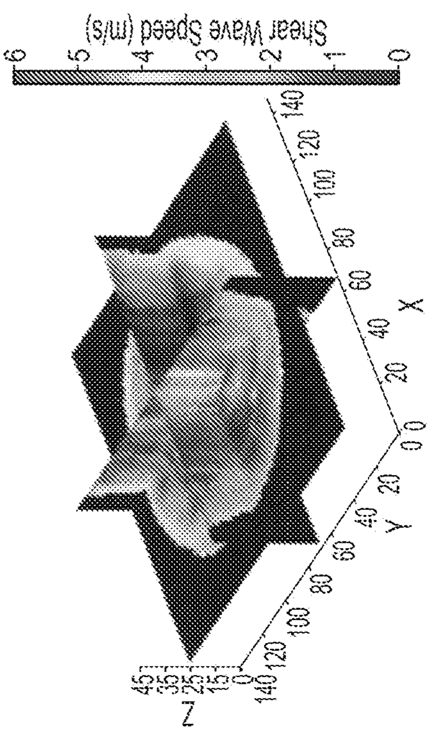

FIG. 75A

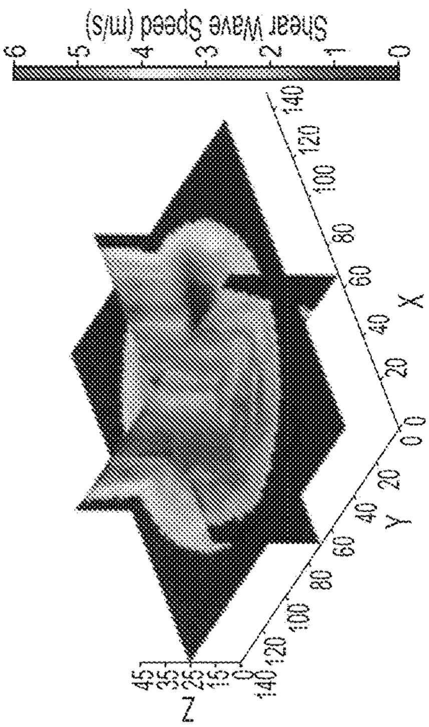

FIG. 75B

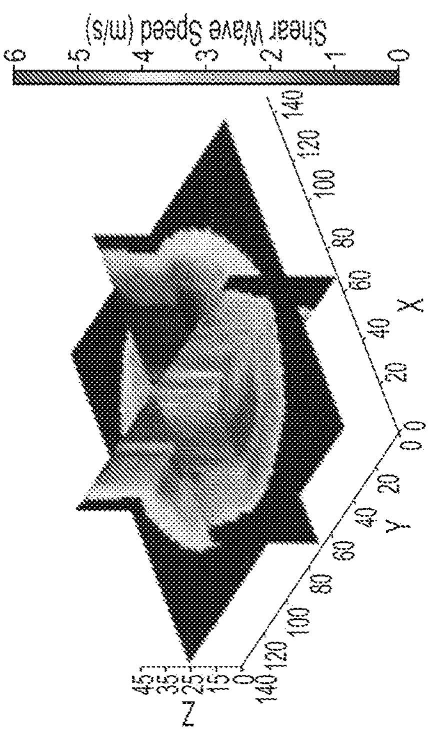

FIG. 75C

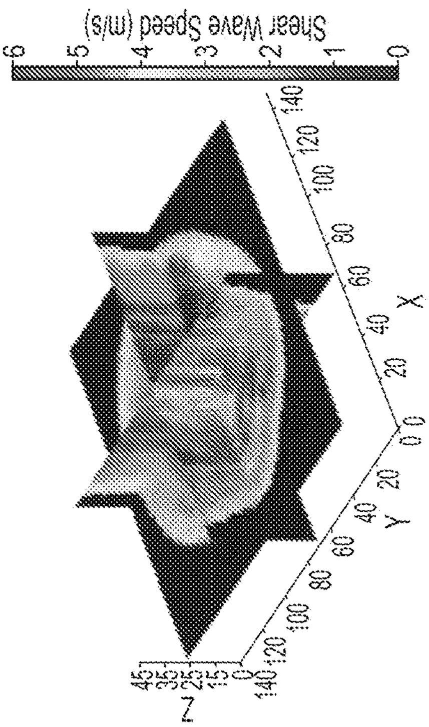

FIG. 75D

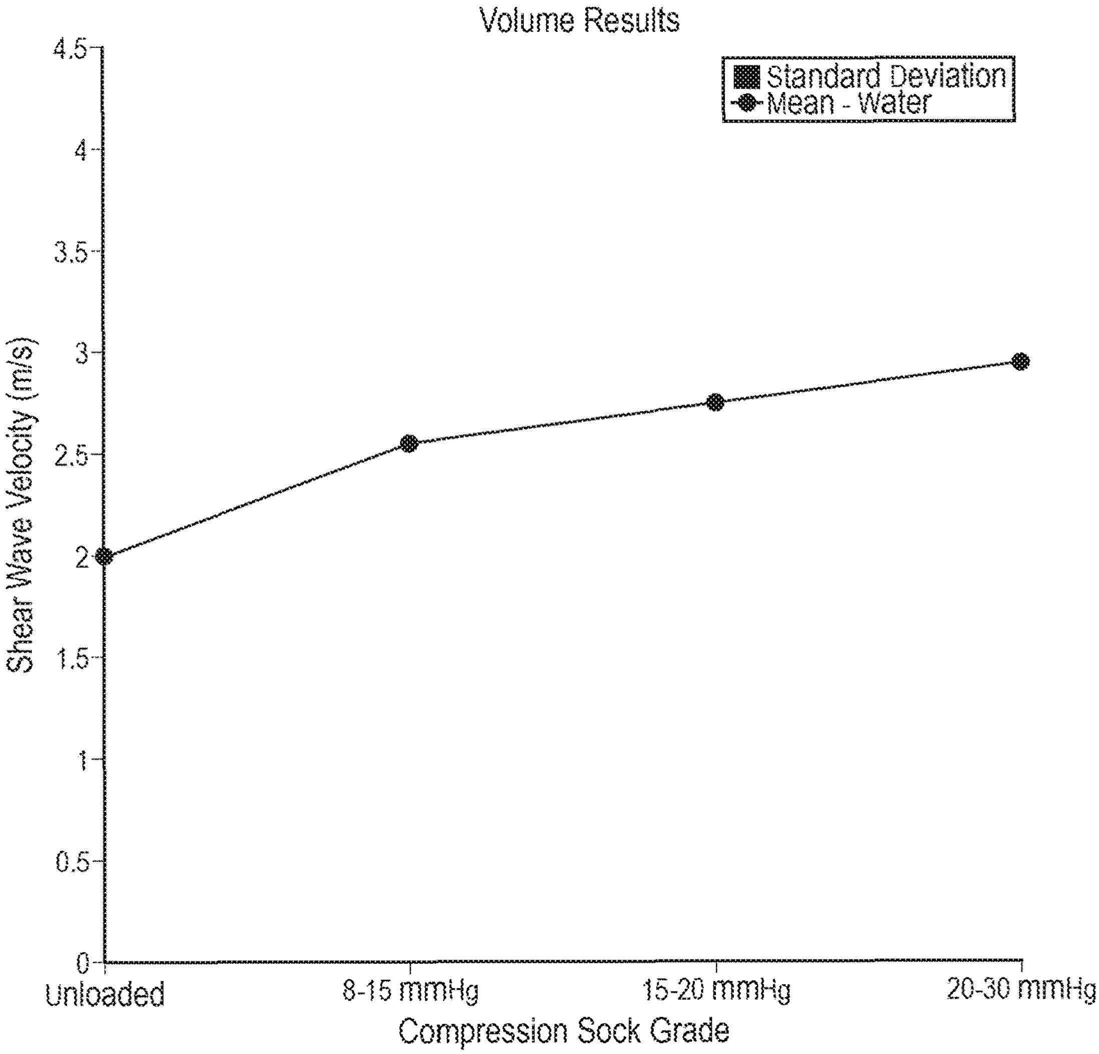
Volume Results
Standard Deviation
Mean - Water
4.5
4
3.5
3
2.5
2
1.5
1
0.5
0
Shear Wave Velocity (m/s)
Unloaded
8-15 mmHg
15-20 mmHg
20-30 mmHg
Compression Sock Grade

FIG. 76

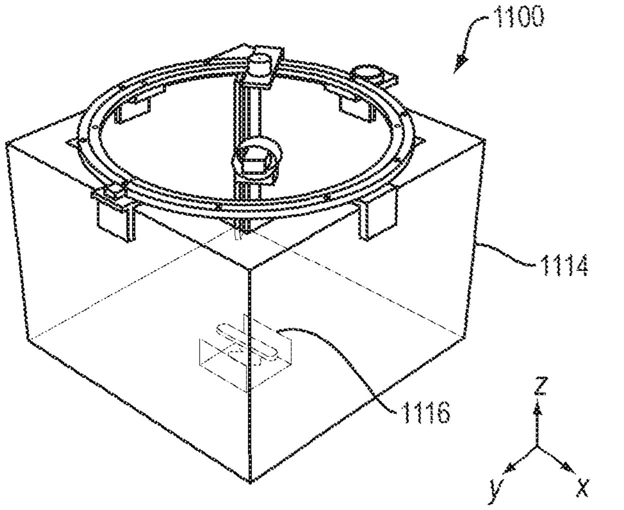


FIG. 77A

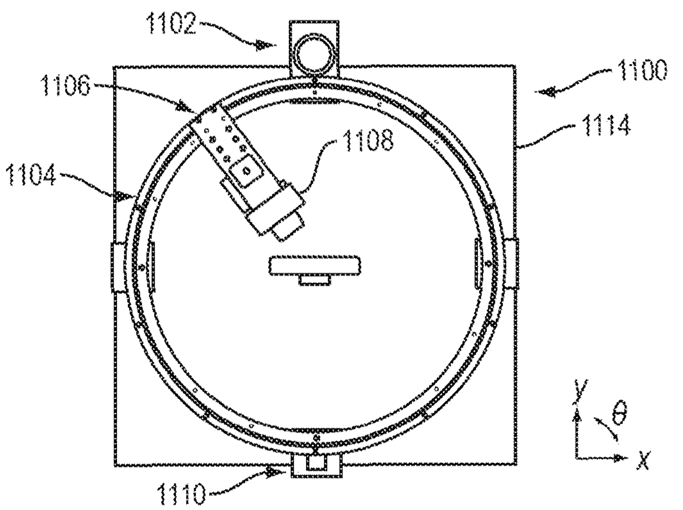


FIG. 77B

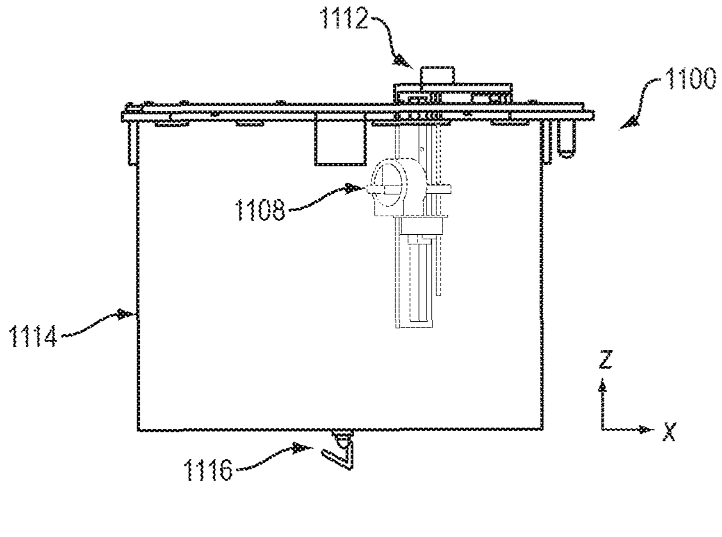


FIG. 77C

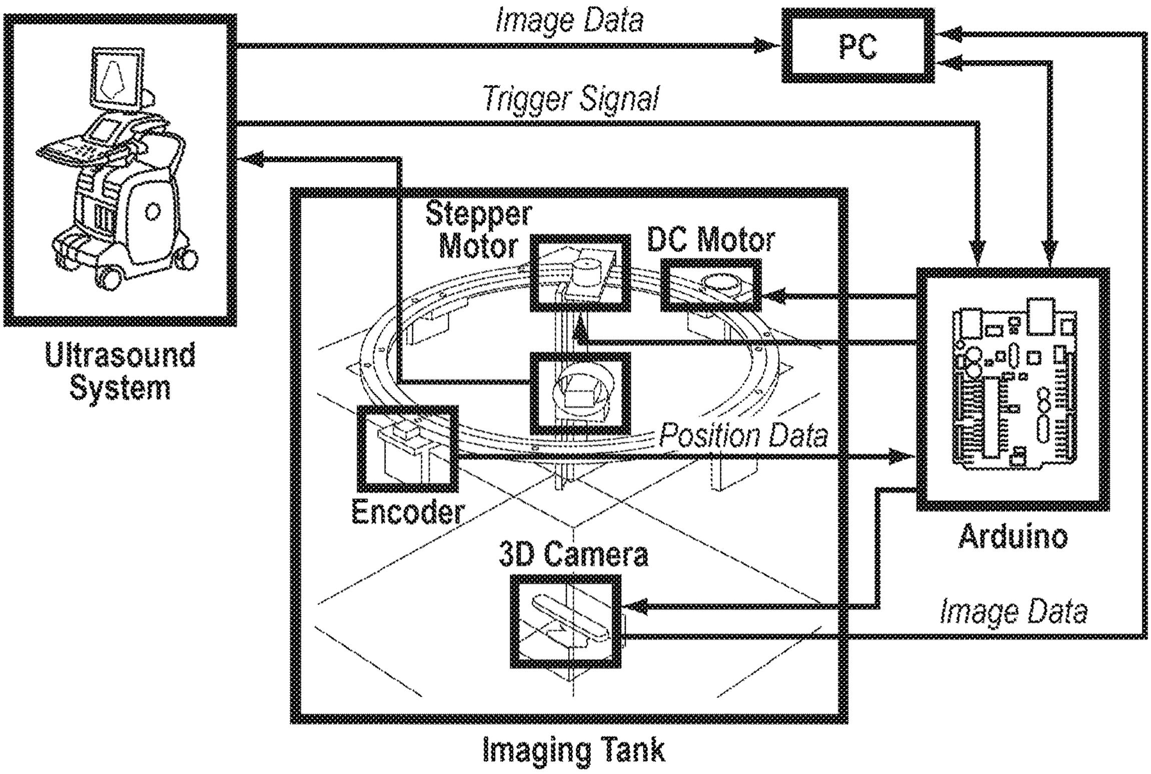
Image Data
PC
Trigger Signal
Stepper Motor
DC Motor
Ultrasound System
Position Data
Encoder
Arduino
3D Camera
Image Data
Imaging Tank

FIG. 78

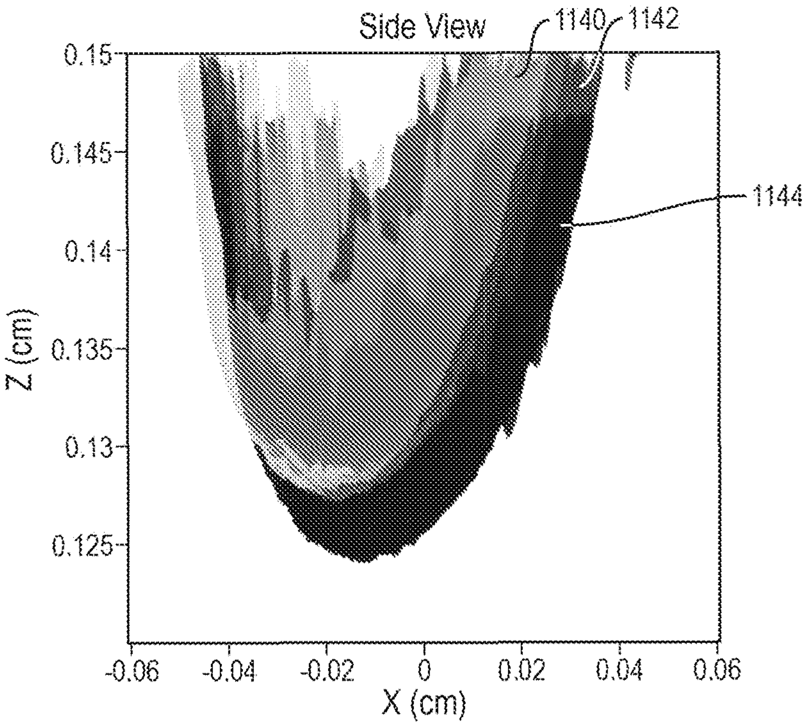


FIG. 79A

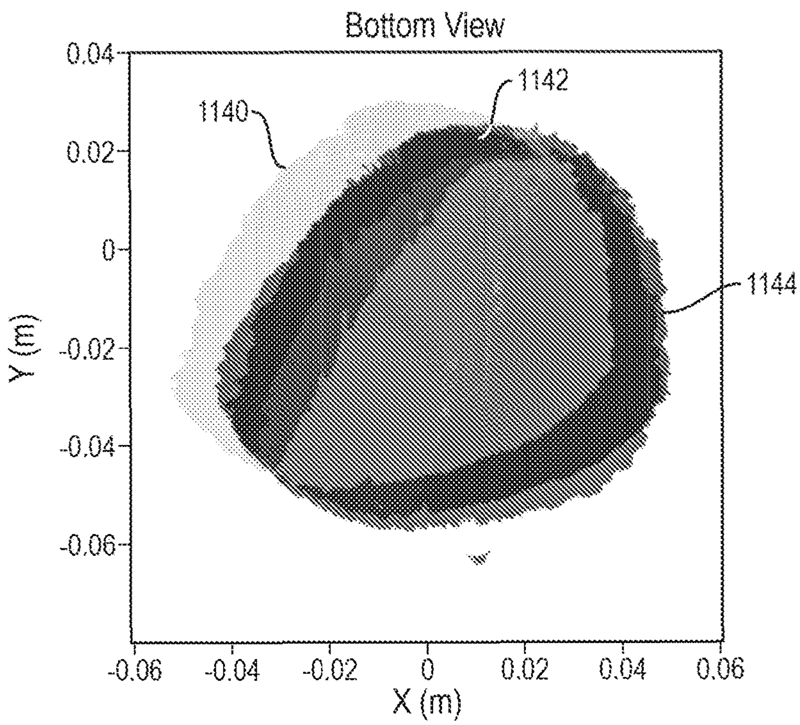


FIG. 79B

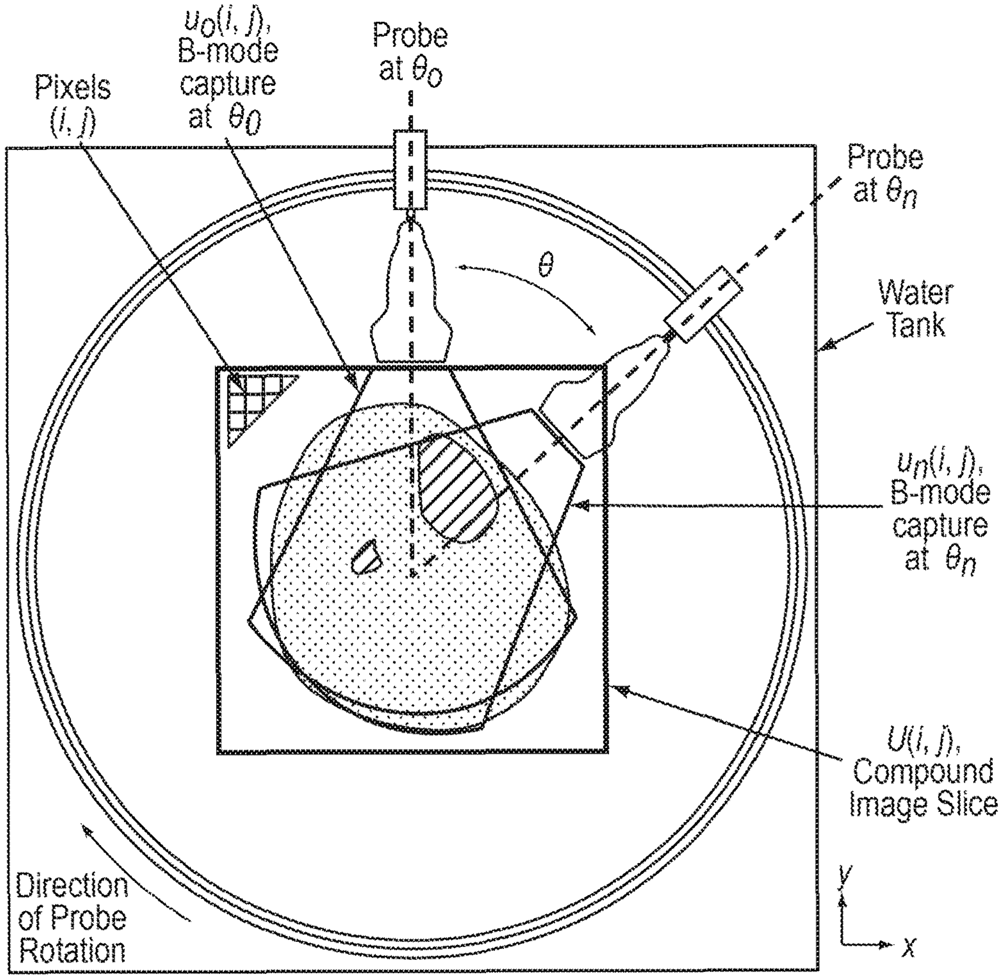

$u_0(i, j)$, B-mode capture at $\theta_0$
Probe at $\theta_0$
Pixels $(i, j)$
Probe at $\theta_n$
$\theta$
Water Tank
$u_n(i, j)$, B-mode capture at $\theta_n$
$U(i, j)$, Compound Image Slice
Direction of Probe Rotation
y
x


FIG. 80

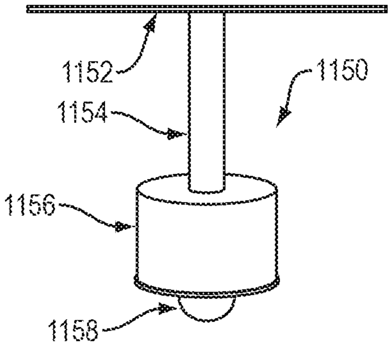


FIG. 81A

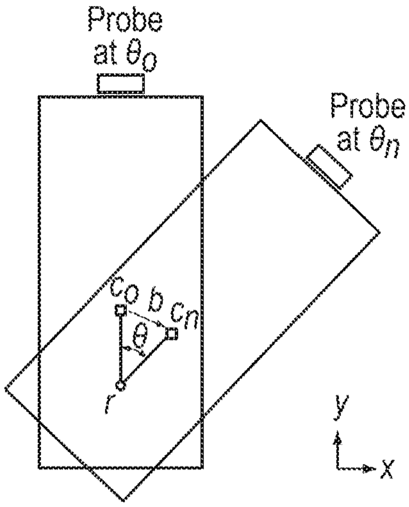


FIG. 81B

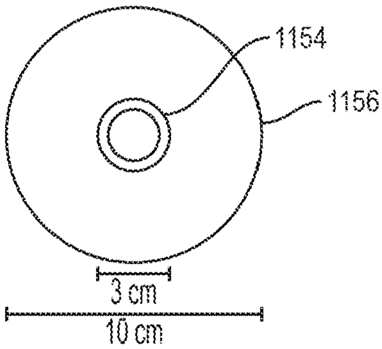


FIG. 81C

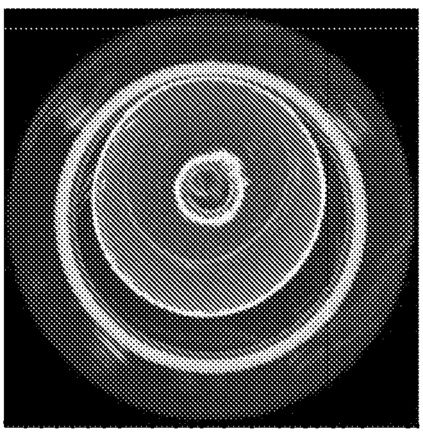

FIG. 81D

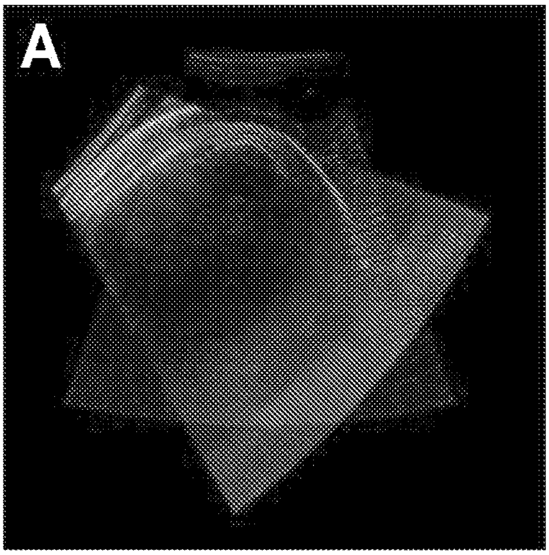


FIG. 82A

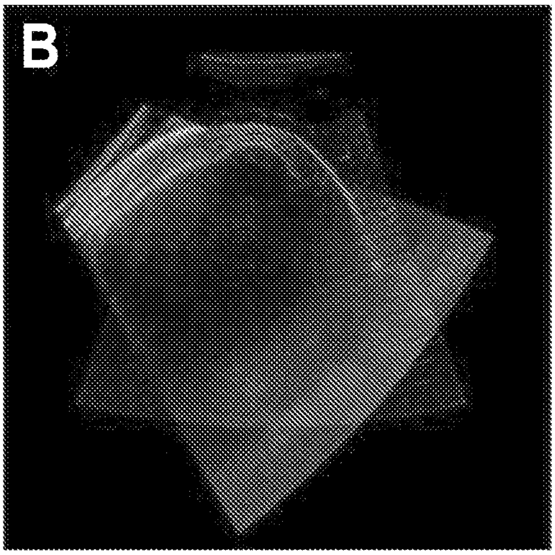


FIG. 82B

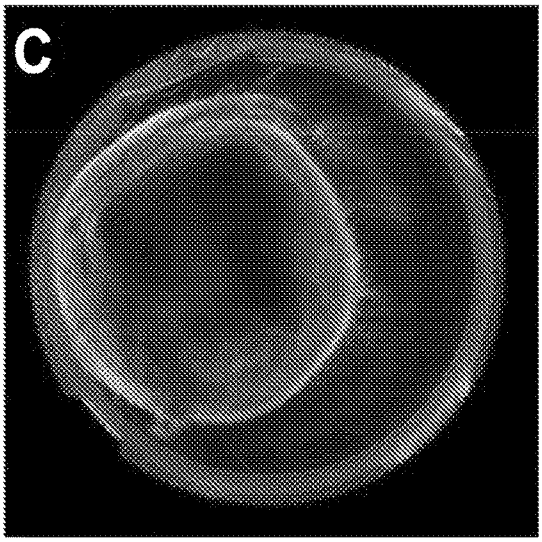


FIG. 82C

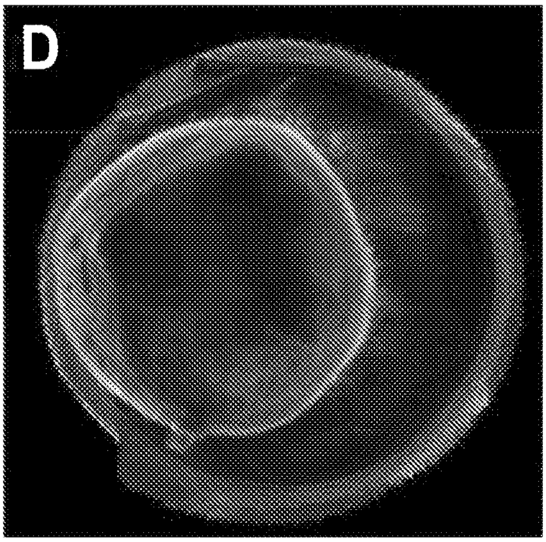


FIG. 82D

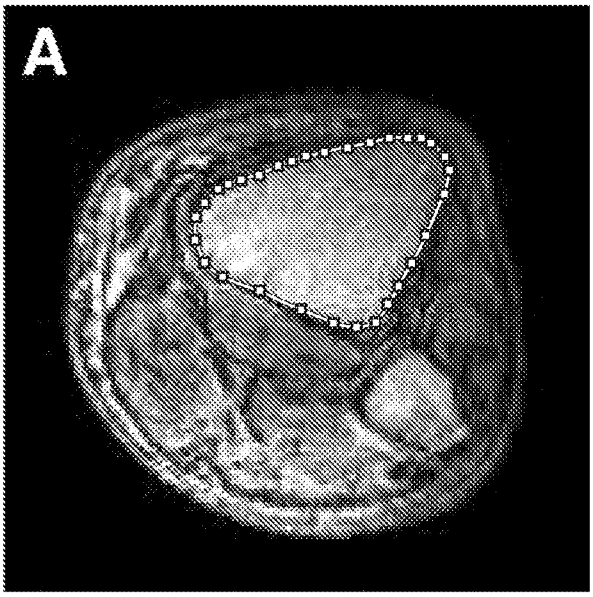


FIG. 83A

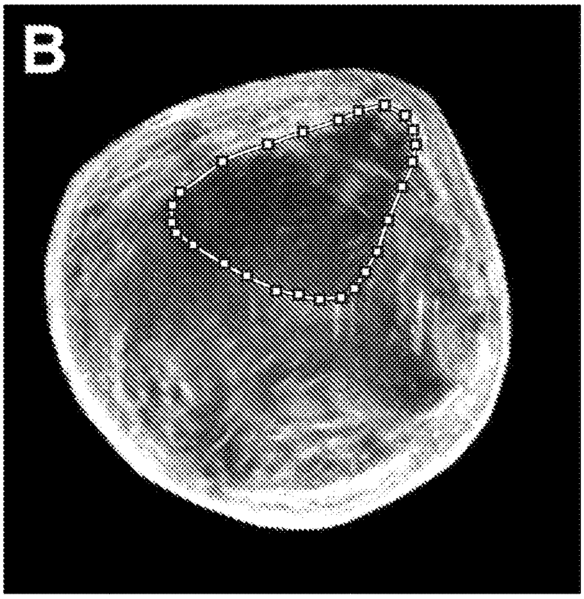


FIG. 83B

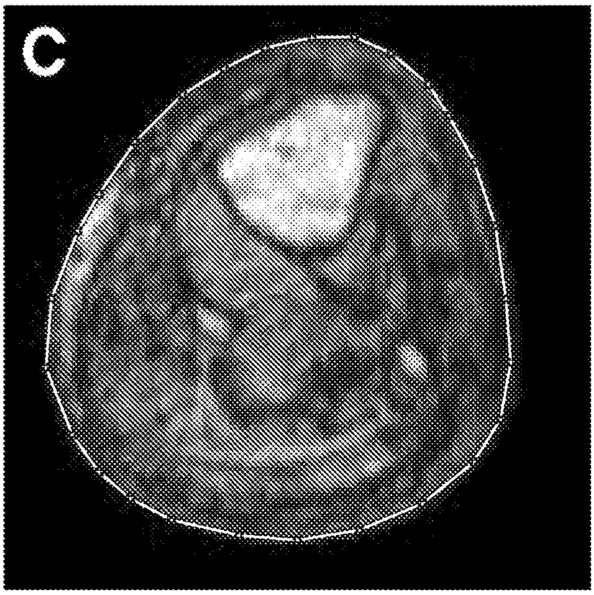


FIG. 83C

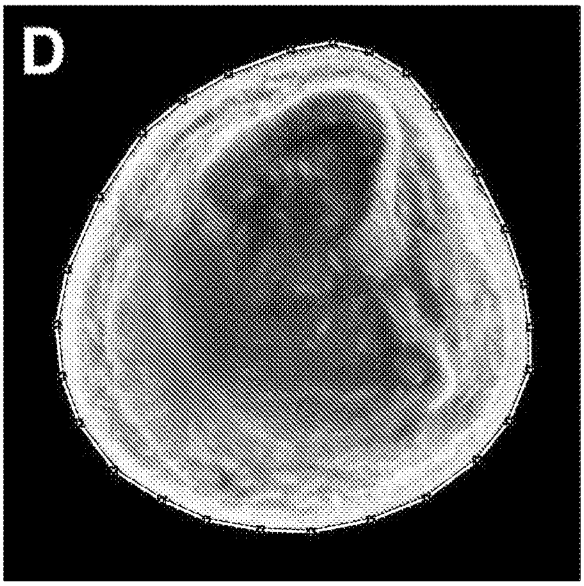


FIG. 83D

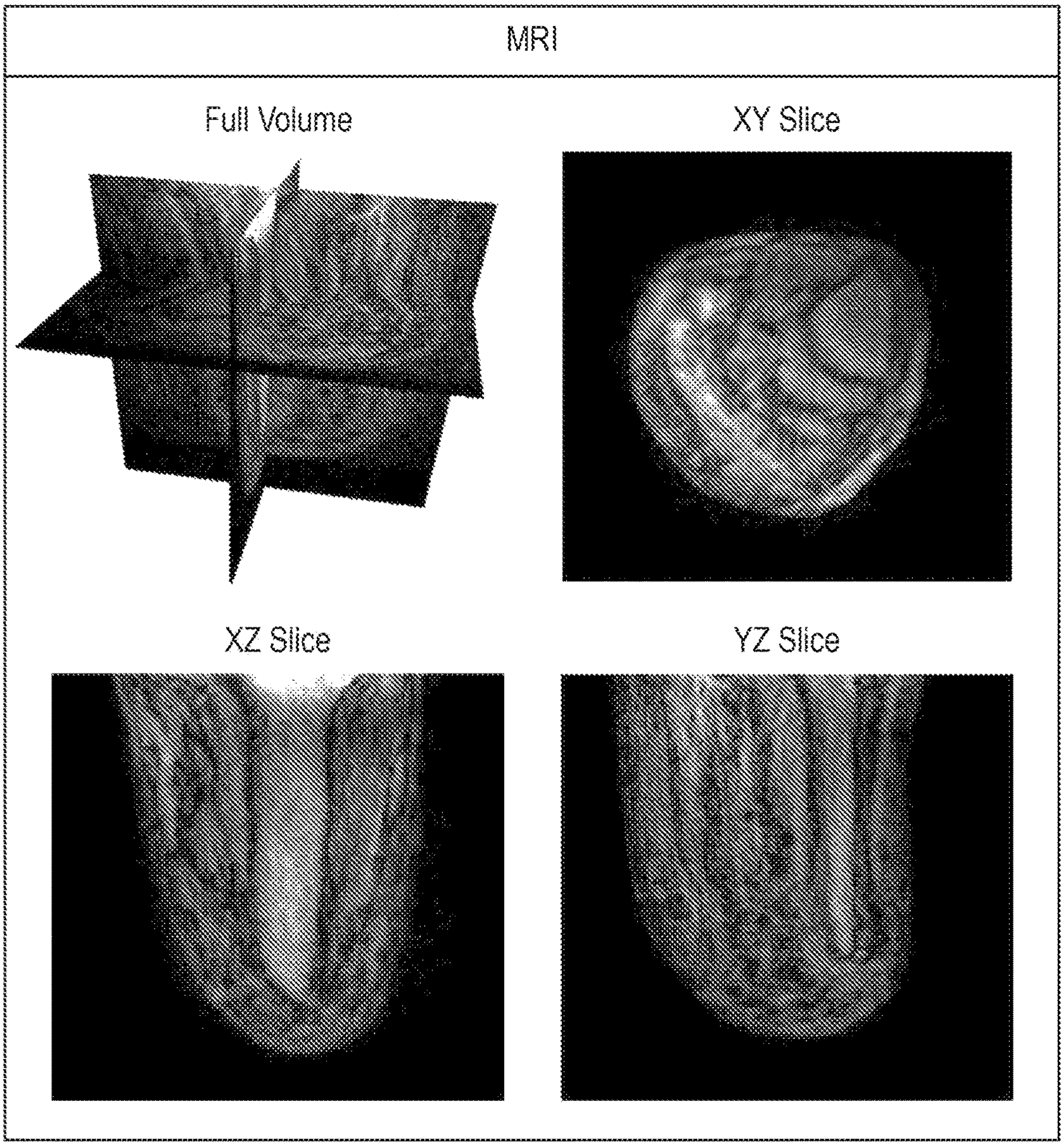


FIG. 84

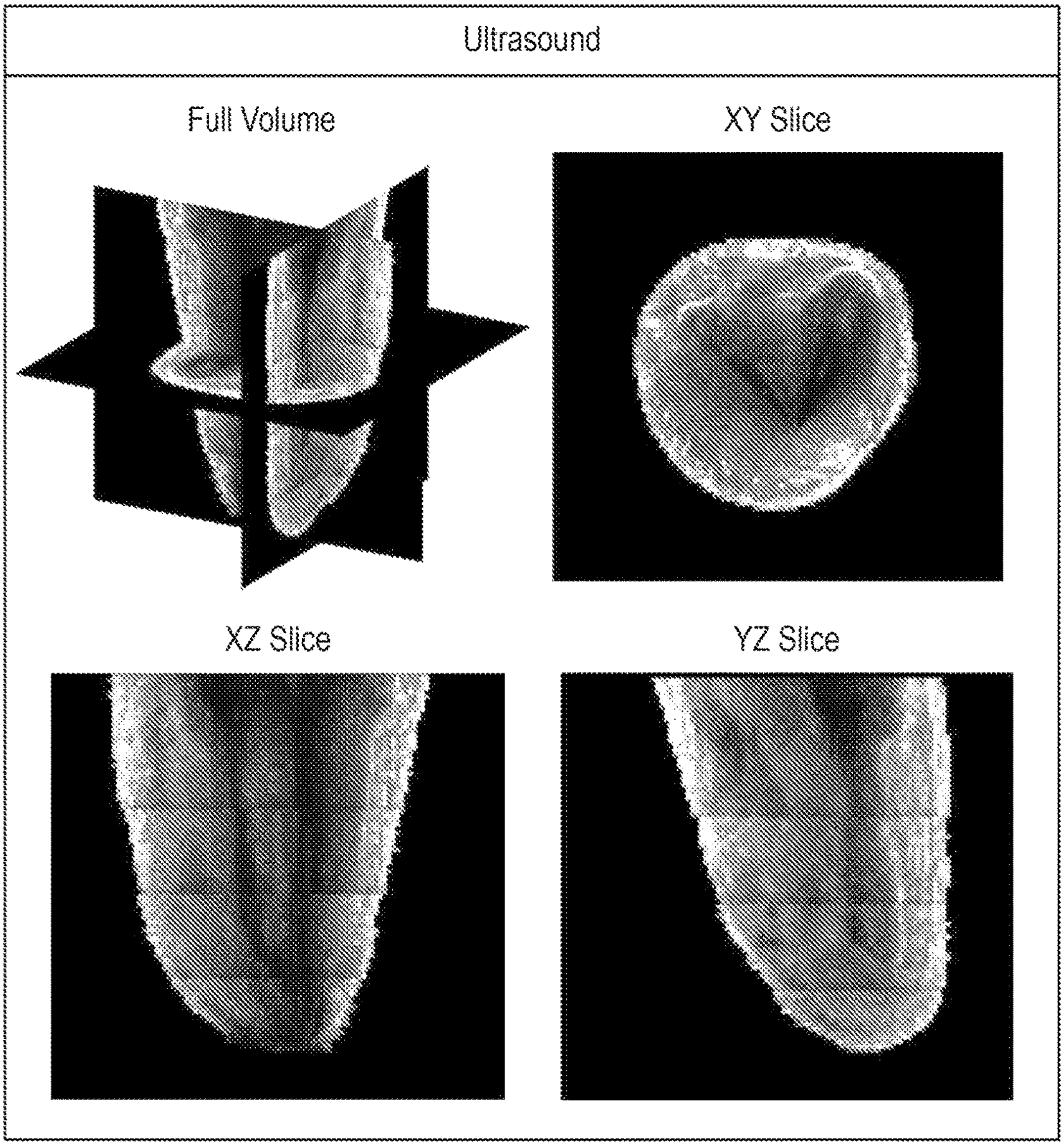


FIG. 85

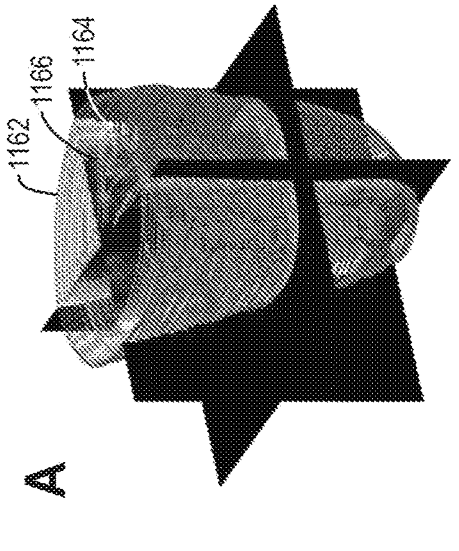


FIG. 86A

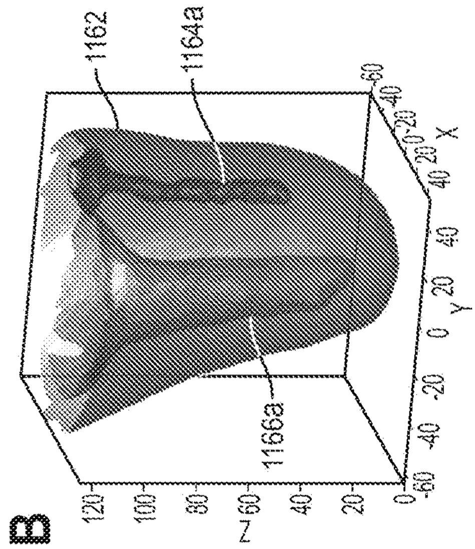


FIG. 86B

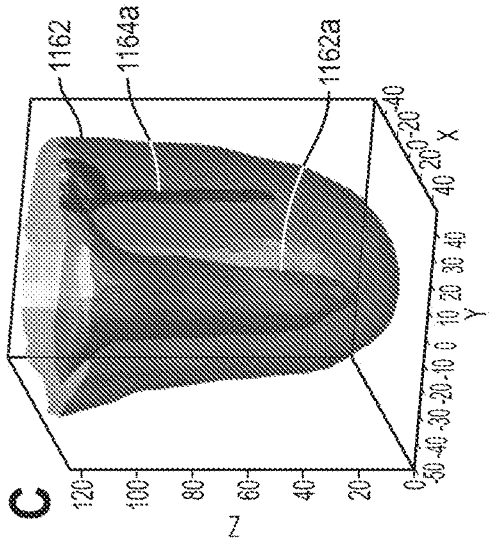


FIG. 86C

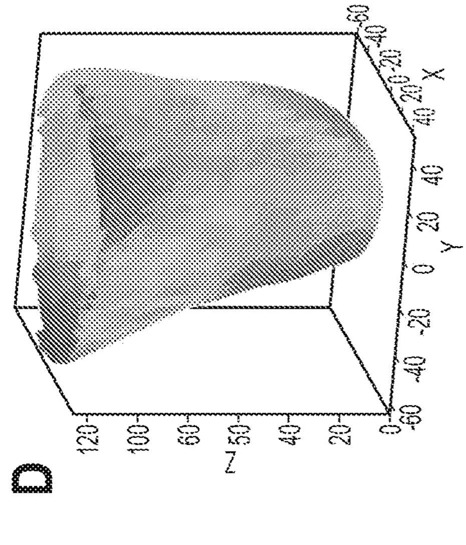


FIG. 86D

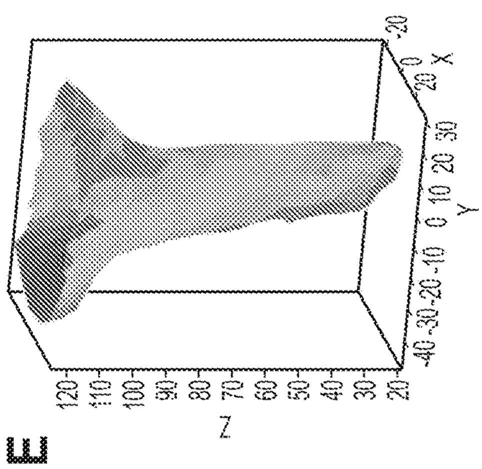


FIG. 86E

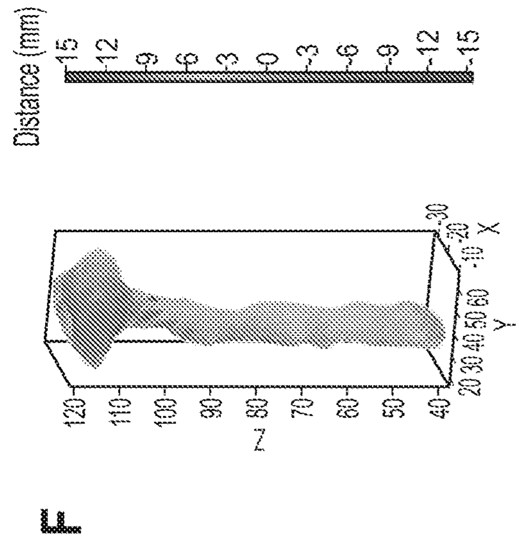


FIG. 86F

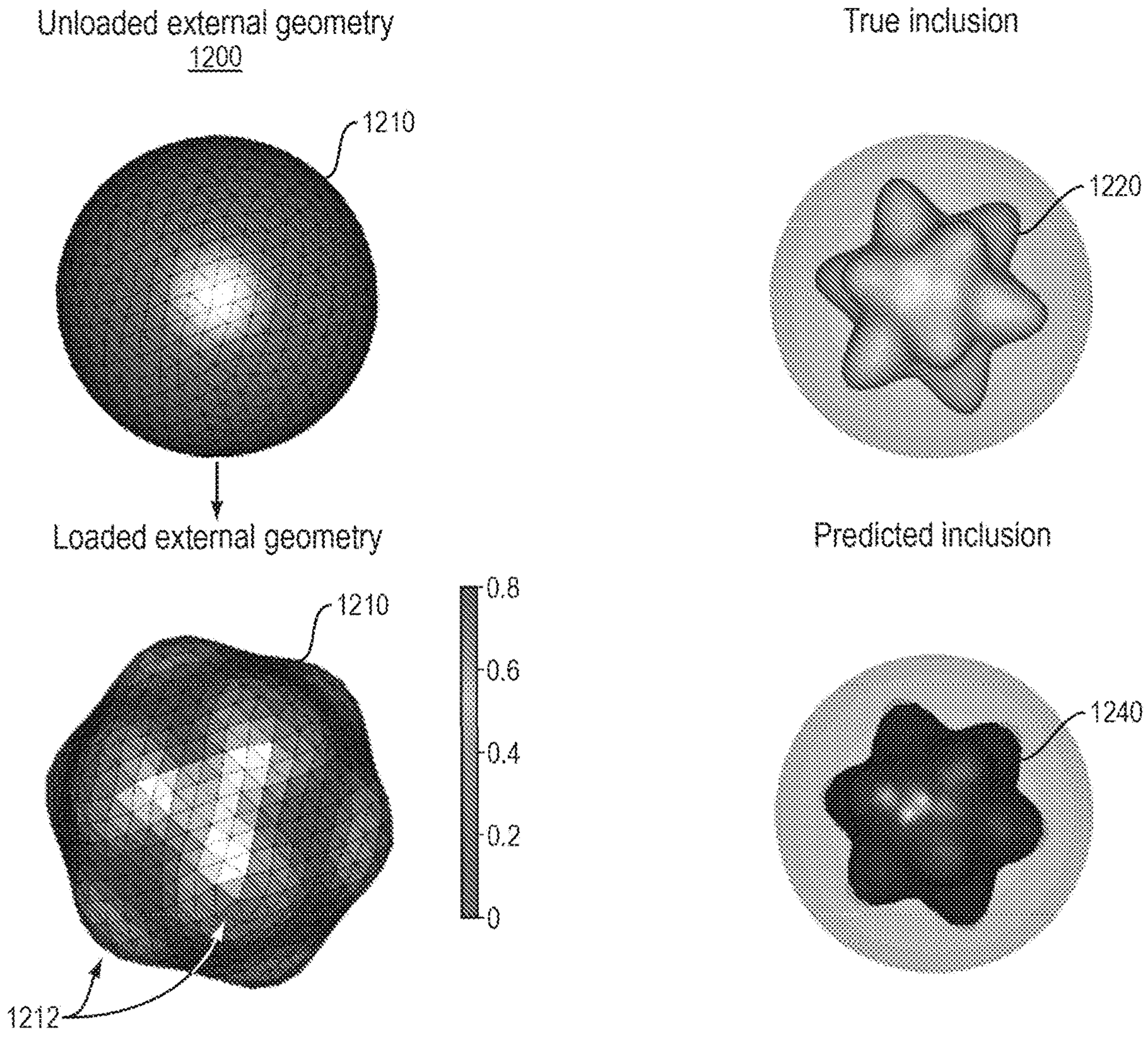

Unloaded external geometry
1200
1210
True inclusion
1220
Loaded external geometry
Predicted inclusion
1210
0.8
0.6
0.4
0.2
0
1240
1212


FIG. 87

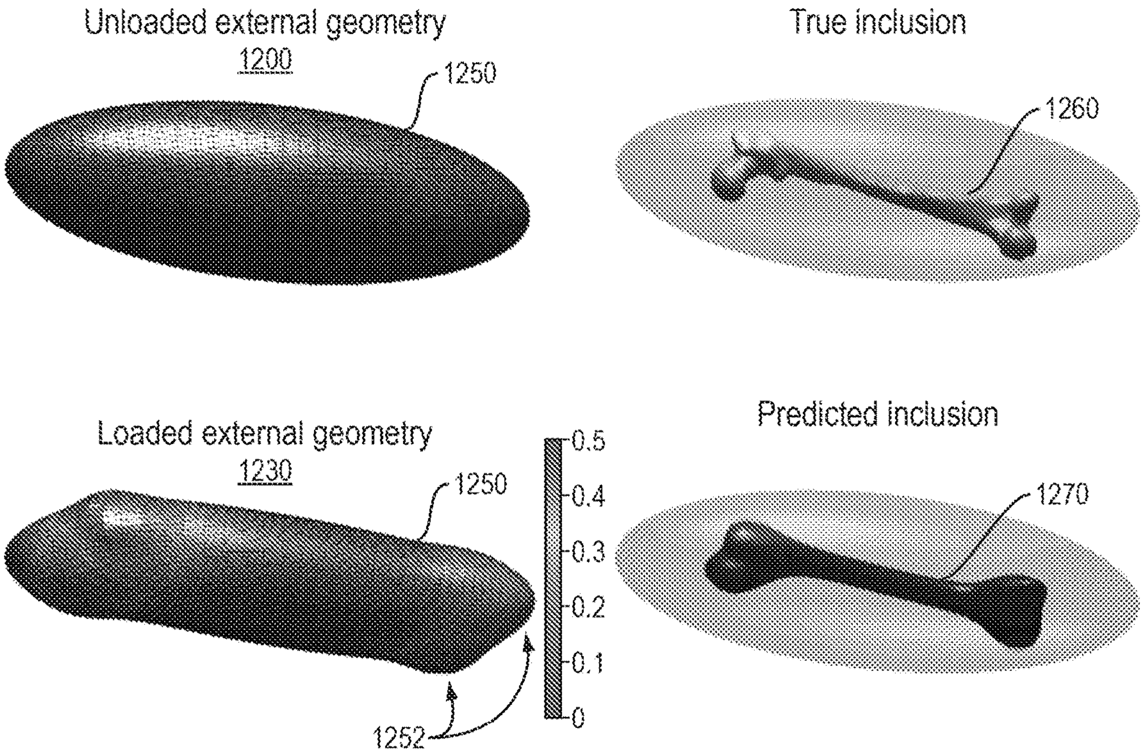
Unloaded external geometry
1200
1250
True inclusion
1260
Loaded external geometry
1230
1250
0.5
0.4
0.3
0.2
0.1
0
1252
Predicted inclusion
1270

FIG. 88

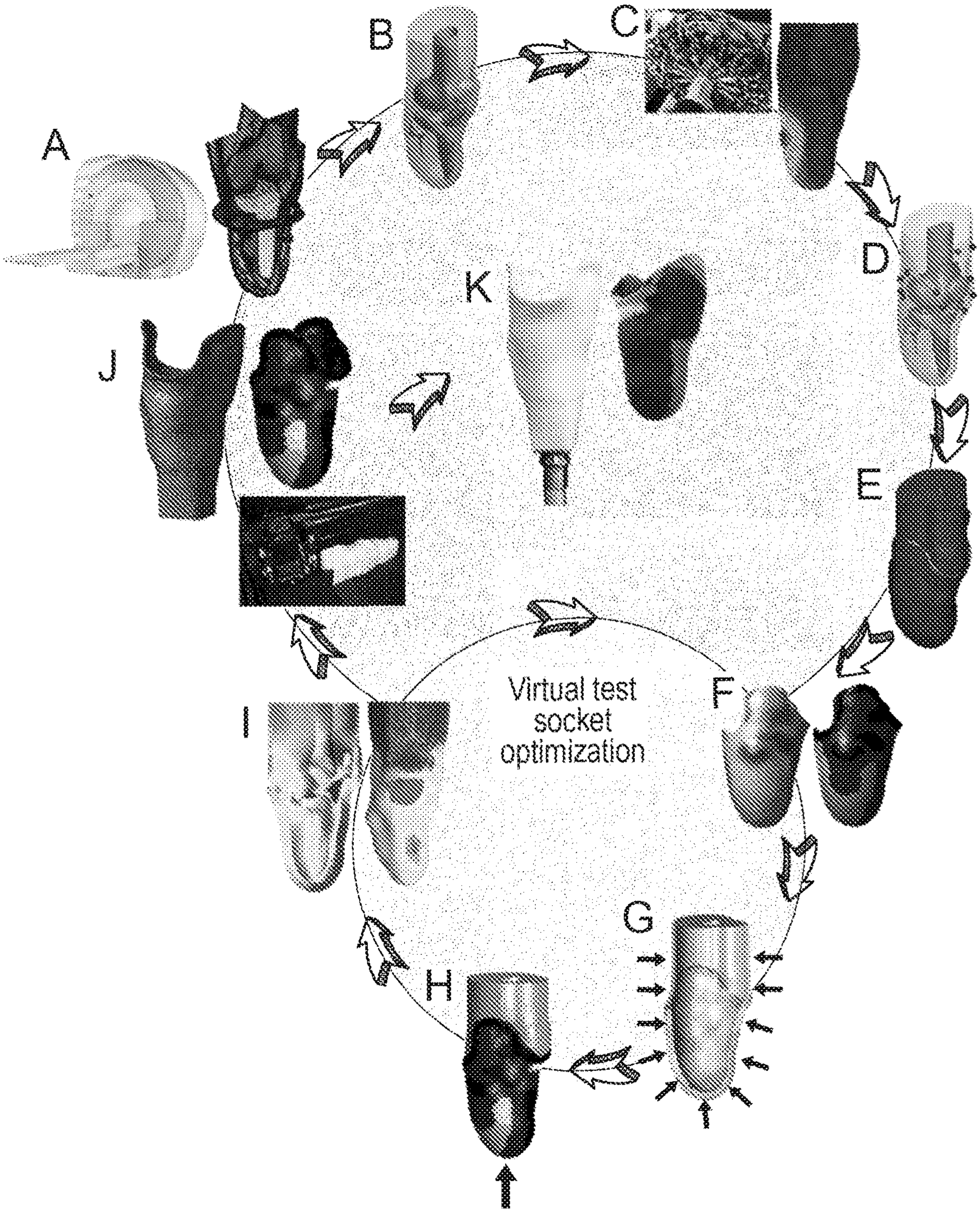


FIG. 89

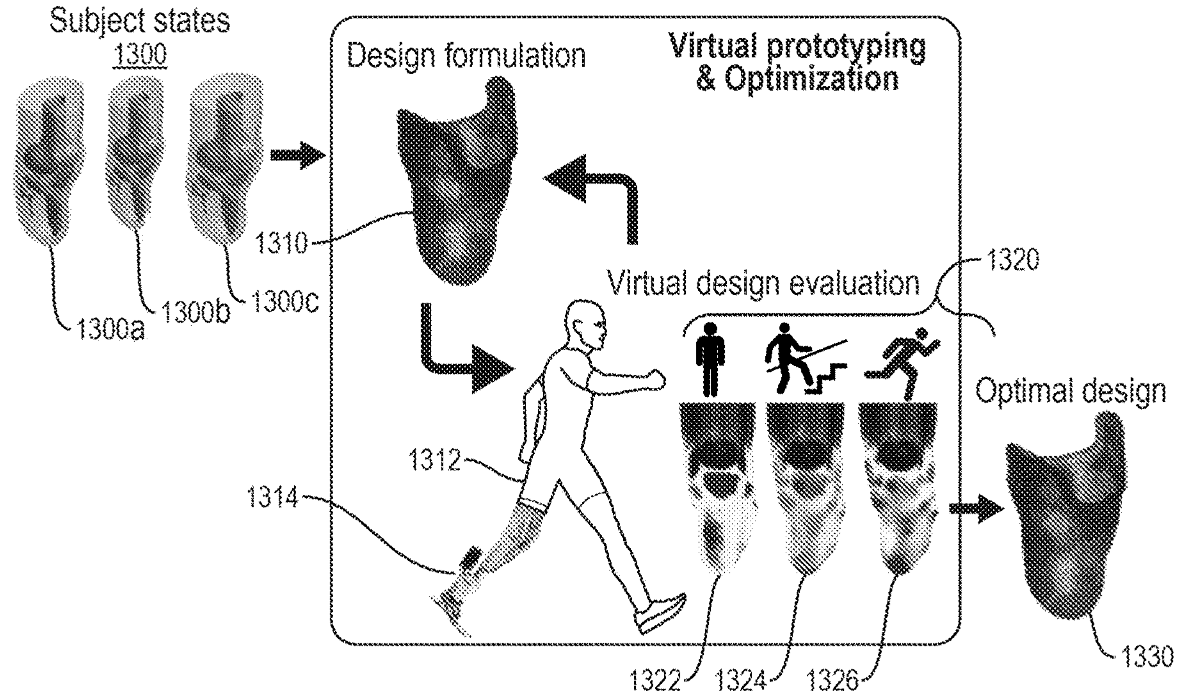
Subject states
1300
1300a
1300b
1300c
Design formulation
Virtual prototyping & Optimization
1310
Virtual design evaluation
1320
1312
1314
Optimal design
1322
1324
1326
1330

FIG. 90

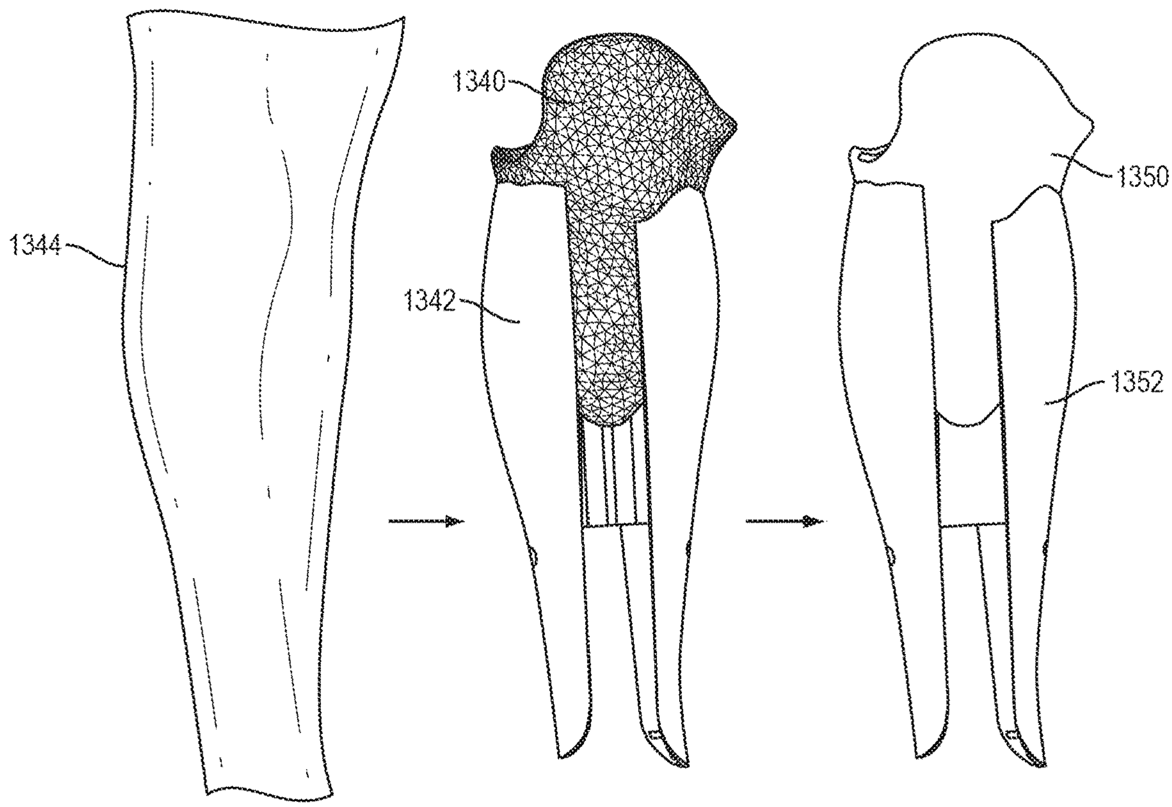


FIG. 91

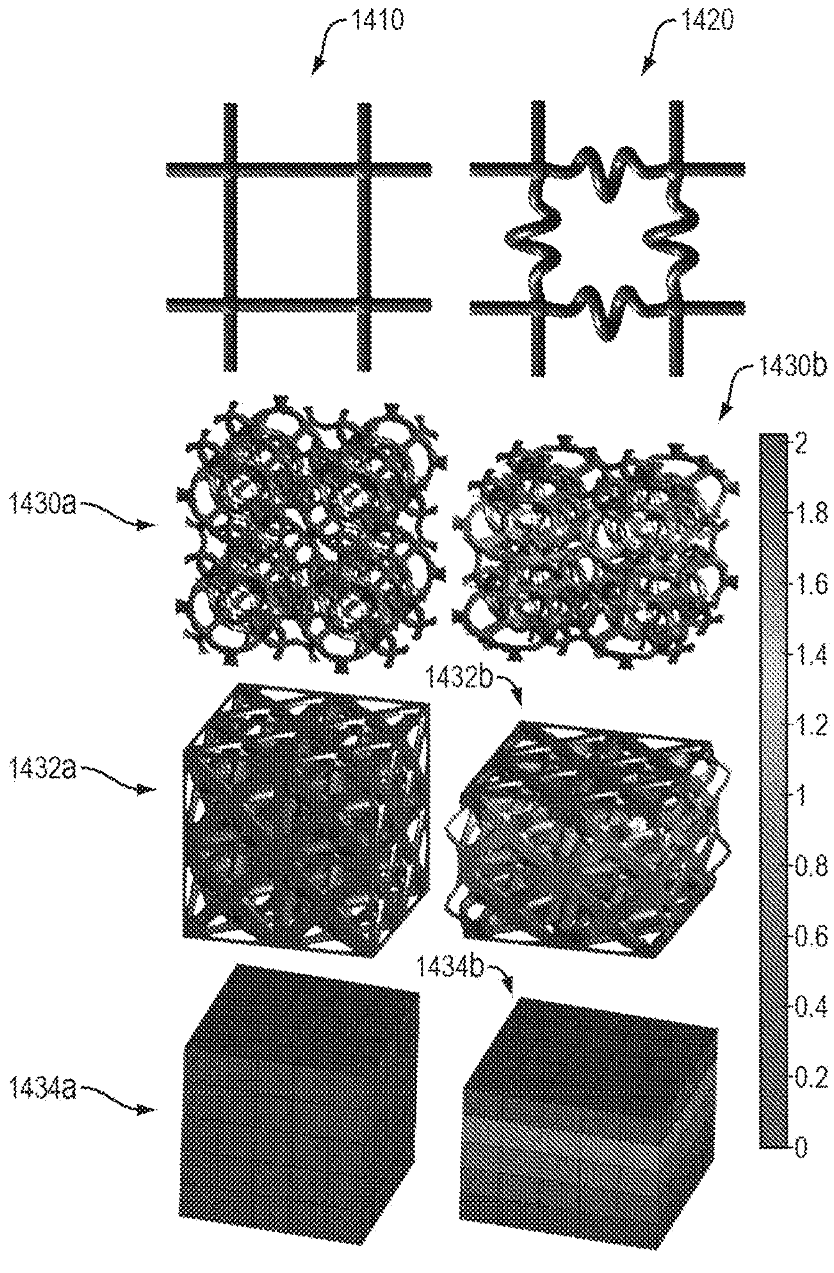
1410
1420
1430b
1430a
1432b
1432a
1434b
1434a
2
1.8
1.6
1.4
1.2
1
0.8
0.6
0.4
0.2
0

FIG. 92A

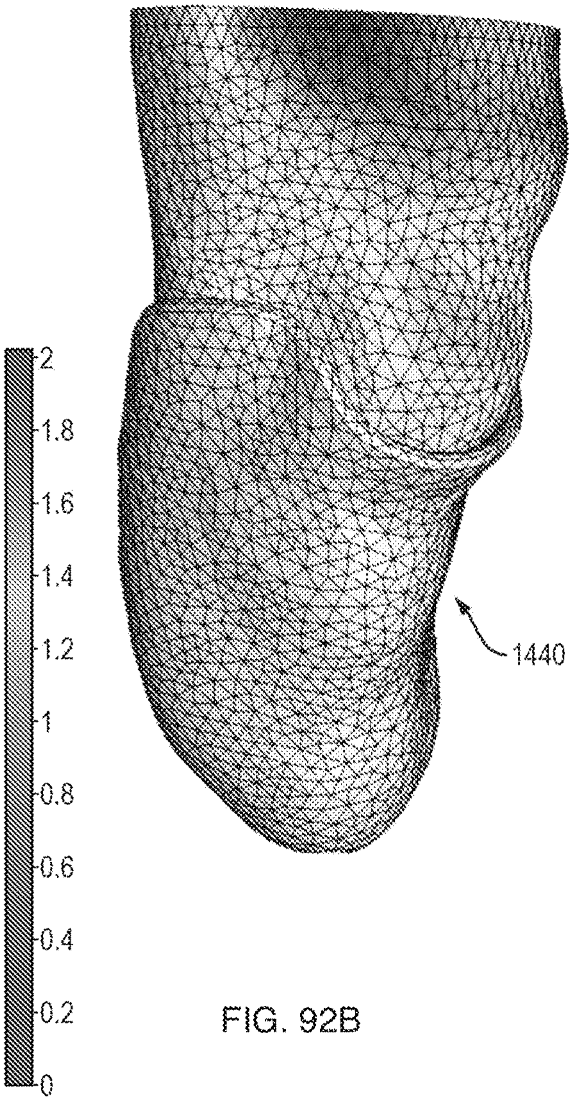


FIG. 92B

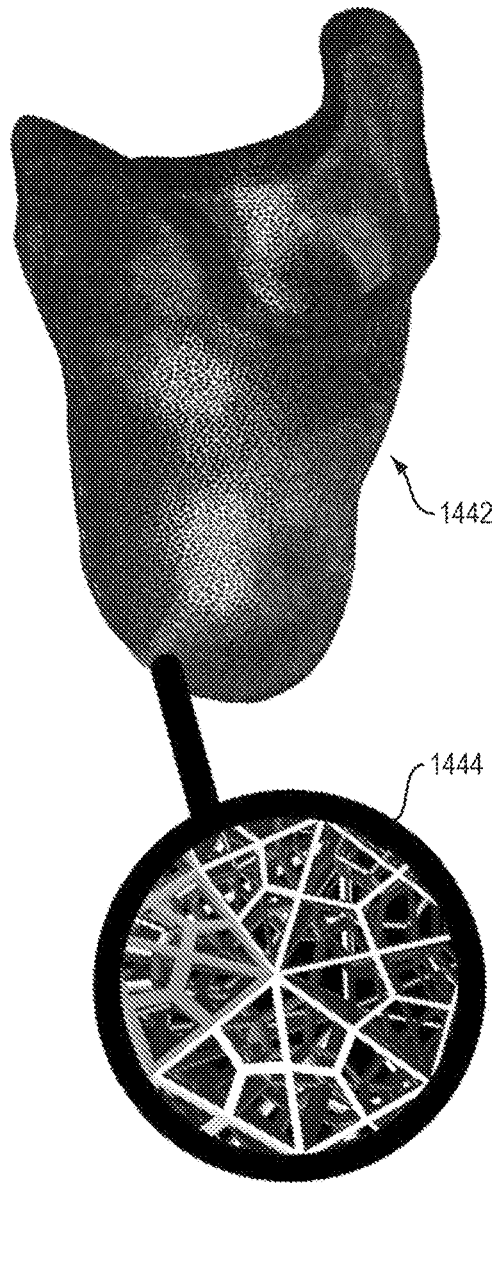


FIG. 92C

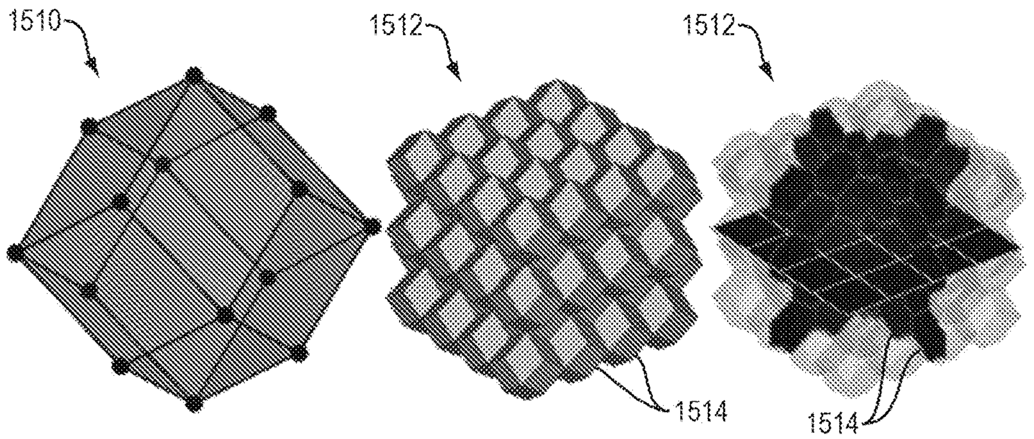


FIG. 93

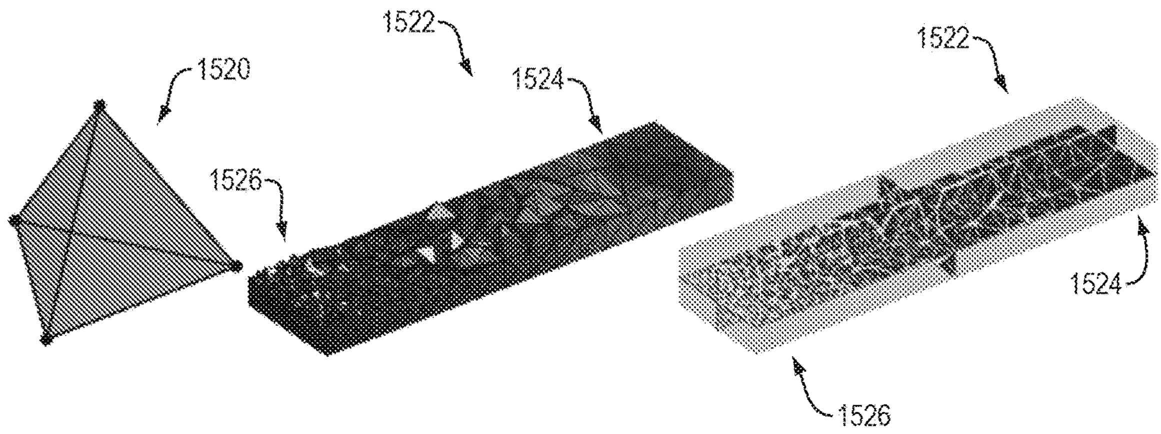


FIG. 94

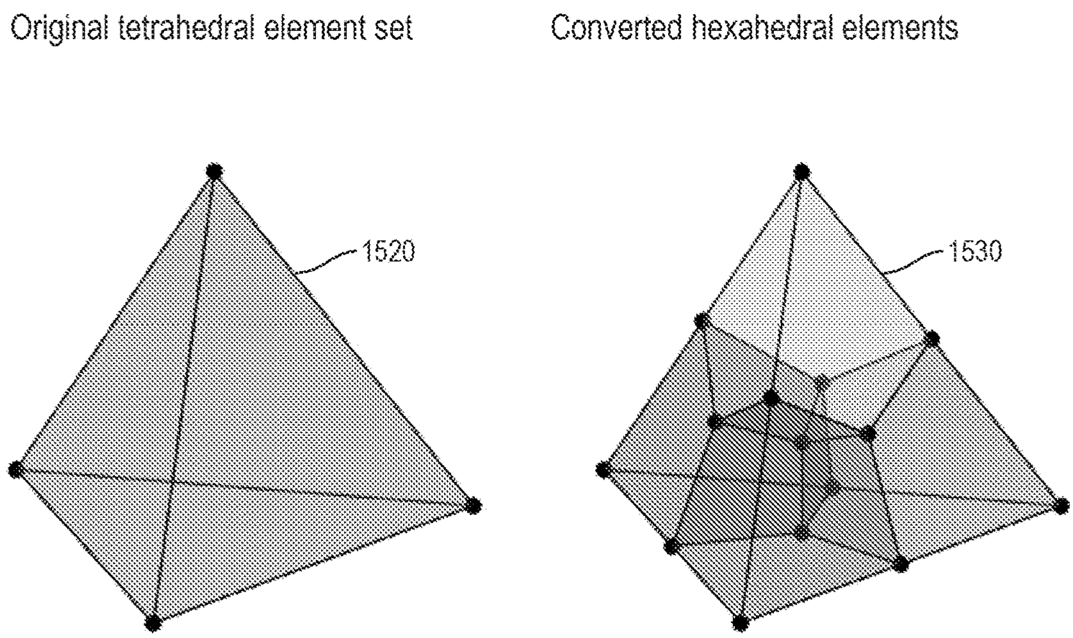
Original tetrahedral element set
Converted hexahedral elements
1520
1530

FIG. 95

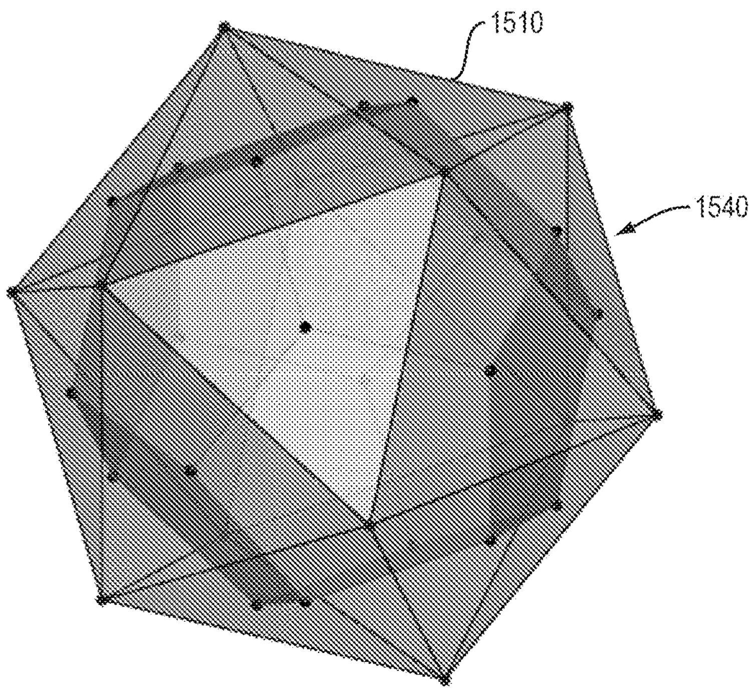
1510
1540

FIG. 96

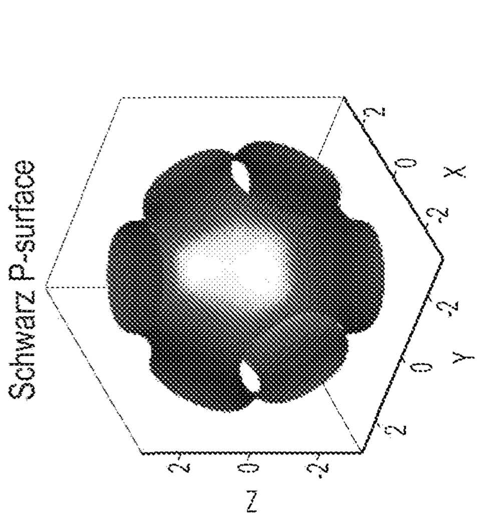

FIG. 97A

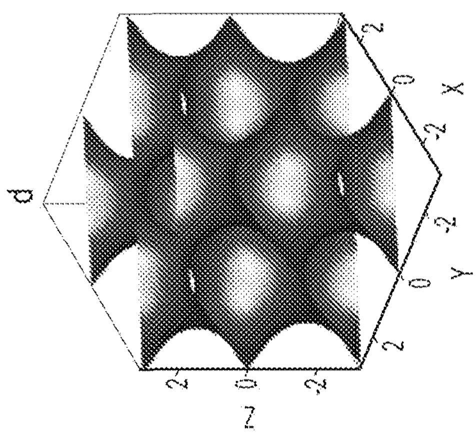

FIG. 97B

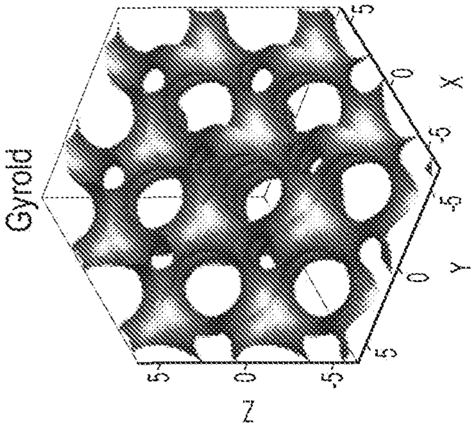

FIG. 97C

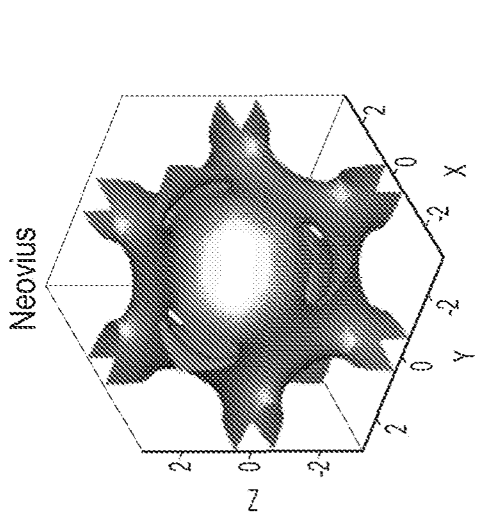

FIG. 97D

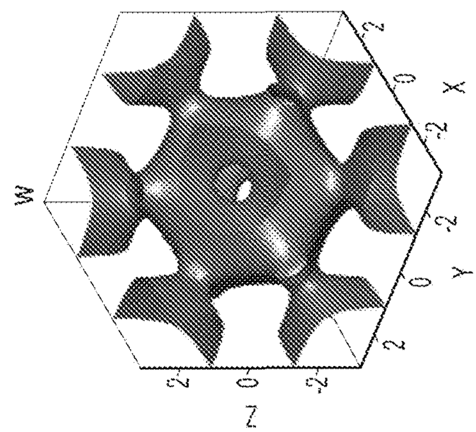

FIG. 97E

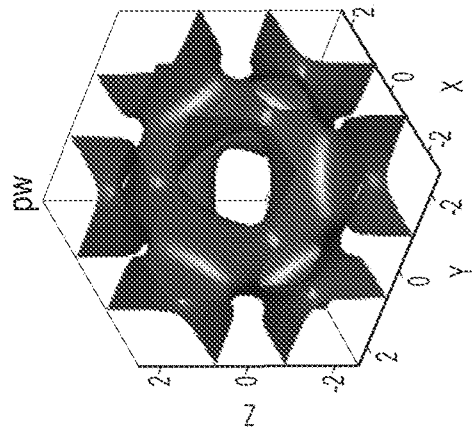

FIG. 97F

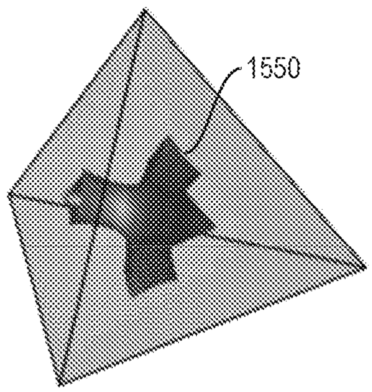


FIG. 98A

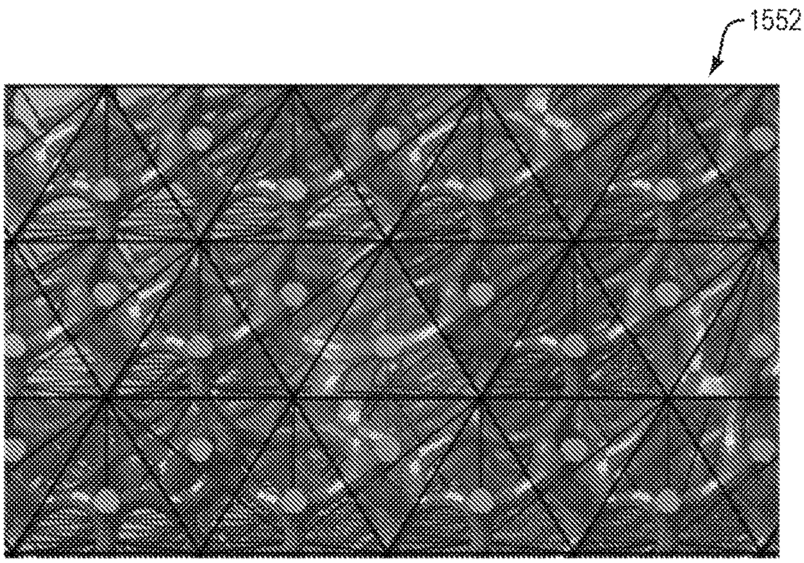


FIG. 98B

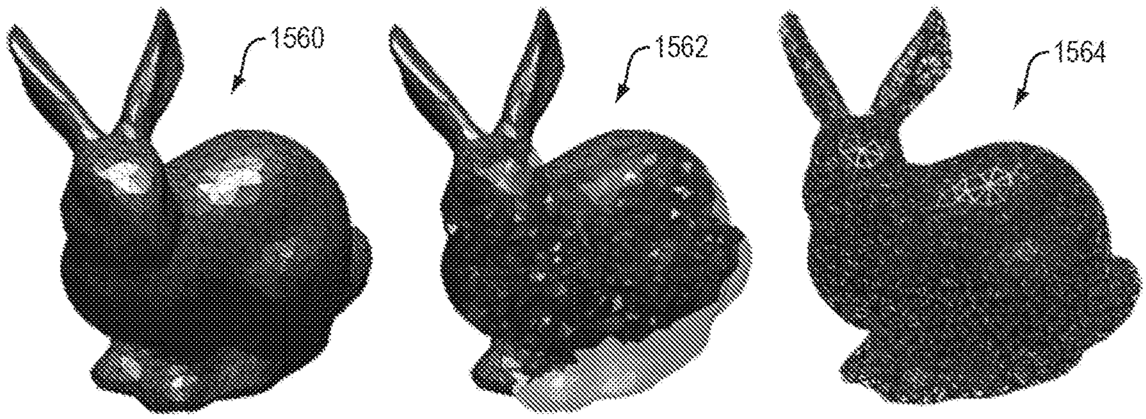


FIG. 99

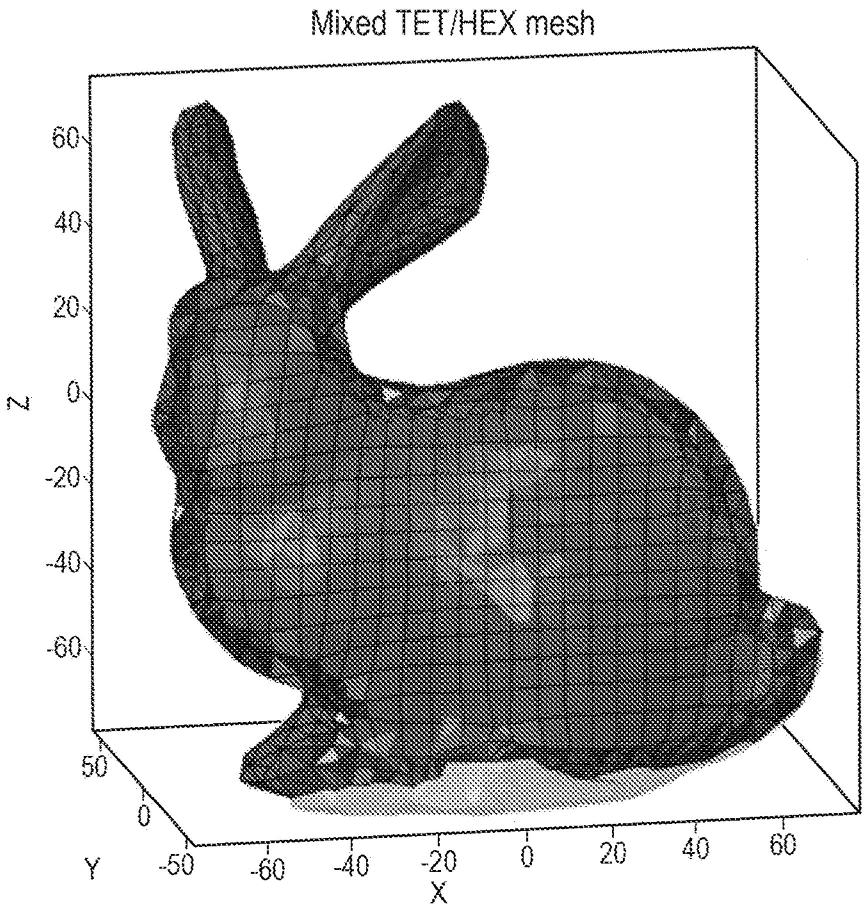


FIG. 100

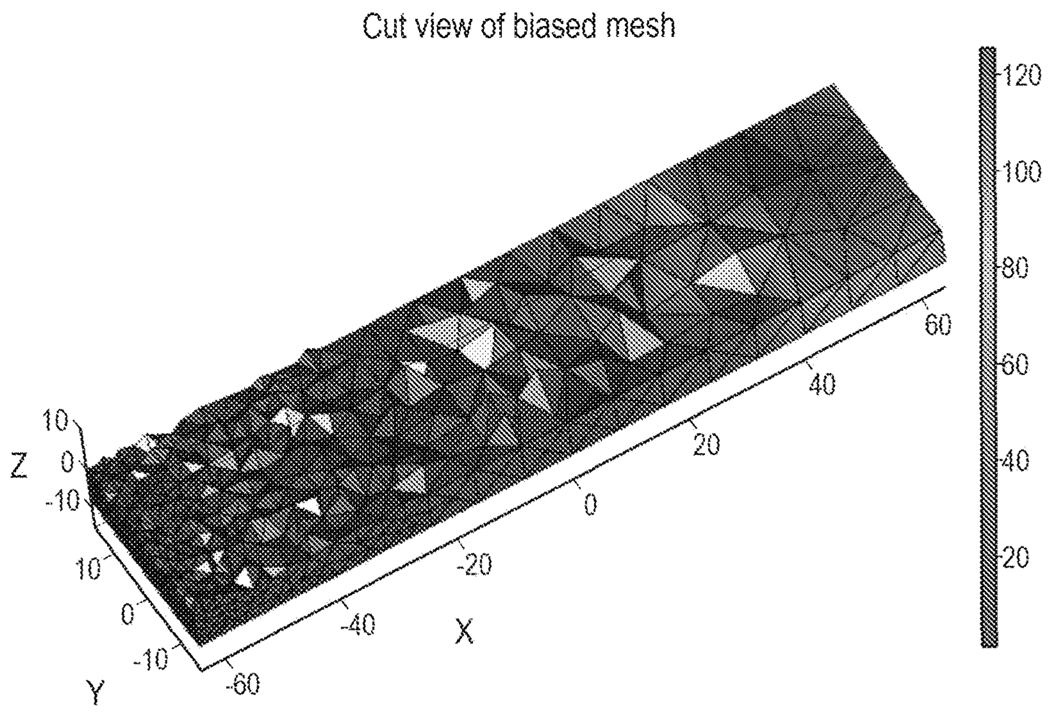


FIG. 101

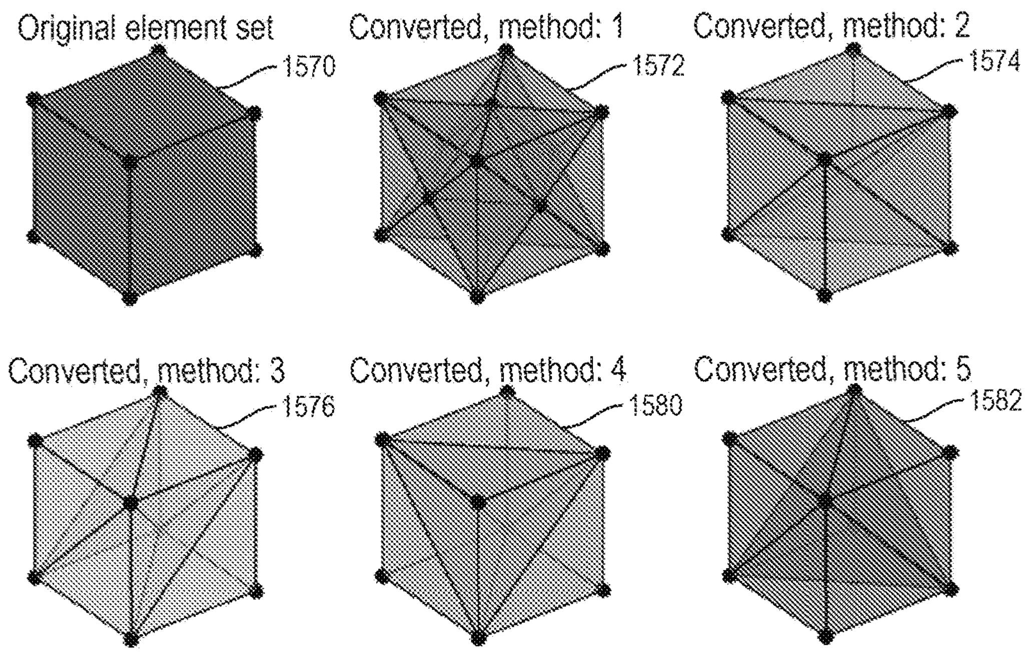


FIG. 102

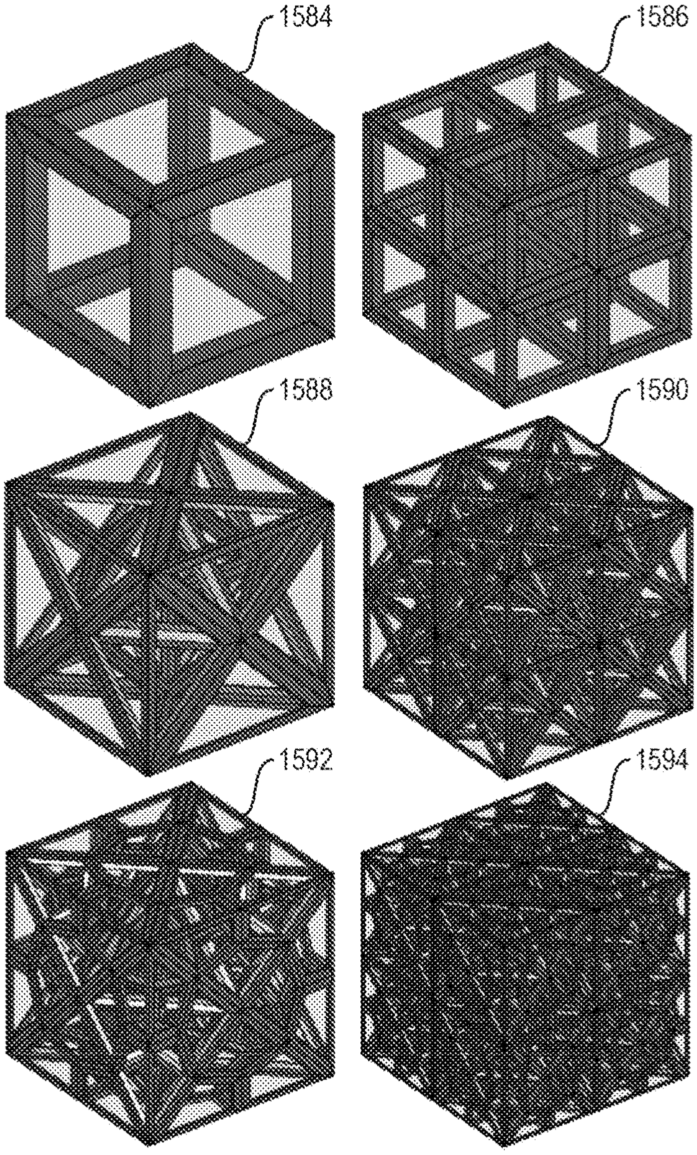


FIG. 103

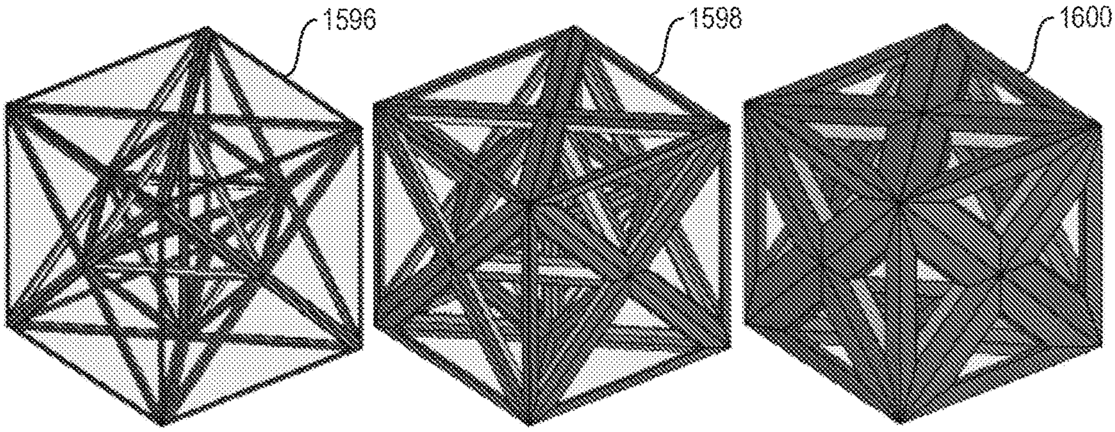


FIG. 104

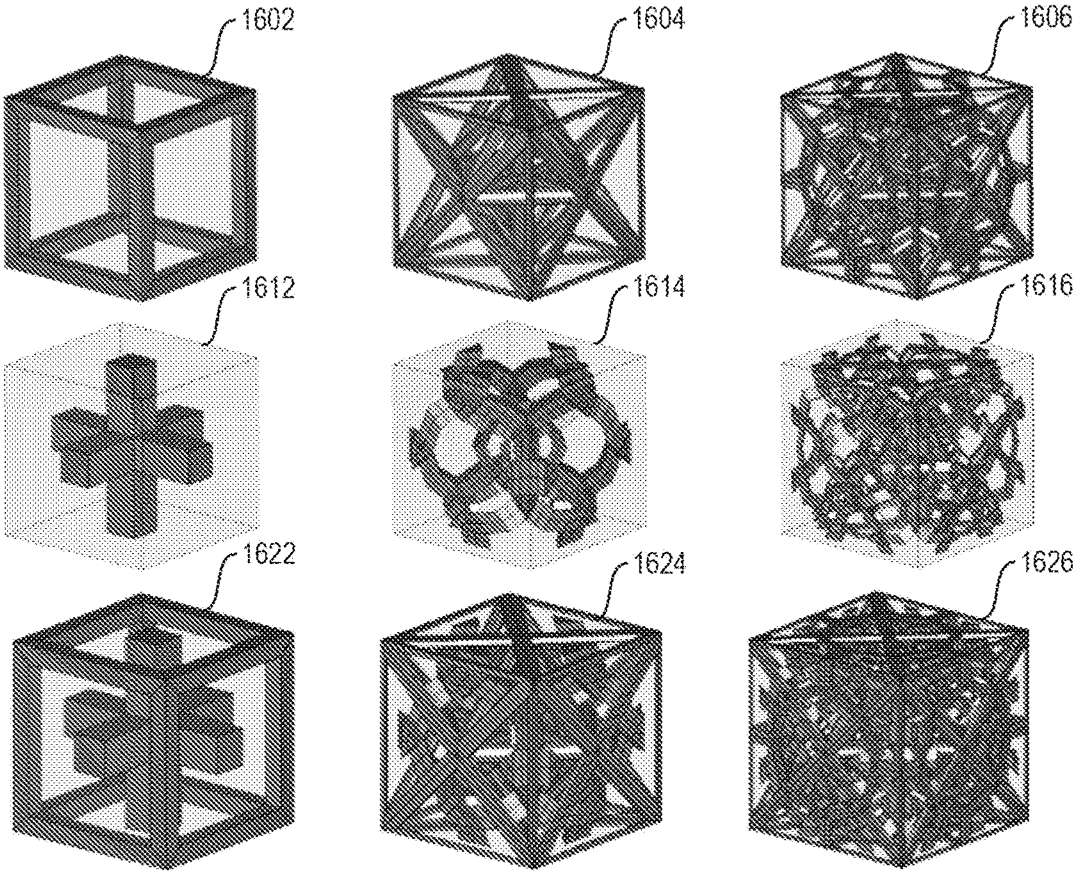


FIG. 105

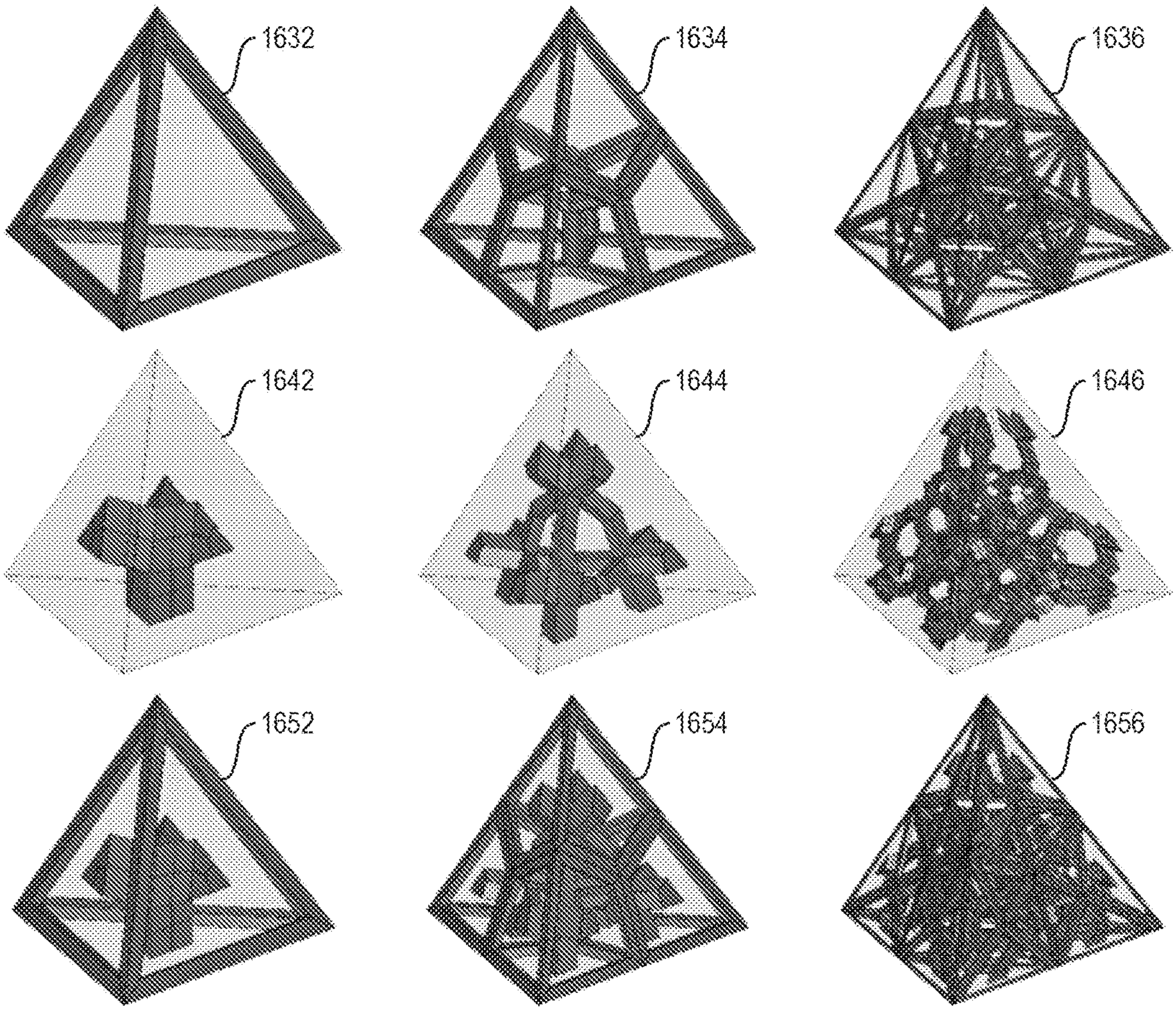


FIG. 106

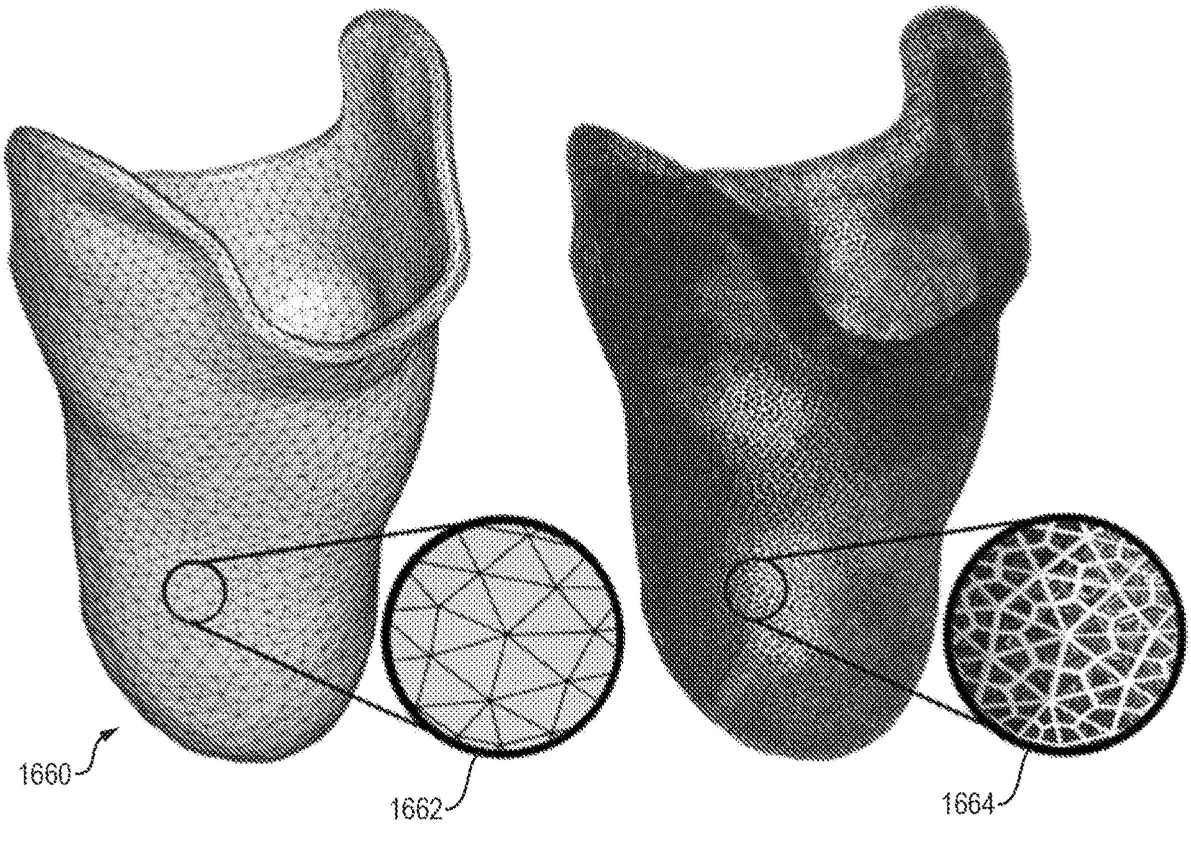


FIG. 107

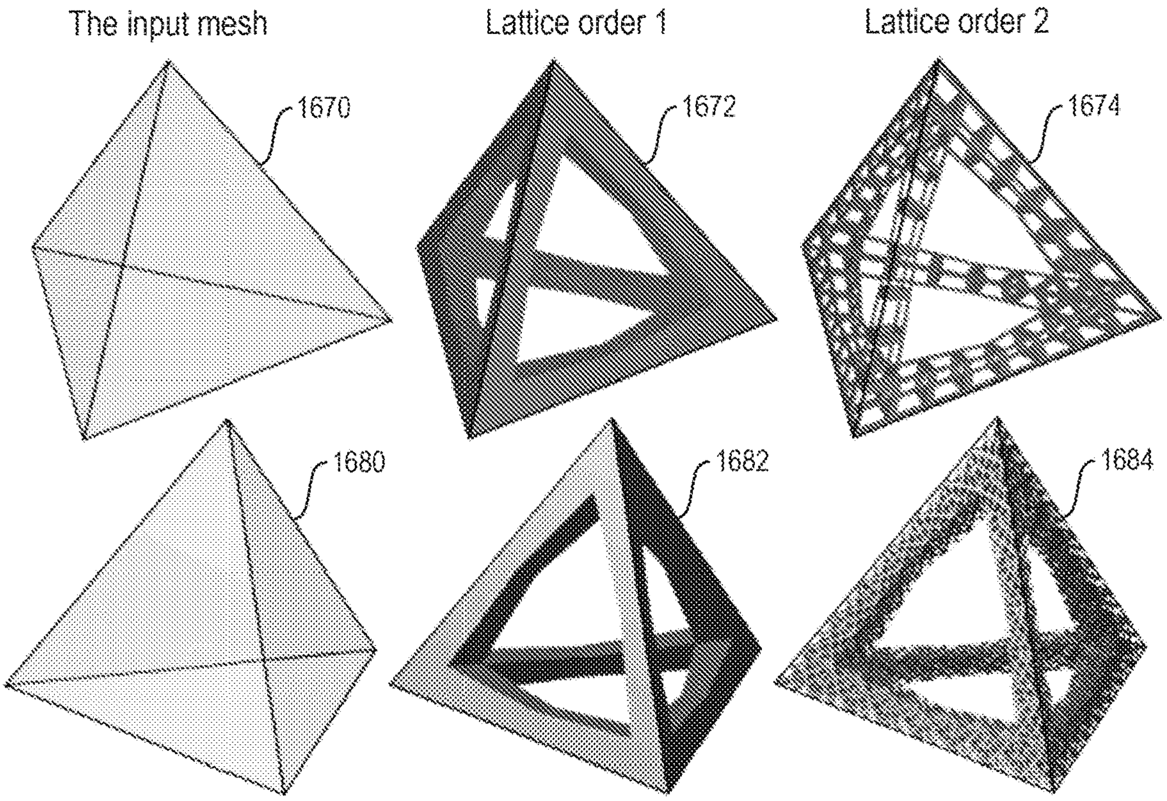
The input mesh
Lattice order 1
Lattice order 2
1670
1672
1674
1680
1682
1684

FIG. 108

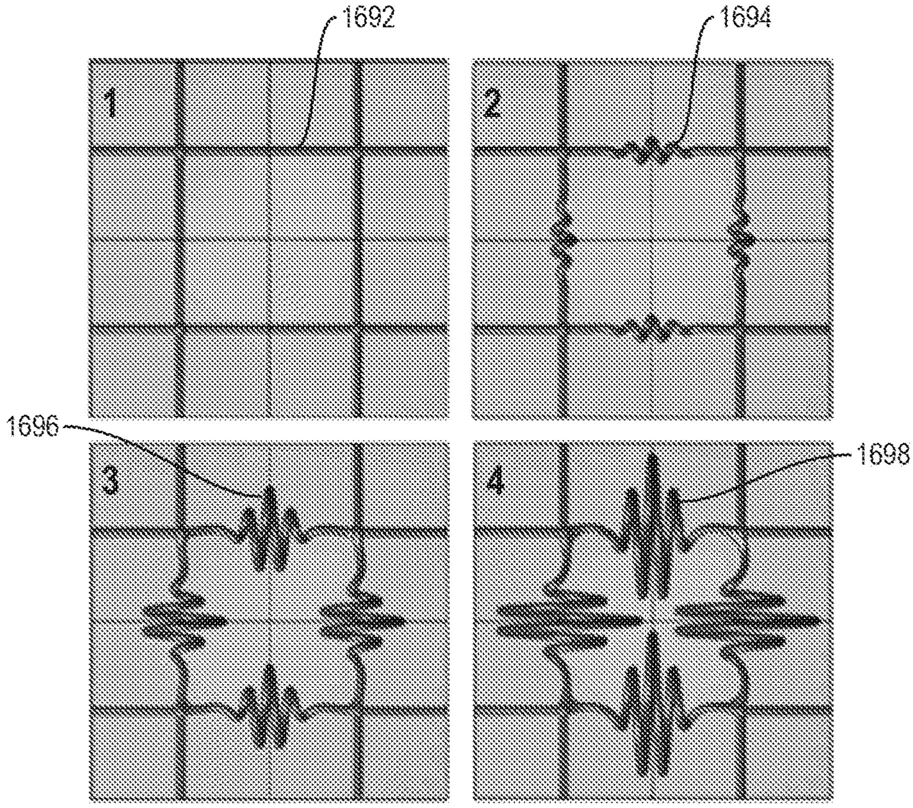


FIG. 109A

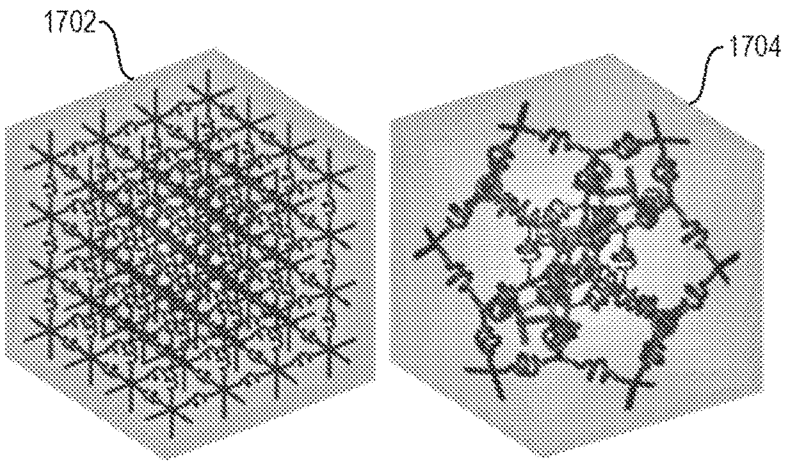


FIG. 109B

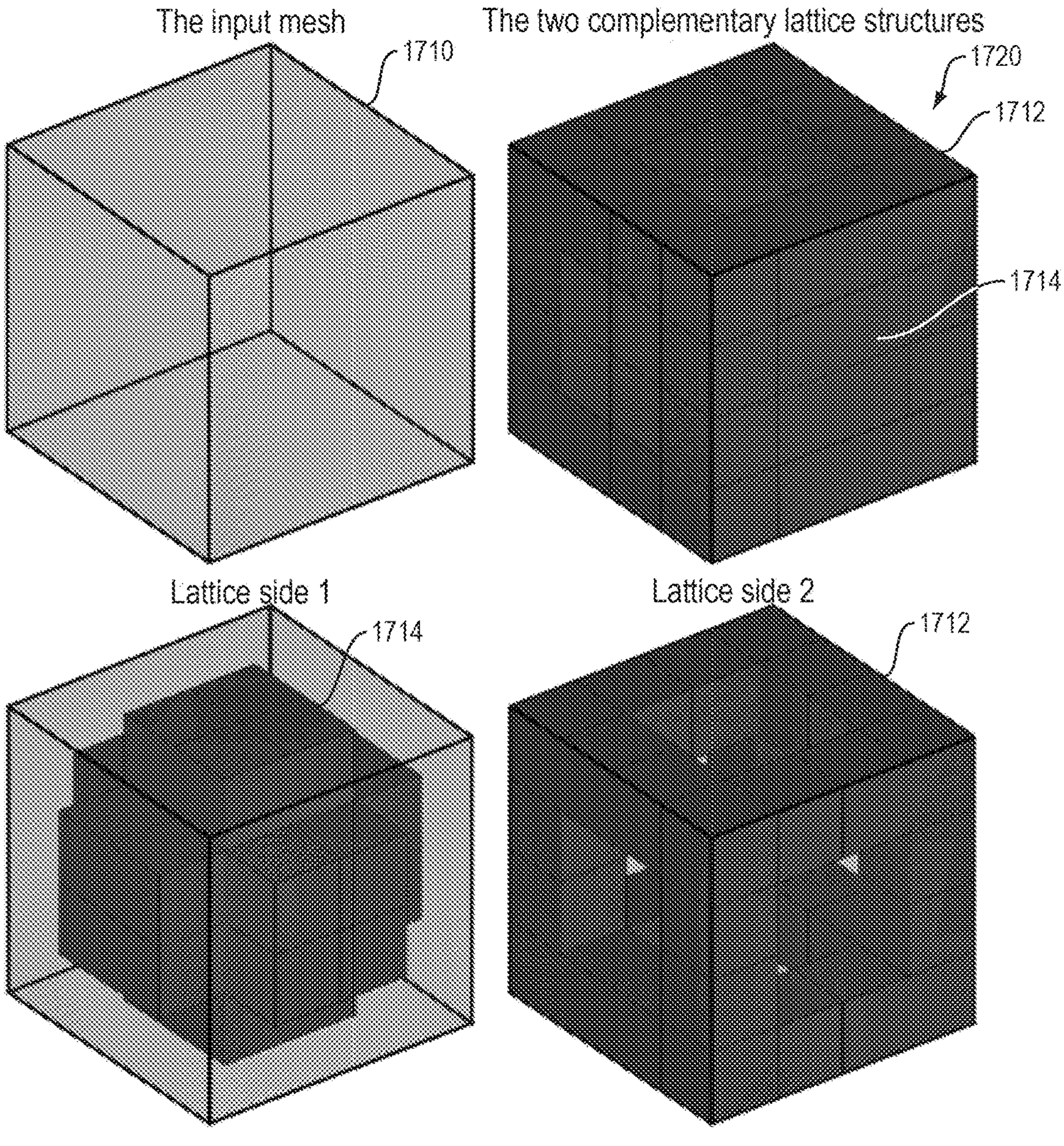
The input mesh
1710
The two complementary lattice structures
1720
1712
1714
Lattice side 1
1714
Lattice side 2
1712

FIG. 110

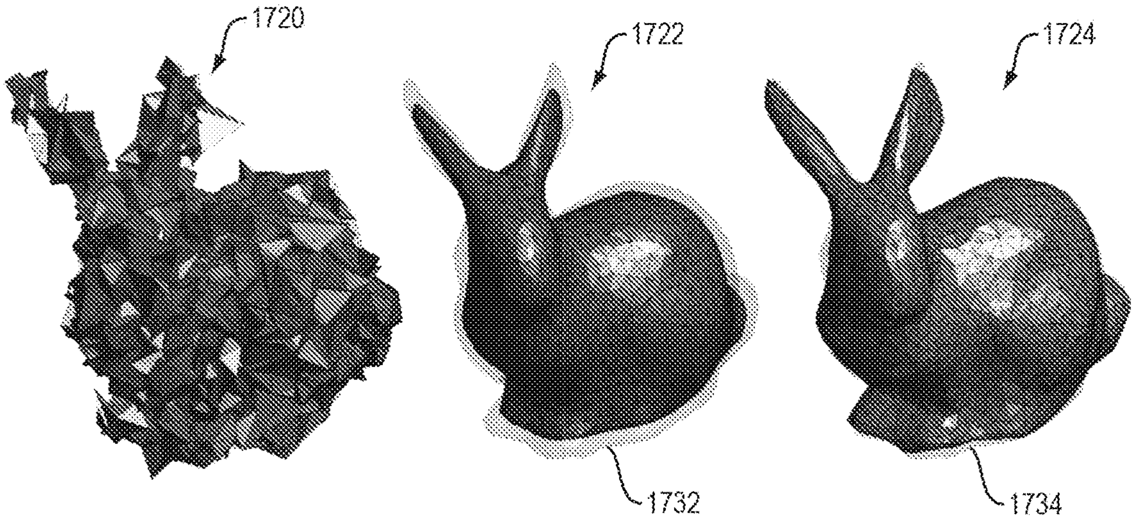


FIG. 111

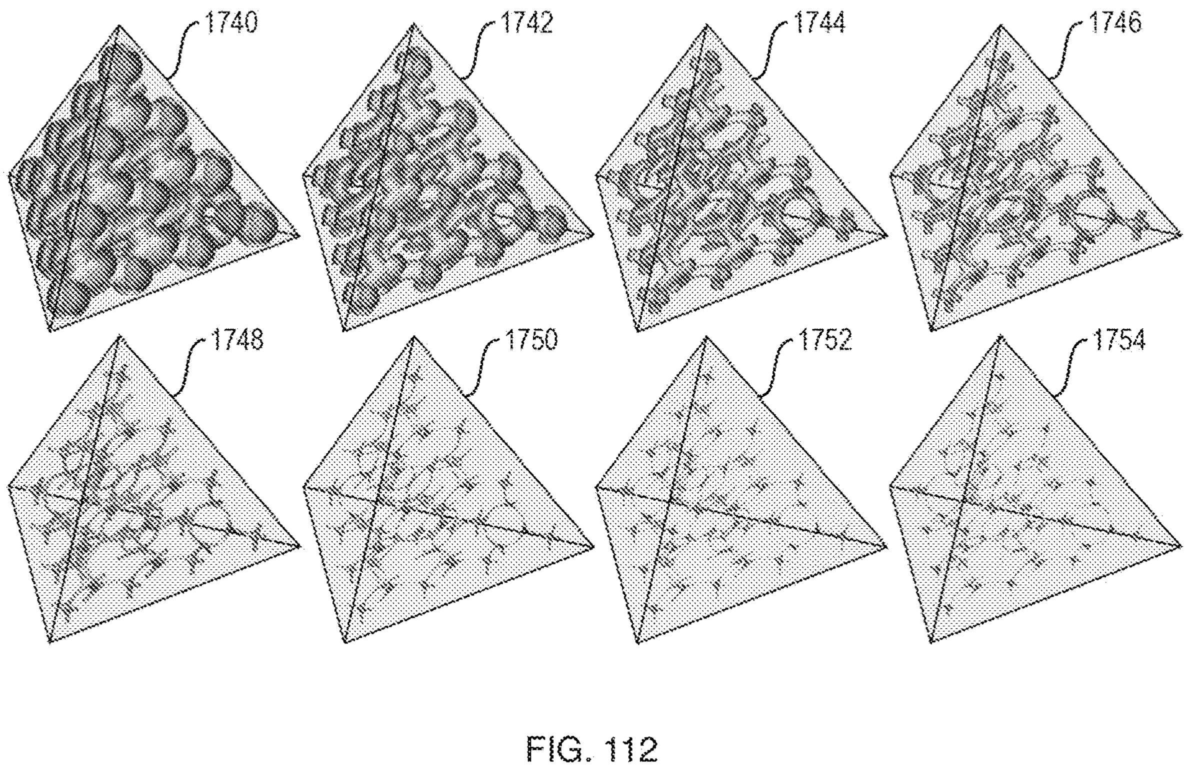


FIG. 112

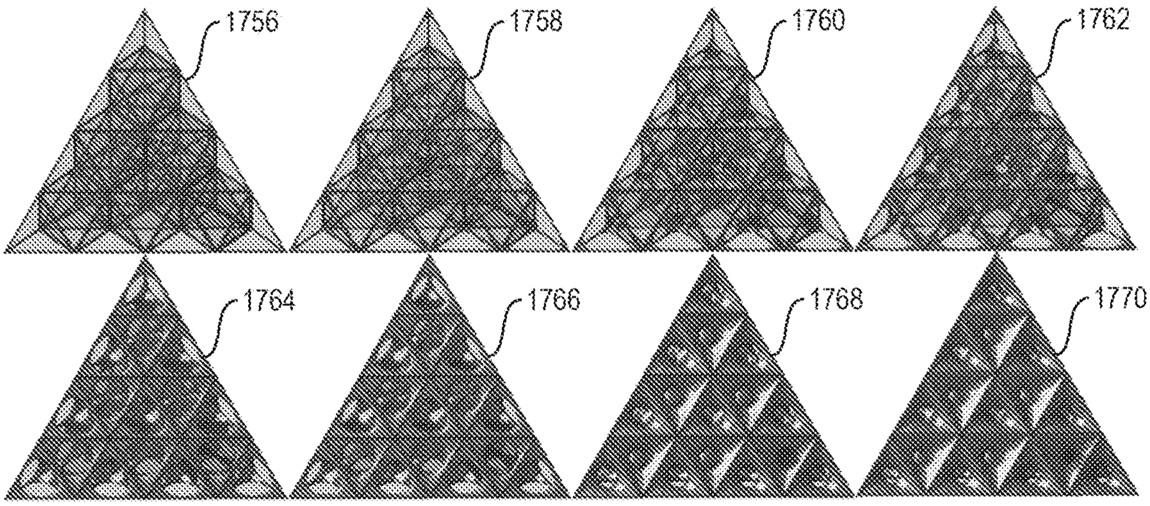


FIG. 113

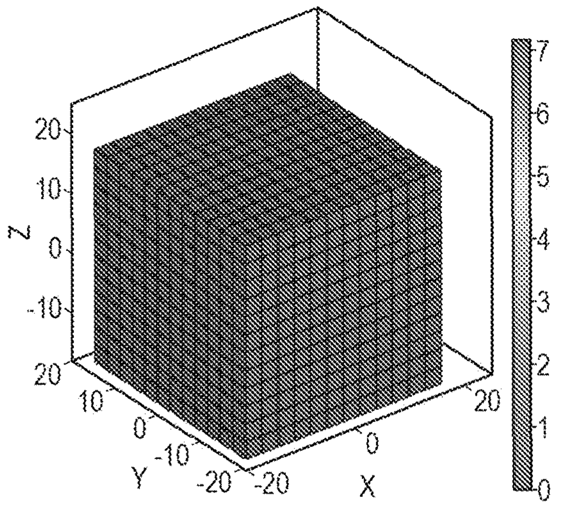


FIG. 114A

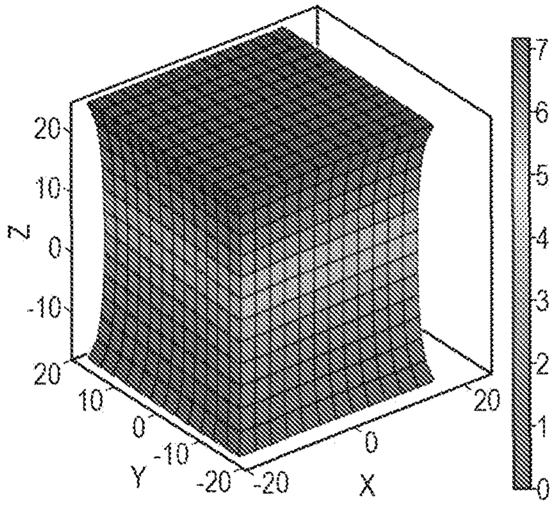


FIG. 114B

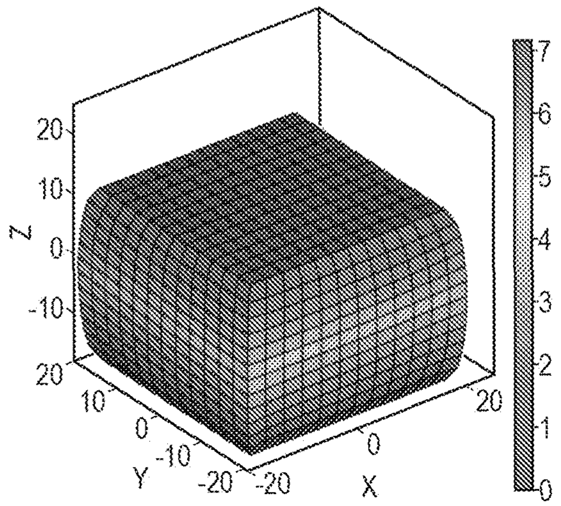


FIG. 114C

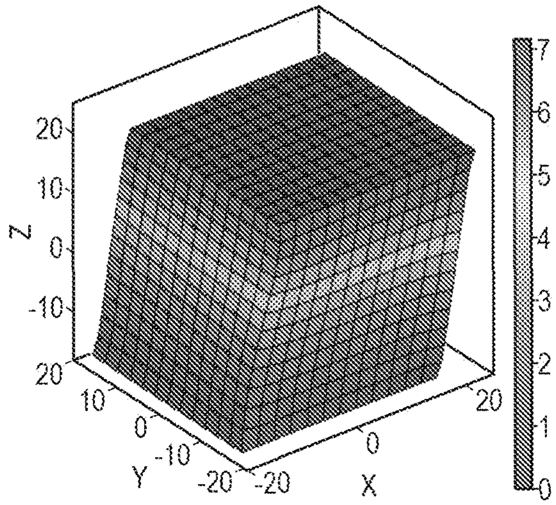


FIG. 114D

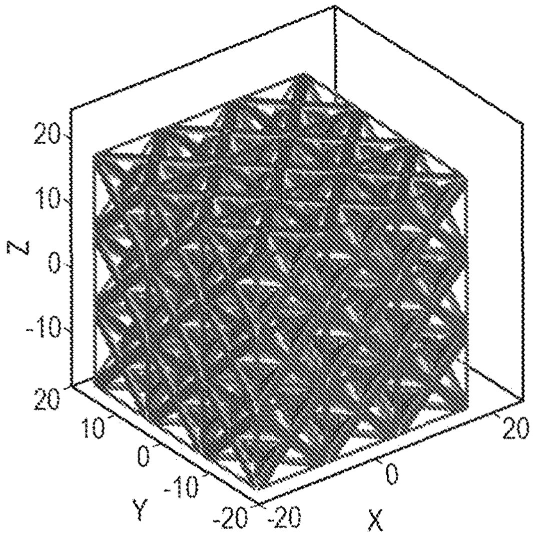


FIG. 115A

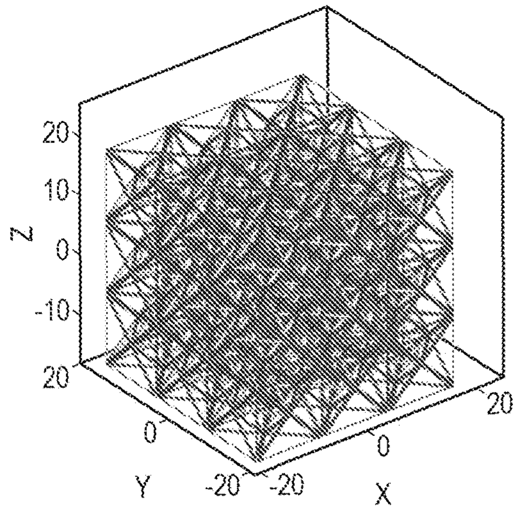


FIG. 115B

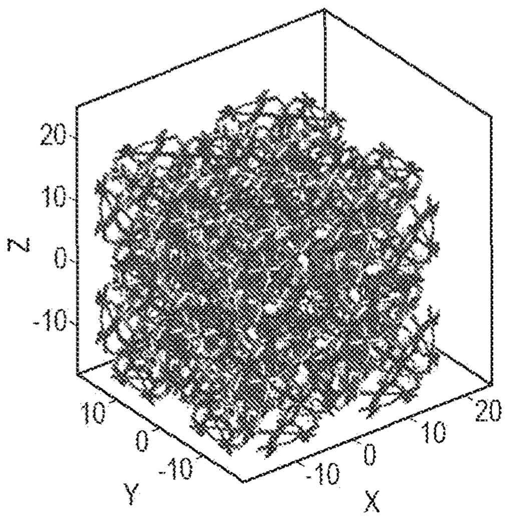


FIG. 115C

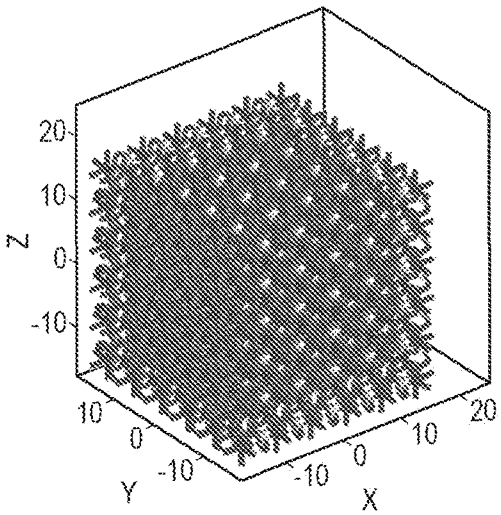


FIG. 115D

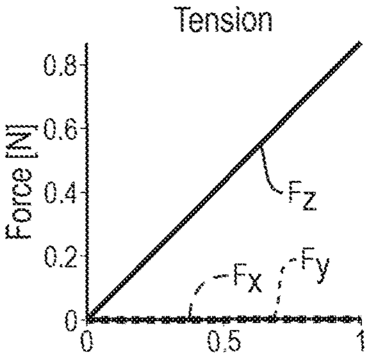


FIG. 116A

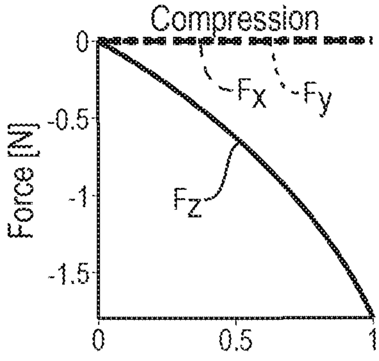


FIG. 116B

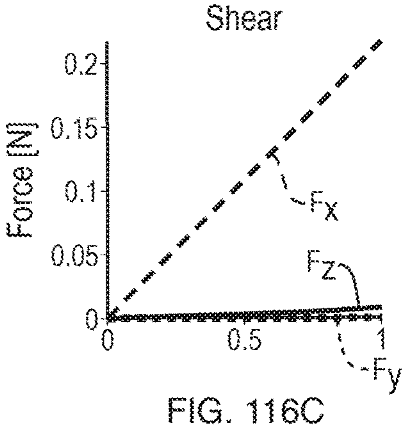


FIG. 116C

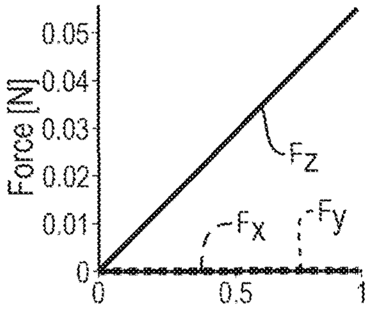


FIG. 116D

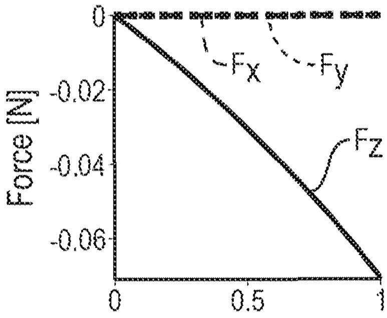


FIG. 116E

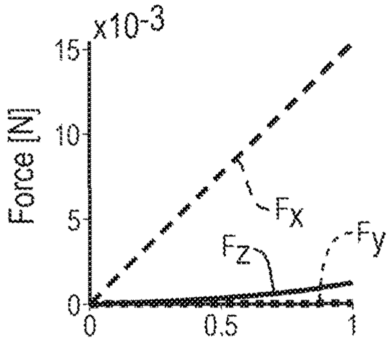


FIG. 116F

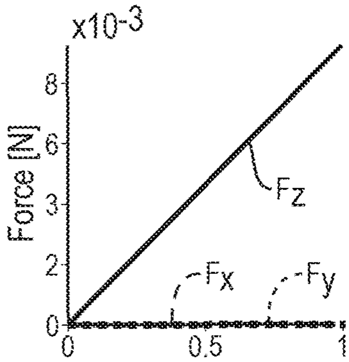


FIG. 116G

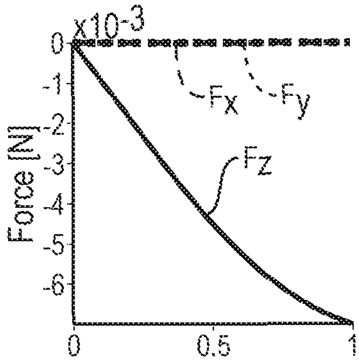


FIG. 116H

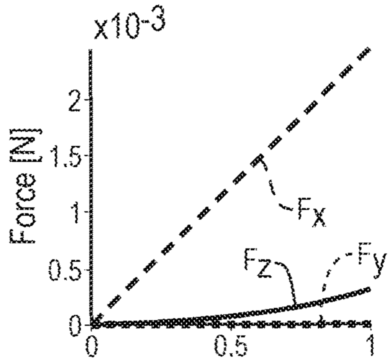


FIG. 116I

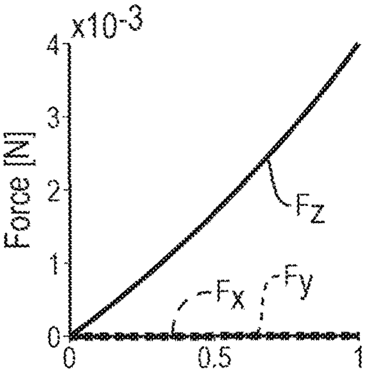


FIG. 116J

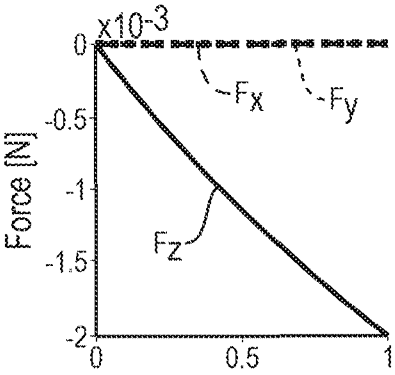


FIG. 116K

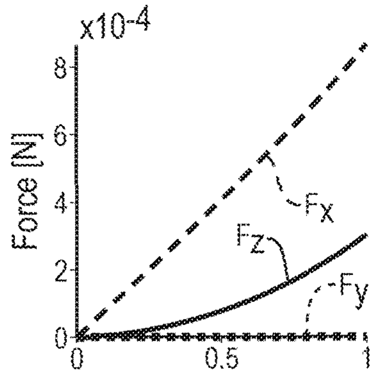


FIG. 116L

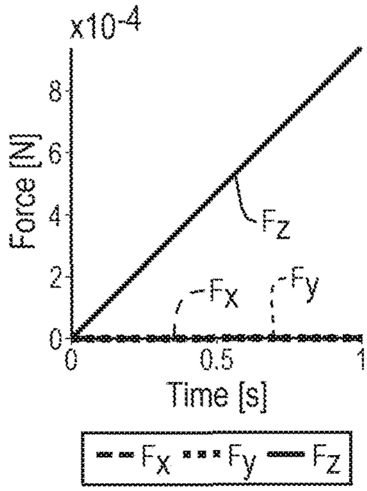


FIG. 116M

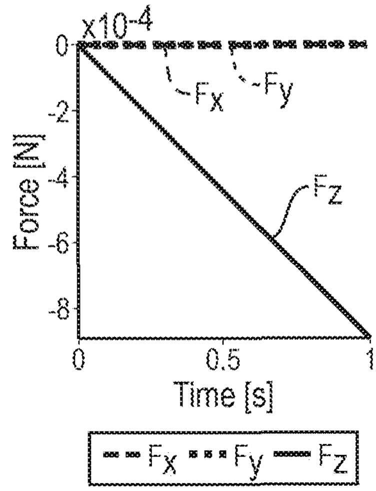


FIG. 116N

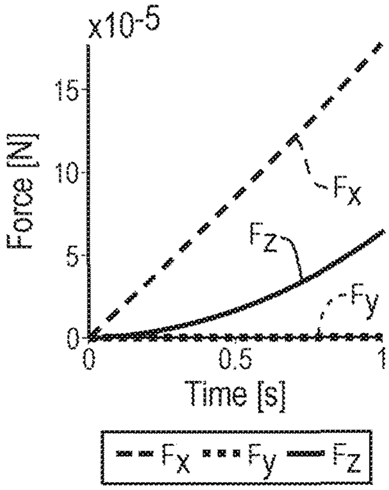


FIG. 116O

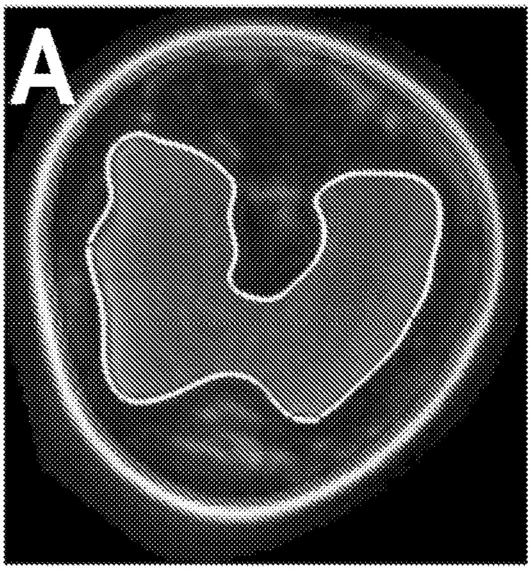


FIG. 117A

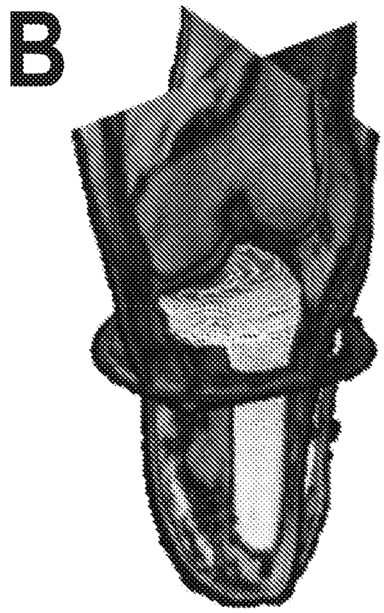


FIG. 117B

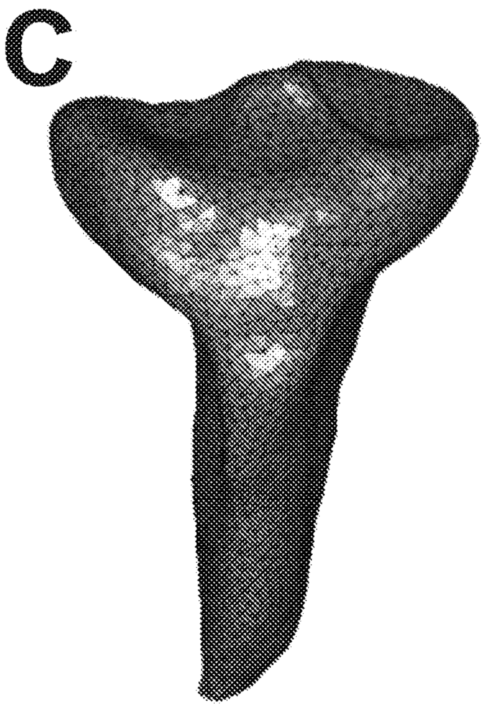


FIG. 117C

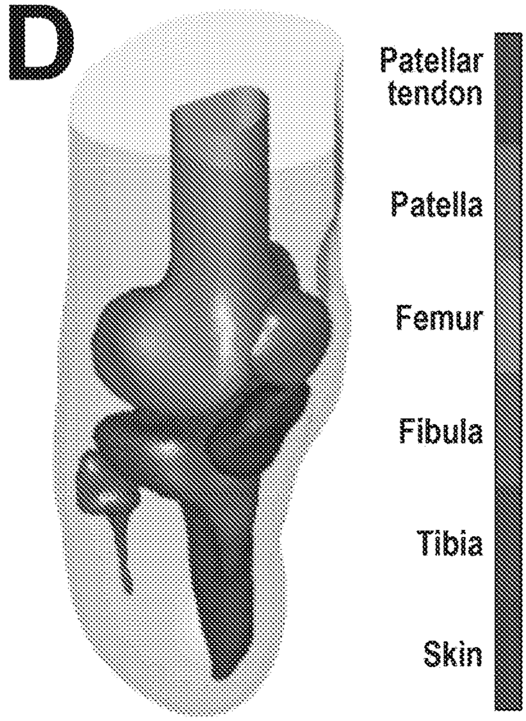


FIG. 117D

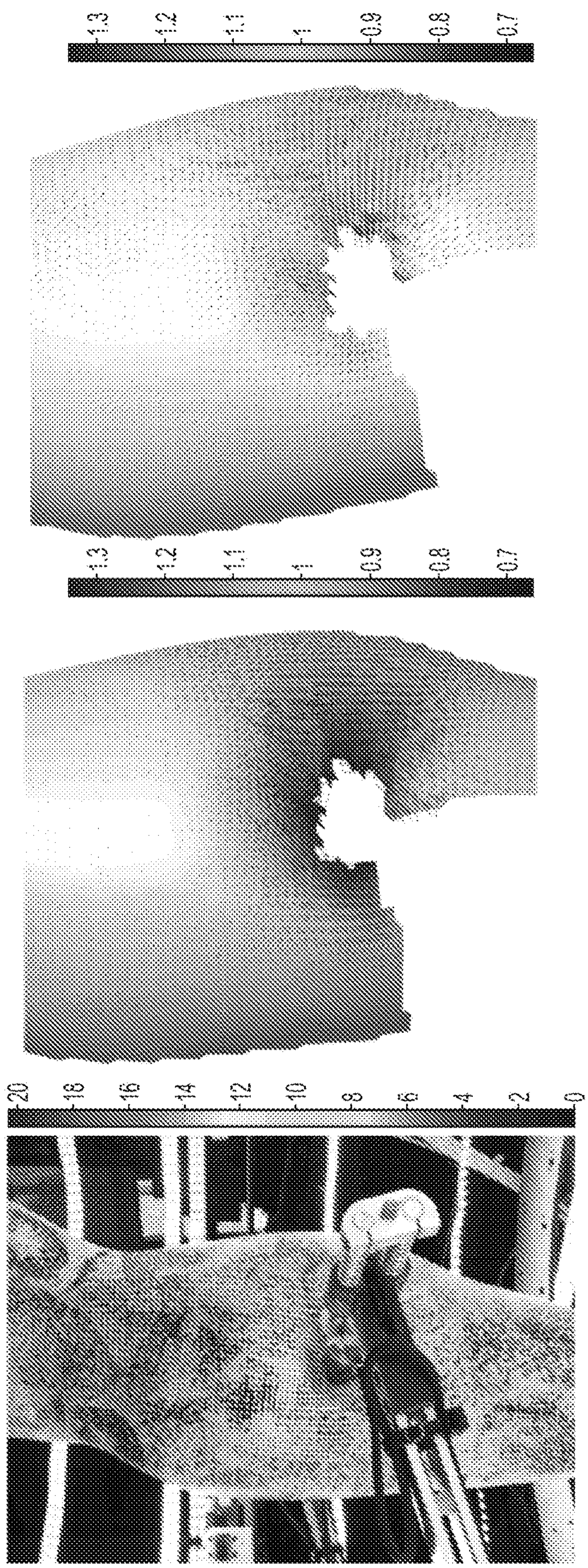


FIG. 118A

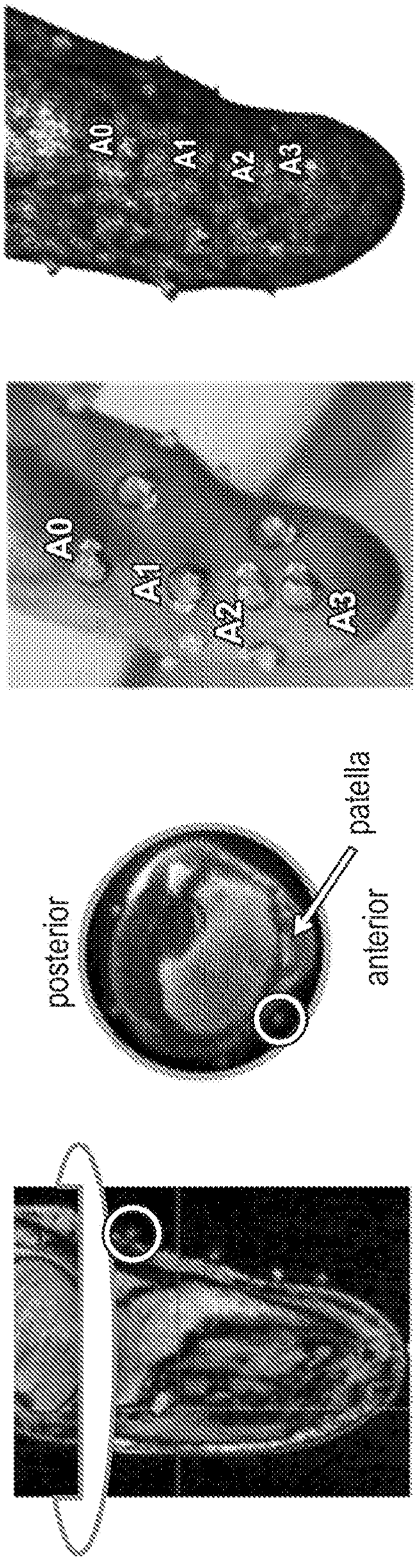


FIG. 118B

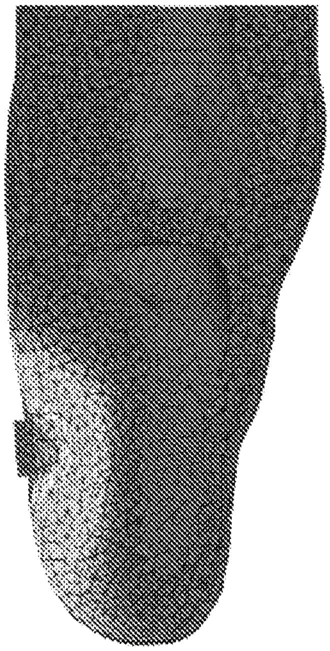

FIG. 118C

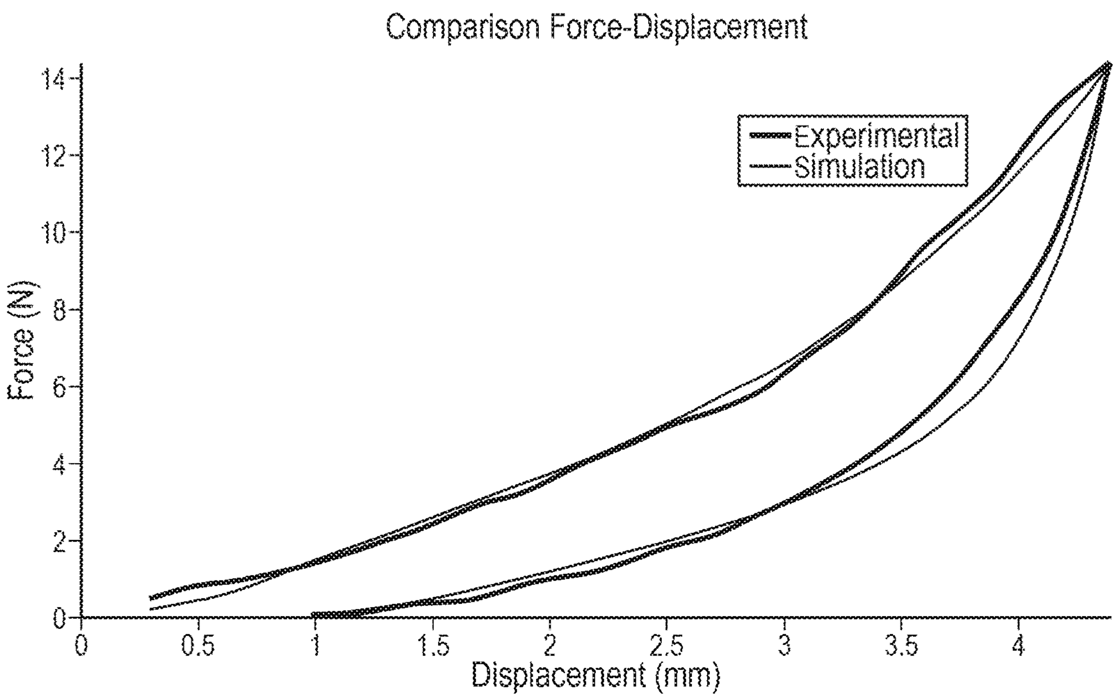


FIG. 118D

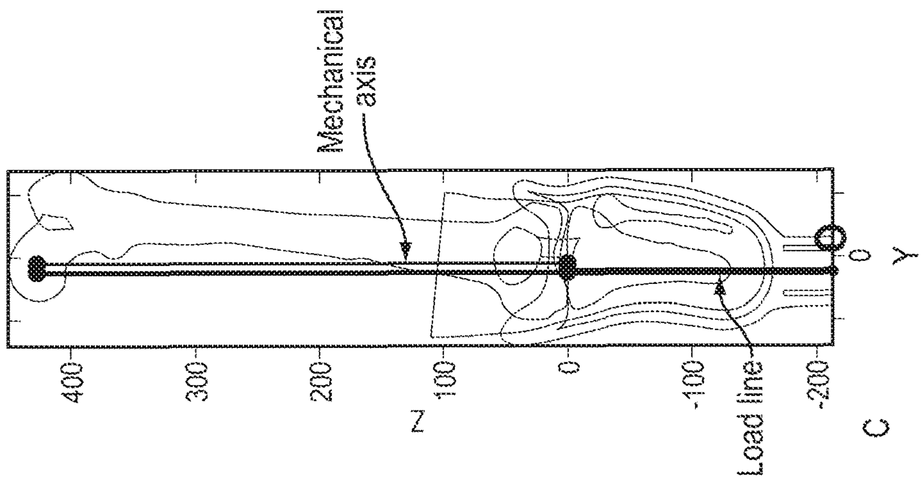


FIG. 119C

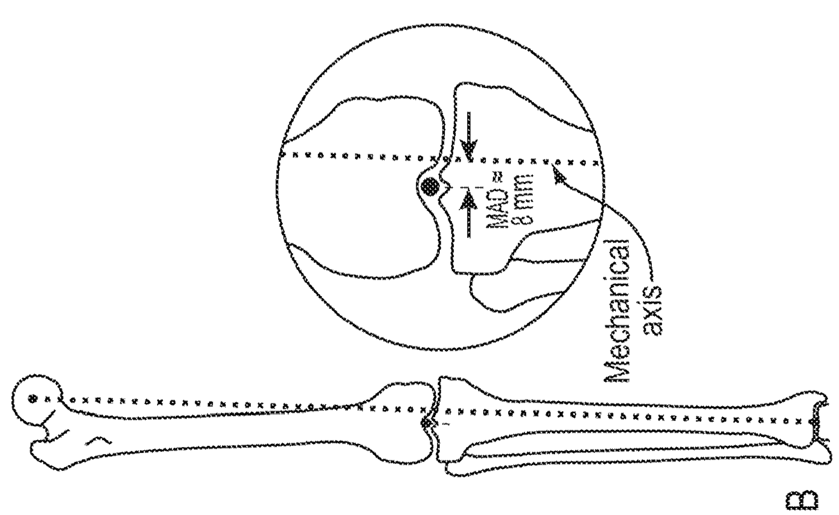


FIG. 119B

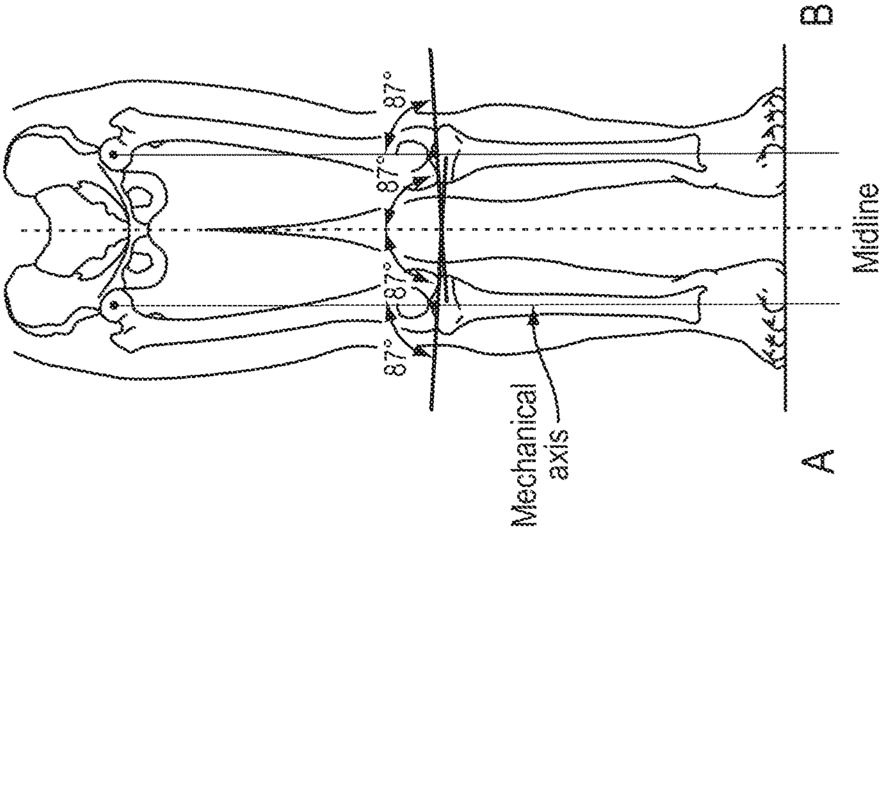


FIG. 119A

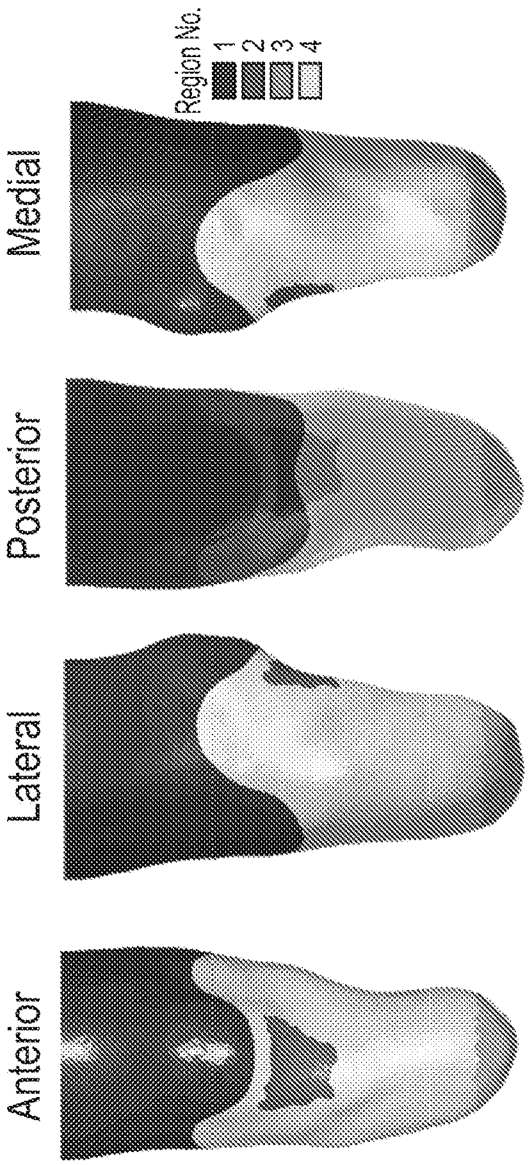


FIG. 120A

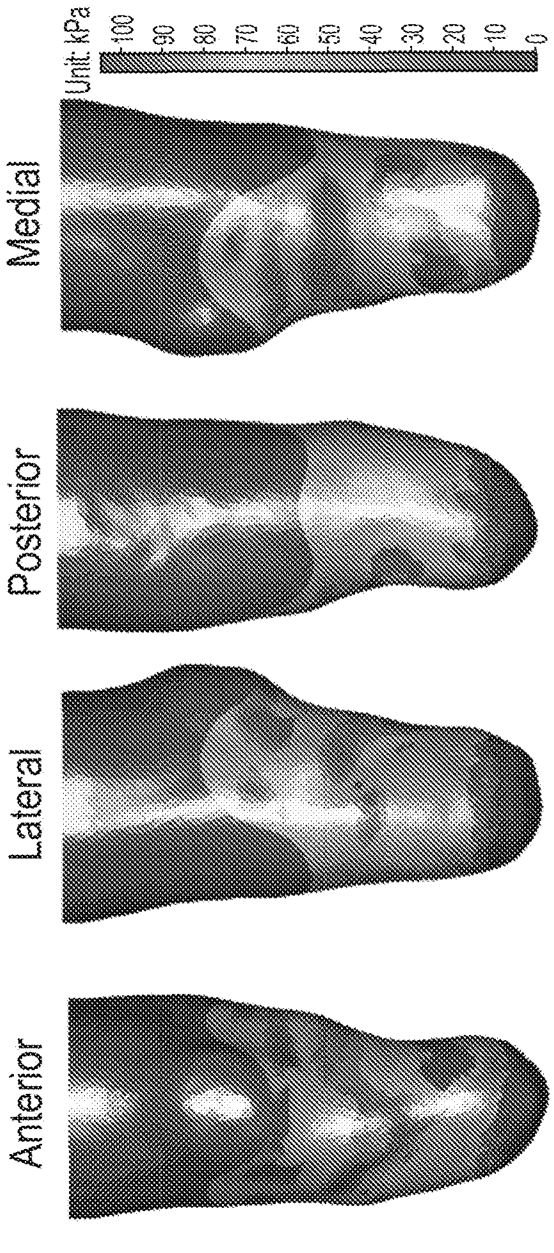
Anterior
Lateral
Posterior
Medial
Unit: kPa
100
90
80
70
60
50
40
30
20
10
0

FIG. 120B

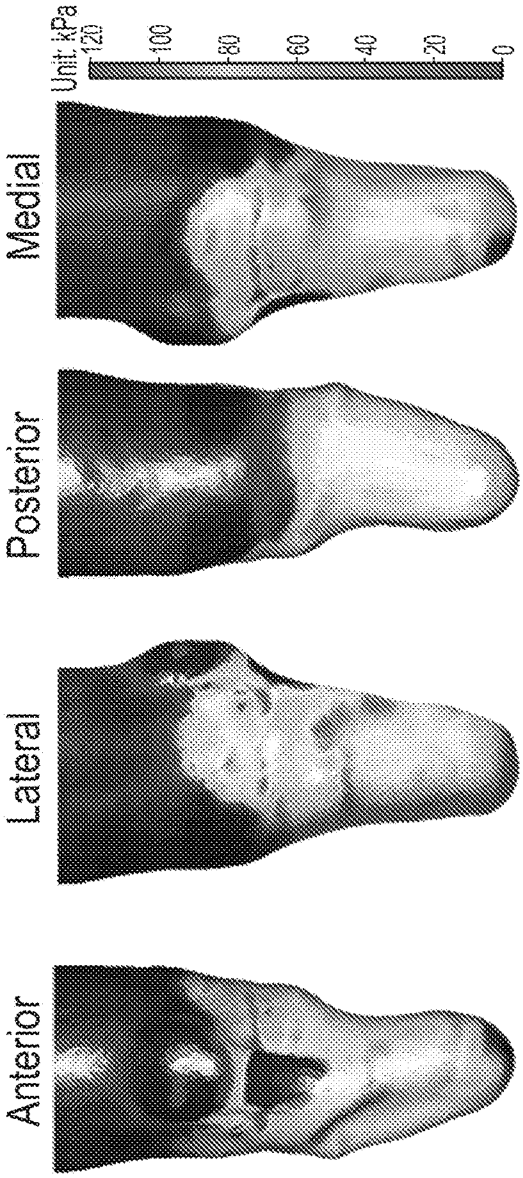
Anterior
Lateral
Posterior
Medial
Unit: kPa
120
100
80
60
40
20
0

FIG. 120C

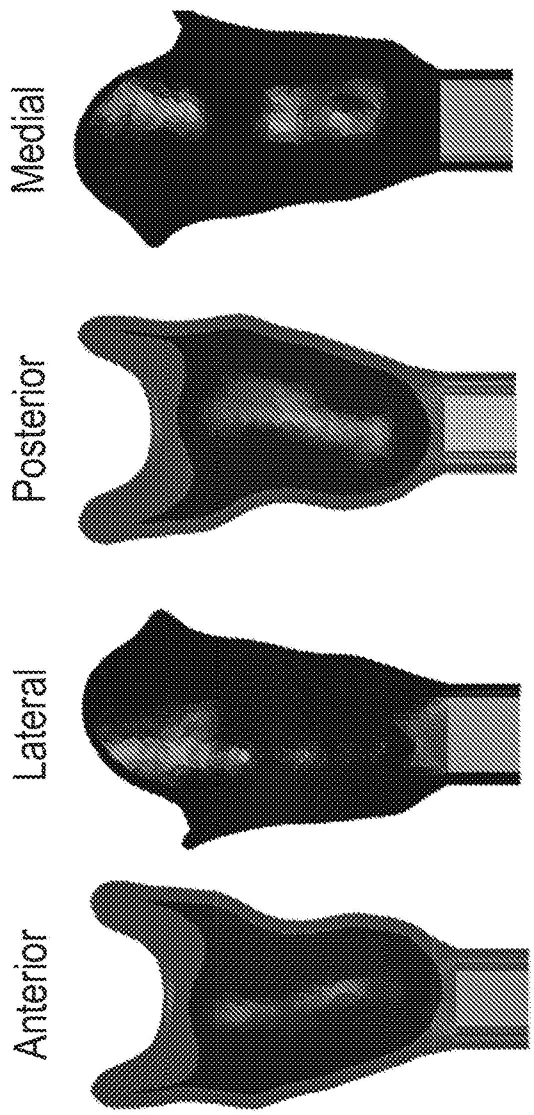


FIG. 120D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62629528
- US 62731376
- US 83683513
- US 20130282141
- US 2017013154 W
- WO 2017123729 A
- US 62278158

### Non-patent literature cited in the description


- *Journal of Rehabilitation Research and Development*, vol. 38 (5), 487-504
- **SOLAV D** ; **MERIC H, RUBIN MB** ; **PRADON D, LOFASO F** ; **WOLF A**. Chest Wall Kinematics Using Triangular Cosserat Point Elements in Healthy and Neuromuscular Subjects.. *Ann Biomed Eng*, 2017, vol. 45, 1963-1973
- **SOLAV D** ; **RUBIN MB** ; **CEREATTI A** ; **CAMOMILLA V** ; **WOLF A**. Bone Pose Estimation in the Presence of Soft Tissue Artifact Using Triangular Cosserat Point Elements.. *Ann Biomed Eng*, 2016, vol. 44, 1181-1190
- **ZHENG YP** ; **MAK A F** ; **LEUNG A K**. State-of-the-art methods for geometric and biomechanical assessments of residual limbs: a review.. *J Rehabil Res Dev*, 2001, vol. 38, 487-504
- **GERE JM** ; **TIMOSHENKO SP.** Mechanics of Materials: Solutions Manual. Nelson Thomes, 2003
- **CHARALAMBIDES A** ; **BERGBREITER S.** A novel all-elastomer MEMS tactile sensor for high dynamic range shear and normal force sensing.. *J. Micromech. Microeng.*, September 2015, vol. 25 (9)
- **SENGEH DM** ; **MOERMAN KM** ; **PETRON A** ; **HERR H**. Multi-Material 3-D Viscoelastic Model of a Transtibial Residuum from In-vivo Indentation and MRI Data. *J Mech Behav Biomed Mater*, 2016
- **RANGER BJ** ; **FEIGIN M** ; **HERR H** ; **ANTHONY BW.** Image registration in a tomographic ultrasound system: comparison between camera-tracking and image-based motion compensation.. *IEEE Int Ultrason Symp*, 2017, 1-4
- **RANGER BJ** ; **FEIGIN M** ; **PESTROV N** ; **ZHANG X** ; **LEMPITSKY V** ; **HERR HM** ; **ANTHONY BW.** Motion compensation in a tomographic ultrasound imaging system: Toward volumetric scans of a limb for prosthetic socket design.. *Proc Annu Int Conf IEEE Eng Med Biol Soc EMBS*, 2015, 7204-7207
- **RANGER BJ** ; **FEIGIN M** ; **ZHANG X** ; **MIREAULT A** ; **RASKAR R** ; **HERR HM** ; **ANTHONY BW.** 3D optical imagery for motion compensation in a limb ultrasound system.. *Proc SPIE*, 2016, 9-12
- **M. FEIGIN** ; **B. J. RANGER** ; **B. W. ANTHONY**. Statistical consensus matching framework for image registration. *In Proceedings of the 23rd IEEE International Conference on Pattern Recognition (ICPR)*, December 2016, 1827-1832
- **LEE, W. C.** ; **ZHANG, M.** ; **MAK, A. F.** Regional differences in pain threshold and tolerance of the transtibial residual limb: including the effects of age and interface material.. *Archives of Physical Medicine and Rehabilitation*, 2005, vol. 86 (4), 641-649
- **SI H**. TetGen, a Delaunay-Based Quality Tetrahedral Mesh Generator.. *ACM Trans Math Softw*, 2015, vol. 41, 1-36